(19) **Europäisches Patentamt / European Patent Office / Office européen des brevets**

(11) **EP 3 282 257 A1**

(12) # EUROPEAN PATENT APPLICATION

(43) Date of publication:
**14.02.2018 Bulletin 2018/07**

(51) Int Cl.:
***G01N 33/68*** (2006.01)

(21) Application number: **17190074.9**

(22) Date of filing: **20.11.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 22.11.2011 US 201161562829 P
22.11.2011 US 201161562883 P
22.11.2011 US 201161562943 P
22.11.2011 US 201161562802 P
22.11.2011 US 201161562916 P
22.11.2011 US 201161562879 P
22.11.2011 US 201161562951 P
22.11.2011 US 201161562778 P
22.11.2011 US 201161562885 P
22.11.2011 US 201161562872 P
22.11.2011 US 201161562947 P
22.11.2011 US 201161562817 P
22.11.2011 US 201161562813 P
22.11.2011 US 201161562824 P

(62) Document number(s) of the earlier application(s) in accordance with Art. 76 EPC:
**12851561.6 / 2 783 213**

(71) Applicant: **Astute Medical, Inc.**
**San Diego, CA 92121 (US)**

(72) Inventors:
- **ANDERBERG, Joseph**
  **Encinitas, CA 92024 (US)**
- **GRAY, Jeff**
  **Buena Park, CA 90520-3143 (US)**
- **MCPHERSON, Paul**
  **Encinitas, CA 92024 (US)**
- **NAKAMURA, Kevin**
  **Cardiff by the Sea, CA 92007 (US)**
- **KAMPF, James, Patrick**
  **San Diego, CA 92130 (US)**

(74) Representative: **Schiweck, Weinzierl & Koch**
**Patentanwälte Partnerschaft mbB**
**Landsberger Straße 98**
**80339 München (DE)**

Remarks:
This application was filed on 08.09.2017 as a divisional application to the application mentioned under INID code 62.

(54) **METHODS AND COMPOSITIONS FOR DIAGNOSIS AND PROGNOSIS OF RENAL INJURY AND RENAL FAILURE**

(57) The present invention relates to methods and compositions for monitoring, diagnosis, prognosis, and determination of treatment regimens in subjects suffering from or suspected of having a renal injury. In particular, the invention relates to using a one or more assays configured to detect Growth/differentiation factor 15, and optionally further a kidney injury marker of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, and Proprotein convertase subtilisin/kexin type 9 as diagnostic and prognostic biomarkers in renal injuries.

EP 3 282 257 A1

**Description**

The present application claims priority to U.S. Provisional Application Nos. 61/562,778 filed November 22, 2011, 61/562,802 filed November 22, 2011, 61/562,813 filed November 22, 2011, 61/562,817 filed November 22, 2011, 61/562,824 filed November 22, 2011, 61/562,829 filed November 22, 2011, 61/562,872 filed November 22, 2011, 61/562,879 filed November 22, 2011, 61/562,883 filed November 22, 2011, 61/562,885 filed November 22, 2011, 61/562,916 filed November 22, 2011, 61/562,943 filed November 22, 2011, 61/562,947 filed November 22, 2011, and 61/562,951 filed November 22, 2011, each of which is hereby incorporated in its entirety including all tables, figures, and claims.

BACKGROUND OF THE INVENTION

The following discussion of the background of the invention is merely provided to aid the reader in understanding the invention and is not admitted to describe or constitute prior art to the present invention.

The kidney is responsible for water and solute excretion from the body. Its functions include maintenance of acid-base balance, regulation of electrolyte concentrations, control of blood volume, and regulation of blood pressure. As such, loss of kidney function through injury and/or disease results in substantial morbidity and mortality. A detailed discussion of renal injuries is provided in Harrison's Principles of Internal Medicine, 17th Ed., McGraw Hill, New York, pages 1741-1830, which are hereby incorporated by reference in their entirety. Renal disease and/or injury may be acute or chronic. Acute and chronic kidney disease are described as follows (from Current Medical Diagnosis & Treatment 2008, 47th Ed, McGraw Hill, New York, pages 785-815, which are hereby incorporated by reference in their entirety): "Acute renal failure is worsening of renal function over hours to days, resulting in the retention of nitrogenous wastes (such as urea nitrogen) and creatinine in the blood. Retention of these substances is called azotemia. Chronic renal failure (chronic kidney disease) results from an abnormal loss of renal function over months to years".

Acute renal failure (ARF, also known as acute kidney injury, or AKI) is an abrupt (typically detected within about 48 hours to 1 week)reduction in glomerular filtration. This loss of filtration capacity results in retention of nitrogenous (urea and creatinine) and non-nitrogenous waste products that are normally excreted by the kidney, a reduction in urine output, or both. It is reported that ARF complicates about 5% of hospital admissions, 4-15% of cardiopulmonary bypass surgeries, and up to 30% of intensive care admissions. ARF may be categorized as prerenal, intrinsic renal, or postrenal in causation. Intrinsic renal disease can be further divided into glomerular, tubular, interstitial, and vascular abnormalities. Major causes of ARF are described in the following table, which is adapted from the Merck Manual, 17th ed., Chapter 222, and which is hereby incorporated by reference in their entirety:

| Type | Risk Factors |
|---|---|
| **Prerenal** | |
| ECF volume depletion | Excessive diuresis, hemorrhage, GI losses, loss of intravascular fluid into the extravascular space (due to ascites, peritonitis, pancreatitis, or burns), loss of skin and mucus membranes, renal salt- and water-wasting states |
| Low cardiac output | Cardiomyopathy, MI, cardiac tamponade, pulmonary embolism, pulmonary hypertension, positive-pressure mechanical ventilation |
| Low systemic vascular resistance | Septic shock, liver failure, antihypertensive drugs |
| Increased renal vascular resistance | NSAIDs, cyclosporines, tacrolimus, hypercalcemia, anaphylaxis, anesthetics, renal artery obstruction, renal vein thrombosis, sepsis, hepatorenal syndrome |
| Decreased efferent arteriolar tone (leading to decreased GFR from reduced glomerular transcapillary pressure, especially in patients with bilateral renal artery stenosis) | ACE inhibitors or angiotensin II receptor blockers |

(continued)

| Intrinsic Renal | |
| --- | --- |
| Acute tubular injury | Ischemia (prolonged or severe prerenal state): surgery, hemorrhage, arterial or venous obstruction; Toxins: NSAIDs, cyclosporines, tacrolimus, aminoglycosides, foscarnet, ethylene glycol, hemoglobin, myoglobin, ifosfamide, heavy metals, methotrexate, radiopaque contrast agents, streptozotocin |
| Acute glomerulonephritis | ANCA-associated: Crescentic glomerulonephritis, polyarteritis nodosa, Wegener's granulomatosis; Anti-GBM glomerulonephritis: Goodpasture's syndrome; Immune-complex: Lupus glomerulonephritis, postinfectious glomerulonephritis, cryoglobulinemic glomerulonephritis |
| Type | Risk Factors |
| Acute tubulointerstitial nephritis | Drug reaction (eg, β-lactams, NSAIDs, sulfonamides, ciprofloxacin, thiazide diuretics, furosemide, phenytoin, allopurinol, pyelonephritis, papillary necrosis |
| Acute vascular nephropathy | Vasculitis, malignant hypertension, thrombotic microangiopathies, scleroderma, atheroembolism |
| Infiltrative diseases | Lymphoma, sarcoidosis, leukemia |
| Postrenal | |
| Tubular precipitation | Uric acid (tumor lysis), sulfonamides, triamterene, acyclovir, indinavir, methotrexate, ethylene glycol ingestion, myeloma protein, myoglobin |
| Ureteral obstruction | Intrinsic: Calculi, clots, sloughed renal tissue, fungus ball, edema, malignancy, congenital defects; Extrinsic: Malignancy, retroperitoneal fibrosis, ureteral trauma during surgery or high impact injury |
| Bladder obstruction | Mechanical: Benign prostatic hyperplasia, prostate cancer, bladder cancer, urethral strictures, phimosis, paraphimosis, urethral valves, obstructed indwelling urinary catheter; Neurogenic: Anticholinergic drugs, upper or lower motor neuron lesion |

In the case of ischemic ARF, the course of the disease may be divided into four phases. During an initiation phase, which lasts hours to days, reduced perfusion of the kidney is evolving into injury. Glomerular ultrafiltration reduces, the flow of filtrate is reduced due to debris within the tubules, and back leakage of filtrate through injured epithelium occurs. Renal injury can be mediated during this phase by reperfusion of the kidney. Initiation is followed by an extension phase which is characterized by continued ischemic injury and inflammation and may involve endothelial damage and vascular congestion. During the maintenance phase, lasting from 1 to 2 weeks, renal cell injury occurs, and glomerular filtration and urine output reaches a minimum. A recovery phase can follow in which the renal epithelium is repaired and GFR gradually recovers. Despite this, the survival rate of subjects with ARF may be as low as about 60%.

Acute kidney injury caused by radiocontrast agents (also called contrast media) and other nephrotoxins such as cyclosporine, antibiotics including aminoglycosides and anticancer drugs such as cisplatin manifests over a period of days to about a week. Contrast induced nephropathy (CIN, which is AKI caused by radiocontrast agents) is thought to be caused by intrarenal vasoconstriction (leading to ischemic injury) and from the generation of reactive oxygen species that are directly toxic to renal tubular epithelial cells. CIN classically presents as an acute (onset within 24-48h) but reversible (peak 3-5 days, resolution within 1 week) rise in blood urea nitrogen and serum creatinine.

A commonly reported criteria for defining and detecting AKI is an abrupt (typically within about 2-7 days or within a period of hospitalization) elevation of serum creatinine. Although the use of serum creatinine elevation to define and detect AKI is well established, the magnitude of the serum creatinine elevation and the time over which it is measured to define AKI varies considerably among publications. Traditionally, relatively large increases in serum creatinine such as 100%, 200%, an increase of at least 100% to a value over 2 mg/dL and other definitions were used to define AKI. However, the recent trend has been towards using smaller serum creatinine rises to define AKI. The relationship between serum creatinine rise, AKI and the associated health risks are reviewed in Praught and Shlipak, Curr Opin Nephrol Hypertens 14:265-270, 2005 and Chertow et al, J Am Soc Nephrol 16: 3365-3370, 2005, which, with the references listed therein, are hereby incorporated by reference in their entirety. As described in these publications, acute worsening renal function (AKI) and increased risk of death and other detrimental outcomes are now known to be associated with very small increases in serum creatinine. These increases may be determined as a relative (percent) value or a nominal value. Relative increases in serum creatinine as small as 20% from the pre-injury value have been reported to indicate acutely worsening renal function (AKI) and increased health risk, but the more commonly reported value to define AKI and increased health risk is a relative increase of at least 25%. Nominal increases as small as 0.3 mg/dL, 0.2 mg/dL or even 0.1 mg/dL have been reported to indicate worsening renal function and increased risk of death. Various time periods for the serum creatinine to rise to these threshold values have been used to define AKI, for example, ranging from 2 days, 3 days, 7 days, or a variable period defined as the time the patient is in the hospital or intensive care unit. These studies indicate there is not a particular threshold serum creatinine rise (or time period for the rise) for worsening renal function or AKI, but rather a continuous increase in risk with increasing magnitude of serum creatinine rise.

One study (Lassnigg et all, J Am Soc Nephrol 15:1597-1605, 2004, hereby incorporated by reference in its entirety) investigated both increases and decreases in serum creatinine. Patients with a mild fall in serum creatinine of -0.1 to -0.3 mg/dL following heart surgery had the lowest mortality rate. Patients with a larger fall in serum creatinine (more than or equal to -0.4 mg/dL) or any increase in serum creatinine had a larger mortality rate. These findings caused the authors to conclude that even very subtle changes in renal function (as detected by small creatinine changes within 48 hours of surgery) seriously effect patient's outcomes. In an effort to reach consensus on a unified classification system for using serum creatinine to define AKI in clinical trials and in clinical practice, Bellomo et al., Crit Care. 8(4):R204-12, 2004, which is hereby incorporated by reference in its entirety, proposes the following classifications for stratifying AKI patients:

"Risk": serum creatinine increased 1.5 fold from baseline OR urine production of <0.5 ml/kg body weight/hr for 6 hours;

"Injury": serum creatinine increased 2.0 fold from baseline OR urine production <0.5 ml/kg/hr for 12 h;

"Failure": serum creatinine increased 3.0 fold from baseline OR creatinine >355 $\mu$mol/l (with a rise of >44) or urine output below 0.3 ml/kg/hr for 24 h or anuria for at least 12 hours;

And included two clinical outcomes:

"Loss": persistent need for renal replacement therapy for more than four weeks.

"ESRD": end stage renal disease-the need for dialysis for more than 3 months.

These criteria are called the RIFLE criteria, which provide a useful clinical tool to classify renal status. As discussed in Kellum, Crit. Care Med. 36: S141-45, 2008 and Ricci et al., Kidney Int. 73, 538-546, 2008, each hereby incorporated by reference in its entirety, the RIFLE criteria provide a uniform definition of AKI which has been validated in numerous studies.
More recently, Mehta et al., Crit. Care 11:R31 (doi:10.1186.cc5713), 2007, hereby incorporated by reference in its entirety, proposes the following similar classifications for stratifying AKI patients, which have been modified from RIFLE:

"Stage I": increase in serum creatinine of more than or equal to 0.3 mg/dL ($\geq$ 26.4 $\mu$mol/L) or increase to more than or equal to 150% (1.5-fold) from baseline OR urine output less than 0.5 mL/kg per hour for more than 6 hours;

"Stage II": increase in serum creatinine to more than 200% (> 2-fold) from baseline OR urine output less than 0.5 mL/kg per hour for more than 12 hours;

"Stage III": increase in serum creatinine to more than 300% (> 3-fold) from baseline OR serum creatinine $\geq$ 354 $\mu$mol/L accompanied by an acute increase of at least 44 $\mu$mol/L OR urine output less than 0.3 mL/kg per hour for 24 hours or anuria for 12 hours.

The CIN Consensus Working Panel (McCollough et al, Rev Cardiovasc Med. 2006; 7(4):177-197, hereby incorporated by reference in its entirety) uses a serum creatinine rise of 25% to define Contrast induced nephropathy (which is a type of AKI).Although various groups propose slightly different criteria for using serum creatinine to detect AKI, the consensus is that small changes in serum creatinine, such as 0.3 mg/dL or 25%, are sufficient to detect AKI (worsening renal function) and that the magnitude of the serum creatinine change is an indicator of the severity of the AKI and mortality risk.

Although serial measurement of serum creatinine over a period of days is an accepted method of detecting and diagnosing AKI and is considered one of the most important tools to evaluate AKI patients, serum creatinine is generally regarded to have several limitations in the diagnosis, assessment and monitoring of AKI patients. The time period for serum creatinine to rise to values (e.g., a 0.3 mg/dL or 25% rise) considered diagnostic for AKI can be 48 hours or longer depending on the definition used. Since cellular injury in AKI can occur over a period of hours, serum creatinine elevations detected at 48 hours or longer can be a late indicator of injury, and relying on serum creatinine can thus delay diagnosis of AKI. Furthermore, serum creatinine is not a good indicator of the exact kidney status and treatment needs during the most acute phases of AKI when kidney function is changing rapidly. Some patients with AKI will recover fully, some will need dialysis (either short term or long term) and some will have other detrimental outcomes including death, major adverse cardiac events and chronic kidney disease. Because serum creatinine is a marker of filtration rate, it does not differentiate between the causes of AKI (pre-renal, intrinsic renal, post-renal obstruction, atheroembolic, etc) or the category or location of injury in intrinsic renal disease (for example, tubular, glomerular or interstitial in origin). Urine output is similarly limited, Knowing these things can be of vital importance in managing and treating patients with AKI.

These limitations underscore the need for better methods to detect and assess AKI, particularly in the early and subclinical stages, but also in later stages when recovery and repair of the kidney can occur. Furthermore, there is a need to better identify patients who are at risk of having an AKI.

BRIEF SUMMARY OF THE INVENTION

It is an object of the invention to provide methods and compositions for evaluating renal function in a subject. As described herein, measurement of one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 (each referred to herein as a "kidney injury marker") can be used for diagnosis, prognosis, risk stratification, staging, monitoring, categorizing and determination of further diagnosis and treatment regimens in subjects suffering or at risk of suffering from an injury to renal function, reduced renal function, and/or acute renal failure (also called acute kidney injury).

The kidney injury markers of the present invention may be used, individually or in panels comprising a plurality of kidney injury markers, for risk stratification (that is, to identify subjects at risk for a future injury to renal function, for future progression to reduced renal function, for future progression to ARF, for future improvement in renal function, *etc.*); for diagnosis of existing disease (that is, to identify subjects who have suffered an injury to renal function, who have progressed to reduced renal function, who have progressed to ARF, *etc.*); for monitoring for deterioration or improvement of renal function; and for predicting a future medical outcome, such as improved or worsening renal function, a decreased or increased mortality risk, a decreased or increased risk that a subject will require renal replacement therapy (*i.e.*, hemodialysis, peritoneal dialysis, hemofiltration, and/or renal transplantation, a decreased or increased risk that a subject will recover from an injury to renal function, a decreased or increased risk that a subject will recover from ARF, a decreased or increased risk that a subject will progress to end stage renal disease, a decreased or increased risk that a subject will progress to chronic renal failure, a decreased or increased risk that a subject will suffer rejection of a transplanted kidney, *etc.*

In a first aspect, the present invention relates to methods for evaluating renal status in a subject. These methods comprise performing an assay method that is configured to detect one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 is/are then correlated to the renal status of the subject. This correlation to renal status may include correlating the assay result(s) to one or more of risk stratification, diagnosis, prognosis, staging, classifying and monitoring of the subject as described herein. Thus, the present invention utilizes one or more kidney injury markers of the present invention for the evaluation of renal injury.

In certain embodiments, the methods for evaluating renal status described herein are methods for risk stratification of the subject; that is, assigning a likelihood of one or more future changes in renal status to the subject. In these embodiments, the assay result(s) is/are correlated to one or more such future changes. The following are preferred risk stratification embodiments.

In preferred risk stratification embodiments, these methods comprise determining a subject's risk for a future injury to renal function, and the assay result(s) is/are correlated to a likelihood of such a future injury to renal function. For example, the measured concentration(s) may each be compared to a threshold value. For a "positive going" kidney injury marker, an increased likelihood of suffering a future injury to renal function is assigned to the subject when the measured concentration is above the threshold, relative to a likelihood assigned when the measured concentration is below the threshold. For a "negative going" kidney injury marker, an increased likelihood of suffering a future injury to renal function is assigned to the subject when the measured concentration is below the threshold, relative to a likelihood assigned when the measured concentration is above the threshold.

In other preferred risk stratification embodiments, these methods comprise determining a subject's risk for future reduced renal function, and the assay result(s) is/are correlated to a likelihood of such reduced renal function. For example, the measured concentrations may each be compared to a threshold value. For a "positive going" kidney injury marker, an increased likelihood of suffering a future reduced renal function is assigned to the subject when the measured concentration is above the threshold, relative to a likelihood assigned when the measured concentration is below the threshold. For a "negative going" kidney injury marker, an increased likelihood of future reduced renal function is assigned to the subject when the measured concentration is below the threshold, relative to a likelihood assigned when the measured concentration is above the threshold.

In still other preferred risk stratification embodiments, these methods comprise determining a subject's likelihood for a future improvement in renal function, and the assay result(s) is/are correlated to a likelihood of such a future improvement in renal function. For example, the measured concentration(s) may each be compared to a threshold value. For a "positive going" kidney injury marker, an increased likelihood of a future improvement in renal function is assigned to the subject when the measured concentration is below the threshold, relative to a likelihood assigned when the measured concentration is above the threshold. For a "negative going" kidney injury marker, an increased likelihood of a future improvement in renal function is assigned to the subject when the measured concentration is above the threshold, relative to a likelihood assigned when the measured concentration is below the threshold.

In yet other preferred risk stratification embodiments, these methods comprise determining a subject's risk for progression to ARF, and the result(s) is/are correlated to a likelihood of such progression to ARF. For example, the measured concentration(s) may each be compared to a threshold value. For a "positive going" kidney injury marker, an increased likelihood of progression to ARF is assigned to the subject when the measured concentration is above the threshold, relative to a likelihood assigned when the measured concentration is below the threshold. For a "negative going" kidney injury marker, an increased likelihood of progression to ARF is assigned to the subject when the measured concentration is below the threshold, relative to a likelihood assigned when the measured concentration is above the threshold.

And in other preferred risk stratification embodiments, these methods comprise determining a subject's outcome risk, and the assay result(s) is/are correlated to a likelihood of the occurrence of a clinical outcome related to a renal injury suffered by the subject. For example, the measured concentration(s) may each be compared to a threshold value. For a "positive going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is above the threshold, relative to a likelihood assigned when the measured concentration is below the threshold. For a "negative going" kidney injury marker, an increased likelihood of one or more of: acute kidney injury, progression to a worsening stage of AKI, mortality, a requirement for renal replacement therapy, a requirement for withdrawal of renal toxins, end stage renal disease, heart failure, stroke, myocardial infarction, progression to chronic kidney disease, etc., is assigned to the subject when the measured concentration is below the threshold, relative to a likelihood assigned when the measured concentration is above the threshold.

In such risk stratification embodiments, preferably the likelihood or risk assigned is that an event of interest is more or less likely to occur within 180 days of the time at which the body fluid sample is obtained from the subject. In particularly preferred embodiments, the likelihood or risk assigned relates to an event of interest occurring within a shorter time period such as 18 months, 120 days, 90 days, 60 days, 45 days, 30 days, 21 days, 14 days, 7 days, 5 days, 96 hours, 72 hours, 48 hours, 36 hours, 24 hours, 12 hours, or less. A risk at 0 hours of the time at which the body fluid sample is obtained from the subject is equivalent to diagnosis of a current condition.

In preferred risk stratification embodiments, the subject is selected for risk stratification based on the pre-existence in the subject of one or more known risk factors for prerenal, intrinsic renal, or postrenal ARF. For example, a subject undergoing or having undergone major vascular surgery, coronary artery bypass, or other cardiac surgery; a subject having pre-existing congestive heart failure, preeclampsia, eclampsia, diabetes mellitus, hypertension, coronary artery disease, proteinuria, renal insufficiency, glomerular filtration below the normal range, cirrhosis, serum creatinine above the normal range, or sepsis; or a subject exposed to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin are all preferred subjects for monitoring risks according to the methods described herein. This list is not meant to be limiting. By "pre-existence" in this context is meant that the risk factor exists at the time the body fluid sample is obtained from the subject. In particularly preferred embodiments, a subject is chosen for risk stratification based on an existing diagnosis of injury to renal function, reduced renal function, or ARF.

In other embodiments, the methods for evaluating renal status described herein are methods for diagnosing a renal injury in the subject; that is, assessing whether or not a subject has suffered from an injury to renal function, reduced renal function, or ARF. In these embodiments, the assay result(s), for example measured concentration(s) of one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 is/are correlated to the occurrence or nonoccurrence of a change in renal status. The following are preferred diagnostic embodiments.

In preferred diagnostic embodiments, these methods comprise diagnosing the occurrence or nonoccurrence of an injury to renal function, and the assay result(s) is/are correlated to the occurrence or nonoccurrence of such an injury. For example, each of the measured concentration(s) may be compared to a threshold value. For a positive going marker, an increased likelihood of the occurrence of an injury to renal function is assigned to the subject when the measured concentration is above the threshold (relative to the likelihood assigned when the measured concentration is below the threshold); alternatively, when the measured concentration is below the threshold, an increased likelihood of the nonoccurrence of an injury to renal function may be assigned to the subject (relative to the likelihood assigned when the measured concentration is above the threshold). For a negative going marker, an increased likelihood of the occurrence of an injury to renal function is assigned to the subject when the measured concentration is below the threshold (relative to the likelihood assigned when the measured concentration is above the threshold); alternatively, when the measured concentration is above the threshold, an increased likelihood of the nonoccurrence of an injury to renal function may be assigned to the subject (relative to the likelihood assigned when the measured concentration is below the threshold).

In other preferred diagnostic embodiments, these methods comprise diagnosing the occurrence or nonoccurrence of reduced renal function, and the assay result(s) is/are correlated to the occurrence or nonoccurrence of an injury causing reduced renal function. For example, each of the measured concentration(s) may be compared to a threshold value. For a positive going marker, an increased likelihood of the occurrence of an injury causing reduced renal function is assigned to the subject when the measured concentration is above the threshold (relative to the likelihood assigned when the measured concentration is below the threshold); alternatively, when the measured concentration is below the threshold, an increased likelihood of the nonoccurrence of an injury causing reduced renal function may be assigned to the subject (relative to the likelihood assigned when the measured concentration is above the threshold). For a negative going marker, an increased likelihood of the occurrence of an injury causing reduced renal function is assigned to the subject when the measured concentration is below the threshold (relative to the likelihood assigned when the measured concentration is above the threshold); alternatively, when the measured concentration is above the threshold, an increased likelihood of the nonoccurrence of an injury causing reduced renal function may be assigned to the subject (relative to the likelihood assigned when the measured concentration is below the threshold).

In yet other preferred diagnostic embodiments, these methods comprise diagnosing the occurrence or nonoccurrence of ARF, and the assay result(s) is/are correlated to the occurrence or nonoccurrence of an injury causing ARF. For example, each of the measured concentration(s) may be compared to a threshold value. For a positive going marker, an increased likelihood of the occurrence of ARF is assigned to the subject when the measured concentration is above the threshold (relative to the likelihood assigned when the measured concentration is below the threshold); alternatively, when the measured concentration is below the threshold, an increased likelihood of the nonoccurrence of ARF may be assigned to the subject (relative to the likelihood assigned when the measured concentration is above the threshold). For a negative going marker, an increased likelihood of the occurrence of ARF is assigned to the subject when the measured concentration is below the threshold (relative to the likelihood assigned when the measured concentration is above the threshold); alternatively, when the measured concentration is above the threshold, an increased likelihood of the nonoccurrence of ARF may be assigned to the subject (relative to the likelihood assigned when the measured concentration is below the threshold).

In still other preferred diagnostic embodiments, these methods comprise diagnosing a subject as being in need of renal replacement therapy, and the assay result(s) is/are correlated to a need for renal replacement therapy. For example, each of the measured concentration(s) may be compared to a threshold value. For a positive going marker, an increased likelihood of the occurrence of an injury creating a need for renal replacement therapy is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal replacement therapy may be assigned to the subject (relative to the likelihood assigned when the measured concentration is above the threshold). For a negative going marker, an increased likelihood of the occurrence of an injury creating a need for renal replacement therapy is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal replacement therapy may be assigned to the subject (relative to the likelihood assigned when the measured concentration is below the threshold).

In still other preferred diagnostic embodiments, these methods comprise diagnosing a subject as being in need of renal transplantation, and the assay result(s0 is/are correlated to a need for renal transplantation. For example, each of the measured concentration(s) may be compared to a threshold value. For a positive going marker, an increased likelihood of the occurrence of an injury creating a need for renal transplantation is assigned to the subject when the measured concentration is above the threshold (relative to the likelihood assigned when the measured concentration is below the threshold); alternatively, when the measured concentration is below the threshold, an increased likelihood of the nonoccurrence of an injury creating a need for renal transplantation may be assigned to the subject (relative to the likelihood assigned when the measured concentration is above the threshold). For a negative going marker, an increased likelihood of the occurrence of an injury creating a need for renal transplantation is assigned to the subject when the measured concentration is below the threshold (relative to the likelihood assigned when the measured concentration is above the threshold); alternatively, when the measured concentration is above the threshold, an increased likelihood of the nonoccurrence of an injury creating a need for renal transplantation may be assigned to the subject (relative to the likelihood assigned when the measured concentration is below the threshold).

In still other embodiments, the methods for evaluating renal status described herein are methods for monitoring a renal injury in the subject; that is, assessing whether or not renal function is improving or worsening in a subject who has suffered from an injury to renal function, reduced renal function, or ARF. In these embodiments, the assay result(s), for example measured concentration(s) of one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 is/are correlated to the occurrence or nonoccurrence of a change in renal status. The following are preferred monitoring embodiments.

In preferred monitoring embodiments, these methods comprise monitoring renal status in a subject suffering from an injury to renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other preferred monitoring embodiments, these methods comprise monitoring renal status in a subject suffering from reduced renal function, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In yet other preferred monitoring embodiments, these methods comprise monitoring renal status in a subject suffering from acute renal failure, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In other additional preferred monitoring embodiments, these methods comprise monitoring renal status in a subject at risk of an injury to renal function due to the pre-existence of one or more known risk factors for prerenal, intrinsic renal, or postrenal ARF, and the assay result(s) is/are correlated to the occurrence or nonoccurrence of a change in renal status in the subject. For example, the measured concentration(s) may be compared to a threshold value. For a positive going marker, when the measured concentration is above the threshold, a worsening of renal function may be assigned to the subject; alternatively, when the measured concentration is below the threshold, an improvement of renal function may be assigned to the subject. For a negative going marker, when the measured concentration is below the threshold, a worsening of renal function may be assigned to the subject; alternatively, when the measured concentration is above the threshold, an improvement of renal function may be assigned to the subject.

In still other embodiments, the methods for evaluating renal status described herein are methods for classifying a renal injury in the subject; that is, determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage. In these embodiments, the assay result(s), for example measured concentration(s) of one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 is/are correlated to a particular class and/or subclass. The following are preferred classification embodiments.

In preferred classification embodiments, these methods comprise determining whether a renal injury in a subject is prerenal, intrinsic renal, or postrenal; and/or further subdividing these classes into subclasses such as acute tubular injury, acute glomerulonephritis acute tubulointerstitial nephritis, acute vascular nephropathy, or infiltrative disease; and/or assigning a likelihood that a subject will progress to a particular RIFLE stage, and the assay result(s) is/are correlated to the injury classification for the subject. For example, the measured concentration may be compared to a threshold value, and when the measured concentration is above the threshold, a particular classification is assigned; alternatively, when the measured concentration is below the threshold, a different classification may be assigned to the subject.

A variety of methods may be used by the skilled artisan to arrive at a desired threshold value for use in these methods. For example, the threshold value may be determined from a population of normal subjects by selecting a concentration representing the 75th, 85th, 90th, 95th, or 99th percentile of a kidney injury marker measured in such normal subjects. Alternatively, the threshold value may be determined from a "diseased" population of subjects, e.g., those suffering from an injury or having a predisposition for an injury (e.g., progression to ARF or some other clinical outcome such as death, dialysis, renal transplantation, etc.), by selecting a concentration representing the 75th, 85th, 90th, 95th, or 99th percentile of a kidney injury marker measured in such subjects. In another alternative, the threshold value may be determined from a prior measurement of a kidney injury marker in the same subject; that is, a temporal change in the level of a kidney injury marker in the subject may be used to assign risk to the subject.

The foregoing discussion is not meant to imply, however, that the kidney injury markers of the present invention must be compared to corresponding individual thresholds. Methods for combining assay results can comprise the use of multivariate logistical regression, loglinear modeling, neural network analysis, n-of-m analysis, decision tree analysis, calculating ratios of markers, etc. This list is not meant to be limiting. In these methods, a composite result which is determined by combining individual markers may be treated as if it is itself a marker; that is, a threshold may be determined for the composite result as described herein for individual markers, and the composite result for an individual patient compared to this threshold.

The ability of a particular test to distinguish two populations can be established using ROC analysis. For example, ROC curves established from a "first" subpopulation which is predisposed to one or more future changes in renal status, and a "second" subpopulation which is not so predisposed can be used to calculate a ROC curve, and the area under the curve provides a measure of the quality of the test. Preferably, the tests described herein provide a ROC curve area greater than 0.5, preferably at least 0.6, more preferably 0.7, still more preferably at least 0.8, even more preferably at least 0.9, and most preferably at least 0.95.

In certain aspects, the measured concentration of one or more kidney injury markers, or a composite of such markers, may be treated as continuous variables. For example, any particular concentration can be converted into a corresponding probability of a future reduction in renal function for the subject, the occurrence of an injury, a classification, etc. In yet another alternative, a threshold that can provide an acceptable level of specificity and sensitivity in separating a population of subjects into "bins" such as a "first" subpopulation (e.g., which is predisposed to one or more future changes in renal status, the occurrence of an injury, a classification, etc.) and a "second" subpopulation which is not so predisposed. A threshold value is selected to separate this first and second population by one or more of the following measures of test accuracy:

an odds ratio greater than 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less;

a specificity of greater than 0.5, preferably at least about 0.6, more preferably at least about 0.7, still more preferably at least about 0.8, even more preferably at least about 0.9 and most preferably at least about 0.95, with a corresponding sensitivity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

a sensitivity of greater than 0.5, preferably at least about 0.6, more preferably at least about 0.7, still more preferably at least about 0.8, even more preferably at least about 0.9 and most preferably at least about 0.95, with a corresponding specificity greater than 0.2, preferably greater than about 0.3, more preferably greater than about 0.4, still more preferably at least about 0.5, even more preferably about 0.6, yet more preferably greater than about 0.7, still more preferably greater than about 0.8, more preferably greater than about 0.9, and most preferably greater than about 0.95;

at least about 75% sensitivity, combined with at least about 75% specificity;

a positive likelihood ratio (calculated as sensitivity/(1-specificity)) of greater than 1, at least about 2, more preferably at least about 3, still more preferably at least about 5, and most preferably at least about 10; or

a negative likelihood ratio (calculated as (1-sensitivity)/specificity) of less than 1, less than or equal to about 0.5, more preferably less than or equal to about 0.3, and most preferably less than or equal to about 0.1.

The term "about" in the context of any of the above measurements refers to +/- 5% of a given measurement.

Multiple thresholds may also be used to assess renal status in a subject. For example, a "first" subpopulation which is predisposed to one or more future changes in renal status, the occurrence of an injury, a classification, etc., and a "second" subpopulation which is not so predisposed can be combined into a single group. This group is then subdivided into three or more equal parts (known as tertiles, quartiles, quintiles, etc., depending on the number of subdivisions). An odds ratio is assigned to subjects based on which subdivision they fall into. If one considers a tertile, the lowest or highest tertile can be used as a reference for comparison of the other subdivisions. This reference subdivision is assigned an odds ratio of 1. The second tertile is assigned an odds ratio that is relative to that first tertile. That is, someone in the second tertile might be 3 times more likely to suffer one or more future changes in renal status in comparison to someone in the first tertile. The third tertile is also assigned an odds ratio that is relative to that first tertile.

In certain embodiments, the assay method is an immunoassay. Antibodies for use in such assays will specifically bind a full length kidney injury marker of interest, and may also bind one or more polypeptides that are "related" thereto, as that term is defined hereinafter. Numerous immunoassay formats are known to those of skill in the art. Preferred body fluid samples are selected from the group consisting of urine, blood, serum, saliva, tears, and plasma. In the case of those kidney injury markers which are membrane proteins as described hereinafter, preferred assays detect soluble forms thereof.

The foregoing method steps should not be interpreted to mean that the kidney injury marker assay result(s) is/are used in isolation in the methods described herein. Rather, additional variables or other clinical indicia may be included in the methods described herein. For example, a risk stratification, diagnostic, classification, monitoring, etc. method may combine the assay result(s) with one or more variables measured for the subject selected from the group consisting of demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score, risk scores of Thakar et al. (J. Am. Soc. Nephrol. 16: 162-68, 2005), Mehran et al. (J. Am. Coll. Cardiol. 44: 1393-99, 2004), Wijeysundera et al. (JAMA 297: 1801-9, 2007), Goldstein and Chawla (Clin. J. Am. Soc. Nephrol. 5: 943-49, 2010), or Chawla et al. (Kidney Intl. 68: 2274-80, 2005)), a glomerular filtration rate, an estimated glomerular filtration rate, a urine production rate, a serum or plasma creatinine concentration, a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, a renal failure index calculated as urine sodium / (urine creatinine / plasma creatinine), a serum or plasma neutrophil gelatinase (NGAL) concentration, a urine NGAL concentration, a serum or plasma cystatin C concentration, a serum or plasma cardiac troponin concentration, a serum or plasma BNP concentration, a serum or plasma NTproBNP concentration, and a serum or plasma proBNP concentration. Other measures of renal function which may be combined with one or more kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

When more than one marker is measured, the individual markers may be measured in samples obtained at the same time, or may be determined from samples obtained at different (e.g., an earlier or later) times. The individual markers may also be measured on the same or different body fluid samples. For example, one kidney injury marker may be measured in a serum or plasma sample and another kidney injury marker may be measured in a urine sample. In addition, assignment of a likelihood may combine an individual kidney injury marker assay result with temporal changes in one or more additional variables.

In various related aspects, the present invention also relates to devices and kits for performing the methods described herein. Suitable kits comprise reagents sufficient for performing an assay for at least one of the described kidney injury markers, together with instructions for performing the described threshold comparisons.

In certain embodiments, reagents for performing such assays are provided in an assay device, and such assay devices may be included in such a kit. Preferred reagents can comprise one or more solid phase antibodies, the solid phase antibody comprising antibody that detects the intended biomarker target(s) bound to a solid support. In the case of sandwich immunoassays, such reagents can also include one or more detectably labeled antibodies, the detectably labeled antibody comprising antibody that detects the intended biomarker target(s) bound to a detectable label. Additional optional elements that may be provided as part of an assay device are described hereinafter.

Detectable labels may include molecules that are themselves detectable (e.g., fluorescent moieties, electrochemical labels, ecl (electrochemical luminescence) labels, metal chelates, colloidal metal particles, etc.) as well as molecules that may be indirectly detected by production of a detectable reaction product (e.g., enzymes such as horseradish peroxidase, alkaline phosphatase, etc.) or through the use of a specific binding molecule which itself may be detectable (e.g., a labeled antibody that binds to the second antibody, biotin, digoxigenin, maltose, oligohistidine, 2,4-dintrobenzene, phenylarsenate, ssDNA, dsDNA, etc.).

Generation of a signal from the signal development element can be performed using various optical, acoustical, and electrochemical methods well known in the art. Examples of detection modes include fluorescence, radiochemical detection, reflectance, absorbance, amperometry, conductance, impedance, interferometry, ellipsometry, etc. In certain of these methods, the solid phase antibody is coupled to a transducer (e.g., a diffraction grating, electrochemical sensor, etc) for generation of a signal, while in others, a signal is generated by a transducer that is spatially separate from the solid phase antibody (e.g., a fluorometer that employs an excitation light source and an optical detector). This list is not meant to be limiting. Antibody-based biosensors may also be employed to determine the presence or amount of analytes that optionally eliminate the need for a labeled molecule.

DETAILED DESCRIPTION OF THE INVENTION

The present invention relates to methods and compositions for diagnosis, differential diagnosis, risk stratification, monitoring, classifying and determination of treatment regimens in subjects suffering or at risk of suffering from injury to renal function, reduced renal function and/or acute renal failure through measurement of one or more kidney injury markers. In various embodiments, a measured concentration of one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 or one or more markers related thereto, are correlated to the renal status of the subject.

For purposes of this document, the following definitions apply:

As used herein, an "injury to renal function" is an abrupt (within 14 days, preferably within 7 days, more preferably within 72 hours, and still more preferably within 48 hours) measurable reduction in a measure of renal function. Such an injury may be identified, for example, by a decrease in glomerular filtration rate or estimated GFR, a reduction in urine output, an increase in serum creatinine, an increase in serum cystatin C, a requirement for renal replacement therapy, *etc.* "Improvement in Renal Function" is an abrupt (within 14 days, preferably within 7 days, more preferably within 72 hours, and still more preferably within 48 hours) measurable increase in a measure of renal function. Preferred methods for measuring and/or estimating GFR are described hereinafter.

As used herein, "reduced renal function" is an abrupt (within 14 days, preferably within 7 days, more preferably within 72 hours, and still more preferably within 48 hours) reduction in kidney function identified by an absolute increase in serum creatinine of greater than or equal to 0.1 mg/dL ($\geq$ 8.8 $\mu$mol/L), a percentage increase in serum creatinine of greater than or equal to 20% (1.2-fold from baseline), or a reduction in urine output (documented oliguria of less than 0. 5 ml/kg per hour).

As used herein, "acute renal failure" or "ARF" is an abrupt (within 14 days, preferably within 7 days, more preferably within 72 hours, and still more preferably within 48 hours) reduction in kidney function identified by an absolute increase in serum creatinine of greater than or equal to 0.3 mg/dl ($\geq$ 26.4 $\mu$mol/l), a percentage increase in serum creatinine of greater than or equal to 50% (1. 5-fold from baseline), or a reduction in urine output (documented oliguria of less than 0.5 ml/kg per hour for at least 6 hours). This term is synonymous with "acute kidney injury" or "AKI."

The following biomarkers (listed with the Swiss-Prot entry number of the human precursor) find use in the present invention as kidney injury markers:

| Swiss-Prot Entry # | Name |
|---|---|
| P52823 | Stanniocalcin-1 |
| P01008 | Antithrombin-III |
| 060603 | Toll-like receptor 2 |
| na | Triiodothyronine (T3) |
| na | Thyroxine (T4) |
| Q16610 | Extracellular matrix protein 1 |
| P00488 | Coagulation factor XIII A chain |
| P05160 | Coagulation factor XIII B chain |
| Q96PD4 | Interleukin-17F |
| Q9GZX6 | Interleukin-22 |
| P04004 | Vitronectin |
| na | Progesterone |
| na | Estradiol |
| Q99988 | Growth/differentiation factor 15 |
| Q8NBP7 | Proprotein convertase subtilisin/kexin type 9 |
| na - not applicable | |

As used herein, the term "Triiodothyroxine" refers to the thyroid hormone also known as T3, while "Thyroxine" refers to the thyroid hormone known as T4. T3 is a product of deiodination of T4 by enzymes in the thyroid. T3 and T4 are carried in the circulation by thyroxine-binding globulins, thyroxine binding prealbumins, and albumins. As a consequence of structural differences, T3 and T4 may be distinguished from one another, for example using antibodies that are specific for differences between the hormones.

Estradiol ((17β)-estra-1,3,5(10)-triene-3,17-diol) is a steroid hormone that is the predominant estrogen in females during reproductive years both in terms of absolute serum levels as well as in terms of estrogenic activity. During menopause, estrone is the predominant circulating estrogen and during pregnancy estriol is the predominant circulating estrogen in terms of serum levels. Estradiol is also present in males, being produced as an active metabolic product of testosterone. The serum levels of estradiol in males (14 -55 pg/mL) are roughly comparable to those of postmenopausal women (< 35 pg/mL).

Progesterone (pregn-4-ene-3,20-dione) is a C-21 steroid hormone. In women, progesterone levels are relatively low during the preovulatory phase of the menstrual cycle, rise after ovulation, and are elevated during the luteal phase. Progesterone levels are relatively low in children and postmenopausal women, while adult males have levels similar to those in women during the follicular phase of the menstrual cycle.

As used herein, the term "Stanniocalcin-1" refers to one or more polypeptides present in a biological sample that are derived from the Stanniocalcin-1 precursor (human precursor: Swiss-Prot P52823 (SEQ ID NO: 1))

```
            10         20         30         40         50         60
     MLQNSAVLLV LVISASATHE AEQNDSVSPR KSRVAAQNSA EVVRCLNSAL QVGCGAFACL

            70         80         90        100        110        120
     ENSTCDTDGM YDICKSFLYS AAKFDTQGKA FVKESLKCIA NGVTSKVFLA IRRCSTFQRM

           130        140        150        160        170        180
     IAEVQEECYS KLNVCSIAKR NPEAITEVVQ LPNHFSNRYY NRLVRSLLEC DEDTVSTIRD

           190        200        210        220        230        240
     SLMEKIGPNM ASLFHILQTD HCAQTHPRAD FNRRRTNEPQ KLKVLLRNLR GEEDSPSHIK

     RTSHESA
```

The following domains have been identified in Stanniocalcin-1:

| Residues | Length | Domain ID |
|----------|--------|-----------|
| 1-17 | 17 | Signal peptide |
| 18-33 | 16 | Propeptide |
| 34-247 | 214 | Stanniocalcin-1 |

As used herein, the term "Proprotein convertase subtilisin/kexin type 9" refers to one or more polypeptides present in a biological sample that are derived from the Proprotein convertase subtilisin/kexin type 9 precursor (human precursor: Swiss-Prot Q8NBP7 (SEQ ID NO: 2))

```
              10         20         30         40         50         60
         MGTVSSRRSW WPLPLLLLLL LLLGPAGARA QEDEDGDYEE LVLALRSEED GLAEAPEHGT

              70         80         90        100        110        120
         TATFHRCAKD PWRLPGTYVV VLKEETHLSQ SERTARRLQA QAARRGYLTK ILHVFHGLLP

             130        140        150        160        170        180
         GFLVKMSGDL LELALKLPHV DYIEEDSSVF AQSIPWNLER ITPPRYRADE YQPPDGGSLV

             190        200        210        220        230        240
         EVYLLDTSIQ SDHREIEGRV MVTDFENVPE EDGTRFHRQA SKCDSHGTHL AGVVSGRDAG

             250        260        270        280        290        300
         VAKGASMRSL RVLNCQGKGT VSGTLIGLEF IRKSQLVQPV GPLVVLLPLA GGYSRVLNAA

             310        320        330        340        350        360
         CQRLARAGVV LVTAAGNFRD DACLYSPASA PEVITVGATN AQDQPVTLGT LGTNFGRCVD

             370        380        390        400        410        420
         LFAPGEDIIG ASSDCSTCFV SQSGTSQAAA HVAGIAAMML SAEPELTLAE LRQRLIHFSA

             430        440        450        460        470        480
         KDVINEAWFP EDQRVLTPNL VAALPPSTHG AGWQLFCRTV WSAHSGPTRM ATAVARCAPD

             490        500        510        520        530        540
         EELLSCSSFS RSGKRRGERM EAQGGKLVCR AHNAFGGEGV YAIARCCLLP QANCSVHTAP

             550        560        570        580        590        600
         PAEASMGTRV HCHQQGHVLT GCSSHWEVED LGTHKPPVLR PRGQPNQCVG HREASIHASC

             610        620        630        640        650        660
         CHAPGLECKV KEHGIPAPQE QVTVACEEGW TLTGCSALPG TSHVLGAYAV DNTCVVRSRD

             670        680        690
         VSTTGSTSEG AVTAVAICCR SRHLAQASQE LQ
```

The following domains have been identified in Proprotein convertase subtilisin/kexin type 9:

| Residues | Length | Domain ID |
|---|---|---|
| 1-30 | 30 | Signal peptide |
| 31-152 | 122 | Propeptide |
| 153-692 | 540 | Proprotein convertase subtilisin/kexin type 9 |
| 1-174 | | →-MSPWK (SEQ ID NO: 3) in isoform 2 |
| 333-365 | | → GRTSLVPPATAAPALCHRVGHHRLLPTWLALQP (SEQ ID NO: 4) in isoform 2 |
| 366-692 | | Missing in isoform 2 |

As used herein, the term "Interleukin-22" refers to one or more polypeptides present in a biological sample that are derived from the Interleukin-22 precursor (human precursor: Swiss-Prot Q9GZX6 (SEQ ID NO: 5))

```
            10          20          30          40          50          60
MAALQKSVSS  FLMGTLATSC  LLLLALLVQG  GAAAPISSHC  RLDKSNFQQP  YITNRTFMLA

            70          80          90         100         110         120
KEASLADNNT  DVRLIGEKLF  HGVSMSERCY  LMKQVLNFTL  EEVLFPQSDR  FQPYMQEVVP

           130         140         150         160         170
FLARLSNRLS  TCHIEGDDLH  IQRNVQKLKD  TVKKLGESGE  IKAIGELDLL  FMSLRNACI
```

The following domains have been identified in Interleukin-22:

| Residues | Length | Domain ID |
|---|---|---|
| 1-33 | 33 | Signal peptide |
| 34-179 | 146 | Interleukin-22 |

As used herein, the term "Coagulation factor XIII A chain" refers to one or more polypeptides present in a biological sample that are derived from the Coagulation factor XIII A chain precursor (human precursor: Swiss-Prot P00488 (SEQ ID NO: 6))

```
            10          20          30          40          50          60
MSETSRTAFG  GRRAVPPNNS  NAAEDDLPTV  ELQGVVPRGV  NLQEFLNVTS  VHLFKERWDT

            70          80          90         100         110         120
NKVDHHTDKY  ENNKLIVRRG  QSFYVQIDFS  RPYDPRRDLF  RVEYVIGRYP  QENKGTYIPV
```

```
         130        140        150        160        170        180
   PIVSELQSGK WGAKIVMRED RSVRLSIQSS PKCIVGKFRM YVAVWTPYGV LRTSRNPETD

         190        200        210        220        230        240
   TYILFNPWCE DDAVYLDNEK EREEYVLNDI GVIFYGEVND IKTRSWSYGQ FEDGILDTCL

         250        260        270        280        290        300
   YVMDRAQMDL SGRGNPIKVS RVGSAMVNAK DDEGVLVGSW DNIYAYGVPP SAWTGSVDIL

         310        320        330        340        350        360
   LEYRSSENPV RYGQCWVFAG VFNTFLRCLG IPARIVTNYF SAHDNDANLQ MDIFLEEDGN

         370        380        390        400        410        420
   VNSKLTKDSV WNYHCWNEAW MTRPDLPVGF GGWQAVDSTP QENSDGMYRC GPASVQAIKH

         430        440        450        460        470        480
   GHVCFQFDAP FVFAEVNSDL IYITAKKDGT HVVENVDATH IGKLIVTKQI GGDGMMDITD

         490        500        510        520        530        540
   TYKFQEGQEE ERLALETALM YGAKKPLNTE GVMKSRSNVD MDFEVENAVL GKDFKLSITF

         550        560        570        580        590        600
   RNNSHNRYTI TAYLSANITF YTGVPKAEFK KETFDVTLEP LSFKKEAVLI QAGEYMGQLL

         610        620        630        640        650        660
   EQASLHFFVT ARINETRDVL AKQKSTVLTI PEIIIKVRGT QVVGSDMTVT VQFTNPLKET

         670        680        690        700        710        720
   LRNVWVHLDG PGVTRPMKKM FREIRPNSTV QWEEVCRPWV SGHRKLIASM SSDSLRHVYG

         730
   ELDVQIQRRP SM
```

The following domains have been identified in Coagulation factor XIII A chain:

| Residues | Length | Domain ID |
|---|---|---|
| 1 | 1 | Initiator methionine |
| 2-38 | 37 | Activation peptide |
| 39-732 | 694 | Coagulation factor XIII A chain |

As used herein, the term "Toll-like receptor 2" refers to one or more polypeptides present in a biological sample that are derived from the Toll-like receptor 2 precursor (human sequence: Swiss-Prot O60603 (SEQ ID NO: 7)):

```
         10         20         30         40         50         60
   MPHTLWMVWV LGVIISLSKE ESSNQASLSC DRNGICKGSS GSLNSIPSGL TEAVKSLDLS

         70         80         90        100        110        120
```

```
NNRITYISNS DLQRCVNLQA LVLTSNGINT IEEDSFSSLG SLEHLDLSYN YLSNLSSSWF

       130        140        150        160        170        180
KPLSSLTFLN LLGNPYKTLG ETSLFSHLTK LQILRVGNMD TFTKIQRKDF AGLTFLEELE

       190        200        210        220        230        240
IDASDLQSYE PKSLKSIQNV SHLILHMKQH ILLLEIFVDV TSSVECLELR DTDLDTFHFS

       250        260        270        280        290        300
ELSTGETNSL IKKFTFRNVK ITDESLFQVM KLLNQISGLL ELEFDDCTLN GVGNFRASDN

       310        320        330        340        350        360
DRVIDPGKVE TLTIRRLHIP RFYLFYDLST LYSLTERVKR ITVENSKVFL VPCLLSQHLK

       370        380        390        400        410        420
SLEYLDLSEN LMVEEYLKNS ACEDAWPSLQ TLILRQNHLA SLEKTGETLL TLKNLTNIDI

       430        440        450        460        470        480
SKNSFHSMPE TCQWPEKMKY LNLSSTRIHS VTGCIPKTLE ILDVSNNNLN LFSLNLPQLK

       490        500        510        520        530        540
ELYISRNKLM TLPDASLLPM LLVLKISRNA ITTFSKEQLD SFHTLKTLEA GGNNFICSCE

       550        560        570        580        590        600
FLSFTQEQQA LAKVLIDWPA NYLCDSPSHV RGQQVQDVRL SVSECHRTAL VSGMCCALFL

       610        620        630        640        650        660
LILLTGVLCH RFHGLWYMKM MWAWLQAKRK PRKAPSRNIC YDAFVSYSER DAYWVENLMV

       670        680        690        700        710        720
QELENFNPPF KLCLHKRDFI PGKWIIDNII DSIEKSHKTV FVLSENFVKS EWCKYELDFS

       730        740        750        760        770        780
HFRLFDENND AAILILLEPI EKKAIPQRFC KLRKIMNTKT YLEWPMDEAQ REGFWVNLRA

AIKS
```

In certain embodiments, the Toll-like receptor 2 assay detects one or more soluble forms of Toll-like receptor 2. Toll-like receptor 2 is a single-pass membrane protein having an extracellular domain which may be found in soluble forms of Toll-like receptor 2 generated by proteolysis of the membrane-bound form or by alternative splicing. In the case of an immunoassay, one or more antibodies that bind to epitopes within an extracellular domain may be used to detect these soluble form(s). The following domains have been identified in Toll-like receptor 2:

| Residues | Length | Domain ID |
|---|---|---|
| 1-18 | 18 | Signal peptide |
| 19-784 | 766 | Toll-like receptor 2 |
| 589-609 | 21 | transmembrane domain |
| 610-784 | 175 | cytoplasmic domain |
| 19-588 | 570 | extracellular domain |

As used herein, the term "Antithrombin-III" refers to one or more polypeptides present in a biological sample that are derived from the Antithrombin-III precursor (human precursor: Swiss-Prot P01008 (SEQ ID NO: 8))

```
        10         20         30         40         50         60
MYSNVIGTVT SGKRKVYLLS LLLIGFWDCV TCHGSPVDIC TAKPRDIPMN PMCIYRSPEK

        70         80         90        100        110        120
KATEDEGSEQ KIPEATNRRV WELSKANSRF ATTFYQHLAD SKNDNDNIFL SPLSISTAFA

       130        140        150        160        170        180
MTKLGACNDT LQQLMEVFKF DTISEKTSDQ IHFFFAKLNC RLYRKANKSS KLVSANRLFG

       190        200        210        220        230        240
DKSLTFNETY QDISELVYGA KLQPLDFKEN AEQSRAAINK WVSNKTEGRI TDVIPSEAIN

       250        260        270        280        290        300
ELTVLVLVNT IYFKGLWKSK FSPENTRKEL FYKADGESCS ASMMYQEGKF RYRRVAEGTQ

       310        320        330        340        350        360
VLELPFKGDD ITMVLILPKP EKSLAKVEKE LTPEVLQEWL DELEEMMLVV HMPRFRIEDG

       370        380        390        400        410        420
FSLKEQLQDM GLVDLFSPEK SKLPGIVAEG RDDLYVSDAF HKAFLEVNEE GSEAAASTAV

       430        440        450        460
VIAGRSLNPN RVTFKANRPF LVFIREVPLN TIIFMGRVAN PCVK
```

The following domains have been identified in Antithrombin-III:

| Residues | Length | Domain ID |
|---|---|---|
| 1-32 | 32 | Signal peptide |
| 33-464 | 432 | Antithrombin-III |

As used herein, the term "Vitronectin" refers to one or more polypeptides present in a biological sample that are derived from the Vitronectin precursor (human precursor: Swiss-Prot P04004 (SEQ ID NO: 9))

```
        10         20         30         40         50         60
MAPLRPLLIL ALLAWVALAD QESCKGRCTE GFNVDKKCQC DELCSYYQSC CTDYTAECKP

        70         80         90        100        110        120
QVTRGDVFTM PEDEYTVYDD GEEKNNATVH EQVGGPSLTS DLQAQSKGNP EQTPVLKPEE
```

```
        130         140         150         160         170         180
   EAPAPEVGAS  KPEGIDSRPE  TLHPGRPQPP  AEEELCSGKP  FDAFTDLKNG  SLFAFRGQYC

        190         200         210         220         230         240
   YELDEKAVRP  GYPKLIRDVW  GIEGPIDAAF  TRINCQGKTY  LFKGSQYWRF  EDGVLDPDYP

        250         260         270         280         290         300
   RNISDGFDGI  PDNVDAALAL  PAHSYSGRER  VYFFKGKQYW  EYQFQHQPSQ  EECEGSSLSA

        310         320         330         340         350         360
   VFEHFAMMQR  DSWEDIFELL  FWGRTSAGTR  QPQFISRDWH  GVPGQVDAAM  AGRIYISGMA

        370         380         390         400         410         420
   PRPSLAKKQR  FRHRNRKGYR  SQRGHSRGRN  QNSRRPSRAT  WLSLFSSEES  NLGANNYDDY

        430         440         450         460         470
   RMDWLVPATC  EPIQSVFFFS  GDKYYRVNLR  TRRVDTVDPP  YPRSIAQYWL  GCPAPGHL
```

The following domains have been identified in Vitronectin:

| Residues | Length | Domain ID |
|---|---|---|
| 1-19 | 19 | Signal peptide |
| 20-478 | 459 | Vitronectin |
| 20-398 | 379 | Vitronectin V65 subunit |
| 20-63 | 44 | Somatomedin-B |
| 399-478 | 80 | Vitronectin V10 subunit |

As used herein, the term "Coagulation factor XIII B chain" refers to one or more polypeptides present in a biological sample that are derived from the Coagulation factor XIII B chain precursor (human precursor: Swiss-Prot P05160 (SEQ ID NO: 10))

```
         10          20          30          40          50          60
   MRLKNLTFII  ILIISGELYA  EEKPCGFPHV  ENGRIAQYYY  TFKSFYFPMS  IDKKLSFFCL

         70          80          90         100         110         120
   AGYTTESGRQ  EEQTTCTTEG  WSPEPRCFKK  CTKPDLSNGY  ISDVKLLYKI  QENMRYGCAS

        130         140         150         160         170         180
   GYKTTGGKDE  EVVQCLSDGW  SSQPTCRKEH  ETCLAPELYN  GNYSTTQKTF  KVKDKVQYEC

        190         200         210         220         230         240
   ATGYYTAGGK  KTEEVECLTY  GWSLTPKCTK  LKCSSLRLIE  NGYFHPVKQT  YEEGDVVQFF

        250         260         270         280         290         300
   CHENYYLSGS  DLIQCYNFGW  YPESPVCEGR  RNRCPPPPLP  INSKIQTHST  TYRHGEIVHI
```

```
        310          320          330          340          350          360
ECELNFEIHG   SAEIRCEDGK   WTEPPKCIEG   QEKVACEEPP   FIENGAANLH   SKIYYNGDKV

        370          380          390          400          410          420
TYACKSGYLL   HGSNEITCNR   GKWTLPPECV   ENNENCKHPP   VVMNGAVADG   ILASYATGSS

        430          440          450          460          470          480
VEYRCNEYYL   LRGSKISRCE   QGKWSSPPVC   LEPCTVNVDY   MNRNNIEMKW   KYEGKVLHGD

        490          500          510          520          530          540
LIDFVCKQGY   DLSPLTPLSE   LSVQCNRGEV   KYPLCTRKES   KGMCTSPPLI   KHGVIISSTV

        550          560          570          580          590          600
DTYENGSSVE   YRCFDHHFLE   GSREAYCLDG   MWTTPPLCLE   PCTLSFTEME   KNNLLLKWDF

        610          620          630          640          650          660
DNRPHILHGE   YIEFICRGDT   YPAELYITGS   ILRMQCDRGQ   LKYPRCIPRQ   STLSYQEPLR

T
```

The following domains have been identified in Coagulation factor XIII B chain:

| Residues | Length | Domain ID |
|----------|--------|-----------|
| 1-20 | 20 | Signal peptide |
| 21-661 | 641 | Coagulation factor XIII B chain |

As used herein, the term "Interleukin-17F" refers to one or more polypeptides present in a biological sample that are derived from the Interleukin-17F precursor (human precursor: Swiss-Prot Q96PD4 (SEQ ID NO: 11))

```
         10           20           30           40           50           60
MTVKTLHGPA   MVKYLLLSIL   GLAFLSEAAA   RKIPKVGHTF   FQKPESCPPV   PGGSMKLDIG

         70           80           90          100          110          120
IINENQRVSM   SRNIESRSTS   PWNYTVTWDP   NRYPSEVVQA   QCRNLGCINA   QGKEDISMNS

        130          140          150          160
VPIQQETLVV   RRKHQGCSVS   FQLEKVLVTV   GCTCVTPVIH   HVQ
```

The following domains have been identified in Interleukin-17F:

| Residues | Length | Domain ID |
|----------|--------|-----------|
| 1-30 | 30 | Signal peptide |
| 31-163 | 133 | Interleukin-17F |

As used herein, the term "Extracellular matrix protein 1" refers to one or more polypeptides present in a biological sample that are derived from the Extracellular matrix protein 1 precursor (human precursor: Swiss-Prot Q16610 (SEQ ID NO: 12))

```
        10         20         30         40         50         60
MGTTARAALV LTYLAVASAA SEGGFTATGQ RQLRPEHFQE VGYAAPPSPP LSRSLPMDHP

        70         80         90        100        110        120
DSSQHGPPFE GQSQVQPPPS QEATPLQQEK LLPAQLPAEK EVGPPLPQEA VPLQKELPSL

       130        140        150        160        170        180
QHPNEQKEGT PAPFGDQSHP EPESWNAAQH CQQDRSQGGW GHRLDGFPPG RPSPDNLNQI

       190        200        210        220        230        240
CLPNRQHVVY GPWNLPQSSY SHLTRQGETL NFLEIGYSRC CHCRSHTNRL ECAKLVWEEA

       250        260        270        280        290        300
MSRFCEAEFS VKTRPHWCCT RQGEARFSCF QEEAPQPHYQ LRACPSHQPD ISSGLELPFP

       310        320        330        340        350        360
PGVPTLDNIK NICHLRRFRS VPRNLPATDP LQRELLALIQ LEREFQRCCR QGNNHTCTWK

       370        380        390        400        410        420
AWEDTLDKYC DREYAVKTHH HLCCRHPPSP TRDECFARRA PYPNYDRDIL TIDIGRVTPN

       430        440        450        460        470        480
LMGHLCGNQR VLTKHKHIPG LIHNMTARCC DLPFPEQACC AEEEKLTFIN DLCGPRRNIW

       490        500        510        520        530        540
RDPALCCYLS PGDEQVNCFN INYLRNVALV SGDTENAKGQ GEQGSTGGTN ISSTSEPKEE
```

The following domains have been identified in Extracellular matrix protein 1:

| Residues | Length | Domain ID |
|---|---|---|
| 1-19 | 19 | Signal peptide |
| 20-540 | 521 | Extracellular matrix protein 1 |
| 237-361 |  | Missing in isoform 2 |
| 1-71 |  | Missing in isoform 3 |
| 72-81 |  | → MALPLRDRVK (SEQ ID NO: 13) in isoform 3 |
| 237-241 |  | →VRLGS (SEQ ID NO: 14) in isoform 3 |
| 242-250 |  | Missing in isoform 3 |
| 74 |  | → GKEGRGPRPHSQPWLGERVGCSHIPPSI (SEQ ID NO: 15) in isoform 4 |

As used herein, the term "Growth/differentiation factor 15" refers to one or more polypeptides present in a biological sample that are derived from the Growth/differentiation factor 15 precursor (human precursor: Swiss-Prot Q99988 (SEQ ID NO: 16))

```
            10          20          30          40          50          60
    MPGQELRTVN  GSQMLLVLLV  LSWLPHGGAL  SLAEASRASF  PGPSELHSED  SRFRELRKRY

            70          80          90         100         110         120
    EDLLTRLRAN  QSWEDSNTDL  VPAPAVRILT  PEVRLGSGGH  LHLRISRAAL  PEGLPEASRL

           130         140         150         160         170         180
    HRALFRLSPT  ASRSWDVTRP  LRRQLSLARP  QAPALHLRLS  PPPSQSDQLL  AESSSARPQL

           190         200         210         220         230         240
    ELHLRPQAAR  GRRRARARNG  DHCPLGPGRC  CRLHTVRASL  EDLGWADWVL  SPREVQVTMC

           250         260         270         280         290         300
    IGACPSQFRA  ANMHAQIKTS  LHRLKPDTVP  APCCVPASYN  PMVLIQKTDT  GVSLQTYDDL


    LAKDCHCI
```

The following domains have been identified in Growth/differentiation factor 15:

| Residues | Length | Domain ID |
| --- | --- | --- |
| 1-29 | 29 | Signal peptide |
| 30-194 | 165 | Propeptide |
| 195-308 | 114 | Growth/differentiation factor 15 |

As used herein, the term "relating a signal to the presence or amount" of an analyte reflects the following understanding. Assay signals are typically related to the presence or amount of an analyte through the use of a standard curve calculated using known concentrations of the analyte of interest. As the term is used herein, an assay is "configured to detect" an analyte if an assay can generate a detectable signal indicative of the presence or amount of a physiologically relevant concentration of the analyte. Because an antibody epitope is on the order of 8 amino acids, an immunoassay configured to detect a marker of interest will also detect polypeptides related to the marker sequence, so long as those polypeptides contain the epitope(s) necessary to bind to the antibody or antibodies used in the assay. The term "related marker" as used herein with regard to a biomarker such as one of the kidney injury markers described herein refers to one or more fragments, variants, etc., of a particular marker or its biosynthetic parent that may be detected as a surrogate for the marker itself or as independent biomarkers. The term also refers to one or more polypeptides present in a biological sample that are derived from the biomarker precursor complexed to additional species, such as binding proteins, receptors, heparin, lipids, sugars, etc.

In this regard, the skilled artisan will understand that the signals obtained from an immunoassay are a direct result of complexes formed between one or more antibodies and the target biomolecule (*i.e.*, the analyte) and polypeptides containing the necessary epitope(s) to which the antibodies bind. While such assays may detect the full length biomarker and the assay result be expressed as a concentration of a biomarker of interest, the signal from the assay is actually a result of all such "immunoreactive" polypeptides present in the sample. Expression of biomarkers may also be determined by means other than immunoassays, including protein measurements (such as dot blots, western blots, chromatographic methods, mass spectrometry, *etc.*) and nucleic acid measurements (mRNA quatitation). This list is not meant to be limiting.

As used herein, the term "hydrocortisone" (also known as cortisol) refers to (11$\beta$)-11,17,21-trihydroxypregn-4-ene-3,20-dione. Hydrocortisone is a steroid hormone, or glucocorticoid, produced by the adrenal gland. It is released in response to stress and a low level of blood glucocorticoids. Its primary functions are to increase blood sugar through gluconeogenesis; suppress the immune system; and aid in fat, protein and carbohydrate metabolism.

The term "positive going" marker as that term is used herein refer to a marker that is determined to be elevated in subjects suffering from a disease or condition, relative to subjects not suffering from that disease or condition. The term "negative going" marker as that term is used herein refer to a marker that is determined to be reduced in subjects suffering from a disease or condition, relative to subjects not suffering from that disease or condition.

The term "subject" as used herein refers to a human or non-human organism. Thus, the methods and compositions described herein are applicable to both human and veterinary disease. Further, while a subject is preferably a living organism, the invention described herein may be used in post-mortem analysis as well. Preferred subjects are humans, and most preferably "patients," which as used herein refers to living humans that are receiving medical care for a disease or condition. This includes persons with no defined illness who are being investigated for signs of pathology.

Preferably, an analyte is measured in a sample. Such a sample may be obtained from a subject, or may be obtained from biological materials intended to be provided to the subject. For example, a sample may be obtained from a kidney being evaluated for possible transplantation into a subject, and an analyte measurement used to evaluate the kidney for preexisting damage. Preferred samples are body fluid samples.

The term "body fluid sample" as used herein refers to a sample of bodily fluid obtained for the purpose of diagnosis, prognosis, classification or evaluation of a subject of interest, such as a patient or transplant donor. In certain embodiments, such a sample may be obtained for the purpose of determining the outcome of an ongoing condition or the effect of a treatment regimen on a condition. Preferred body fluid samples include blood, serum, plasma, cerebrospinal fluid, urine, saliva, sputum, and pleural effusions. In addition, one of skill in the art would realize that certain body fluid samples would be more readily analyzed following a fractionation or purification procedure, for example, separation of whole blood into serum or plasma components.

The term "diagnosis" as used herein refers to methods by which the skilled artisan can estimate and/or determine the probability ("a likelihood") of whether or not a patient is suffering from a given disease or condition. In the case of the present invention, "diagnosis" includes using the results of an assay, most preferably an immunoassay, for a kidney injury marker of the present invention, optionally together with other clinical characteristics, to arrive at a diagnosis (that is, the occurrence or nonoccurrence) of an acute renal injury or ARF for the subject from which a sample was obtained and assayed. That such a diagnosis is "determined" is not meant to imply that the diagnosis is 100% accurate. Many biomarkers are indicative of multiple conditions. The skilled clinician does not use biomarker results in an informational vacuum, but rather test results are used together with other clinical indicia to arrive at a diagnosis. Thus, a measured biomarker level on one side of a predetermined diagnostic threshold indicates a greater likelihood of the occurrence of disease in the subject relative to a measured level on the other side of the predetermined diagnostic threshold.

Similarly, a prognostic risk signals a probability ("a likelihood") that a given course or outcome will occur. A level or a change in level of a prognostic indicator, which in turn is associated with an increased probability of morbidity (e.g., worsening renal function, future ARF, or death) is referred to as being "indicative of an increased likelihood" of an adverse outcome in a patient.

Marker Assays

In general, immunoassays involve contacting a sample containing or suspected of containing a biomarker of interest with at least one antibody that specifically binds to the biomarker. A signal is then generated indicative of the presence or amount of complexes formed by the binding of polypeptides in the sample to the antibody. The signal is then related to the presence or amount of the biomarker in the sample. Numerous methods and devices are well known to the skilled artisan for the detection and analysis of biomarkers. *See, e.g.,* U.S. Patents 6,143,576; 6,113,855; 6,019,944; 5,985,579; 5,947,124; 5,939,272; 5,922,615; 5,885,527; 5,851,776; 5,824,799; 5,679,526; 5,525,524; and 5,480,792, and The Immunoassay Handbook, David Wild, ed. Stockton Press, New York, 1994, each of which is hereby incorporated by reference in its entirety, including all tables, figures and claims.

The assay devices and methods known in the art can utilize labeled molecules in various sandwich, competitive, or non-competitive assay formats, to generate a signal that is related to the presence or amount of the biomarker of interest. Suitable assay formats also include chromatographic, mass spectrographic, and protein "blotting" methods. Additionally, certain methods and devices, such as biosensors and optical immunoassays, may be employed to determine the presence or amount of analytes without the need for a labeled molecule. *See, e.g.,* U.S. Patents 5,631,171; and 5,955,377, each of which is hereby incorporated by reference in its entirety, including all tables, figures and claims. One skilled in the art also recognizes that robotic instrumentation including but not limited to Beckman ACCESS®, Abbott AXSYM®, Roche ELECSYS®, Dade Behring STRATUS® systems are among the immunoassay analyzers that are capable of performing immunoassays. But any suitable immunoassay may be utilized, for example, enzyme-linked immunoassays (ELISA), radioimmunoassays (RIAs), competitive binding assays, and the like.

Antibodies or other polypeptides may be immobilized onto a variety of solid supports for use in assays. Solid phases that may be used to immobilize specific binding members include include those developed and/or used as solid phases in solid phase binding assays. Examples of suitable solid phases include membrane filters, cellulose-based papers, beads (including polymeric, latex and paramagnetic particles), glass, silicon wafers, microparticles, nanoparticles, TentaGels, AgroGels, PEGA gels, SPOCC gels, and multiple-well plates. An assay strip could be prepared by coating the antibody or a plurality of antibodies in an array on solid support. This strip could then be dipped into the test sample and then processed quickly through washes and detection steps to generate a measurable signal, such as a colored spot. Antibodies or other polypeptides may be bound to specific zones of assay devices either by conjugating directly to an assay device surface, or by indirect binding. In an example of the later case, antibodies or other polypeptides may be immobilized on particles or other solid supports, and that solid support immobilized to the device surface.

Biological assays require methods for detection, and one of the most common methods for quantitation of results is to conjugate a detectable label to a protein or nucleic acid that has affinity for one of the components in the biological system being studied. Detectable labels may include molecules that are themselves detectable (*e.g.,* fluorescent moieties, electrochemical labels, metal chelates, *etc*.) as well as molecules that may be indirectly detected by production of a detectable reaction product (*e.g.,* enzymes such as horseradish peroxidase, alkaline phosphatase, *etc*.) or by a specific binding molecule which itself may be detectable (e.g., biotin, digoxigenin, maltose, oligohistidine, 2,4-dintrobenzene, phenylarsenate, ssDNA, dsDNA, etc.).

Preparation of solid phases and detectable label conjugates often comprise the use of chemical cross-linkers. Cross-linking reagents contain at least two reactive groups, and are divided generally into homofunctional cross-linkers (containing identical reactive groups) and heterofunctional cross-linkers (containing non-identical reactive groups). Homobifunctional cross-linkers that couple through amines, sulfhydryls or react non-specifically are available from many commercial sources. Maleimides, alkyl and aryl halides, alpha-haloacyls and pyridyl disulfides are thiol reactive groups. Maleimides, alkyl and aryl halides, and alpha-haloacyls react with sulfhydryls to form thiol ether bonds, while pyridyl disulfides react with sulfhydryls to produce mixed disulfides. The pyridyl disulfide product is cleavable. Imidoesters are also very useful for protein-protein cross-links. A variety of heterobifunctional cross-linkers, each combining different attributes for successful conjugation, are commercially available.

In certain aspects, the present invention provides kits for the analysis of the described kidney injury markers. The kit comprises reagents for the analysis of at least one test sample which comprise at least one antibody that a kidney injury marker. The kit can also include devices and instructions for performing one or more of the diagnostic and/or prognostic correlations described herein. Preferred kits will comprise an antibody pair for performing a sandwich assay, or a labeled species for performing a competitive assay, for the analyte. Preferably, an antibody pair comprises a first antibody conjugated to a solid phase and a second antibody conjugated to a detectable label, wherein each of the first and second antibodies that bind a kidney injury marker. Most preferably each of the antibodies are monoclonal antibodies. The instructions for use of the kit and performing the correlations can be in the form of labeling, which refers to any written or recorded material that is attached to, or otherwise accompanies a kit at any time during its manufacture, transport, sale or use. For example, the term labeling encompasses advertising leaflets and brochures, packaging materials, instructions, audio or video cassettes, computer discs, as well as writing imprinted directly on kits.

Antibodies

The term "antibody" as used herein refers to a peptide or polypeptide derived from, modeled after or substantially encoded by an immunoglobulin gene or immunoglobulin genes, or fragments thereof, capable of specifically binding an antigen or epitope. *See, e.g.* Fundamental Immunology, 3rd Edition, W.E. Paul, ed., Raven Press, N.Y. (1993); Wilson (1994; J. Immunol. Methods 175:267-273; Yarmush (1992) J. Biochem. Biophys. Methods 25:85-97. The term antibody includes antigen-binding portions, i.e., "antigen binding sites," (e.g., fragments, subsequences, complementarity determining regions (CDRs)) that retain capacity to bind antigen, including (i) a Fab fragment, a monovalent fragment consisting of the VL, VH, CL and CHl domains; (ii) a F(ab')2 fragment, a bivalent fragment comprising two Fab fragments linked by a disulfide bridge at the hinge region; (iii) a Fd fragment consisting of the VH and CHl domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Single chain antibodies are also included by reference in the term "antibody."

Antibodies used in the immunoassays described herein preferably specifically bind to a kidney injury marker of the present invention. The term "specifically binds" is not intended to indicate that an antibody binds exclusively to its intended target since, as noted above, an antibody binds to any polypeptide displaying the epitope(s) to which the antibody binds. Rather, an antibody "specifically binds" if its affinity for its intended target is about 5-fold greater when compared to its affinity for a non-target molecule which does not display the appropriate epitope(s). Preferably the affinity of the antibody will be at least about 5 fold, preferably 10 fold, more preferably 25-fold, even more preferably 50-fold, and most preferably 100-fold or more, greater for a target molecule than its affinity for a non-target molecule. In preferred embodiments, Preferred antibodies bind with affinities of at least about $10^7$ $M^{-1}$, and preferably between about $10^8$ $M^{-1}$ to about $10^9$ $M^{-1}$, about $10^9$ $M^{-1}$ to about $10^{10}$ $M^{-1}$, or about $10^{10}$ $M^{-1}$ to about $10^{12}$ $M^{-1}$ .

Affinity is calculated as $K_d = k_{off}/k_{on}$ ($k_{off}$ is the dissociation rate constant, $K_{on}$ is the association rate constant and $K_d$ is the equilibrium constant). Affinity can be determined at equilibrium by measuring the fraction bound (r) of labeled ligand at various concentrations (c). The data are graphed using the Scatchard equation: r/c = K(n-r): where r = moles of bound ligand/mole of receptor at equilibrium; c = free ligand concentration at equilibrium; K = equilibrium association constant; and n = number of ligand binding sites per receptor molecule. By graphical analysis, r/c is plotted on the Y-axis versus r on the X-axis, thus producing a Scatchard plot. Antibody affinity measurement by Scatchard analysis is well known in the art. *See, e.g.,* van Erp et al., J. Immunoassay 12: 425-43, 1991; Nelson and Griswold, Comput. Methods Programs Biomed. 27: 65-8, 1988.

The term "epitope" refers to an antigenic determinant capable of specific binding to an antibody. Epitopes usually consist of chemically active surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Numerous publications discuss the use of phage display technology to produce and screen libraries of polypeptides for binding to a selected analyte. *See, e.g,* Cwirla et al., Proc. Natl. Acad. Sci. USA 87, 6378-82, 1990; Devlin et al., Science 249, 404-6, 1990, Scott and Smith, Science 249, 386-88, 1990; and Ladner et al., U.S. Pat. No. 5,571,698. A basic concept of phage display methods is the establishment of a physical association between DNA encoding a polypeptide to be screened and the polypeptide. This physical association is provided by the phage particle, which displays a polypeptide as part of a capsid enclosing the phage genome which encodes the polypeptide. The establishment of a physical association between polypeptides and their genetic material allows simultaneous mass screening of very large numbers of phage bearing different polypeptides. Phage displaying a polypeptide with affinity to a target bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means. *See, e.g.,* U.S. Patent No. 6,057,098, which is hereby incorporated in its entirety, including all tables, figures, and claims.

The antibodies that are generated by these methods may then be selected by first screening for affinity and specificity with the purified polypeptide of interest and, if required, comparing the results to the affinity and specificity of the antibodies with polypeptides that are desired to be excluded from binding. The screening procedure can involve immobilization of the purified polypeptides in separate wells of microtiter plates. The solution containing a potential antibody or groups of antibodies is then placed into the respective microtiter wells and incubated for about 30 min to 2 h. The microtiter wells are then washed and a labeled secondary antibody (for example, an anti-mouse antibody conjugated to alkaline phosphatase if the raised antibodies are mouse antibodies) is added to the wells and incubated for about 30 min and then washed. Substrate is added to the wells and a color reaction will appear where antibody to the immobilized polypeptide(s) are present.

The antibodies so identified may then be further analyzed for affinity and specificity in the assay design selected. In the development of immunoassays for a target protein, the purified target protein acts as a standard with which to judge the sensitivity and specificity of the immunoassay using the antibodies that have been selected. Because the binding affinity of various antibodies may differ; certain antibody pairs (e.g., in sandwich assays) may interfere with one another sterically, etc., assay performance of an antibody may be a more important measure than absolute affinity and specificity of an antibody.

While the present application describes antibody-based binding assays in detail, alternatives to antibodies as binding species in assays are well known in the art. These include receptors for a particular target, aptamers, etc. Aptamers are oligonucleic acid or peptide molecules that bind to a specific target molecule. Aptamers are usually created by selecting them from a large random sequence pool, but natural aptamers also exist. High-affinity aptamers containing modified nucleotides conferring improved characteristics on the ligand, such as improved in vivo stability or improved delivery characteristics. Examples of such modifications include chemical substitutions at the ribose and/or phosphate and/or base positions, and may include amino acid side chain functionalities.

Assay Correlations

The term "correlating" as used herein in reference to the use of biomarkers refers to comparing the presence or amount of the biomarker(s) in a patient to its presence or amount in persons known to suffer from, or known to be at risk of, a given condition; or in persons known to be free of a given condition. Often, this takes the form of comparing an assay result in the form of a biomarker concentration to a predetermined threshold selected to be indicative of the occurrence or nonoccurrence of a disease or the likelihood of some future outcome.

Selecting a diagnostic threshold involves, among other things, consideration of the probability of disease, distribution of true and false diagnoses at different test thresholds, and estimates of the consequences of treatment (or a failure to treat) based on the diagnosis. For example, when considering administering a specific therapy which is highly efficacious and has a low level of risk, few tests are needed because clinicians can accept substantial diagnostic uncertainty. On the other hand, in situations where treatment options are less effective and more risky, clinicians often need a higher degree of diagnostic certainty. Thus, cost/benefit analysis is involved in selecting a diagnostic threshold.

Suitable thresholds may be determined in a variety of ways. For example, one recommended diagnostic threshold for the diagnosis of acute myocardial infarction using cardiac troponin is the 97.5th percentile of the concentration seen in a normal population. Another method may be to look at serial samples from the same patient, where a prior "baseline" result is used to monitor for temporal changes in a biomarker level.

Population studies may also be used to select a decision threshold. Reciever Operating Characteristic ("ROC") arose from the field of signal dectection theroy developed during World War II for the analysis of radar images, and ROC analysis is often used to select a threshold able to best distinguish a "diseased" subpopulation from a "nondiseased" subpopulation. A false positive in this case occurs when the person tests positive, but actually does not have the disease. A false negative, on the other hand, occurs when the person tests negative, suggesting they are healthy, when they actually do have the disease. To draw a ROC curve, the true positive rate (TPR) and false positive rate (FPR) are determined as the decision threshold is varied continuously. Since TPR is equivalent with sensitivity and FPR is equal to 1 - specificity, the ROC graph is sometimes called the sensitivity vs (1 - specificity) plot. A perfect test will have an area under the ROC curve of 1.0; a random test will have an area of 0.5. A threshold is selected to provide an acceptable level of specificity and sensitivity.

In this context, "diseased" is meant to refer to a population having one characteristic (the presence of a disease or condition or the occurrence of some outcome) and "nondiseased" is meant to refer to a population lacking the characteristic. While a single decision threshold is the simplest application of such a method, multiple decision thresholds may be used. For example, below a first threshold, the absence of disease may be assigned with relatively high confidence, and above a second threshold the presence of disease may also be assigned with relatively high confidence. Between the two thresholds may be considered indeterminate. This is meant to be exemplary in nature only.

In addition to threshold comparisons, other methods for correlating assay results to a patient classification (occurrence or nonoccurrence of disease, likelihood of an outcome, etc.) include decision trees, rule sets, Bayesian methods, and neural network methods. These methods can produce probability values representing the degree to which a subject belongs to one classification out of a plurality of classifications.

Measures of test accuracy may be obtained as described in Fischer et al., Intensive Care Med. 29: 1043-51, 2003, and used to determine the effectiveness of a given biomarker. These measures include sensitivity and specificity, predictive values, likelihood ratios, diagnostic odds ratios, and ROC curve areas. The area under the curve ("AUC") of a ROC plot is equal to the probability that a classifier will rank a randomly chosen positive instance higher than a randomly chosen negative one. The area under the ROC curve may be thought of as equivalent to the Mann-Whitney U test, which tests for the median difference between scores obtained in the two groups considered if the groups are of continuous data, or to the Wilcoxon test of ranks.

As discussed above, suitable tests may exhibit one or more of the following results on these various measures: a specificity of greater than 0.5, preferably at least 0.6, more preferably at least 0.7, still more preferably at least 0.8, even more preferably at least 0.9 and most preferably at least 0.95, with a corresponding sensitivity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; a sensitivity of greater than 0.5, preferably at least 0.6, more preferably at least 0.7, still more preferably at least 0.8, even more preferably at least 0.9 and most preferably at least 0.95, with a corresponding specificity greater than 0.2, preferably greater than 0.3, more preferably greater than 0.4, still more preferably at least 0.5, even more preferably 0.6, yet more preferably greater than 0.7, still more preferably greater than 0.8, more preferably greater than 0.9, and most preferably greater than 0.95; at least 75% sensitivity, combined with at least 75% specificity; a ROC curve area of greater than 0.5, preferably at least 0.6, more preferably 0.7, still more preferably at least 0.8, even more preferably at least 0.9, and most preferably at least 0.95; an odds ratio different from 1, preferably at least about 2 or more or about 0.5 or less, more preferably at least about 3 or more or about 0.33 or less, still more preferably at least about 4 or more or about 0.25 or less, even more preferably at least about 5 or more or about 0.2 or less, and most preferably at least about 10 or more or about 0.1 or less; a positive likelihood ratio (calculated as sensitivity/(l-specificity)) of greater than 1, at least 2, more preferably at least 3, still more preferably at least 5, and most preferably at least 10; and or a negative likelihood ratio (calculated as (1-sensitivity)/specificity) of less than 1, less than or equal to 0.5, more preferably less than or equal to 0.3, and most preferably less than or equal to 0.1

Additional clinical indicia may be combined with the kidney injury marker assay result(s) of the present invention. These include other biomarkers related to renal status. Examples include the following, which recite the common biomarker name, followed by the Swiss-Prot entry number for that biomarker or its parent: Actin (P68133); Adenosine deaminase binding protein (DPP4, P27487); Alpha-1-acid glycoprotein 1 (P02763); Alpha-1-microglobulin (P02760); Albumin (P02768); Angiotensinogenase (Renin, P00797); Annexin A2 (P07355); Beta-glucuronidase (P08236); B-2-microglobulin (P61679); Beta-galactosidase (P16278); BMP-7 (P18075); Brain natriuretic peptide (proBNP, BNP-32, NTproBNP; P16860); Calcium-binding protein Beta (S100-beta, P04271); Carbonic anhydrase (Q16790); Casein Kinase 2 (P68400); Ceruloplasmin (P00450); Clusterin (P10909); Complement C3 (P01024); Cysteine-rich protein (CYR61, 000622); Cytochrome C (P99999); Epidermal growth factor (EGF, P01133); Endothelin-1 (P05305); Exosomal Fetuin-A (P02765); Fatty acid-binding protein, heart (FABP3, P05413); Fatty acid-binding protein, liver (P07148); Ferritin (light chain, P02793; heavy chain P02794); Fructose-1,6-biphosphatase (P09467); GRO-alpha (CXCL1, (P09341); Growth Hormone (P01241); Hepatocyte growth factor (P14210); Insulin-like growth factor I (P01343); Immunoglobulin G; Immunoglobulin Light Chains (Kappa and Lambda); Interferon gamma (P01308); Lysozyme (P61626); Interleukin-1alpha (P01583); Interleukin-2 (P60568); Interleukin-4 (P60568); Interleukin-9 (P15248); Interleukin-12p40 (P29460); Interleukin-13 (P35225); Interleukin-16 (Q14005); L1 cell adhesion molecule (P32004); Lactate dehydrogenase (P00338); Leucine Aminopeptidase (P28838); Meprin A-alpha subunit (Q16819); Meprin A-beta subunit (Q16820); Midkine (P21741); MIP2-alpha (CXCL2, P19875); MMP-2 (P08253); MMP-9 (P14780); Netrin-1 (095631); Neutral endopeptidase (P08473); Osteopontin (P10451); Renal papillary antigen 1 (RPA1); Renal papillary antigen 2 (RPA2); Retinol binding protein (P09455); Ribonuclease; S100 calcium-binding protein A6 (P06703); Serum Amyloid P Component (P02743); Sodium/Hydrogen exchanger isoform (NHE3, P48764); Spermidine/spermine N1-acetyltransferase (P21673); TGF-Beta1 (P01137); Transferrin (P02787); Trefoil factor 3 (TFF3, Q07654); Toll-Like protein 4 (000206); Total protein; Tubulointerstitial nephritis antigen (Q9UJW2); Uromodulin (Tamm-Horsfallprotein, P07911).

For purposes of risk stratification, Adiponectin (Q15848); Alkaline phosphatase (P05186); Aminopeptidase N (P15144); CalbindinD28k (P05937); Cystatin C (P01034); 8 subunit of FIFO ATPase (P03928); Gamma-glutamyltransferase (P19440); GSTa (alpha-glutathione-S-transferase, P08263); GSTpi (Glutathione-S-transferase P; GST class-pi; P09211); IGFBP-1 (P08833); IGFBP-2 (P18065); IGFBP-6 (P24592); Integral membrane protein 1 (Itm1, P46977); Interleukin-6 (P05231); Interleukin-8 (P10145); Interleukin-18 (Q14116); IP-10 (10 kDa interferon-gamma-induced protein, P02778); IRPR (IFRD1, 000458); Isovaleryl-CoA dehydrogenase (IVD, P26440); I-TAC/CXCL11 (014625); Keratin 19 (P08727); Kim-1 (Hepatitis A virus cellular receptor 1, 043656); L-arginine:glycine amidinotransferase (P50440); Leptin (P41159); Lipocalin2 (NGAL, P80188); MCP-1 (P13500); MIG (Gamma-interferon-induced monokine Q07325); MIP-1a (P10147); MIP-3a (P78556); MIP-1beta (P13236); MIP-1d (Q16663); NAG (N-acetyl-beta-D-glucosaminidase, P54802); Organic ion transporter (OCT2, 015244); Osteoprotegerin (014788); P8 protein (060356); Plasminogen activator inhibitor 1 (PAI-1, P05121); ProANP(1-98) (P01160); Protein phosphatase 1-beta (PPI-beta, P62140); Rab GDI-beta (P50395); Renal kallikrein (Q86U61); RT1.B-1 (alpha) chain of the integral membrane protein (Q5Y7A8); Soluble tumor necrosis factor receptor superfamily member 1A (sTNFR-I, P19438); Soluble tumor necrosis factor receptor superfamily member 1B (sTNFR-II, P20333); Tissue inhibitor of metalloproteinases 3 (TIMP-3, P35625); uPAR (Q03405) may be combined with the kidney injury marker assay result(s) of the present invention.

Other clinical indicia which may be combined with the kidney injury marker assay result(s) of the present invention includes demographic information (e.g., weight, sex, age, race), medical history (e.g., family history, type of surgery, pre-existing disease such as aneurism, congestive heart failure, preeclampsia, eclampsia, diabetes mellitus, hypertension, coronary artery disease, proteinuria, renal insufficiency, or sepsis, type of toxin exposure such as NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin), clinical variables (e.g., blood pressure, temperature, respiration rate), risk scores (APACHE score, PREDICT score, TIMI Risk Score for UA/NSTEMI, Framingham Risk Score), a urine total protein measurement, a glomerular filtration rate, an estimated glomerular filtration rate, a urine production rate, a serum or plasma creatinine concentration, a renal papillary antigen 1 (RPA1) measurement; a renal papillary antigen 2 (RPA2) measurement; a urine creatinine concentration, a fractional excretion of sodium, a urine sodium concentration, a urine creatinine to serum or plasma creatinine ratio, a urine specific gravity, a urine osmolality, a urine urea nitrogen to plasma urea nitrogen ratio, a plasma BUN to creatnine ratio, and/or a renal failure index calculated as urine sodium / (urine creatinine / plasma creatinine). Other measures of renal function which may be combined with the kidney injury marker assay result(s) are described hereinafter and in Harrison's Principles of Internal Medicine, 17th Ed., McGraw Hill, New York, pages 1741-1830, and Current Medical Diagnosis & Treatment 2008, 47th Ed, McGraw Hill, New York, pages 785-815, each of which are hereby incorporated by reference in their entirety.

Combining assay results/clinical indicia in this manner can comprise the use of multivariate logistical regression, loglinear modeling, neural network analysis, n-of-m analysis, decision tree analysis, etc. This list is not meant to be limiting.

Diagnosis of Acute Renal Failure

As noted above, the terms "acute renal (or kidney) injury" and "acute renal (or kidney) failure" as used herein are defined in part in terms of changes in serum creatinine from a baseline value. Most definitions of ARF have common elements, including the use of serum creatinine and, often, urine output. Patients may present with renal dysfunction without an available baseline measure of renal function for use in this comparison. In such an event, one may estimate a baseline serum creatinine value by assuming the patient initially had a normal GFR. Glomerular filtration rate (GFR) is the volume of fluid filtered from the renal (kidney) glomerular capillaries into the Bowman's capsule per unit time. Glomerular filtration rate (GFR) can be calculated by measuring any chemical that has a steady level in the blood, and is freely filtered but neither reabsorbed nor secreted by the kidneys. GFR is typically expressed in units of ml/min:

$$GFR = \frac{\text{Urine Concentration} \times \text{Urine Flow}}{\text{Plasma Concentration}}$$

By normalizing the GFR to the body surface area, a GFR of approximately 75-100 ml/min per 1.73 m$^2$ can be assumed. The rate therefore measured is the quantity of the substance in the urine that originated from a calculable volume of blood.

There are several different techniques used to calculate or estimate the glomerular filtration rate (GFR or eGFR). In clinical practice, however, creatinine clearance is used to measure GFR. Creatinine is produced naturally by the body (creatinine is a metabolite of creatine, which is found in muscle). It is freely filtered by the glomerulus, but also actively secreted by the renal tubules in very small amounts such that creatinine clearance overestimates actual GFR by 10-20%. This margin of error is acceptable considering the ease with which creatinine clearance is measured.

Creatinine clearance (CCr) can be calculated if values for creatinine's urine concentration (U$_{Cr}$), urine flow rate (V), and creatinine's plasma concentration (P$_{Cr}$) are known. Since the product of urine concentration and urine flow rate yields creatinine's excretion rate, creatinine clearance is also said to be its excretion rate (U$_{Cr}$×V) divided by its plasma concentration. This is commonly represented mathematically as:

$$C_{Cr} = \frac{U_{Cr} \times V}{P_{Cr}}$$

Commonly a 24 hour urine collection is undertaken, from empty-bladder one morning to the contents of the bladder the following morning, with a comparative blood test then taken:

$$C_{Cr} = \frac{U_{Cr} \times \text{24-hour volume}}{P_{Cr} \times 24 \times 60 mins}$$

To allow comparison of results between people of different sizes, the CCr is often corrected for the body surface area (BSA) and expressed compared to the average sized man as ml/min/1.73 m2. While most adults have a BSA that approaches 1.7 (1.6-1.9), extremely obese or slim patients should have their CCr corrected for their actual BSA:

$$C_{Cr-corrected} = \frac{C_{Cr} \times 1.73}{BSA}$$

The accuracy of a creatinine clearance measurement (even when collection is complete) is limited because as glomerular filtration rate (GFR) falls creatinine secretion is increased, and thus the rise in serum creatinine is less. Thus, creatinine excretion is much greater than the filtered load, resulting in a potentially large overestimation of the GFR (as much as a twofold difference). However, for clinical purposes it is important to determine whether renal function is stable or getting worse or better. This is often determined by monitoring serum creatinine alone. Like creatinine clearance, the serum creatinine will not be an accurate reflection of GFR in the non-steady-state condition of ARF. Nonetheless, the degree to which serum creatinine changes from baseline will reflect the change in GFR. Serum creatinine is readily and easily measured and it is specific for renal function.

For purposes of determining urine output on a Urine output on a mL/kg/hr basis, hourly urine collection and measurement is adequate. In the case where, for example, only a cumulative 24-h output was available and no patient weights are provided, minor modifications of the RIFLE urine output criteria have been described. For example, Bagshaw et al., Nephrol. Dial. Transplant. 23: 1203-1210, 2008, assumes an average patient weight of 70 kg, and patients are assigned a RIFLE classification based on the following: <35 mL/h (Risk), <21 mL/h (Injury) or <4 mL/h (Failure).

Selecting a Treatment Regimen

Once a diagnosis is obtained, the clinician can readily select a treatment regimen that is compatible with the diagnosis, such as initiating renal replacement therapy, withdrawing delivery of compounds that are known to be damaging to the kidney, kidney transplantation, delaying or avoiding procedures that are known to be damaging to the kidney, modifying diuretic administration, initiating goal directed therapy, etc. The skilled artisan is aware of appropriate treatments for numerous diseases discussed in relation to the methods of diagnosis described herein. See, e.g., Merck Manual of Diagnosis and Therapy, 17th Ed. Merck Research Laboratories, Whitehouse Station, NJ, 1999. In addition, since the methods and compositions described herein provide prognostic information, the markers of the present invention may be used to monitor a course of treatment. For example, improved or worsened prognostic state may indicate that a particular treatment is or is not efficacious.

One skilled in the art readily appreciates that the present invention is well adapted to carry out the objects and obtain the ends and advantages mentioned, as well as those inherent therein. The examples provided herein are representative of preferred embodiments, are exemplary, and are not intended as limitations on the scope of the invention.

Example 1: Contrast-induced nephropathy sample collection

The objective of this sample collection study is to collect samples of plasma and urine and clinical data from patients before and after receiving intravascular contrast media. Approximately 250 adults undergoing radiographic/angiographic procedures involving intravascular administration of iodinated contrast media are enrolled. To be enrolled in the study, each patient must meet all of the following inclusion criteria and none of the following exclusion criteria:

Inclusion Criteria

males and females 18 years of age or older;

undergoing a radiographic / angiographic procedure (such as a CT scan or coronary intervention) involving the intravascular administration of contrast media;

expected to be hospitalized for at least 48 hours after contrast administration.

able and willing to provide written informed consent for study participation and to comply with all study procedures.

Exclusion Criteria

renal transplant recipients;

acutely worsening renal function prior to the contrast procedure;

already receiving dialysis (either acute or chronic) or in imminent need of dialysis at enrollment;

expected to undergo a major surgical procedure (such as involving cardiopulmonary bypass) or an additional imaging procedure with contrast media with significant risk for further renal insult within the 48 hrs following contrast administration;

participation in an interventional clinical study with an experimental therapy within the previous 30 days;

known infection with human immunodeficiency virus (HIV) or a hepatitis virus.

Immediately prior to the first contrast administration (and after any pre-procedure hydration), an EDTA anti-coagulated blood sample (10 mL) and a urine sample (10 mL) are collected from each patient. Blood and urine samples are then collected at 4 ($\pm$0.5), 8 ($\pm$1), 24 ($\pm$2) 48 ($\pm$2), and 72 ($\pm$2) hrs following the last administration of contrast media during the index contrast procedure. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Serum creatinine is assessed at the site immediately prior to the first contrast administration (after any pre-procedure hydration) and at 4 ($\pm$0.5), 8 ($\pm$1), 24 ($\pm$2) and 48 ($\pm$2)), and 72 ($\pm$2) hours following the last administration of contrast (ideally at the same time as the study samples are obtained). In addition, each patient's status is evaluated through day 30 with regard to additional serum and urine creatinine measurements, a need for dialysis, hospitalization status, and adverse clinical outcomes (including mortality).

Prior to contrast administration, each patient is assigned a risk based on the following assessment: systolic blood pressure <80 mm Hg = 5 points; intra-arterial balloon pump = 5 points; congestive heart failure (Class III-IV or history of pulmonary edema) = 5 points; age >75 yrs = 4 points; hematocrit level <39% for men, <35% for women = 3 points; diabetes = 3 points; contrast media volume = 1 point for each 100 mL; serum creatinine level >1.5 g/dL = 4 points OR estimated GFR 40-60 mL/min/1.73 $m^2$ = 2 points, 20-40 mL/min/1.73 $m^2$ = 4 points, < 20 mL/min/1.73 $m^2$ = 6 points. The risks assigned are as follows: risk for CIN and dialysis: 5 or less total points = risk of CIN - 7.5%, risk of dialysis - 0.04%; 6-10 total points = risk of CIN - 14%, risk of dialysis - 0.12%; 11-16 total points = risk of CIN - 26.1%, risk of dialysis - 1.09%; >16 total points = risk of CIN - 57.3%, risk of dialysis - 12.8%.

Example 2: Cardiac surgery sample collection

The objective of this sample collection study is to collect samples of plasma and urine and clinical data from patients before and after undergoing cardiovascular surgery, a procedure known to be potentially damaging to kidney function. Approximately 900 adults undergoing such surgery are enrolled. To be enrolled in the study, each patient must meet all of the following inclusion criteria and none of the following exclusion criteria:

Inclusion Criteria

males and females 18 years of age or older;

undergoing cardiovascular surgery;

Toronto/Ottawa Predictive Risk Index for Renal Replacement risk score of at least 2 (Wijeysundera et al., JAMA 297: 1801-9, 2007); and

able and willing to provide written informed consent for study participation and to comply with all study procedures.

Exclusion Criteria

known pregnancy;

previous renal transplantation;

acutely worsening renal function prior to enrollment (e.g., any category of RIFLE criteria);

already receiving dialysis (either acute or chronic) or in imminent need of dialysis at enrollment;

currently enrolled in another clinical study or expected to be enrolled in another clinical study within 7 days of cardiac surgery that involves drug infusion or a therapeutic intervention for AKI;

known infection with human immunodeficiency virus (HIV) or a hepatitis virus.

Within 3 hours prior to the first incision (and after any pre-procedure hydration), an EDTA anti-coagulated blood sample (10 mL), whole blood (3 mL), and a urine sample (35 mL) are collected from each patient. Blood and urine samples are then collected at 3 ($\pm$0.5), 6 ($\pm$0.5), 12 ($\pm$1), 24 ($\pm$2) and 48 ($\pm$2) hrs following the procedure and then daily on days 3 through 7 if the subject remains in the hospital. Blood is collected via direct venipuncture or via other available venous access, such as an existing femoral sheath, central venous line, peripheral intravenous line or hep-lock. These study blood samples are frozen and shipped to Astute Medical, Inc., San Diego, CA. The study urine samples are frozen and shipped to Astute Medical, Inc.

Example 3: Acutely ill subject sample collection

The objective of this study is to collect samples from acutely ill patients. Approximately 1900 adults expected to be in the ICU for at least 48 hours will be enrolled. To be enrolled in the study, each patient must meet all of the following inclusion criteria and none of the following exclusion criteria:

Inclusion Criteria

males and females 18 years of age or older;

Study population 1: approximately 300 patients that have at least one of: shock (SBP < 90 mmHg and/or need for vasopressor support to maintain MAP > 60 mmHg and/or documented drop in SBP of at least 40 mmHg); and

sepsis;

Study population 2: approximately 300 patients that have at least one of: IV antibiotics ordered in computerized physician order entry (CPOE) within 24 hours of enrollment;

contrast media exposure within 24 hours of enrollment;

increased Intra-Abdominal Pressure with acute decompensated heart failure; and severe trauma as the primary reason for ICU admission and likely to be hospitalized in the ICU for 48 hours after enrollment;

Study population 3: approximately 300 patients expected to be hospitalized through acute care setting (ICU or ED) with a known risk factor for acute renal injury (e.g. sepsis, hypotension/shock (Shock = systolic BP < 90 mmHg and/or the need for vasopressor support to maintain a MAP > 60 mmHg and/or a documented drop in SBP > 40 mmHg), major trauma, hemorrhage, or major surgery); and/or expected to be hospitalized to the ICU for at least 24 hours after enrollment;

Study population 4: approximately 1000 patients that are 21 years of age or older, within 24 hours of being admitted into the ICU, expected to have an indwelling urinary catheter for at least 48 hours after enrollment, and have at least one of the following acute conditions within 24 hours prior to enrollment:

(i) respiratory SOFA score of $\geq 2$ (PaO2/FiO2 <300), (ii) cardiovascular SOFA score of $\geq 1$ (MAP < 70 mm Hg and/or any vasopressor required).

Exclusion Criteria

known pregnancy;

institutionalized individuals;

previous renal transplantation;

known acutely worsening renal function prior to enrollment (e.g., any category of RIFLE criteria);

received dialysis (either acute or chronic) within 5 days prior to enrollment or in imminent need of dialysis at the time of enrollment;

known infection with human immunodeficiency virus (HIV) or a hepatitis virus;

meets any of the following:

(i) active bleeding with an anticipated need for > 4 units PRBC in a day;

(ii) hemoglobin < 7 g/dL;

(iii) any other condition that in the physician's opinion would contraindicate drawing serial blood samples for clinical study purposes;

meets only the SBP < 90 mmHg inclusion criterion set forth above, and does not have shock in the attending physician's or principal investigator's opinion;

After obtaining informed consent, an EDTA anti-coagulated blood sample (10 mL) and a urine sample (25-50 mL) are collected from each patient. Blood and urine samples are then collected at 4 ($\pm$ 0.5) and 8 ($\pm$ 1) hours after contrast administration (if applicable); at 12 ($\pm$ 1), 24 ($\pm$ 2), 36 ($\pm$ 2), 48 ($\pm$ 2), 60 ($\pm$ 2), 72 ($\pm$ 2), and 84 ($\pm$ 2) hours after enrollment, and thereafter daily up to day 7 to day 14 while the subject is hospitalized. Blood is collected via direct venipuncture or via other available venous access, such as an existing femoral sheath, central venous line, peripheral intravenous line or hep-lock. These study blood samples are processed to plasma at the clinical site, frozen and shipped to Astute Medical, Inc., San Diego, CA. The study urine samples are frozen and shipped to Astute Medical, Inc.

Example 4. Immunoassay format

Analytes are measured using standard sandwich enzyme immunoassay techniques. A first antibody which binds the analyte is immobilized in wells of a 96 well polystyrene microplate. Analyte standards and test samples are pipetted into the appropriate wells and any analyte present is bound by the immobilized antibody. After washing away any unbound substances, a horseradish peroxidase-conjugated second antibody which binds the analyte is added to the wells, thereby forming sandwich complexes with the analyte (if present) and the first antibody. Following a wash to remove any unbound antibody-enzyme reagent, a substrate solution comprising tetramethylbenzidine and hydrogen peroxide is added to the wells. Color develops in proportion to the amount of analyte present in the sample. The color development is stopped and the intensity of the color is measured at 540 nm or 570 nm. An analyte concentration is assigned to the test sample by comparison to a standard curve determined from the analyte standards.

Units for the concentrations reported in the following data tables are as follows: Stanninocalcin-1 - ng/mL, Antithrombin-III - ng/mL, Toll-like receptor 2 - ng/mL, Triiodothyronine (T3) - ng/mL, Thyroxine (T4) - ng/mL, Extracellular matrix protein 1 - ng/mL, Coagulation factor XIII A chain/Coagulation factor XIII B chain - ng/mL (assay detects both chains), Interleukin-17F - ng/mL, Interleukin-22 - ng/mL, Vitronectin - ng/mL, Progesterone - ng/mL, Estradiol - ng/mL, Growth/differentiation factor 15 - pg/mL, Proprotein convertase subtilisin/kexin type 9.

In the case of those kidney injury markers which are membrane proteins as described herein, the assays used in these examples detect soluble forms thereof.

Example 5. Apparently Healthy Donor and Chronic Disease Patient Samples

Human urine samples from donors with no known chronic or acute disease ("Apparently Healthy Donors") were purchased from two vendors (Golden West Biologicals, Inc., 27625 Commerce Center Dr., Temecula, CA 92590 and Virginia Medical Research, Inc., 915 First Colonial Rd., Virginia Beach, VA 23454). The urine samples were shipped and stored frozen at less than -20° C. The vendors supplied demographic information for the individual donors including gender, race (Black /White), smoking status and age.

Human urine samples from donors with various chronic diseases ("Chronic Disease Patients") including congestive heart failure, coronary artery disease, chronic kidney disease, chronic obstructive pulmonary disease, diabetes mellitus and hypertension were purchased from Virginia Medical Research, Inc., 915 First Colonial Rd., Virginia Beach, VA 23454. The urine samples were shipped and stored frozen at less than -20 degrees centigrade. The vendor provided a case report form for each individual donor with age, gender, race (Black/White), smoking status and alcohol use, height, weight, chronic disease(s) diagnosis, current medications and previous surgeries.

Example 6. Use of Kidney Injury Markers for evaluating renal status in patients

Patients from the intensive care unit (ICU) were enrolled in the following study. Each patient was classified by kidney status as non-injury (0), risk of injury (R), injury (I), and failure (F) according to the maximum stage reached within 7 days of enrollment as determined by the RIFLE criteria. EDTA anti-coagulated blood samples (10 mL) and a urine samples (25-30 mL) were collected from each patient at enrollment, 4 ($\pm$ 0.5) and 8 ($\pm$ 1) hours after contrast administration (if applicable); at 12 ($\pm$ 1), 24 ($\pm$ 2), and 48 ($\pm$ 2) hours after enrollment, and thereafter daily up to day 7 to day 14 while the subject is hospitalized. Markers were each measured by standard immunoassay methods using commercially available assay reagents in the urine samples and the plasma component of the blood samples collected.

Two cohorts were defined to represent a "diseased" and a "normal" population. While these terms are used for convenience, "diseased" and "normal" simply represent two cohorts for comparison (say RIFLE 0 vs RIFLE R, I and F; RIFLE 0 vs RIFLE R; RIFLE 0 and R vs RIFLE I and F; etc.). The time "prior max stage" represents the time at which a sample is collected, relative to the time a particular patient reaches the lowest disease stage as defined for that cohort, binned into three groups which are +/- 12 hours. For example, "24 hr prior" which uses 0 vs R, I, F as the two cohorts would mean 24 hr (+/- 12 hours) prior to reaching stage R (or I if no sample at R, or F if no sample at R or I).

A receiver operating characteristic (ROC) curve was generated for each biomarker measured and the area under each ROC curve (AUC) is determined. Patients in Cohort 2 were also separated according to the reason for adjudication to cohort 2 as being based on serum creatinine measurements (sCr), being based on urine output (UO), or being based on either serum creatinine measurements or urine output. Using the same example discussed above (0 vs R, I, F), for those patients adjudicated to stage R, I, or F on the basis of serum creatinine measurements alone, the stage 0 cohort may include patients adjudicated to stage R, I, or F on the basis of urine output; for those patients adjudicated to stage R, I, or F on the basis of urine output alone, the stage 0 cohort may include patients adjudicated to stage R, I, or F on the basis of serum creatinine measurements; and for those patients adjudicated to stage R, I, or F on the basis of serum creatinine measurements or urine output, the stage 0 cohort contains only patients in stage 0 for both serum creatinine measurements and urine output. Also, in the data for patients adjudicated on the basis of serum creatinine measurements or urine output, the adjudication method which yielded the most severe RIFLE stage is used.

The ability to distinguish cohort 1 from Cohort 2 was determined using ROC analysis. SE is the standard error of the AUC, n is the number of sample or individual patients ("pts," as indicated). Standard errors are calculated as described in Hanley, J. A., and McNeil, B.J., The meaning and use of the area under a receiver operating characteristic (ROC) curve. Radiology (1982) 143: 29-36; p values are calculated with a two-tailed Z-test. An AUC < 0.5 is indicative of a negative going marker for the comparison, and an AUC > 0.5 is indicative of a positive going marker for the comparison.

Various threshold (or "cutoff") concentrations were selected, and the associated sensitivity and specificity for distinguishing cohort 1 from cohort 2 are determined. OR is the odds ratio calculated for the particular cutoff concentration, and 95% CI is the confidence interval for the odds ratio.

Table 1: Comparison of marker levels in urine samples collected from Cohort 1 (patients that did not progress beyond RIFLE stage 0) and in urine samples collected from subjects at 0, 24 hours, and 48 hours prior to reaching stage R, I or F in Cohort 2.

**Stanniocalcin-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0962 | 0.179 | 0.0962 | 0.131 | 0.0962 | 0.218 |
| Average | 0.385 | 0.522 | 0.385 | 0.267 | 0.385 | 0.297 |
| Stdev | 0.978 | 1.26 | 0.978 | 0.334 | 0.978 | 0.241 |
| p(t-test) | | 0.62 | | 0.60 | | 0.84 |
| Min | 0.00189 | 0.0152 | 0.00189 | 0.0176 | 0.00189 | 0.0874 |
| Max | 6.00 | 6.00 | 6.00 | 1.25 | 6.00 | 0.682 |
| n (Samp) | 47 | 22 | 47 | 20 | 47 | 5 |
| n (Patient) | 22 | 22 | 22 | 20 | 22 | 5 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.120 | 0.132 | 0.120 | 0.106 | 0.120 | 0.160 |
| Average | 0.388 | 0.158 | 0.388 | 0.169 | 0.388 | 0.169 |
| Stdev | 0.914 | 0.109 | 0.914 | 0.140 | 0.914 | 0.133 |
| p(t-test) | | 0.58 | | 0.50 | | 0.60 |
| Min | 0.00189 | 0.0152 | 0.00189 | 0.0295 | 0.00189 | 0.0371 |
| Max | 6.00 | 0.299 | 6.00 | 0.410 | 6.00 | 0.367 |
| n (Samp) | 98 | 5 | 98 | 8 | 98 | 5 |
| n (Patient) | 47 | 5 | 47 | 8 | 47 | 5 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0894 | 0.189 | 0.0894 | 0.131 | 0.0894 | 0.156 |
| Average | 0.364 | 0.575 | 0.364 | 0.249 | 0.364 | 0.196 |
| Stdev | 0.976 | 1.32 | 0.976 | 0.333 | 0.976 | 0.213 |
| p(t-test) | | 0.47 | | 0.61 | | 0.63 |
| Min | 0.00189 | 0.0289 | 0.00189 | 0.0176 | 0.00189 | 0.0181 |
| Max | 6.00 | 6.00 | 6.00 | 1.25 | 6.00 | 0.682 |
| n (Samp) | 47 | 20 | 47 | 20 | 47 | 8 |
| n (Patient) | 22 | 20 | 22 | 20 | 22 | 8 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.63 | 0.51 | 0.69 | 0.56 | 0.49 | 0.58 | 0.71 | 0.50 | 0.57 |
| SE | 0.074 | 0.13 | 0.075 | 0.078 | 0.11 | 0.078 | 0.14 | 0.13 | 0.11 |
| p | 0.091 | 0.93 | 0.013 | 0.41 | 0.91 | 0.29 | 0.13 | 0.99 | 0.56 |
| nCohort 1 | 47 | 98 | 47 | 47 | 98 | 47 | 47 | 98 | 47 |
| nCohort 2 | 22 | 5 | 20 | 20 | 8 | 20 | 5 | 5 | 8 |
| Cutoff 1 | 0.117 | 0.117 | 0.119 | 0.0690 | 0.0690 | 0.0690 | 0.122 | 0.0592 | 0.0760 |
| Sens 1 | 73% | 80% | 70% | 70% | 75% | 70% | 80% | 80% | 75% |
| Spec 1 | 60% | 49% | 66% | 43% | 33% | 47% | 64% | 29% | 49% |
| Cutoff 2 | 0.0760 | 0.117 | 0.0895 | 0.0515 | 0.0481 | 0.0515 | 0.122 | 0.0592 | 0.0191 |
| Sens 2 | 82% | 80% | 80% | 80% | 88% | 80% | 80% | 80% | 88% |
| Spec 2 | 45% | 49% | 53% | 32% | 23% | 36% | 64% | 29% | 11% |
| Cutoff 3 | 0.0296 | 0.0132 | 0.0592 | 0.0289 | 0.0289 | 0.0296 | 0.0760 | 0.0357 | 0.0152 |
| Sens 3 | 91% | 100% | 90% | 90% | 100% | 90% | 100% | 100% | 100% |
| Spec 3 | 17% | 3% | 43% | 15% | 13% | 23% | 45% | 15% | 9% |
| Cutoff 4 | 0.179 | 0.221 | 0.146 | 0.179 | 0.221 | 0.146 | 0.179 | 0.221 | 0.146 |
| Sens 4 | 50% | 40% | 60% | 40% | 38% | 45% | 60% | 20% | 50% |
| Spec 4 | 70% | 70% | 70% | 70% | 70% | 70% | 70% | 70% | 70% |
| Cutoff 5 | 0.292 | 0.495 | 0.307 | 0.292 | 0.495 | 0.307 | 0.292 | 0.495 | 0.307 |
| Sens 5 | 23% | 0% | 25% | 30% | 0% | 20% | 40% | 0% | 12% |
| Spec 5 | 81% | 81% | 81% | 81% | 81% | 81% | 81% | 81% | 81% |
| Cutoff 6 | 0.976 | 0.912 | 0.675 | 0.976 | 0.912 | 0.675 | 0.976 | 0.912 | 0.675 |
| Sens 6 | 14% | 0% | 15% | 5% | 0% | 15% | 0% | 0% | 12% |
| Spec 6 | 91% | 91% | 91% | 91% | 91% | 91% | 91% | 91% | 91% |
| OR Quart 2 | 1.0 | 0.96 | 1.5 | 2.4 | 1.0 | 1.8 | >1.1 | 0.96 | 0.42 |
| p Value | 1.0 | 0.98 | 0.68 | 0.29 | 0.97 | 0.48 | <0.96 | 0.98 | 0.51 |
| 95% CI of OR Quart2 | 0.17 | 0.057 | 0.22 | 0.48 | 0.14 | 0.35 | >0.061 | 0.057 | 0.034 |
| | 5.8 | 16 | 10 | 12 | 8.0 | 9.2 | na | 16 | 5.3 |
| OR Quart 3 | 6.7 | 2.0 | 7.9 | 1.8 | 1.0 | 3.0 | >2.4 | 2.0 | 1.5 |
| p Value | 0.018 | 0.58 | 0.022 | 0.48 | 1.0 | 0.17 | <0.51 | 0.58 | 0.69 |
| 95% CI of OR Quart3 | 1.4 | 0.17 | 1.4 | 0.35 | 0.13 | 0.62 | >0.19 | 0.17 | 0.21 |
| | 32 | 24 | 46 | 9.2 | 7.7 | 15 | na | 24 | 11 |
| OR Quart 4 | 2.3 | 0.96 | 3.8 | 2.4 | 1.0 | 1.8 | >2.4 | 0.96 | 0.92 |
| p Value | 0.30 | 0.98 | 0.14 | 0.29 | 0.97 | 0.48 | <0.51 | 0.98 | 0.94 |
| 95% CI of OR Quart4 | 0.48 | 0.057 | 0.64 | 0.48 | 0.14 | 0.35 | >0.19 | 0.057 | 0.11 |
| | 11 | 16 | 23 | 12 | 8.0 | 9.2 | na | 16 | 7.7 |

**Extracellular matrix protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.347 | 0.560 | 0.347 | 0.736 | 0.347 | 0.337 |
| Average | 0.701 | 2.60 | 0.701 | 1.10 | 0.701 | 0.686 |
| Stdev | 0.991 | 14.3 | 0.991 | 1.12 | 0.991 | 0.829 |
| p(t-test) | | 0.054 | | 0.0041 | | 0.96 |
| Min | 0.000528 | 0.00745 | 0.000528 | 0.000528 | 0.000528 | 0.0222 |
| Max | 7.81 | 150 | 7.81 | 4.80 | 7.81 | 2.46 |
| n (Samp) | 212 | 111 | 212 | 74 | 212 | 14 |
| n (Patient) | 106 | 111 | 106 | 74 | 106 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.416 | 0.703 | 0.416 | 0.871 | 0.416 | 0.668 |
| Average | 1.89 | 0.978 | 1.89 | 1.97 | 1.89 | 1.38 |
| Stdev | 11.4 | 0.892 | 11.4 | 2.84 | 11.4 | 1.65 |
| p(t-test) | | 0.66 | | 0.97 | | 0.87 |
| Min | 0.000528 | 0.0190 | 0.000528 | 0.0170 | 0.000528 | 0.0306 |
| Max | 150 | 3.08 | 150 | 10.9 | 150 | 6.00 |
| n (Samp) | 509 | 30 | 509 | 25 | 509 | 14 |
| n (Patient) | 217 | 30 | 217 | 25 | 217 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.362 | 0.569 | 0.362 | 0.780 | 0.362 | 0.378 |
| Average | 0.737 | 2.83 | 0.737 | 1.09 | 0.737 | 1.07 |
| Stdev | 0.988 | 15.1 | 0.988 | 1.01 | 0.988 | 1.71 |
| p(t-test) | | 0.026 | | 0.0075 | | 0.22 |
| Min | 0.000528 | 0.00745 | 0.000528 | 0.000528 | 0.000528 | 0.0222 |
| Max | 7.81 | 150 | 7.81 | 4.11 | 7.81 | 6.57 |
| n (Samp) | 259 | 99 | 259 | 76 | 259 | 16 |
| n (Patient) | 117 | 99 | 117 | 76 | 117 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.55 | 0.61 | 0.62 | 0.58 | 0.62 | 0.49 | 0.58 | 0.51 |
| SE | 0.034 | 0.056 | 0.034 | 0.039 | 0.061 | 0.038 | 0.080 | 0.081 | 0.075 |
| p | 8.2E-4 | 0.33 | 7.9E-4 | 0.0014 | 0.20 | 0.0012 | 0.91 | 0.30 | 0.86 |
| nCohort 1 | 212 | 509 | 259 | 212 | 509 | 259 | 212 | 509 | 259 |
| nCohort 2 | 111 | 30 | 99 | 74 | 25 | 76 | 14 | 14 | 16 |
| Cutoff 1 | 0.277 | 0.330 | 0.310 | 0.282 | 0.274 | 0.282 | 0.198 | 0.393 | 0.137 |
| Sens 1 | 70% | 70% | 71% | 70% | 72% | 71% | 71% | 71% | 75% |
| Spec 1 | 42% | 42% | 44% | 43% | 36% | 44% | 35% | 48% | 26% |
| Cutoff 2 | 0.198 | 0.195 | 0.241 | 0.172 | 0.172 | 0.182 | 0.0800 | 0.230 | 0.0928 |
| Sens 2 | 80% | 80% | 81% | 81% | 80% | 80% | 86% | 86% | 81% |
| Spec 2 | 35% | 28% | 38% | 31% | 24% | 32% | 15% | 30% | 18% |
| Cutoff 3 | 0.0928 | 0.0847 | 0.108 | 0.0553 | 0.0421 | 0.0702 | 0.0290 | 0.0413 | 0.0290 |
| Sens 3 | 90% | 90% | 91% | 91% | 92% | 91% | 93% | 93% | 94% |
| Spec 3 | 17% | 11% | 20% | 11% | 5% | 14% | 6% | 5% | 6% |
| Cutoff 4 | 0.747 | 1.01 | 0.860 | 0.747 | 1.01 | 0.860 | 0.747 | 1.01 | 0.860 |
| Sens 4 | 44% | 43% | 43% | 49% | 40% | 46% | 29% | 43% | 31% |
| Spec 4 | 70% | 70% | 70% | 70% | 70% | 70% | 70% | 70% | 70% |
| Cutoff 5 | 1.01 | 1.46 | 1.15 | 1.01 | 1.46 | 1.15 | 1.01 | 1.46 | 1.15 |
| Sens 5 | 39% | 27% | 37% | 39% | 28% | 38% | 29% | 36% | 25% |
| Spec 5 | 80% | 80% | 80% | 80% | 80% | 80% | 80% | 80% | 80% |
| Cutoff 6 | 2.04 | 2.35 | 2.11 | 2.04 | 2.35 | 2.11 | 2.04 | 2.35 | 2.11 |
| Sens 6 | 18% | 10% | 18% | 20% | 28% | 18% | 14% | 21% | 19% |
| Spec 6 | 90% | 90% | 90% | 90% | 90% | 90% | 90% | 90% | 90% |
| OR Quart 2 | 1.7 | 0.82 | 2.2 | 0.80 | 0.48 | 0.99 | 0.75 | 1.5 | 0.73 |
| p Value | 0.13 | 0.75 | 0.037 | 0.63 | 0.31 | 0.97 | 0.72 | 0.66 | 0.68 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart2 | 0.86 3.5 | 0.24 2.8 | 1.0 4.5 | 0.33 1.9 | 0.12 2.0 | 0.42 2.3 | 0.16 3.5 | 0.25 9.1 | 0.16 3.4 |
| OR Quart 3 | 1.5 | 1.5 | 1.8 | 1.9 | 1.2 | 2.2 | 0.74 | 2.0 | 0.98 |
| p Value | 0.30 | 0.44 | 0.14 | 0.12 | 0.78 | 0.043 | 0.70 | 0.42 | 0.98 |
| 95% CI of OR Quart3 | 0.71 2.9 | 0.53 4.4 | 0.83 3.7 | 0.85 4.1 | 0.38 3.6 | 1.0 4.9 | 0.16 3.4 | 0.36 11 | 0.24 4.1 |
| OR Quart 4 | 3.7 | 1.7 | 3.7 | 3.0 | 1.5 | 3.1 | 1.0 | 2.5 | 1.2 |
| p Value | 1.7E-4 | 0.31 | 2.6E-4 | 0.0047 | 0.44 | 0.0033 | 0.98 | 0.27 | 0.75 |
| 95% CI of OR Quart4 | 1.9 7.3 | 0.60 4.8 | 1.8 7.6 | 1.4 6.5 | 0.53 4.4 | 1.5 6.7 | 0.24 4.3 | 0.48 13 | 0.32 4.9 |

**Coagulation factor XIII A and chains**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.98 | 4.46 | 2.98 | 4.61 | 2.98 | 3.06 |
| Average | 6.77 | 8.82 | 6.77 | 8.20 | 6.77 | 6.87 |
| Stdev | 10.7 | 11.6 | 10.7 | 8.99 | 10.7 | 8.40 |
| p(t-test) | | 0.11 | | 0.30 | | 0.97 |
| Min | 0.000120 | 0.000327 | 0.000120 | 0.000303 | 0.000120 | 0.000324 |
| Max | 84.2 | 82.5 | 84.2 | 46.4 | 84.2 | 29.4 |
| n (Samp) | 212 | 111 | 212 | 74 | 212 | 14 |
| n (Patient) | 106 | 111 | 106 | 74 | 106 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.00 | 3.20 | 4.00 | 7.73 | 4.00 | 7.48 |
| Average | 7.90 | 5.79 | 7.90 | 10.5 | 7.90 | 8.65 |
| Stdev | 12.0 | 6.57 | 12.0 | 11.0 | 12.0 | 10.00 |
| p(t-test) | | 0.34 | | 0.29 | | 0.82 |
| Min | 0.000120 | 0.000327 | 0.000120 | 0.000458 | 0.000120 | 0.000180 |
| Max | 143 | 21.8 | 143 | 35.4 | 143 | 30.6 |
| n (Samp) | 509 | 30 | 509 | 25 | 509 | 14 |
| n (Patient) | 217 | 30 | 217 | 25 | 217 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.28 | 4.56 | 3.28 | 4.34 | 3.28 | 2.58 |
| Average | 6.66 | 8.95 | 6.66 | 7.55 | 6.66 | 6.87 |
| Stdev | 10.0 | 11.9 | 10.0 | 8.30 | 10.0 | 8.26 |
| p(t-test) | | 0.067 | | 0.48 | | 0.93 |
| Min | 0.000120 | 0.000443 | 0.000120 | 0.000303 | 0.000120 | 0.000324 |
| Max | 84.2 | 82.5 | 84.2 | 46.4 | 84.2 | 22.9 |
| n (Samp) | 259 | 99 | 259 | 76 | 259 | 16 |
| n (Patient) | 117 | 99 | 117 | 76 | 117 | 16 |

| | 0hr prior to AKI stage | 24hr prior to AKI stage | 48hr prior to AKI stage |
|---|---|---|---|

| | sCr or UO | sCr only | UO only | sCr or UO | sCr only | UO only | sCr or UO | sCr only | UO only |
|---|---|---|---|---|---|---|---|---|---|
| AUC | 0.57 | 0.46 | 0.56 | 0.58 | 0.57 | 0.57 | 0.54 | 0.53 | 0.51 |
| SE | 0.034 | 0.055 | 0.034 | 0.039 | 0.061 | 0.038 | 0.081 | 0.079 | 0.075 |
| p | 0.047 | 0.46 | 0.063 | 0.036 | 0.25 | 0.082 | 0.65 | 0.73 | 0.93 |
| nCohort 1 | 212 | 509 | 259 | 212 | 509 | 259 | 212 | 509 | 259 |
| nCohort 2 | 111 | 30 | 99 | 74 | 25 | 76 | 14 | 14 | 16 |
| Cutoff 1 | 1.68 | 1.79 | 1.67 | 2.47 | 1.99 | 2.54 | 1.62 | 1.79 | 1.58 |
| Sens 1 | 70% | 70% | 71% | 70% | 72% | 71% | 71% | 71% | 75% |
| Spec 1 | 35% | 30% | 34% | 43% | 32% | 44% | 34% | 30% | 32% |
| Cutoff 2 | 0.873 | 0.737 | 0.900 | 1.30 | 1.45 | 1.35 | 0.826 | 0.258 | 1.39 |
| Sens 2 | 80% | 80% | 81% | 81% | 80% | 80% | 86% | 86% | 81% |
| Spec 2 | 24% | 17% | 23% | 28% | 26% | 29% | 24% | 10% | 29% |
| Cutoff 3 | 0.521 | 0.493 | 0.529 | 0.0786 | 0.000620 | 0.493 | 0.435 | 0.000324 | 0.435 |
| Sens 3 | 90% | 90% | 91% | 91% | 92% | 91% | 93% | 93% | 94% |
| Spec 3 | 18% | 13% | 17% | 10% | 7% | 16% | 17% | 2% | 15% |
| Cutoff 4 | 6.63 | 7.46 | 7.10 | 6.63 | 7.46 | 7.10 | 6.63 | 7.46 | 7.10 |
| Sens 4 | 35% | 23% | 35% | 42% | 52% | 37% | 43% | 50% | 31% |
| Spec 4 | 70% | 70% | 70% | 70% | 70% | 70% | 70% | 70% | 70% |
| Cutoff 5 | 9.06 | 13.0 | 9.23 | 9.06 | 13.0 | 9.23 | 9.06 | 13.0 | 9.23 |
| Sens 5 | 31% | 17% | 31% | 36% | 32% | 29% | 21% | 21% | 31% |
| Spec 5 | 80% | 80% | 80% | 80% | 80% | 80% | 80% | 80% | 80% |
| Cutoff 6 | 18.0 | 19.9 | 17.9 | 18.0 | 19.9 | 17.9 | 18.0 | 19.9 | 17.9 |
| Sens 6 | 18% | 7% | 17% | 12% | 16% | 12% | 14% | 14% | 19% |
| Spec 6 | 90% | 90% | 90% | 90% | 90% | 90% | 90% | 90% | 90% |
| OR Quart 2 | 0.77 | 0.85 | 0.99 | 1.2 | 0.99 | 1.4 | 1.3 | 0.49 | 2.4 |
| p Value | 0.46 | 0.78 | 0.97 | 0.71 | 0.99 | 0.35 | 0.72 | 0.41 | 0.21 |
| 95% CI of OR Quart2 | 0.39 1.5 | 0.28 2.6 | 0.50 1.9 | 0.52 2.6 | 0.28 3.5 | 0.67 3.1 | 0.28 6.2 | 0.088 2.7 | 0.61 9.9 |
| OR Quart 3 | 1.2 | 1.3 | 1.1 | 1.3 | 0.59 | 1.4 | 1.0 | 0.99 | 0.32 |
| p Value | 0.66 | 0.61 | 0.73 | 0.54 | 0.48 | 0.35 | 1.0 | 0.99 | 0.33 |
| 95% CI of OR Quart3 | 0.60 2.2 | 0.47 3.6 | 0.57 2.2 | 0.58 2.9 | 0.14 2.5 | 0.67 3.1 | 0.19 5.2 | 0.24 4.1 | 0.032 3.1 |
| OR Quart 4 | 1.5 | 1.2 | 1.6 | 2.4 | 2.5 | 2.0 | 1.3 | 0.99 | 1.7 |
| p Value | 0.21 | 0.78 | 0.16 | 0.020 | 0.091 | 0.074 | 0.72 | 0.99 | 0.48 |
| 95% CI of OR Quart4 | 0.79 2.9 | 0.41 3.3 | 0.84 3.1 | 1.1 5.2 | 0.86 7.4 | 0.94 4.2 | 0.28 6.2 | 0.24 4.1 | 0.39 7.4 |

**Vitronectin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 25.1 | 37.8 | 25.1 | 33.1 | 25.1 | 25.2 |
| Average | 62.1 | 105 | 62.1 | 80.0 | 62.1 | 39.1 |
| Stdev | 121 | 173 | 121 | 152 | 121 | 37.5 |
| p(t-test) | | 0.0094 | | 0.31 | | 0.48 |
| Min | 0.112 | 0.0795 | 0.112 | 2.95 | 0.112 | 4.01 |
| Max | 750 | 750 | 750 | 750 | 750 | 140 |
| n (Samp) | 212 | 111 | 212 | 74 | 212 | 14 |
| n (Patient) | 106 | 111 | 106 | 74 | 106 | 14 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 30.8 | 24.4 | 30.8 | 26.7 | 30.8 | 36.0 |
| Average | 75.8 | 37.0 | 75.8 | 74.7 | 75.8 | 146 |
| Stdev | 137 | 35.1 | 137 | 152 | 137 | 259 |
| p(t-test) | | 0.12 | | 0.97 | | 0.066 |
| Min | 0.0795 | 0.119 | 0.0795 | 3.61 | 0.0795 | 4.01 |
| Max | 750 | 138 | 750 | 750 | 750 | 750 |
| n (Samp) | 509 | 30 | 509 | 25 | 509 | 14 |
| n (Patient) | 217 | 30 | 217 | 25 | 217 | 14 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 24.5 | 41.7 | 24.5 | 28.7 | 24.5 | 18.8 |
| Average | 56.4 | 118 | 56.4 | 76.8 | 56.4 | 27.4 |
| Stdev | 112 | 181 | 112 | 150 | 112 | 21.9 |
| p(t-test) | | 1.3E-4 | | 0.20 | | 0.30 |
| Min | 0.112 | 0.0795 | 0.112 | 2.95 | 0.112 | 4.01 |
| Max | 750 | 750 | 750 | 750 | 750 | 74.8 |
| n (Samp) | 259 | 99 | 259 | 76 | 259 | 16 |
| n (Patient) | 117 | 99 | 117 | 76 | 117 | 16 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.60 | 0.43 | 0.65 | 0.54 | 0.47 | 0.54 | 0.50 | 0.55 | 0.44 |
| SE | 0.034 | 0.056 | 0.034 | 0.039 | 0.060 | 0.038 | 0.080 | 0.080 | 0.077 |
| p | 0.0025 | 0.23 | 1.2E-5 | 0.27 | 0.67 | 0.34 | 0.96 | 0.50 | 0.40 |
| nCohort 1 | 212 | 509 | 259 | 212 | 509 | 259 | 212 | 509 | 259 |
| nCohort 2 | 111 | 30 | 99 | 74 | 25 | 76 | 14 | 14 | 16 |
| Cutoff 1 | 22.2 | 13.3 | 24.7 | 15.4 | 14.4 | 16.9 | 16.0 | 26.5 | 10.1 |
| Sens 1 | 70% | 70% | 71% | 70% | 72% | 71% | 71% | 71% | 75% |
| Spec 1 | 42% | 21% | 51% | 29% | 23% | 36% | 30% | 46% | 18% |
| Cutoff 2 | 16.0 | 9.74 | 18.6 | 11.9 | 12.6 | 9.38 | 8.64 | 6.01 | 8.64 |
| Sens 2 | 80% | 80% | 81% | 81% | 80% | 80% | 86% | 86% | 81% |
| Spec 2 | 30% | 16% | 39% | 20% | 20% | 15% | 14% | 8% | 14% |
| Cutoff 3 | 8.10 | 7.75 | 9.55 | 7.00 | 9.92 | 6.91 | 4.35 | 4.09 | 4.35 |
| Sens 3 | 90% | 90% | 91% | 91% | 92% | 91% | 93% | 93% | 94% |
| Spec 3 | 13% | 12% | 16% | 12% | 16% | 12% | 8% | 5% | 7% |
| Cutoff 4 | 40.5 | 54.2 | 40.3 | 40.5 | 54.2 | 40.3 | 40.5 | 54.2 | 40.3 |
| Sens 4 | 47% | 30% | 53% | 42% | 32% | 37% | 43% | 43% | 31% |
| Spec 4 | 70% | 70% | 70% | 70% | 70% | 70% | 70% | 70% | 70% |
| Cutoff 5 | 64.6 | 76.6 | 59.7 | 64.6 | 76.6 | 59.7 | 64.6 | 76.6 | 59.7 |
| Sens 5 | 31% | 10% | 35% | 24% | 20% | 25% | 21% | 29% | 6% |
| Spec 5 | 80% | 80% | 80% | 80% | 80% | 80% | 80% | 80% | 80% |
| Cutoff 6 | 104 | 169 | 99.3 | 104 | 169 | 99.3 | 104 | 169 | 99.3 |
| Sens 6 | 23% | 0% | 27% | 15% | 8% | 17% | 7% | 14% | 0% |
| Spec 6 | 90% | 90% | 90% | 90% | 90% | 90% | 90% | 90% | 90% |
| OR Quart 2 | 1.2 | 1.2 | 1.5 | 0.77 | 1.2 | 0.98 | 0.15 | 0.66 | 0.49 |
| p Value | 0.64 | 0.78 | 0.28 | 0.52 | 0.75 | 0.97 | 0.089 | 0.65 | 0.41 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart2 | 0.60 2.3 | 0.38 3.6 | 0.72 3.1 | 0.35 1.7 | 0.36 4.1 | 0.47 2.1 | 0.018 1.3 | 0.11 4.0 | 0.086 2.7 |
| OR Quart 3 | 1.4 | 1.0 | 2.1 | 0.86 | 1.0 | 0.98 | 0.47 | 0.99 | 1.3 |
| p Value | 0.33 | 1.0 | 0.036 | 0.69 | 1.0 | 0.97 | 0.31 | 0.99 | 0.73 |
| 95% CI of OR Quart3 | 0.71 2.7 | 0.31 3.2 | 1.1 4.4 | 0.40 1.9 | 0.28 3.5 | 0.47 2.1 | 0.11 2.0 | 0.20 5.0 | 0.33 4.9 |
| OR Quart 4 | 2.1 | 1.9 | 3.3 | 1.6 | 1.9 | 1.3 | 0.65 | 2.0 | 1.3 |
| p Value | 0.026 | 0.21 | 8.1E-4 | 0.22 | 0.27 | 0.50 | 0.53 | 0.32 | 0.71 |
| 95% CI of OR Quart4 | 1.1 4.1 | 0.69 5.4 | 1.6 6.6 | 0.76 3.2 | 0.61 5.7 | 0.63 2.6 | 0.17 2.5 | 0.50 8.3 | 0.33 5.0 |

**Estradiol**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.48 | 9.47 | 6.48 | 9.50 | 6.48 | 8.04 |
| Average | 9.98 | 11.7 | 9.98 | 10.5 | 9.98 | 8.04 |
| Stdev | 16.6 | 8.08 | 16.6 | 7.34 | 16.6 | 2.54 |
| p(t-test) | | 0.67 | | 0.89 | | 0.87 |
| Min | 0.143 | 1.46 | 0.143 | 1.18 | 0.143 | 6.25 |
| Max | 128 | 26.7 | 128 | 28.3 | 128 | 9.84 |
| n (Samp) | 62 | 19 | 62 | 18 | 62 | 2 |
| n (Patient) | 50 | 19 | 50 | 18 | 50 | 2 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 7.13 | 9.69 | 7.13 | 7.90 | 7.13 | 14.0 |
| Average | 11.2 | 10.1 | 11.2 | 11.2 | 11.2 | 14.0 |
| Stdev | 14.8 | 6.53 | 14.8 | 10.5 | 14.8 | 5.89 |
| p(t-test) | | 0.87 | | 0.99 | | 0.79 |
| Min | 0.143 | 1.93 | 0.143 | 1.18 | 0.143 | 9.84 |
| Max | 128 | 19.9 | 128 | 28.3 | 128 | 18.2 |
| n (Samp) | 101 | 5 | 101 | 5 | 101 | 2 |
| n (Patient) | 81 | 5 | 81 | 5 | 81 | 2 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.88 | 9.47 | 5.88 | 9.28 | 5.88 | 6.93 |
| Average | 7.34 | 12.1 | 7.34 | 11.9 | 7.34 | 7.41 |
| Stdev | 6.17 | 8.43 | 6.17 | 8.70 | 6.17 | 3.06 |
| p(t-test) | | 0.018 | | 0.017 | | 0.98 |
| Min | 0.143 | 1.46 | 0.143 | 1.23 | 0.143 | 4.27 |
| Max | 31.6 | 26.7 | 31.6 | 34.2 | 31.6 | 11.5 |
| n (Samp) | 53 | 15 | 53 | 19 | 53 | 4 |
| n (Patient) | 42 | 15 | 42 | 19 | 42 | 4 |

| | 0hr prior to AKI stage | 24hr prior to AKI stage | 48hr prior to AKI stage |
|---|---|---|---|

| | sCr or UO | sCr only | UO only | sCr or UO | sCr only | UO only | sCr or UO | sCr only | UO only |
|---|---|---|---|---|---|---|---|---|---|
| AUC | 0.64 | 0.57 | 0.69 | 0.62 | 0.53 | 0.70 | 0.58 | 0.75 | 0.58 |
| SE | 0.076 | 0.14 | 0.083 | 0.078 | 0.13 | 0.074 | 0.22 | 0.20 | 0.16 |
| p | 0.065 | 0.63 | 0.023 | 0.14 | 0.82 | 0.0075 | 0.71 | 0.22 | 0.58 |
| nCohort 1 | 62 | 101 | 53 | 62 | 101 | 53 | 62 | 101 | 53 |
| nCohort 2 | 19 | 5 | 15 | 18 | 5 | 19 | 2 | 2 | 4 |
| Cutoff 1 | 6.77 | 7.54 | 6.77 | 5.44 | 5.41 | 6.61 | 5.88 | 9.74 | 5.88 |
| Sens 1 | 74% | 80% | 73% | 72% | 80% | 74% | 100% | 100% | 75% |
| Spec 1 | 56% | 52% | 62% | 44% | 35% | 60% | 45% | 64% | 51% |
| Cutoff 2 | 4.66 | 7.54 | 5.44 | 4.53 | 5.41 | 5.44 | 5.88 | 9.74 | 3.62 |
| Sens 2 | 84% | 80% | 80% | 83% | 80% | 84% | 100% | 100% | 100% |
| Spec 2 | 39% | 52% | 49% | 37% | 35% | 49% | 45% | 64% | 30% |
| Cutoff 3 | 1.89 | 1.89 | 3.47 | 1.18 | 0.944 | 3.62 | 5.88 | 9.74 | 3.62 |
| Sens 3 | 95% | 100% | 93% | 94% | 100% | 95% | 100% | 100% | 100% |
| Spec 3 | 10% | 8% | 28% | 3% | 3% | 30% | 45% | 64% | 30% |
| Cutoff 4 | 9.38 | 11.0 | 7.96 | 9.38 | 11.0 | 7.96 | 9.38 | 11.0 | 7.96 |
| Sens 4 | 53% | 40% | 53% | 50% | 40% | 58% | 50% | 50% | 25% |
| Spec 4 | 71% | 70% | 72% | 71% | 70% | 72% | 71% | 70% | 72% |
| Cutoff 5 | 11.6 | 14.2 | 9.96 | 11.6 | 14.2 | 9.96 | 11.6 | 14.2 | 9.96 |
| Sens 5 | 37% | 20% | 47% | 28% | 20% | 42% | 0% | 50% | 25% |
| Spec 5 | 81% | 80% | 81% | 81% | 80% | 81% | 81% | 80% | 81% |
| Cutoff 6 | 17.0 | 24.4 | 14.2 | 17.0 | 24.4 | 14.2 | 17.0 | 24.4 | 14.2 |
| Sens 6 | 26% | 0% | 27% | 17% | 20% | 26% | 0% | 0% | 0% |
| Spec 6 | 90% | 90% | 91% | 90% | 90% | 91% | 90% | 90% | 91% |
| OR Quart 2 | 0.63 | 0 | 1.0 | 2.2 | 0.96 | 1.6 | >1.1 | >0 | >1.1 |
| p Value | 0.63 | na | 1.0 | 0.38 | 0.98 | 0.63 | <0.96 | <na | <0.96 |
| 95% Cl of | 0.093 | na | 0.12 | 0.36 | 0.057 | 0.23 | >0.061 | >na | >0.061 |
| OR Quart2 | 4.2 | na | 8.1 | 14 | 16 | 11 | na | na | na |
| OR Quart 3 | 2.4 | 3.3 | 2.3 | 3.0 | 1.0 | 4.0 | >1.1 | >1.0 | >2.3 |
| p Value | 0.26 | 0.32 | 0.38 | 0.23 | 1.0 | 0.12 | <0.96 | <1.0 | <0.51 |
| 95% Cl of | 0.51 | 0.32 | 0.36 | 0.51 | 0.059 | 0.68 | >0.061 | >0.059 | >0.19 |
| OR Quart3 | 12 | 34 | 15 | 18 | 17 | 23 | na | na | na |
| OR Quart 4 | 3.5 | 0.96 | 5.2 | 4.8 | 2.0 | 6.4 | >0 | >1.0 | >1.0 |
| p Value | 0.11 | 0.98 | 0.065 | 0.073 | 0.58 | 0.036 | <na | <1.0 | <1.0 |
| 95% Cl of | 0.77 | 0.057 | 0.90 | 0.86 | 0.17 | 1.1 | >na | >0.059 | >0.057 |
| OR Quart4 | 16 | 16 | 31 | 27 | 23 | 36 | na | na | na |

**Progesterone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.1 | 20.3 | 21.1 | 34.6 | 21.1 | 81.5 |
| Average | 28.4 | 32.6 | 28.4 | 42.7 | 28.4 | 81.5 |
| Stdev | 25.5 | 32.4 | 25.5 | 28.2 | 25.5 | 88.8 |
| p(t-test) | | 0.55 | | 0.044 | | 0.0098 |
| Min | 2.91 | 3.98 | 2.91 | 4.04 | 2.91 | 18.7 |
| Max | 152 | 140 | 152 | 113 | 152 | 144 |
| n (Samp) | 62 | 19 | 62 | 18 | 62 | 2 |
| n (Patient) | 50 | 19 | 50 | 18 | 50 | 2 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 23.8 | 7.44 | 23.8 | 19.4 | 23.8 | 82.6 |
| Average | 36.3 | 11.1 | 36.3 | 24.6 | 36.3 | 82.6 |
| Stdev | 34.7 | 7.22 | 34.7 | 20.2 | 34.7 | 87.3 |
| p(t-test) | | 0.11 | | 0.46 | | 0.072 |
| Min | 2.91 | 5.03 | 2.91 | 4.04 | 2.91 | 20.9 |
| Max | 228 | 20.3 | 228 | 58.5 | 228 | 144 |
| n (Samp) | 101 | 5 | 101 | 5 | 101 | 2 |
| n (Patient) | 81 | 5 | 81 | 5 | 81 | 2 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 18.8 | 27.5 | 18.8 | 35.9 | 18.8 | 18.0 |
| Average | 30.4 | 38.1 | 30.4 | 43.9 | 30.4 | 21.6 |
| Stdev | 33.4 | 34.4 | 33.4 | 25.7 | 33.4 | 16.9 |
| p(t-test) | | 0.44 | | 0.12 | | 0.60 |
| Min | 2.91 | 3.98 | 2.91 | 6.29 | 2.91 | 5.03 |
| Max | 152 | 140 | 152 | 113 | 152 | 45.3 |
| n (Samp) | 53 | 15 | 53 | 19 | 53 | 4 |
| n (Patient) | 42 | 15 | 42 | 19 | 42 | 4 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.19 | 0.60 | 0.68 | 0.41 | 0.73 | 0.71 | 0.70 | 0.45 |
| SE | 0.077 | 0.12 | 0.086 | 0.076 | 0.14 | 0.072 | 0.21 | 0.21 | 0.15 |
| p | 0.85 | 0.0085 | 0.24 | 0.018 | 0.51 | 0.0015 | 0.32 | 0.35 | 0.76 |
| nCohort 1 | 62 | 101 | 53 | 62 | 101 | 53 | 62 | 101 | 53 |
| nCohort 2 | 19 | 5 | 15 | 18 | 5 | 19 | 2 | 2 | 4 |
| Cutoff 1 | 14.6 | 5.18 | 17.2 | 31.0 | 18.5 | 31.2 | 18.5 | 20.3 | 17.4 |
| Sens 1 | 74% | 80% | 73% | 72% | 80% | 74% | 100% | 100% | 75% |
| Spec 1 | 27% | 6% | 43% | 74% | 37% | 75% | 44% | 42% | 45% |
| Cutoff 2 | 10.2 | 5.18 | 14.6 | 18.5 | 18.5 | 23.5 | 18.5 | 20.3 | 4.62 |
| Sens 2 | 84% | 80% | 80% | 83% | 80% | 84% | 100% | 100% | 100% |
| Spec 2 | 19% | 6% | 32% | 44% | 37% | 64% | 44% | 42% | 4% |
| Cutoff 3 | 4.62 | 4.62 | 10.2 | 6.00 | 3.98 | 11.4 | 18.5 | 20.3 | 4.62 |
| Sens 3 | 95% | 100% | 93% | 94% | 100% | 95% | 100% | 100% | 100% |
| Spec 3 | 5% | 4% | 23% | 10% | 2% | 26% | 44% | 42% | 4% |
| Cutoff 4 | 30.0 | 40.0 | 28.1 | 30.0 | 40.0 | 28.1 | 30.0 | 40.0 | 28.1 |
| Sens 4 | 37% | 0% | 47% | 72% | 20% | 79% | 50% | 50% | 25% |
| Spec 4 | 71% | 70% | 72% | 71% | 70% | 72% | 71% | 70% | 72% |
| Cutoff 5 | 35.0 | 57.6 | 35.0 | 35.0 | 57.6 | 35.0 | 35.0 | 57.6 | 35.0 |
| Sens 5 | 37% | 0% | 47% | 50% | 20% | 53% | 50% | 50% | 25% |
| Spec 5 | 81% | 80% | 81% | 81% | 80% | 81% | 81% | 80% | 81% |
| Cutoff 6 | 61.1 | 75.2 | 68.1 | 61.1 | 75.2 | 68.1 | 61.1 | 75.2 | 68.1 |
| Sens 6 | 11% | 0% | 13% | 17% | 0% | 16% | 50% | 50% | 0% |
| Spec 6 | 90% | 90% | 91% | 90% | 90% | 91% | 90% | 90% | 91% |
| OR Quart 2 | 1.0 | >0 | 1.0 | 0.63 | 0 | 0.47 | >1.1 | >1.0 | 1.1 |
| p Value | 1.0 | <na | 1.0 | 0.63 | na | 0.55 | <0.96 | <1.0 | 0.96 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart2 | 0.24 | >na | 0.17 | 0.093 | na | 0.039 | >0.061 | >0.059 | 0.061 |
| | 4.2 | na | 5.8 | 4.2 | na | 5.7 | na | na | 19 |
| OR Quart 3 | 0.33 | >2.2 | 0.62 | 1.9 | 3.2 | 6.4 | >0 | >0 | 1.1 |
| p Value | 0.23 | <0.54 | 0.63 | 0.43 | 0.32 | 0.036 | <na | <na | 0.96 |
| 95% CI of OR Quart3 | 0.056 | >0.18 | 0.090 | 0.38 | 0.32 | 1.1 | >na | >na | 0.061 |
| | 2.0 | na | 4.3 | 9.3 | 33 | 36 | na | na | 19 |
| OR Quart 4 | 1.5 | >3.5 | 3.3 | 3.8 | 1.0 | 6.4 | >1.1 | >1.0 | 1.1 |
| p Value | 0.56 | <0.29 | 0.14 | 0.086 | 0.98 | 0.036 | <0.96 | <1.0 | 0.96 |
| 95% CI of OR Quart4 | 0.39 | >0.34 | 0.67 | 0.83 | 0.062 | 1.1 | >0.061 | >0.059 | 0.061 |
| | 5.8 | na | 16 | 17 | 18 | 36 | na | na | 19 |

**T3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.12 | 3.40 | 3.12 | 5.70 | 3.12 | 2.72 |
| Average | 4.28 | 3.94 | 4.28 | 6.00 | 4.28 | 2.72 |
| Stdev | 4.22 | 2.88 | 4.22 | 3.89 | 4.22 | 1.07 |
| p(t-test) | | 0.74 | | 0.13 | | 0.61 |
| Min | 0.000958 | 0.533 | 0.000958 | 0.569 | 0.000958 | 1.97 |
| Max | 20.4 | 10.2 | 20.4 | 14.4 | 20.4 | 3.48 |
| n (Samp) | 62 | 19 | 62 | 18 | 62 | 2 |
| n (Patient) | 50 | 19 | 50 | 18 | 50 | 2 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.57 | 2.23 | 3.57 | 2.59 | 3.57 | 1.98 |
| Average | 4.74 | 2.45 | 4.74 | 2.89 | 4.74 | 1.98 |
| Stdev | 3.92 | 0.772 | 3.92 | 1.78 | 3.92 | 0.0187 |
| p(t-test) | | 0.20 | | 0.30 | | 0.32 |
| Min | 0.000958 | 1.59 | 0.000958 | 0.569 | 0.000958 | 1.97 |
| Max | 20.4 | 3.40 | 20.4 | 4.81 | 20.4 | 2.00 |
| n (Samp) | 101 | 5 | 101 | 5 | 101 | 2 |
| n (Patient) | 81 | 5 | 81 | 5 | 81 | 2 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2.96 | 3.62 | 2.96 | 6.52 | 2.96 | 3.44 |
| Average | 3.93 | 4.38 | 3.93 | 6.14 | 3.93 | 3.72 |
| Stdev | 3.53 | 3.09 | 3.53 | 3.82 | 3.53 | 1.74 |
| p(t-test) | | 0.65 | | 0.025 | | 0.91 |
| Min | 0.00599 | 0.533 | 0.00599 | 0.757 | 0.00599 | 1.92 |
| Max | 19.3 | 10.2 | 19.3 | 14.4 | 19.3 | 6.10 |
| n (Samp) | 53 | 15 | 53 | 19 | 53 | 4 |
| n (Patient) | 42 | 15 | 42 | 19 | 42 | 4 |

| | 0hr prior to AKI stage | 24hr prior to AKI stage | 48hr prior to AKI stage |
|---|---|---|---|

|  | sCr or UO | sCr only | UO only | sCr or UO | sCr only | UO only | sCr or UO | sCr only | UO only |
|---|---|---|---|---|---|---|---|---|---|
| AUC | 0.50 | 0.30 | 0.56 | 0.67 | 0.36 | 0.71 | 0.45 | 0.25 | 0.58 |
| SE | 0.076 | 0.13 | 0.086 | 0.077 | 0.14 | 0.074 | 0.21 | 0.20 | 0.16 |
| p | 0.96 | 0.12 | 0.49 | 0.025 | 0.31 | 0.0049 | 0.82 | 0.21 | 0.58 |
| nCohort 1 | 62 | 101 | 53 | 62 | 101 | 53 | 62 | 101 | 53 |
| nCohort 2 | 19 | 5 | 15 | 18 | 5 | 19 | 2 | 2 | 4 |
| Cutoff 1 | 1.87 | 1.87 | 2.62 | 3.35 | 1.93 | 3.35 | 1.95 | 1.95 | 3.35 |
| Sens 1 | 74% | 80% | 73% | 72% | 80% | 74% | 100% | 100% | 75% |
| Spec 1 | 29% | 23% | 42% | 56% | 24% | 60% | 32% | 25% | 60% |
| Cutoff 2 | 1.37 | 1.87 | 1.60 | 2.49 | 1.93 | 2.77 | 1.95 | 1.95 | 1.78 |
| Sens 2 | 84% | 80% | 80% | 83% | 80% | 84% | 100% | 100% | 100% |
| Spec 2 | 16% | 23% | 19% | 39% | 24% | 45% | 32% | 25% | 26% |
| Cutoff 3 | 0.533 | 1.42 | 0.533 | 0.746 | 0.533 | 0.757 | 1.95 | 1.95 | 1.78 |
| Sens 3 | 95% | 100% | 93% | 94% | 100% | 95% | 100% | 100% | 100% |
| Spec 3 | 5% | 14% | 2% | 8% | 4% | 6% | 32% | 25% | 26% |
| Cutoff 4 | 4.65 | 5.80 | 4.54 | 4.65 | 5.80 | 4.54 | 4.65 | 5.80 | 4.54 |
| Sens 4 | 26% | 0% | 33% | 61% | 0% | 63% | 0% | 0% | 25% |
| Spec 4 | 71% | 70% | 72% | 71% | 70% | 72% | 71% | 70% | 72% |
| Cutoff 5 | 5.80 | 6.84 | 5.79 | 5.80 | 6.84 | 5.79 | 5.80 | 6.84 | 5.79 |
| Sens 5 | 26% | 0% | 33% | 50% | 0% | 53% | 0% | 0% | 25% |
| Spec 5 | 81% | 80% | 81% | 81% | 80% | 81% | 81% | 80% | 81% |
| Cutoff 6 | 7.12 | 8.80 | 7.09 | 7.12 | 8.80 | 7.09 | 7.12 | 8.80 | 7.09 |
| Sens 6 | 16% | 0% | 20% | 33% | 0% | 37% | 0% | 0% | 0% |
| Spec 6 | 90% | 90% | 91% | 90% | 90% | 91% | 90% | 90% | 91% |
| OR Quart 2 | 0.75 | >0 | 0.43 | 1.6 | >2.2 | 1.6 | >1.1 | >0 | >1.1 |
| p Value | 0.71 | <na | 0.38 | 0.63 | <0.52 | 0.63 | <0.96 | <na | <0.96 |
| 95% CI of OR Quart2 | 0.17 3.3 | >na na | 0.068 2.8 | 0.24 11 | >0.19 na | 0.23 11 | >0.061 na | >na na | >0.061 na |
| OR Quart 3 | 1.0 | >3.4 | 1.0 | 2.2 | >1.0 | 2.3 | >1.1 | >2.2 | >2.3 |
| p Value | 1.0 | <0.31 | 1.0 | 0.38 | <0.98 | 0.38 | <0.96 | <0.54 | <0.51 |
| 95% CI of OR Quart3 | 0.24 4.2 | >0.33 na | 0.20 4.9 | 0.36 14 | >0.062 na | 0.36 14 | >0.061 na | >0.18 na | >0.19 na |
| OR Quart 4 | 0.94 | >2.2 | 1.4 | 7.4 | >2.2 | 10 | >0 | >0 | >1.0 |
| p Value | 0.93 | <0.52 | 0.70 | 0.022 | <0.52 | 0.0095 | <na | <na | <1.0 |
| 95% CI of OR Quart4 | 0.23 3.9 | >0.19 na | 0.29 6.3 | 1.3 41 | >0.19 na | 1.8 57 | >na na | >na na | >0.057 na |

## Growth/differentiation factor 15

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 18600 | 23400 | 18600 | 58000 | 18600 | 54200 |
| Average | 73200 | 70600 | 73200 | 95300 | 73200 | 54200 |
| Stdev | 151000 | 74800 | 151000 | 95600 | 151000 | 48900 |
| p(t-test) |  | 0.94 |  | 0.56 |  | 0.86 |
| Min | 641 | 4750 | 641 | 1700 | 641 | 19600 |
| Max | 810000 | 221000 | 810000 | 341000 | 810000 | 88800 |
| n (Samp) | 62 | 19 | 62 | 18 | 62 | 2 |
| n (Patient) | 50 | 19 | 50 | 18 | 50 | 2 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 40200 | 19900 | 40200 | 49500 | 40200 | 29100 |
| Average | 83500 | 35000 | 83500 | 38900 | 83500 | 29100 |
| Stdev | 130000 | 39300 | 130000 | 26600 | 130000 | 13400 |
| p(t-test) | | 0.41 | | 0.45 | | 0.56 |
| Min | 641 | 4750 | 641 | 1700 | 641 | 19600 |
| Max | 810000 | 103000 | 810000 | 62700 | 810000 | 38600 |
| n (Samp) | 101 | 5 | 101 | 5 | 101 | 2 |
| n (Patient) | 81 | 5 | 81 | 5 | 81 | 2 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 17400 | 33300 | 17400 | 88100 | 17400 | 95700 |
| Average | 44400 | 80000 | 44400 | 112000 | 44400 | 110000 |
| Stdev | 73700 | 79400 | 73700 | 92600 | 73700 | 92100 |
| p(t-test) | | 0.11 | | 0.0020 | | 0.097 |
| Min | 641 | 5560 | 641 | 5180 | 641 | 13700 |
| Max | 326000 | 221000 | 326000 | 341000 | 326000 | 235000 |
| n (Samp) | 53 | 15 | 53 | 19 | 53 | 4 |
| n (Patient) | 42 | 15 | 42 | 19 | 42 | 4 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.57 | 0.39 | 0.64 | 0.68 | 0.44 | 0.81 | 0.67 | 0.44 | 0.79 |
| SE | 0.077 | 0.14 | 0.085 | 0.076 | 0.14 | 0.065 | 0.21 | 0.21 | 0.14 |
| p | 0.40 | 0.43 | 0.090 | 0.021 | 0.67 | 1.8E-6 | 0.43 | 0.78 | 0.034 |
| nCohort 1 | 62 | 101 | 53 | 62 | 101 | 53 | 62 | 101 | 53 |
| nCohort 2 | 19 | 5 | 15 | 18 | 5 | 19 | 2 | 2 | 4 |
| Cutoff 1 | 10800 | 12900 | 10700 | 42000 | 19000 | 49600 | 19000 | 19000 | 70800 |
| Sens 1 | 74% | 80% | 73% | 72% | 80% | 74% | 100% | 100% | 75% |
| Spec 1 | 32% | 29% | 38% | 66% | 39% | 83% | 52% | 39% | 89% |
| Cutoff 2 | 7930 | 12900 | 8210 | 19000 | 19000 | 29800 | 19000 | 19000 | 12900 |
| Sens 2 | 84% | 80% | 80% | 83% | 80% | 84% | 100% | 100% | 100% |
| Spec 2 | 24% | 29% | 32% | 52% | 39% | 66% | 52% | 39% | 43% |
| Cutoff 3 | 5470 | 4510 | 5570 | 5070 | 641 | 12900 | 19000 | 19000 | 12900 |
| Sens 3 | 95% | 100% | 93% | 94% | 100% | 95% | 100% | 100% | 100% |
| Spec 3 | 18% | 6% | 26% | 15% | 1% | 43% | 52% | 39% | 43% |
| Cutoff 4 | 49600 | 88800 | 37400 | 49600 | 88800 | 37400 | 49600 | 88800 | 37400 |
| Sens 4 | 42% | 20% | 47% | 61% | 0% | 79% | 50% | 0% | 75% |
| Spec 4 | 71% | 70% | 72% | 71% | 70% | 72% | 71% | 70% | 72% |
| Cutoff 5 | 76400 | 116000 | 49500 | 76400 | 116000 | 49500 | 76400 | 116000 | 49500 |
| Sens 5 | 42% | 0% | 47% | 39% | 0% | 74% | 50% | 0% | 75% |
| Spec 5 | 81% | 80% | 81% | 81% | 80% | 81% | 81% | 80% | 81% |
| Cutoff 6 | 205000 | 218000 | 93200 | 205000 | 218000 | 93200 | 205000 | 218000 | 93200 |
| Sens 6 | 11% | 0% | 47% | 17% | 0% | 47% | 0% | 0% | 50% |
| Spec 6 | 90% | 90% | 91% | 90% | 90% | 91% | 90% | 90% | 91% |
| OR Quart 2 | 1.0 | >2.2 | 2.3 | 1.6 | >3.5 | 1.0 | >0 | >1.0 | >1.1 |
| p Value | 1.0 | <0.52 | 0.38 | 0.63 | <0.29 | 1.0 | <na | <0.98 | <0.96 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 0.21 | >0.19 | 0.36 | 0.24 | >0.34 | 0.058 | >na | >0.062 | >0.061 |
| OR Quart2 | 4.7 | na | 15 | 11 | na | 17 | na | na | na |
| OR Quart 3 | 0.71 | >2.2 | 1.0 | 3.9 | >1.0 | 8.5 | >1.1 | >1.0 | >0 |
| p Value | 0.68 | <0.54 | 1.0 | 0.13 | <0.98 | 0.061 | <0.96 | <0.98 | <na |
| 95% CI of | 0.14 | >0.18 | 0.12 | 0.67 | >0.062 | 0.90 | >0.061 | >0.062 | >na |
| OR Quart3 | 3.7 | na | 8.1 | 22 | na | 80 | na | na | na |
| OR Quart 4 | 2.5 | >1.1 | 5.2 | 4.8 | >1.1 | 27 | >1.1 | >0 | >3.5 |
| p Value | 0.21 | <0.96 | 0.065 | 0.073 | <0.96 | 0.0039 | <0.96 | <na | <0.30 |
| 95% CI of | 0.60 | >0.064 | 0.90 | 0.86 | >0.064 | 2.9 | >0.061 | >na | >0.32 |
| OR Quart4 | 10 | na | 31 | 27 | na | 250 | na | na | na |

**Proprotein convertase subtilisin/kexin type 9**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 359 | 944 | 359 | 882 | 359 | 580 |
| Average | 3220 | 2850 | 3220 | 3140 | 3220 | 580 |
| Stdev | 13100 | 4820 | 13100 | 3890 | 13100 | 0 |
| p(t-test) |  | 0.90 |  | 0.98 |  | 0.78 |
| Min | 81.8 | 120 | 81.8 | 70.6 | 81.8 | 580 |
| Max | 96400 | 19200 | 96400 | 11400 | 96400 | 580 |
| n (Samp) | 62 | 19 | 62 | 18 | 62 | 2 |
| n (Patient) | 50 | 19 | 50 | 18 | 50 | 2 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 419 | 1190 | 419 | 3260 | 419 | 586 |
| Average | 2890 | 2180 | 2890 | 4450 | 2890 | 586 |
| Stdev | 10600 | 2410 | 10600 | 4180 | 10600 | 8.37 |
| p(t-test) |  | 0.88 |  | 0.74 |  | 0.76 |
| Min | 48.1 | 285 | 48.1 | 121 | 48.1 | 580 |
| Max | 96400 | 6340 | 96400 | 11400 | 96400 | 592 |
| n (Samp) | 101 | 5 | 101 | 5 | 101 | 2 |
| n (Patient) | 81 | 5 | 81 | 5 | 81 | 2 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 366 | 436 | 366 | 897 | 366 | 1740 |
| Average | 2730 | 2960 | 2730 | 2550 | 2730 | 2600 |
| Stdev | 13200 | 5310 | 13200 | 3270 | 13200 | 2560 |
| p(t-test) |  | 0.95 |  | 0.95 |  | 0.98 |
| Min | 95.1 | 120 | 95.1 | 70.6 | 95.1 | 580 |
| Max | 96400 | 19200 | 96400 | 11400 | 96400 | 6340 |
| n (Samp) | 53 | 15 | 53 | 19 | 53 | 4 |
| n (Patient) | 42 | 15 | 42 | 19 | 42 | 4 |

| 0hr prior to AKI stage | 24hr prior to AKI stage | 48hr prior to AKI stage |
|---|---|---|

| | sCr or UO | sCr only | UO only | sCr or UO | sCr only | UO only | sCr or UO | sCr only | UO only |
|---|---|---|---|---|---|---|---|---|---|
| AUC | 0.61 | 0.71 | 0.58 | 0.62 | 0.73 | 0.65 | 0.67 | 0.63 | 0.83 |
| SE | 0.077 | 0.13 | 0.086 | 0.078 | 0.13 | 0.077 | 0.21 | 0.21 | 0.13 |
| p | 0.15 | 0.12 | 0.37 | 0.13 | 0.075 | 0.058 | 0.43 | 0.55 | 0.010 |
| nCohort 1 | 62 | 101 | 53 | 62 | 101 | 53 | 62 | 101 | 53 |
| nCohort 2 | 19 | 5 | 15 | 18 | 5 | 19 | 2 | 2 | 4 |
| Cutoff 1 | 285 | 1010 | 247 | 296 | 3030 | 430 | 575 | 575 | 1360 |
| Sens 1 | 74% | 80% | 73% | 72% | 80% | 74% | 100% | 100% | 75% |
| Spec 1 | 40% | 72% | 36% | 47% | 87% | 57% | 66% | 61% | 85% |
| Cutoff 2 | 148 | 1010 | 148 | 148 | 3030 | 187 | 575 | 575 | 575 |
| Sens 2 | 84% | 80% | 80% | 83% | 80% | 84% | 100% | 100% | 100% |
| Spec 2 | 15% | 72% | 19% | 15% | 87% | 21% | 66% | 61% | 60% |
| Cutoff 3 | 115 | 279 | 115 | 114 | 120 | 114 | 575 | 575 | 575 |
| Sens 3 | 100% | 100% | 100% | 94% | 100% | 95% | 100% | 100% | 100% |
| Spec 3 | 8% | 36% | 9% | 6% | 10% | 8% | 66% | 61% | 60% |
| Cutoff 4 | 633 | 944 | 944 | 633 | 944 | 944 | 633 | 944 | 944 |
| Sens 4 | 53% | 80% | 40% | 56% | 80% | 37% | 0% | 0% | 75% |
| Spec 4 | 71% | 71% | 72% | 71% | 71% | 72% | 71% | 71% | 72% |
| Cutoff 5 | 1300 | 1450 | 1300 | 1300 | 1450 | 1300 | 1300 | 1450 | 1300 |
| Sens 5 | 37% | 40% | 33% | 44% | 80% | 37% | 0% | 0% | 75% |
| Spec 5 | 81% | 80% | 81% | 81% | 80% | 81% | 81% | 80% | 81% |
| Cutoff 6 | 2690 | 4940 | 2690 | 2690 | 4940 | 2690 | 2690 | 4940 | 2690 |
| Sens 6 | 26% | 20% | 27% | 39% | 20% | 32% | 0% | 0% | 25% |
| Spec 6 | 90% | 90% | 91% | 90% | 90% | 91% | 90% | 90% | 91% |
| OR Quart 2 | 1.0 | >1.0 | 0.70 | 0.16 | 0 | 0.44 | >0 | >0 | >0 |
| p Value | 1.0 | <1.0 | 0.67 | 0.11 | na | 0.38 | <na | <na | <na |
| 95% CI of OR Quart2 | 0.21 | >0.059 | 0.13 | 0.017 | na | 0.069 | >na | >na | >na |
| | 4.7 | na | 3.7 | 1.5 | na | 2.8 | na | na | na |
| OR Quart 3 | 0.71 | >2.2 | 0.43 | 0.75 | 0 | 1.8 | >2.3 | >2.1 | >1.1 |
| p Value | 0.68 | <0.54 | 0.38 | 0.71 | na | 0.46 | <0.52 | <0.56 | <0.96 |
| 95% CI of OR Quart3 | 0.14 | >0.18 | 0.068 | 0.17 | na | 0.40 | >0.19 | >0.18 | >0.061 |
| | 3.7 | na | 2.8 | 3.3 | na | 7.7 | na | na | na |
| OR Quart 4 | 2.5 | >2.1 | 1.8 | 2.0 | 4.3 | 2.2 | >0 | >0 | >3.5 |
| p Value | 0.21 | <0.56 | 0.45 | 0.31 | 0.20 | 0.28 | <na | <na | <0.30 |
| 95% CI of OR Quart4 | 0.60 | >0.18 | 0.40 | 0.52 | 0.45 | 0.52 | >na | >na | >0.32 |
| | 10 | na | 7.9 | 7.7 | 42 | 9.6 | na | na | na |

Table 2: Comparison of marker levels in urine samples collected from Cohort 1 (patients that did not progress beyond RIFLE stage 0 or R) and in urine samples collected from subjects at 0, 24 hours, and 48 hours prior to reaching stage I or F in Cohort 2.

**Toll-like receptor 2**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 0.336 | 0.398 | 0.336 | 0.592 |
| Average | 0.485 | 0.648 | 0.485 | 0.690 |
| Stdev | 0.498 | 0.622 | 0.498 | 0.516 |
| p(t-test) | | 0.23 | | 0.10 |
| Min | 0.0538 | 0.0738 | 0.0538 | 0.0621 |

| sCr or UO | 0hr prior to AKI stage | | 24hr prior to AKI stage | |
| --- | --- | --- | --- | --- |
| | Cohort 1 | Cohort 2 | Cohort 1 | Cohort 2 |
| Max | 3.06 | 2.43 | 3.06 | 2.03 |
| n (Samp) | 92 | 18 | 92 | 20 |
| n (Patient) | 44 | 18 | 44 | 20 |

| sCr only | 0hr prior to AKI stage | | 24hr prior to AKI stage | |
| --- | --- | --- | --- | --- |
| | Cohort 1 | Cohort 2 | Cohort 1 | Cohort 2 |
| Median | 0.358 | 0.253 | 0.358 | 1.04 |
| Average | 0.519 | 0.253 | 0.519 | 1.19 |
| Stdev | 0.496 | 0.164 | 0.496 | 1.17 |
| p(t-test) | | 0.45 | | 0.027 |
| Min | 0.0538 | 0.136 | 0.0538 | 0.0988 |
| Max | 3.06 | 0.369 | 3.06 | 2.43 |
| n (Samp) | 124 | 2 | 124 | 3 |
| n (Patient) | 60 | 2 | 60 | 3 |

| UO only | 0hr prior to AKI stage | | 24hr prior to AKI stage | |
| --- | --- | --- | --- | --- |
| | Cohort 1 | Cohort 2 | Cohort 1 | Cohort 2 |
| Median | 0.326 | 0.426 | 0.326 | 0.662 |
| Average | 0.491 | 0.678 | 0.491 | 0.721 |
| Stdev | 0.519 | 0.627 | 0.519 | 0.511 |
| p(t-test) | | 0.19 | | 0.084 |
| Min | 0.0538 | 0.0738 | 0.0538 | 0.0621 |
| Max | 3.06 | 2.43 | 3.06 | 2.03 |
| n (Samp) | 84 | 17 | 84 | 19 |
| n (Patient) | 40 | 17 | 40 | 19 |

| | 0hr prior to AKI stage | | | 24hr prior to AKI stage | | |
| --- | --- | --- | --- | --- | --- | --- |
| | sCr or UO | sCr only | UO only | sCr or UO | sCr only | UO only |
| AUC | 0.57 | 0.31 | 0.60 | 0.63 | 0.65 | 0.66 |
| SE | 0.076 | 0.21 | 0.079 | 0.072 | 0.17 | 0.074 |
| p | 0.38 | 0.36 | 0.22 | 0.074 | 0.40 | 0.029 |
| nCohort 1 | 92 | 124 | 84 | 92 | 124 | 84 |
| nCohort 2 | 18 | 2 | 17 | 20 | 3 | 19 |
| Cutoff 1 | 0.214 | 0.131 | 0.269 | 0.375 | 0.0901 | 0.375 |
| Sens 1 | 72% | 100% | 71% | 70% | 100% | 74% |
| Spec 1 | 26% | 10% | 37% | 58% | 7% | 60% |
| Cutoff 2 | 0.174 | 0.131 | 0.200 | 0.214 | 0.0901 | 0.211 |
| Sens 2 | 83% | 100% | 82% | 80% | 100% | 84% |
| Spec 2 | 20% | 10% | 26% | 26% | 7% | 27% |
| Cutoff 3 | 0.0812 | 0.131 | 0.0812 | 0.111 | 0.0901 | 0.111 |
| Sens 3 | 94% | 100% | 94% | 90% | 100% | 95% |
| Spec 3 | 7% | 10% | 6% | 9% | 7% | 10% |
| Cutoff 4 | 0.469 | 0.522 | 0.449 | 0.469 | 0.522 | 0.449 |
| Sens 4 | 44% | 0% | 47% | 60% | 67% | 63% |
| Spec 4 | 71% | 70% | 70% | 71% | 70% | 70% |
| Cutoff 5 | 0.626 | 0.728 | 0.641 | 0.626 | 0.728 | 0.641 |
| Sens 5 | 39% | 0% | 41% | 50% | 67% | 53% |

|  | 0hr prior to AKI stage |  |  | 24hr prior to AKI stage |  |  |
|---|---|---|---|---|---|---|
|  | sCr or UO | sCr only | UO only | sCr or UO | sCr only | UO only |
| Spec 5 | 80% | 81% | 81% | 80% | 81% | 81% |
| Cutoff 6 | 1.01 | 1.17 | 1.09 | 1.01 | 1.17 | 1.09 |
| Sens 6 | 17% | 0% | 18% | 30% | 33% | 21% |
| Spec 6 | 90% | 90% | 90% | 90% | 90% | 90% |
| OR Quart 2 | 0.34 | >1.1 | 1.0 | 0.46 | 0 | 0.61 |
| p Value | 0.22 | <0.96 | 1.0 | 0.40 | na | 0.61 |
| 95% CI of | 0.060 | >0.064 | 0.18 | 0.077 | na | 0.093 |
| OR Quart2 | 1.9 | na | 5.5 | 2.8 | na | 4.0 |
| OR Quart 3 | 0.55 | >0 | 1.0 | 1.0 | 0 | 1.3 |
| p Value | 0.45 | <na | 1.0 | 1.0 | na | 0.73 |
| 95% CI of | 0.12 | >na | 0.18 | 0.22 | na | 0.27 |
| OR Quart3 | 2.6 | na | 5.5 | 4.5 | na | 6.7 |
| OR Quart 4 | 1.8 | >1.1 | 3.3 | 3.3 | 2.0 | 4.6 |
| p Value | 0.38 | <0.96 | 0.11 | 0.072 | 0.58 | 0.039 |
| 95% CI of | 0.49 | >0.064 | 0.75 | 0.90 | 0.17 | 1.1 |
| OR Quart4 | 6.3 | na | 14 | 12 | 23 | 19 |

**Antithrombin-III**

| sCr or UO | 0hr prior to AKI stage |  | 24hr prior to AKI stage |  | 48hr prior to AKI stage |  |
|---|---|---|---|---|---|---|
|  | Cohort 1 | Cohort 2 | Cohort 1 | Cohort 2 | Cohort 1 | Cohort 2 |
| Median | 111 | 132 | 111 | 121 | 111 | 54.3 |
| Average | 340 | 217 | 340 | 590 | 340 | 198 |
| Stdev | 896 | 253 | 896 | 1350 | 896 | 435 |
| p(t-test) |  | 0.34 |  | 0.056 |  | 0.43 |
| Min | 0.0182 | 4.10 | 0.0182 | 4.48 | 0.0182 | 0.347 |
| Max | 6000 | 1120 | 6000 | 6000 | 6000 | 2060 |
| n (Samp) | 465 | 48 | 465 | 61 | 465 | 25 |
| n (Patient) | 212 | 48 | 212 | 61 | 212 | 25 |

| sCr only | 0hr prior to AKI stage |  | 24hr prior to AKI stage |  | 48hr prior to AKI stage |  |
|---|---|---|---|---|---|---|
|  | Cohort 1 | Cohort 2 | Cohort 1 | Cohort 2 | Cohort 1 | Cohort 2 |
| Median | 107 | 119 | 107 | 176 | 107 | 112 |
| Average | 367 | 136 | 367 | 221 | 367 | 255 |
| Stdev | 947 | 71.1 | 947 | 226 | 947 | 312 |
| p(t-test) |  | 0.52 |  | 0.57 |  | 0.74 |
| Min | 0.0182 | 36.9 | 0.0182 | 11.5 | 0.0182 | 8.92 |
| Max | 6000 | 244 | 6000 | 908 | 6000 | 930 |
| n (Samp) | 637 | 7 | 637 | 14 | 637 | 8 |
| n (Patient) | 267 | 7 | 267 | 14 | 267 | 8 |

| UO only | 0hr prior to AKI stage |  | 24hr prior to AKI stage |  | 48hr prior to AKI stage |  |
|---|---|---|---|---|---|---|
|  | Cohort 1 | Cohort 2 | Cohort 1 | Cohort 2 | Cohort 1 | Cohort 2 |
| Median | 110 | 125 | 110 | 117 | 110 | 54.3 |
| Average | 332 | 222 | 332 | 602 | 332 | 185 |
| Stdev | 878 | 261 | 878 | 1370 | 878 | 424 |
| p(t-test) |  | 0.40 |  | 0.038 |  | 0.43 |
| Min | 0.0182 | 4.10 | 0.0182 | 4.48 | 0.0182 | 0.347 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 6000 | 1120 | 6000 | 6000 | 6000 | 2060 |
| n (Samp) | 486 | 45 | 486 | 59 | 486 | 23 |
| n (Patient) | 209 | 45 | 209 | 59 | 209 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.53 | 0.54 | 0.53 | 0.56 | 0.58 | 0.56 | 0.36 | 0.53 | 0.38 |
| SE | 0.044 | 0.11 | 0.046 | 0.040 | 0.081 | 0.041 | 0.061 | 0.10 | 0.064 |
| p | 0.52 | 0.74 | 0.56 | 0.14 | 0.33 | 0.15 | 0.023 | 0.77 | 0.052 |
| nCohort 1 | 465 | 637 | 486 | 465 | 637 | 486 | 465 | 637 | 486 |
| nCohort 2 | 48 | 7 | 45 | 61 | 14 | 59 | 25 | 8 | 23 |
| Cutoff 1 | 64.8 | 106 | 59.8 | 82.8 | 96.5 | 78.6 | 42.4 | 58.2 | 42.4 |
| Sens 1 | 71% | 71% | 71% | 70% | 71% | 71% | 72% | 75% | 74% |
| Spec 1 | 33% | 50% | 30% | 41% | 47% | 40% | 20% | 30% | 20% |
| Cutoff 2 | 36.8 | 79.7 | 49.0 | 62.2 | 74.9 | 55.5 | 24.2 | 56.5 | 24.2 |
| Sens 2 | 81% | 86% | 80% | 80% | 86% | 81% | 80% | 88% | 83% |
| Spec 2 | 18% | 40% | 24% | 32% | 38% | 28% | 11% | 28% | 12% |
| Cutoff 3 | 28.0 | 36.8 | 28.0 | 29.1 | 27.1 | 29.1 | 12.1 | 8.57 | 12.5 |
| Sens 3 | 92% | 100% | 91% | 90% | 93% | 92% | 92% | 100% | 91% |
| Spec 3 | 12% | 18% | 12% | 12% | 11% | 13% | 3% | 2% | 5% |
| Cutoff 4 | 189 | 189 | 188 | 189 | 189 | 188 | 189 | 189 | 188 |
| Sens 4 | 33% | 29% | 36% | 43% | 50% | 41% | 24% | 38% | 26% |
| Spec 4 | 70% | 70% | 70% | 70% | 70% | 70% | 70% | 70% | 70% |
| Cutoff 5 | 281 | 282 | 279 | 281 | 282 | 279 | 281 | 282 | 279 |
| Sens 5 | 19% | 0% | 22% | 28% | 21% | 27% | 12% | 38% | 13% |
| Spec 5 | 80% | 80% | 80% | 80% | 80% | 80% | 80% | 80% | 80% |
| Cutoff 6 | 590 | 625 | 572 | 590 | 625 | 572 | 590 | 625 | 572 |
| Sens 6 | 10% | 0% | 11% | 13% | 7% | 15% | 8% | 12% | 4% |
| Spec 6 | 90% | 90% | 90% | 90% | 90% | 90% | 90% | 90% | 90% |
| OR Quart 2 | 0.80 | 2.0 | 0.80 | 1.6 | 2.0 | 1.9 | 1.4 | 3.0 | 1.4 |
| p Value | 0.64 | 0.57 | 0.63 | 0.24 | 0.42 | 0.12 | 0.69 | 0.34 | 0.69 |
| 95% CI of OR Quart2 | 0.32 | 0.18 | 0.32 | 0.72 | 0.36 | 0.85 | 0.30 | 0.31 | 0.30 |
| | 2.0 | 22 | 2.0 | 3.6 | 11 | 4.3 | 6.2 | 30 | 6.2 |
| OR Quart 3 | 1.4 | 3.0 | 1.1 | 1.3 | 1.5 | 1.3 | 3.2 | 1.0 | 2.8 |
| p Value | 0.41 | 0.34 | 0.84 | 0.53 | 0.66 | 0.51 | 0.091 | 1.0 | 0.13 |
| 95% CI of OR Quart3 | 0.62 | 0.31 | 0.46 | 0.57 | 0.25 | 0.56 | 0.83 | 0.062 | 0.73 |
| | 3.2 | 30 | 2.6 | 3.0 | 9.1 | 3.2 | 12 | 16 | 11 |
| OR Quart 4 | 1.2 | 1.0 | 1.2 | 1.8 | 2.5 | 1.9 | 3.2 | 3.0 | 2.8 |
| p Value | 0.68 | 1.0 | 0.68 | 0.13 | 0.27 | 0.12 | 0.088 | 0.34 | 0.13 |
| 95% CI of OR Quart4 | 0.51 | 0.062 | 0.51 | 0.84 | 0.48 | 0.85 | 0.84 | 0.31 | 0.73 |
| | 2.8 | 16 | 2.8 | 4.0 | 13 | 4.3 | 12 | 29 | 11 |

**Extracellular matrix protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.409 | 0.659 | 0.409 | 1.18 | 0.409 | 0.316 |
| Average | 0.871 | 4.11 | 0.871 | 1.85 | 0.871 | 1.11 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 1.19 | 19.4 | 1.19 | 2.39 | 1.19 | 1.82 |
| p(t-test) | | 3.8E-4 | | 2.9E-7 | | 0.34 |
| Min | 0.000528 | 0.00180 | 0.000528 | 0.00789 | 0.000528 | 0.0117 |
| Max | 10.9 | 135 | 10.9 | 13.8 | 10.9 | 6.57 |
| n (Samp) | 466 | 48 | 466 | 61 | 466 | 25 |
| n (Patient) | 212 | 48 | 212 | 61 | 212 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.495 | 0.459 | 0.495 | 1.31 | 0.495 | 1.01 |
| Average | 1.76 | 0.776 | 1.76 | 1.52 | 1.76 | 2.27 |
| Stdev | 10.2 | 0.823 | 10.2 | 1.30 | 10.2 | 2.77 |
| p(t-test) | | 0.80 | | 0.93 | | 0.89 |
| Min | 0.000528 | 0.0250 | 0.000528 | 0.128 | 0.000528 | 0.272 |
| Max | 150 | 2.38 | 150 | 4.67 | 150 | 7.31 |
| n (Samp) | 638 | 7 | 638 | 14 | 638 | 8 |
| n (Patient) | 267 | 7 | 267 | 14 | 267 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.463 | 0.643 | 0.463 | 0.795 | 0.463 | 0.311 |
| Average | 0.893 | 4.34 | 0.893 | 1.82 | 0.893 | 0.986 |
| Stdev | 1.20 | 20.0 | 1.20 | 2.45 | 1.20 | 1.59 |
| p(t-test) | | 1.9E-4 | | 1.7E-6 | | 0.72 |
| Min | 0.000528 | 0.00180 | 0.000528 | 0.00419 | 0.000528 | 0.0117 |
| Max | 10.9 | 135 | 10.9 | 13.8 | 10.9 | 6.57 |
| n (Samp) | 486 | 45 | 486 | 59 | 486 | 23 |
| n (Patient) | 209 | 45 | 209 | 59 | 209 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.49 | 0.59 | 0.66 | 0.66 | 0.63 | 0.46 | 0.68 | 0.44 |
| SE | 0.045 | 0.11 | 0.046 | 0.040 | 0.080 | 0.041 | 0.061 | 0.11 | 0.063 |
| p | 0.019 | 0.94 | 0.041 | 7.5E-5 | 0.049 | 0.0011 | 0.50 | 0.091 | 0.35 |
| nCohort 1 | 466 | 638 | 486 | 466 | 638 | 486 | 466 | 638 | 486 |
| nCohort 2 | 48 | 7 | 45 | 61 | 14 | 59 | 25 | 8 | 23 |
| Cutoff 1 | 0.351 | 0.390 | 0.312 | 0.422 | 0.725 | 0.382 | 0.163 | 0.560 | 0.0936 |
| Sens 1 | 71% | 71% | 71% | 70% | 71% | 71% | 72% | 75% | 74% |
| Spec 1 | 45% | 45% | 40% | 51% | 59% | 46% | 26% | 54% | 14% |
| Cutoff 2 | 0.263 | 0.177 | 0.263 | 0.274 | 0.274 | 0.269 | 0.0837 | 0.362 | 0.0800 |
| Sens 2 | 81% | 86% | 80% | 80% | 86% | 81% | 80% | 88% | 83% |
| Spec 2 | 36% | 24% | 35% | 38% | 35% | 36% | 12% | 43% | 12% |
| Cutoff 3 | 0.129 | 0.0241 | 0.129 | 0.132 | 0.132 | 0.124 | 0.0290 | 0.271 | 0.0290 |
| Sens 3 | 92% | 100% | 91% | 90% | 93% | 92% | 92% | 100% | 91% |
| Spec 3 | 20% | 3% | 20% | 20% | 17% | 19% | 4% | 35% | 4% |
| Cutoff 4 | 0.987 | 1.09 | 1.00 | 0.987 | 1.09 | 1.00 | 0.987 | 1.09 | 1.00 |
| Sens 4 | 46% | 29% | 47% | 52% | 64% | 47% | 24% | 50% | 26% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Spec 4 | 70% | 70% | 70% | 70% | 70% | 70% | 70% | 70% | 70% |
| Cutoff 5 | 1.29 | 1.49 | 1.32 | 1.29 | 1.49 | 1.32 | 1.29 | 1.49 | 1.32 |
| Sens 5 | 38% | 14% | 33% | 46% | 43% | 39% | 24% | 38% | 26% |
| Spec 5 | 80% | 80% | 80% | 80% | 80% | 80% | 80% | 80% | 80% |
| Cutoff 6 | 2.28 | 2.45 | 2.29 | 2.28 | 2.45 | 2.29 | 2.28 | 2.45 | 2.29 |
| Sens 6 | 15% | 0% | 16% | 28% | 21% | 29% | 16% | 25% | 13% |
| Spec 6 | 90% | 90% | 90% | 90% | 90% | 90% | 90% | 90% | 90% |
| OR Quart 2 | 2.8 | 0.50 | 2.8 | 1.4 | 1.0 | 1.7 | 0.49 | >2.0 | 0.33 |
| p Value | 0.061 | 0.57 | 0.062 | 0.49 | 1.0 | 0.26 | 0.32 | <0.57 | 0.17 |
| 95% CI of | 0.95 | 0.045 | 0.95 | 0.54 | 0.14 | 0.68 | 0.12 | >0.18 | 0.064 |
| OR Quart2 | 8.0 | 5.6 | 8.0 | 3.6 | 7.2 | 4.2 | 2.0 | na | 1.6 |
| OR Quart 3 | 2.5 | 1.0 | 2.1 | 1.7 | 1.5 | 1.5 | 1.5 | >2.0 | 1.4 |
| p Value | 0.088 | 1.00 | 0.20 | 0.27 | 0.65 | 0.36 | 0.43 | <0.57 | 0.57 |
| 95% CI of | 0.87 | 0.14 | 0.69 | 0.67 | 0.25 | 0.61 | 0.53 | >0.18 | 0.46 |
| OR Quart3 | 7.4 | 7.2 | 6.2 | 4.2 | 9.2 | 3.9 | 4.5 | na | 4.1 |
| OR Quart 4 | 4.0 | 1.0 | 3.7 | 4.3 | 3.6 | 3.7 | 1.2 | >4.1 | 1.2 |
| p Value | 0.0081 | 1.00 | 0.012 | 5.0E-4 | 0.11 | 0.0019 | 0.76 | <0.21 | 0.77 |
| 95% CI of | 1.4 | 0.14 | 1.3 | 1.9 | 0.74 | 1.6 | 0.39 | >0.45 | 0.39 |
| OR Quart4 | 11 | 7.2 | 10 | 9.9 | 18 | 8.6 | 3.6 | na | 3.6 |

**Coagulation factor XIII A and B chains**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.84 | 3.83 | 3.84 | 6.25 | 3.84 | 2.26 |
| Average | 7.04 | 7.74 | 7.04 | 11.6 | 7.04 | 6.20 |
| Stdev | 9.23 | 9.70 | 9.23 | 20.1 | 9.23 | 8.57 |
| p(t-test) | | 0.62 | | 0.0027 | | 0.66 |
| Min | 0.000120 | 0.000180 | 0.000120 | 0.000189 | 0.000120 | 0.000180 |
| Max | 84.2 | 44.0 | 84.2 | 143 | 84.2 | 30.6 |
| n (Samp) | 466 | 48 | 466 | 61 | 466 | 25 |
| n (Patient) | 212 | 48 | 212 | 61 | 212 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.11 | 0.952 | 4.11 | 4.70 | 4.11 | 4.04 |
| Average | 7.96 | 3.34 | 7.96 | 6.30 | 7.96 | 9.07 |
| Stdev | 11.4 | 5.42 | 11.4 | 5.09 | 11.4 | 10.6 |
| p(t-test) | | 0.29 | | 0.59 | | 0.79 |
| Min | 0.000120 | 0.000303 | 0.000120 | 0.579 | 0.000120 | 0.000180 |
| Max | 143 | 15.2 | 143 | 17.8 | 143 | 30.6 |
| n (Samp) | 638 | 7 | 638 | 14 | 638 | 8 |
| n (Patient) | 267 | 7 | 267 | 14 | 267 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.74 | 4.80 | 3.74 | 6.27 | 3.74 | 2.54 |
| Average | 6.96 | 8.29 | 6.96 | 11.7 | 6.96 | 5.53 |

EP 3 282 257 A1

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 9.13 | 10.0 | 9.13 | 20.5 | 9.13 | 7.09 |
| p(t-test) | | 0.36 | | 0.0017 | | 0.46 |
| Min | 0.000120 | 0.000180 | 0.000120 | 0.000189 | 0.000120 | 0.000327 |
| Max | 84.2 | 44.0 | 84.2 | 143 | 84.2 | 22.9 |
| n (Samp) | 486 | 45 | 486 | 59 | 486 | 23 |
| n (Patient) | 209 | 45 | 209 | 59 | 209 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.51 | 0.30 | 0.53 | 0.57 | 0.53 | 0.57 | 0.43 | 0.52 | 0.43 |
| SE | 0.044 | 0.11 | 0.046 | 0.040 | 0.079 | 0.041 | 0.061 | 0.10 | 0.063 |
| p | 0.75 | 0.076 | 0.45 | 0.068 | 0.68 | 0.086 | 0.23 | 0.85 | 0.29 |
| nCohort 1 | 466 | 638 | 486 | 466 | 638 | 486 | 466 | 638 | 486 |
| nCohort 2 | 48 | 7 | 45 | 61 | 14 | 59 | 25 | 8 | 23 |
| Cutoff 1 | 1.96 | 0.649 | 1.96 | 2.03 | 2.47 | 1.67 | 0.844 | 1.99 | 0.844 |
| Sens 1 | 71% | 71% | 71% | 70% | 71% | 71% | 72% | 75% | 74% |
| Spec 1 | 33% | 14% | 33% | 34% | 36% | 30% | 19% | 32% | 20% |
| Cutoff 2 | 0.900 | 0.115 | 1.35 | 1.27 | 1.89 | 1.25 | 0.470 | 1.79 | 0.470 |
| Sens 2 | 81% | 86% | 80% | 82% | 86% | 81% | 80% | 88% | 83% |
| Spec 2 | 20% | 9% | 26% | 23% | 30% | 24% | 13% | 29% | 13% |
| Cutoff 3 | 0.0139 | 0.000189 | 0.0139 | 0.564 | 1.45 | 0.353 | 0.000324 | 0.000145 | 0.427 |
| Sens 3 | 94% | 100% | 93% | 90% | 93% | 92% | 96% | 100% | 91% |
| Spec 3 | 6% | 1% | 7% | 15% | 26% | 12% | 2% | 1% | 13% |
| Cutoff 4 | 7.46 | 7.93 | 7.46 | 7.46 | 7.93 | 7.46 | 7.46 | 7.93 | 7.46 |
| Sens 4 | 33% | 14% | 36% | 46% | 29% | 46% | 28% | 38% | 26% |
| Spec 4 | 70% | 70% | 70% | 70% | 70% | 70% | 70% | 70% | 70% |
| Cutoff 5 | 11.0 | 13.2 | 10.8 | 11.0 | 13.2 | 10.8 | 11.0 | 13.2 | 10.8 |
| Sens 5 | 27% | 14% | 29% | 30% | 7% | 31% | 20% | 25% | 17% |
| Spec 5 | 80% | 80% | 80% | 80% | 80% | 80% | 80% | 80% | 80% |
| Cutoff 6 | 18.9 | 20.0 | 18.8 | 18.9 | 20.0 | 18.8 | 18.9 | 20.0 | 18.8 |
| Sens 6 | 10% | 0% | 13% | 16% | 0% | 17% | 8% | 12% | 4% |
| Spec 6 | 90% | 90% | 90% | 90% | 90% | 90% | 90% | 90% | 90% |
| OR Quart 2 | 1.2 | 0 | 1.4 | 0.83 | 4.1 | 0.62 | 0.79 | 3.0 | 1.3 |
| p Value | 0.68 | na | 0.51 | 0.65 | 0.21 | 0.28 | 0.73 | 0.34 | 0.73 |
| 95% CI of | 0.51 | na | 0.55 | 0.36 | 0.45 | 0.26 | 0.21 | 0.31 | 0.33 |
| OR Quart2 | 2.8 | na | 3.3 | 1.9 | 37 | 1.5 | 3.0 | 29 | 4.8 |
| OR Quart 3 | 1.0 | 2.0 | 1.2 | 1.3 | 6.2 | 1.2 | 1.4 | 1.0 | 1.5 |
| p Value | 1.0 | 0.57 | 0.65 | 0.45 | 0.093 | 0.57 | 0.56 | 1.0 | 0.51 |
| 95% CI of | 0.42 | 0.18 | 0.49 | 0.62 | 0.74 | 0.59 | 0.44 | 0.062 | 0.42 |
| OR Quart3 | 2.4 | 23 | 3.1 | 2.9 | 52 | 2.6 | 4.6 | 16 | 5.6 |
| OR Quart 4 | 1.2 | 4.1 | 1.5 | 1.6 | 3.0 | 1.4 | 1.9 | 3.0 | 2.1 |
| p Value | 0.68 | 0.21 | 0.39 | 0.20 | 0.34 | 0.37 | 0.27 | 0.34 | 0.24 |
| 95% CI of | 0.51 | 0.45 | 0.61 | 0.77 | 0.31 | 0.67 | 0.61 | 0.31 | 0.61 |
| OR Quart4 | 2.8 | 37 | 3.6 | 3.4 | 30 | 2.9 | 5.8 | 29 | 7.1 |

52

**Vitronectin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 29.7 | 54.6 | 29.7 | 36.0 | 29.7 | 16.5 |
| Average | 71.8 | 103 | 71.8 | 77.7 | 71.8 | 66.6 |
| Stdev | 137 | 139 | 137 | 125 | 137 | 152 |
| p(t-test) | | 0.14 | | 0.75 | | 0.86 |
| Min | 0.112 | 0.119 | 0.112 | 3.33 | 0.112 | 0.237 |
| Max | 750 | 750 | 750 | 750 | 750 | 750 |
| n (Samp) | 466 | 48 | 466 | 61 | 466 | 25 |
| n (Patient) | 212 | 48 | 212 | 61 | 212 | 25 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 31.1 | 24.5 | 31.1 | 55.7 | 31.1 | 22.5 |
| Average | 73.4 | 40.0 | 73.4 | 63.6 | 73.4 | 211 |
| Stdev | 131 | 45.3 | 131 | 60.3 | 131 | 334 |
| p(t-test) | | 0.50 | | 0.78 | | 0.0042 |
| Min | 0.0795 | 0.119 | 0.0795 | 5.44 | 0.0795 | 4.15 |
| Max | 750 | 133 | 750 | 228 | 750 | 750 |
| n (Samp) | 638 | 7 | 638 | 14 | 638 | 8 |
| n (Patient) | 267 | 7 | 267 | 14 | 267 | 8 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 29.1 | 57.2 | 29.1 | 36.0 | 29.1 | 16.5 |
| Average | 70.4 | 111 | 70.4 | 78.5 | 70.4 | 39.3 |
| Stdev | 137 | 144 | 137 | 127 | 137 | 55.5 |
| p(t-test) | | 0.061 | | 0.67 | | 0.28 |
| Min | 0.112 | 1.17 | 0.112 | 2.03E-5 | 0.112 | 0.237 |
| Max | 750 | 750 | 750 | 750 | 750 | 216 |
| n (Samp) | 486 | 45 | 486 | 59 | 486 | 23 |
| n (Patient) | 209 | 45 | 209 | 59 | 209 | 23 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.43 | 0.63 | 0.58 | 0.58 | 0.59 | 0.41 | 0.51 | 0.40 |
| SE | 0.045 | 0.11 | 0.046 | 0.040 | 0.081 | 0.041 | 0.061 | 0.10 | 0.064 |
| p | 0.016 | 0.53 | 0.0058 | 0.054 | 0.30 | 0.035 | 0.13 | 0.94 | 0.12 |
| nCohort 1 | 466 | 638 | 486 | 466 | 638 | 486 | 466 | 638 | 486 |
| nCohort 2 | 48 | 7 | 45 | 61 | 14 | 59 | 25 | 8 | 23 |
| Cutoff 1 | 23.6 | 12.9 | 23.6 | 23.0 | 26.5 | 23.0 | 9.66 | 16.0 | 8.72 |
| Sens 1 | 71% | 71% | 71% | 70% | 71% | 71% | 72% | 75% | 74% |
| Spec 1 | 42% | 20% | 43% | 41% | 45% | 42% | 16% | 27% | 15% |
| Cutoff 2 | 15.0 | 9.97 | 15.5 | 18.0 | 15.6 | 18.5 | 7.53 | 9.74 | 7.53 |
| Sens 2 | 81% | 86% | 80% | 80% | 86% | 81% | 80% | 88% | 83% |
| Spec 2 | 26% | 15% | 29% | 32% | 26% | 35% | 12% | 15% | 13% |
| Cutoff 3 | 6.27 | 0.112 | 6.27 | 14.1 | 8.72 | 14.1 | 4.09 | 4.09 | 4.94 |
| Sens 3 | 92% | 100% | 91% | 90% | 93% | 92% | 92% | 100% | 91% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 3 | 8% | 0% | 9% | 23% | 13% | 25% | 5% | 4% | 7% |
| Cutoff 4 | 48.2 | 50.6 | 48.3 | 48.2 | 50.6 | 48.3 | 48.2 | 50.6 | 48.3 |
| Sens 4 | 52% | 29% | 53% | 41% | 57% | 37% | 28% | 38% | 26% |
| Spec 4 | 70% | 70% | 70% | 70% | 70% | 70% | 70% | 70% | 70% |
| Cutoff 5 | 68.6 | 74.8 | 67.1 | 68.6 | 74.8 | 67.1 | 68.6 | 74.8 | 67.1 |
| Sens 5 | 46% | 14% | 49% | 28% | 21% | 27% | 16% | 38% | 13% |
| Spec 5 | 80% | 80% | 80% | 80% | 80% | 80% | 80% | 80% | 80% |
| Cutoff 6 | 147 | 168 | 139 | 147 | 168 | 139 | 147 | 168 | 139 |
| Sens 6 | 21% | 0% | 24% | 10% | 7% | 14% | 12% | 25% | 9% |
| Spec 6 | 90% | 90% | 90% | 90% | 90% | 90% | 90% | 90% | 90% |
| OR Quart 2 | 0.99 | 2.0 | 0.99 | 2.6 | 2.0 | 3.5 | 0.79 | 1.5 | 1.0 |
| p Value | 0.99 | 0.57 | 0.99 | 0.031 | 0.42 | 0.0095 | 0.73 | 0.66 | 0.99 |
| 95% CI of | 0.38 | 0.18 | 0.36 | 1.1 | 0.37 | 1.4 | 0.21 | 0.25 | 0.25 |
| OR Quart2 | 2.6 | 23 | 2.7 | 6.1 | 11 | 9.1 | 3.0 | 9.1 | 4.1 |
| OR Quart 3 | 0.88 | 1.0 | 0.86 | 1.8 | 1.0 | 2.9 | 1.2 | 0 | 1.3 |
| p Value | 0.80 | 1.00 | 0.78 | 0.19 | 1.0 | 0.032 | 0.76 | na | 0.73 |
| 95% CI of | 0.33 | 0.062 | 0.30 | 0.74 | 0.14 | 1.1 | 0.36 | na | 0.33 |
| OR Quart3 | 2.4 | 16 | 2.4 | 4.5 | 7.2 | 7.6 | 4.1 | na | 4.8 |
| OR Quart 4 | 2.7 | 3.1 | 3.1 | 2.7 | 3.1 | 3.3 | 2.1 | 1.5 | 2.6 |
| p Value | 0.017 | 0.34 | 0.0095 | 0.021 | 0.17 | 0.015 | 0.19 | 0.66 | 0.11 |
| 95% CI of | 1.2 | 0.31 | 1.3 | 1.2 | 0.61 | 1.3 | 0.70 | 0.25 | 0.81 |
| OR Quart4 | 6.2 | 30 | 7.2 | 6.5 | 15 | 8.5 | 6.4 | 9.1 | 8.7 |

**Interleukin-17F**

| sCr or UO | 0hr prior to AKI stage | | 24hr prior to AKI stage | |
|---|---|---|---|---|
|  | Cohort 1 | Cohort 2 | Cohort 1 | Cohort 2 |
| Median | 0.00406 | 0.00382 | 0.00406 | 0.00530 |
| Average | 0.00500 | 0.00565 | 0.00500 | 0.00780 |
| Stdev | 0.00437 | 0.00481 | 0.00437 | 0.00841 |
| p(t-test) |  | 0.57 |  | 0.034 |
| Min | 2.12E-6 | 9.15E-5 | 2.12E-6 | 9.15E-5 |
| Max | 0.0239 | 0.0199 | 0.0239 | 0.0300 |
| n (Samp) | 92 | 18 | 92 | 20 |
| n (Patient) | 44 | 18 | 44 | 20 |

| sCr only | 0hr prior to AKI stage | | 24hr prior to AKI stage | |
|---|---|---|---|---|
|  | Cohort 1 | Cohort 2 | Cohort 1 | Cohort 2 |
| Median | 0.00406 | 0.00451 | 0.00406 | 0.00238 |
| Average | 0.00533 | 0.00451 | 0.00533 | 0.00811 |
| Stdev | 0.00516 | 0.00274 | 0.00516 | 0.0102 |
| p(t-test) |  | 0.82 |  | 0.37 |
| Min | 2.12E-6 | 0.00257 | 2.12E-6 | 0.00208 |
| Max | 0.0300 | 0.00644 | 0.0300 | 0.0199 |
| n (Samp) | 124 | 2 | 124 | 3 |
| n (Patient) | 60 | 2 | 60 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.00406 | 0.00358 | 0.00406 | 0.00604 |
| Average | 0.00490 | 0.00561 | 0.00490 | 0.00809 |
| Stdev | 0.00427 | 0.00495 | 0.00427 | 0.00854 |
| p(t-test) | | 0.55 | | 0.020 |
| Min | 2.12E-6 | 9.15E-5 | 2.12E-6 | 9.15E-5 |
| Max | 0.0239 | 0.0199 | 0.0239 | 0.0300 |
| n (Samp) | 84 | 17 | 84 | 19 |
| n (Patient) | 40 | 17 | 40 | 19 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.55 | 0.52 | 0.54 | 0.59 | 0.50 | 0.60 |
| SE | 0.076 | 0.21 | 0.078 | 0.073 | 0.17 | 0.075 |
| p | 0.54 | 0.92 | 0.61 | 0.24 | 1.0 | 0.16 |
| nCohort 1 | 92 | 124 | 84 | 92 | 124 | 84 |
| nCohort 2 | 18 | 2 | 17 | 20 | 3 | 19 |
| Cutoff 1 | 0.00282 | 0.00238 | 0.00282 | 0.00232 | 0.00198 | 0.00232 |
| Sens 1 | 72% | 100% | 71% | 80% | 100% | 79% |
| Spec 1 | 37% | 29% | 37% | 30% | 24% | 30% |
| Cutoff 2 | 0.00238 | 0.00238 | 0.00238 | 0.00232 | 0.00198 | 0.00198 |
| Sens 2 | 89% | 100% | 88% | 80% | 100% | 84% |
| Spec 2 | 30% | 29% | 31% | 30% | 24% | 27% |
| Cutoff 3 | 0.00232 | 0.00238 | 0.00232 | 0.000770 | 0.00198 | 9.15E-5 |
| Sens 3 | 94% | 100% | 94% | 90% | 100% | 95% |
| Spec 3 | 30% | 29% | 30% | 13% | 24% | 8% |
| Cutoff 4 | 0.00555 | 0.00579 | 0.00555 | 0.00555 | 0.00579 | 0.00555 |
| Sens 4 | 33% | 50% | 29% | 50% | 33% | 53% |
| Spec 4 | 71% | 70% | 70% | 71% | 70% | 70% |
| Cutoff 5 | 0.00770 | 0.00770 | 0.00770 | 0.00770 | 0.00770 | 0.00770 |
| Sens 5 | 17% | 0% | 18% | 30% | 33% | 32% |
| Spec 5 | 80% | 81% | 81% | 80% | 81% | 81% |
| Cutoff 6 | 0.0109 | 0.0118 | 0.0100 | 0.0109 | 0.0118 | 0.0100 |
| Sens 6 | 17% | 0% | 18% | 25% | 33% | 26% |
| Spec 6 | 90% | 90% | 90% | 90% | 90% | 90% |
| OR Quart 2 | 12 | >1.0 | 11 | 1.8 | 0 | 1.3 |
| p Value | 0.022 | <1.0 | 0.029 | 0.45 | na | 0.73 |
| 95% CI of OR Quart2 | 1.4 110 | >0.060 na | 1.3 99 | 0.39 8.4 | na na | 0.27 6.7 |
| OR Quart 3 | 4.5 | >1.0 | 4.6 | 1.8 | 2.1 | 1.7 |
| p Value | 0.19 | <0.98 | 0.19 | 0.45 | 0.56 | 0.48 |
| 95% CI of OR Quart3 | 0.47 43 | >0.062 na | 0.47 44 | 0.39 8.4 | 0.18 24 | 0.37 8.2 |
| OR Quart 4 | 4.3 | >0 | 4.4 | 2.8 | 0 | 2.7 |
| p Value | 0.20 | <na | 0.20 | 0.17 | na | 0.19 |
| 95% CI of OR Quart4 | 0.45 42 | >na na | 0.45 42 | 0.64 12 | na na | 0.61 12 |

**Interleukin-22**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 1.78E-5 | 1.78E-5 | 1.78E-5 | 1.78E-5 |
| Average | 0.000752 | 0.00799 | 0.000752 | 0.0138 |
| Stdev | 0.00339 | 0.0192 | 0.00339 | 0.0522 |
| p(t-test) | | 9.1E-4 | | 0.018 |
| Min | 4.41E-6 | 4.41E-6 | 4.41E-6 | 4.41E-6 |
| Max | 0.0214 | 0.0725 | 0.0214 | 0.235 |
| n (Samp) | 92 | 18 | 92 | 20 |
| n (Patient) | 44 | 18 | 44 | 20 |

| sCr only | 0hr prior to AKI stage | | 24hr prior to AKI stage | |
|---|---|---|---|---|
| | Cohort 1 | Cohort 2 | Cohort 1 | Cohort 2 |
| Median | 1.78E-5 | 1.11E-5 | 1.78E-5 | 1.78E-5 |
| Average | 0.00347 | 1.11E-5 | 0.00347 | 0.0122 |
| Stdev | 0.0223 | 9.44E-6 | 0.0223 | 0.0212 |
| p(t-test) | | 0.83 | | 0.50 |
| Min | 4.41E-6 | 4.41E-6 | 4.41E-6 | 4.41E-6 |
| Max | 0.235 | 1.78E-5 | 0.235 | 0.0367 |
| n (Samp) | 124 | 2 | 124 | 3 |
| n (Patient) | 60 | 2 | 60 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 1.78E-5 | 1.78E-5 | 1.78E-5 | 1.78E-5 |
| Average | 0.000582 | 0.00845 | 0.000582 | 0.0145 |
| Stdev | 0.00323 | 0.0197 | 0.00323 | 0.0535 |
| p(t-test) | | 6.8E-4 | | 0.018 |
| Min | 4.41E-6 | 4.41E-6 | 4.41E-6 | 4.41E-6 |
| Max | 0.0214 | 0.0725 | 0.0214 | 0.235 |
| n (Samp) | 84 | 17 | 84 | 19 |
| n (Patient) | 40 | 17 | 40 | 19 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.58 | 0.45 | 0.62 | 0.61 | 0.62 | 0.65 |
| SE | 0.076 | 0.21 | 0.078 | 0.073 | 0.18 | 0.074 |
| p | 0.30 | 0.80 | 0.14 | 0.12 | 0.48 | 0.042 |
| nCohort 1 | 92 | 124 | 84 | 92 | 124 | 84 |
| nCohort 2 | 18 | 2 | 17 | 20 | 3 | 19 |
| Cutoff 1 | 0 | 0 | 0 | 0 | 0 | 0 |
| Sens 1 | 100% | 100% | 100% | 100% | 100% | 100% |
| Spec 1 | 0% | 0% | 0% | 0% | 0% | 0% |
| Cutoff 2 | 0 | 0 | 0 | 0 | 0 | 0 |
| Sens 2 | 100% | 100% | 100% | 100% | 100% | 100% |
| Spec 2 | 0% | 0% | 0% | 0% | 0% | 0% |
| Cutoff 3 | 0 | 0 | 0 | 0 | 0 | 0 |
| Sens 3 | 100% | 100% | 100% | 100% | 100% | 100% |

| | 0hr prior to AKI stage | | | 24hr prior to AKI stage | | |
|---|---|---|---|---|---|---|
| | sCr or UO | sCr only | UO only | sCr or UO | sCr only | UO only |
| Spec 3 | 0% | 0% | 0% | 0% | 0% | 0% |
| Cutoff 4 | 1.78E-5 | 1.78E-5 | 1.78E-5 | 1.78E-5 | 1.78E-5 | 1.78E-5 |
| Sens 4 | 22% | 0% | 24% | 30% | 33% | 32% |
| Spec 4 | 93% | 90% | 95% | 93% | 90% | 95% |
| Cutoff 5 | 1.78E-5 | 1.78E-5 | 1.78E-5 | 1.78E-5 | 1.78E-5 | 1.78E-5 |
| Sens 5 | 22% | 0% | 24% | 30% | 33% | 32% |
| Spec 5 | 93% | 90% | 95% | 93% | 90% | 95% |
| Cutoff 6 | 1.78E-5 | 0.000334 | 1.78E-5 | 1.78E-5 | 0.000334 | 1.78E-5 |
| Sens 6 | 22% | 0% | 24% | 30% | 33% | 32% |
| Spec 6 | 93% | 90% | 95% | 93% | 90% | 95% |
| OR Quart 2 | >15 | >1.1 | >12 | >16 | >2.1 | >11 |
| p Value | <0.013 | <0.96 | <0.026 | <0.012 | <0.56 | <0.029 |
| 95% CI of | >1.8 | >0.064 | >1.3 | >1.8 | >0.18 | >1.3 |
| OR Quart2 | na | na | na | na | na | na |
| OR Quart 3 | >4.7 | >1.0 | >6.2 | >4.7 | >0 | >6.0 |
| p Value | <0.18 | <0.98 | <0.11 | <0.18 | <na | <0.12 |
| 95% CI of | >0.49 | >0.062 | >0.67 | >0.49 | >na | >0.64 |
| OR Quart3 | na | na | na | na | na | na |
| OR Quart 4 | >4.5 | >0 | >4.5 | >7.6 | >1.0 | >7.5 |
| p Value | <0.19 | <na | <0.19 | <0.069 | <1.0 | <0.072 |
| 95% CI of | >0.47 | >na | >0.47 | >0.86 | >0.060 | >0.83 |
| OR Quart4 | na | na | na | na | na | na |

**T3**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | nd | nd | 3.40 | 3.25 |
| Average | nd | nd | 4.53 | 3.98 |
| Stdev | nd | nd | 3.85 | 2.74 |
| p(t-test) | nd | nd | | 0.61 |
| Min | nd | nd | 0.000958 | 0.792 |
| Max | nd | nd | 20.4 | 9.76 |
| n (Samp) | nd | nd | 106 | 14 |
| n (Patient) | nd | nd | 83 | 14 |

| sCr only | 0hr prior to AKI stage | | 24hr prior to AKI stage | |
|---|---|---|---|---|
| | Cohort 1 | Cohort 2 | Cohort 1 | Cohort 2 |
| Median | nd | nd | 3.43 | 3.09 |
| Average | nd | nd | 4.51 | 3.57 |
| Stdev | nd | nd | 3.75 | 1.91 |
| p(t-test) | nd | nd | | 0.67 |
| Min | nd | nd | 0.000958 | 1.94 |
| Max | nd | nd | 20.4 | 5.67 |
| n (Samp) | nd | nd | 118 | 3 |
| n (Patient) | nd | nd | 93 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | nd | nd | 3.39 | 2.84 |
| Average | nd | nd | 4.45 | 3.81 |
| Stdev | nd | nd | 3.52 | 2.82 |
| p(t-test) | nd | nd | | 0.53 |
| Min | nd | nd | 0.00599 | 0.792 |
| Max | nd | nd | 19.3 | 9.76 |
| n (Samp) | nd | nd | 90 | 13 |
| n (Patient) | nd | nd | 70 | 13 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | nd | nd | nd | 0.48 | 0.45 | 0.45 |
| SE | nd | nd | nd | 0.083 | 0.17 | 0.088 |
| p | nd | nd | nd | 0.78 | 0.79 | 0.56 |
| nCohort 1 | nd | nd | nd | 106 | 118 | 90 |
| nCohort 2 | nd | nd | nd | 14 | 3 | 13 |
| Cutoff 1 | nd | nd | nd | 2.36 | 1.93 | 1.97 |
| Sens 1 | nd | nd | nd | 71% | 100% | 77% |
| Spec 1 | nd | nd | nd | 32% | 25% | 24% |
| Cutoff 2 | nd | nd | nd | 1.60 | 1.93 | 1.60 |
| Sens 2 | nd | nd | nd | 86% | 100% | 85% |
| Spec 2 | nd | nd | nd | 15% | 25% | 17% |
| Cutoff 3 | nd | nd | nd | 0.862 | 1.93 | 0.862 |
| Sens 3 | nd | nd | nd | 93% | 100% | 92% |
| Spec 3 | nd | nd | nd | 9% | 25% | 9% |
| Cutoff 4 | nd | nd | nd | 5.72 | 5.72 | 5.32 |
| Sens 4 | nd | nd | nd | 21% | 0% | 23% |
| Spec 4 | nd | nd | nd | 71% | 70% | 70% |
| Cutoff 5 | nd | nd | nd | 6.71 | 6.79 | 6.71 |
| Sens 5 | nd | nd | nd | 21% | 0% | 23% |
| Spec 5 | nd | nd | nd | 80% | 81% | 80% |
| Cutoff 6 | nd | nd | nd | 8.14 | 8.37 | 7.57 |
| Sens 6 | nd | nd | nd | 14% | 0% | 15% |
| Spec 6 | nd | nd | nd | 91% | 91% | 90% |
| OR Quart 2 | nd | nd | nd | 1.4 | >1.1 | 1.0 |
| p Value | nd | nd | nd | 0.69 | <0.96 | 1.0 |
| 95% CI of OR Quart2 | nd | nd | nd | 0.28 | >0.064 | 0.18 |
| | nd | nd | nd | 6.8 | na | 5.5 |
| OR Quart 3 | nd | nd | nd | 1.4 | >1.1 | 1.4 |
| p Value | nd | nd | nd | 0.69 | <0.96 | 0.69 |
| 95% CI of OR Quart3 | nd | nd | nd | 0.28 | >0.064 | 0.28 |
| | nd | nd | nd | 6.8 | na | 7.0 |
| OR Quart 4 | nd | nd | nd | 1.0 | >1.1 | 1.0 |
| p Value | nd | nd | nd | 1.0 | <0.96 | 0.96 |
| 95% CI of OR Quart4 | nd | nd | nd | 0.19 | >0.064 | 0.19 |
| | nd | nd | nd | 5.4 | na | 5.7 |

**T4**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | nd | nd | 4.45 | 4.67 |
| Average | nd | nd | 5.38 | 5.73 |
| Stdev | nd | nd | 5.83 | 4.58 |
| p(t-test) | nd | nd | | 0.83 |
| Min | nd | nd | 0.00501 | 0.428 |
| Max | nd | nd | 36.2 | 15.5 |
| n (Samp) | nd | nd | 106 | 14 |
| n (Patient) | nd | nd | 83 | 14 |

| sCr only | 0hr prior to AKI stage | | 24hr prior to AKI stage | |
|---|---|---|---|---|
| | Cohort 1 | Cohort 2 | Cohort 1 | Cohort 2 |
| Median | nd | nd | 4.43 | 4.69 |
| Average | nd | nd | 5.31 | 4.96 |
| Stdev | nd | nd | 5.73 | 3.89 |
| p(t-test) | nd | nd | | 0.92 |
| Min | nd | nd | 0.00501 | 1.20 |
| Max | nd | nd | 36.2 | 8.98 |
| n (Samp) | nd | nd | 118 | 3 |
| n (Patient) | nd | nd | 93 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | nd | nd | 4.47 | 4.01 |
| Average | nd | nd | 5.47 | 5.63 |
| Stdev | nd | nd | 5.71 | 4.55 |
| p(t-test) | nd | nd | | 0.92 |
| Min | nd | nd | 0.00501 | 0.428 |
| Max | nd | nd | 36.2 | 15.5 |
| n (Samp) | nd | nd | 90 | 13 |
| n (Patient) | nd | nd | 70 | 13 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | nd | nd | nd | 0.56 | 0.56 | 0.54 |
| SE | nd | nd | nd | 0.084 | 0.17 | 0.087 |
| p | nd | nd | nd | 0.49 | 0.75 | 0.66 |
| nCohort 1 | nd | nd | nd | 106 | 118 | 90 |
| nCohort 2 | nd | nd | nd | 14 | 3 | 13 |
| Cutoff 1 | nd | nd | nd | 2.01 | 1.19 | 2.01 |
| Sens 1 | nd | nd | nd | 71% | 100% | 77% |
| Spec 1 | nd | nd | nd | 33% | 27% | 33% |
| Cutoff 2 | nd | nd | nd | 1.20 | 1.19 | 1.67 |
| Sens 2 | nd | nd | nd | 86% | 100% | 85% |
| Spec 2 | nd | nd | nd | 27% | 27% | 30% |
| Cutoff 3 | nd | nd | nd | 1.19 | 1.19 | 1.19 |
| Sens 3 | nd | nd | nd | 93% | 100% | 92% |

| | 0hr prior to AKI stage | | | 24hr prior to AKI stage | | |
|---|---|---|---|---|---|---|
| | sCr or UO | sCr only | UO only | sCr or UO | sCr only | UO only |
| Spec 3 | nd | nd | nd | 27% | 27% | 29% |
| Cutoff 4 | nd | nd | nd | 6.52 | 6.47 | 6.89 |
| Sens 4 | nd | nd | nd | 36% | 33% | 31% |
| Spec 4 | nd | nd | nd | 71% | 70% | 70% |
| Cutoff 5 | nd | nd | nd | 8.39 | 8.39 | 8.80 |
| Sens 5 | nd | nd | nd | 29% | 33% | 23% |
| Spec 5 | nd | nd | nd | 80% | 81% | 80% |
| Cutoff 6 | nd | nd | nd | 12.5 | 12.5 | 12.5 |
| Sens 6 | nd | nd | nd | 7% | 0% | 8% |
| Spec 6 | nd | nd | nd | 91% | 91% | 90% |
| OR Quart 2 | nd | nd | nd | 7.2 | >1.0 | 7.2 |
| p Value | nd | nd | nd | 0.076 | <0.98 | 0.078 |
| 95% CI of | nd | nd | nd | 0.82 | >0.062 | 0.80 |
| OR Quart2 | nd | nd | nd | 64 | na | 65 |
| OR Quart 3 | nd | nd | nd | 2.1 | >1.0 | 2.0 |
| p Value | nd | nd | nd | 0.56 | <0.98 | 0.58 |
| 95% CI of | nd | nd | nd | 0.18 | >0.062 | 0.17 |
| OR Quart3 | nd | nd | nd | 24 | na | 24 |
| OR Quart 4 | nd | nd | nd | 5.8 | >1.0 | 4.4 |
| p Value | nd | nd | nd | 0.12 | <1.0 | 0.20 |
| 95% CI of | nd | nd | nd | 0.63 | >0.060 | 0.45 |
| OR Quart4 | nd | nd | nd | 53 | na | 42 |

**Proprotein convertase subtilisin/kexin type 9**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | nd | nd | 470 | 829 |
| Average | nd | nd | 2870 | 1920 |
| Stdev | nd | nd | 10300 | 3050 |
| p(t-test) | nd | nd | | 0.73 |
| Min | nd | nd | 70.6 | 250 |
| Max | nd | nd | 96400 | 11400 |
| n (Samp) | nd | nd | 106 | 14 |
| n (Patient) | nd | nd | 83 | 14 |

| sCr only | 0hr prior to AKI stage | | 24hr prior to AKI stage | |
|---|---|---|---|---|
| | Cohort 1 | Cohort 2 | Cohort 1 | Cohort 2 |
| Median | nd | nd | 470 | 1190 |
| Average | nd | nd | 2760 | 1750 |
| Stdev | nd | nd | 9830 | 1320 |
| p(t-test) | nd | nd | | 0.86 |
| Min | nd | nd | 48.1 | 813 |
| Max | nd | nd | 96400 | 3260 |
| n (Samp) | nd | nd | 118 | 3 |
| n (Patient) | nd | nd | 93 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | nd | nd | 470 | 592 |
| Average | nd | nd | 2630 | 1930 |
| Stdev | nd | nd | 10400 | 3190 |
| p(t-test) | nd | nd | | 0.81 |
| Min | nd | nd | 70.6 | 220 |
| Max | nd | nd | 96400 | 11400 |
| n (Samp) | nd | nd | 90 | 13 |
| n (Patient) | nd | nd | 70 | 13 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | nd | nd | nd | 0.62 | 0.74 | 0.58 |
| SE | nd | nd | nd | 0.084 | 0.17 | 0.088 |
| p | nd | nd | nd | 0.15 | 0.14 | 0.36 |
| nCohort 1 | nd | nd | nd | 106 | 118 | 90 |
| nCohort 2 | nd | nd | nd | 14 | 3 | 13 |
| Cutoff 1 | nd | nd | nd | 497 | 804 | 353 |
| Sens 1 | nd | nd | nd | 71% | 100% | 77% |
| Spec 1 | nd | nd | nd | 52% | 64% | 44% |
| Cutoff 2 | nd | nd | nd | 353 | 804 | 250 |
| Sens 2 | nd | nd | nd | 86% | 100% | 85% |
| Spec 2 | nd | nd | nd | 43% | 64% | 31% |
| Cutoff 3 | nd | nd | nd | 258 | 804 | 247 |
| Sens 3 | nd | nd | nd | 93% | 100% | 92% |
| Spec 3 | nd | nd | nd | 31% | 64% | 31% |
| Cutoff 4 | nd | nd | nd | 1060 | 1040 | 1170 |
| Sens 4 | nd | nd | nd | 29% | 67% | 23% |
| Spec 4 | nd | nd | nd | 71% | 70% | 70% |
| Cutoff 5 | nd | nd | nd | 2060 | 2060 | 2140 |
| Sens 5 | nd | nd | nd | 21% | 33% | 23% |
| Spec 5 | nd | nd | nd | 80% | 81% | 80% |
| Cutoff 6 | nd | nd | nd | 4940 | 5260 | 4770 |
| Sens 6 | nd | nd | nd | 14% | 0% | 15% |
| Spec 6 | nd | nd | nd | 91% | 91% | 90% |
| OR Quart 2 | nd | nd | nd | >6.0 | >0 | 4.4 |
| p Value | nd | nd | nd | <0.11 | <na | 0.20 |
| 95% CI of | nd | nd | nd | >0.66 | >na | 0.45 |
| OR Quart2 | nd | nd | nd | na | na | 42 |
| OR Quart 3 | nd | nd | nd | >7.5 | >2.1 | 5.7 |
| p Value | nd | nd | nd | <0.071 | <0.54 | 0.12 |
| 95% CI of | nd | nd | nd | >0.84 | >0.18 | 0.62 |
| OR Quart3 | nd | nd | nd | na | na | 53 |
| OR Quart 4 | nd | nd | nd | >3.3 | >1.0 | 3.1 |
| p Value | nd | nd | nd | <0.31 | <1.0 | 0.34 |
| 95% CI of | nd | nd | nd | >0.33 | >0.060 | 0.30 |
| OR Quart4 | nd | nd | nd | na | na | 32 |

Table 3: Comparison of marker levels in urine samples collected within 12 hours of reaching stage R from Cohort 1 (patients that reached, but did not progress beyond, RIFLE stage R) and from Cohort 2 (patients that reached RIFLE stage I or F).

**Estradiol**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 10.8 | 7.02 | nd | nd | 11.6 | 6.20 |
| Average | 14.5 | 9.36 | nd | nd | 16.6 | 6.58 |
| Stdev | 13.1 | 8.41 | nd | nd | 14.2 | 1.83 |
| p(t-test) |  | 0.25 | nd | nd |  | 0.14 |
| Min | 1.46 | 4.27 | nd | nd | 1.46 | 4.77 |
| Max | 55.8 | 34.2 | nd | nd | 55.8 | 9.47 |
| n (Samp) | 23 | 11 | nd | nd | 17 | 5 |
| n (Patient) | 23 | 11 | nd | nd | 17 | 5 |

|  | At Enrollment | | |
|---|---|---|---|
|  | sCr or UO | sCr only | UO only |
| AUC | 0.36 | nd | 0.21 |
| SE | 0.11 | nd | 0.13 |
| p | 0.17 | nd | 0.028 |
| nCohort 1 | 23 | nd | 17 |
| nCohort 2 | 11 | nd | 5 |
| Cutoff 1 | 5.56 | nd | 4.77 |
| Sens 1 | 73% | nd | 80% |
| Spec 1 | 22% | nd | 12% |
| Cutoff 2 | 4.77 | nd | 4.77 |
| Sens 2 | 82% | nd | 80% |
| Spec 2 | 17% | nd | 12% |
| Cutoff 3 | 4.27 | nd | 3.57 |
| Sens 3 | 91% | nd | 100% |
| Spec 3 | 17% | nd | 12% |
| Cutoff 4 | 19.9 | nd | 20.2 |
| Sens 4 | 9% | nd | 0% |
| Spec 4 | 74% | nd | 71% |
| Cutoff 5 | 24.6 | nd | 25.9 |
| Sens 5 | 9% | nd | 0% |
| Spec 5 | 83% | nd | 82% |
| Cutoff 6 | 26.7 | nd | 38.9 |
| Sens 6 | 9% | nd | 0% |
| Spec 6 | 91% | nd | 94% |
| OR Quart 2 | 2.7 | nd | >1.5 |
| p Value | 0.46 | nd | <0.79 |
| 95% CI of | 0.19 | nd | >0.071 |
| OR Quart2 | 37 | nd | na |
| OR Quart 3 | 10 | nd | >3.0 |
| p Value | 0.067 | nd | <0.43 |
| 95% CI of | 0.85 | nd | >0.20 |
| OR Quart3 | 120 | nd | na |

| | At Enrollment | | |
|---|---|---|---|
| | sCr or UO | sCr only | UO only |
| OR Quart 4 | 4.8 | nd | >4.0 |
| p Value | 0.22 | nd | <0.33 |
| 95% CI of | 0.38 | nd | >0.25 |
| OR Quart4 | 60 | nd | na |

**Growth/differentiation factor 15**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 37400 | 79100 | nd | nd | 108000 | 11400 |
| Average | 82500 | 71000 | nd | nd | 105000 | 28900 |
| Stdev | 81100 | 69100 | nd | nd | 83100 | 31300 |
| p(t-test) | | 0.69 | nd | nd | | 0.061 |
| Min | 4550 | 5170 | nd | nd | 5720 | 5560 |
| Max | 284000 | 235000 | nd | nd | 284000 | 79100 |
| n (Samp) | 23 | 11 | nd | nd | 17 | 5 |
| n (Patient) | 23 | 11 | nd | nd | 17 | 5 |

| | At Enrollment | | |
|---|---|---|---|
| | sCr or UO | sCr only | UO only |
| AUC | 0.44 | nd | 0.19 |
| SE | 0.11 | nd | 0.13 |
| p | 0.60 | nd | 0.013 |
| nCohort 1 | 23 | nd | 17 |
| nCohort 2 | 11 | nd | 5 |
| Cutoff 1 | 11100 | nd | 5720 |
| Sens 1 | 73% | nd | 80% |
| Spec 1 | 22% | nd | 6% |
| Cutoff 2 | 5720 | nd | 5720 |
| Sens 2 | 82% | nd | 80% |
| Spec 2 | 13% | nd | 6% |
| Cutoff 3 | 5170 | nd | 0 |
| Sens 3 | 91% | nd | 100% |
| Spec 3 | 9% | nd | 0% |
| Cutoff 4 | 115000 | nd | 116000 |
| Sens 4 | 9% | nd | 0% |
| Spec 4 | 74% | nd | 71% |
| Cutoff 5 | 151000 | nd | 172000 |
| Sens 5 | 9% | nd | 0% |
| Spec 5 | 83% | nd | 82% |
| Cutoff 6 | 208000 | nd | 221000 |
| Sens 6 | 9% | nd | 0% |
| Spec 6 | 91% | nd | 94% |
| OR Quart 2 | 13 | nd | >0 |
| p Value | 0.044 | nd | <na |
| 95% CI of | 1.1 | nd | >na |
| OR Quart2 | 170 | nd | na |
| OR Quart 3 | 2.3 | nd | >6.0 |

|  | At Enrollment |  |  |
|---|---|---|---|
|  | sCr or UO | sCr only | UO only |
| p Value | 0.53 | nd | <0.19 |
| 95% CI of | 0.17 | nd | >0.42 |
| OR Quart3 | 31 | nd | na |
| OR Quart 4 | 4.8 | nd | >4.0 |
| p Value | 0.22 | nd | <0.33 |
| 95% CI of | 0.38 | nd | >0.25 |
| OR Quart4 | 60 | nd | na |

**Proprotein convertase subtilisin/kexin type 9**

|  | sCr or UO |  | sCr only |  | UO only |  |
|---|---|---|---|---|---|---|
|  | Cohort 1 | Cohort 2 | Cohort 1 | Cohort 2 | Cohort 1 | Cohort 2 |
| Median | 897 | 521 | nd | nd | 944 | 355 |
| Average | 2310 | 1320 | nd | nd | 2820 | 364 |
| Stdev | 4360 | 1860 | nd | nd | 4990 | 129 |
| p(t-test) |  | 0.48 | nd | nd |  | 0.29 |
| Min | 104 | 133 | nd | nd | 120 | 176 |
| Max | 19200 | 6340 | nd | nd | 19200 | 521 |
| n (Samp) | 23 | 11 | nd | nd | 17 | 5 |
| n (Patient) | 23 | 11 | nd | nd | 17 | 5 |

|  | At Enrollment |  |  |
|---|---|---|---|
|  | sCr or UO | sCr only | UO only |
| AUC | 0.49 | nd | 0.38 |
| SE | 0.11 | nd | 0.15 |
| p | 0.93 | nd | 0.41 |
| nCohort 1 | 23 | nd | 17 |
| nCohort 2 | 11 | nd | 5 |
| Cutoff 1 | 353 | nd | 293 |
| Sens 1 | 73% | nd | 80% |
| Spec 1 | 39% | nd | 35% |
| Cutoff 2 | 293 | nd | 293 |
| Sens 2 | 82% | nd | 80% |
| Spec 2 | 35% | nd | 35% |
| Cutoff 3 | 170 | nd | 170 |
| Sens 3 | 91% | nd | 100% |
| Spec 3 | 22% | nd | 24% |
| Cutoff 4 | 2060 | nd | 2250 |
| Sens 4 | 18% | nd | 0% |
| Spec 4 | 74% | nd | 71% |
| Cutoff 5 | 2510 | nd | 3030 |
| Sens 5 | 18% | nd | 0% |
| Spec 5 | 83% | nd | 82% |
| Cutoff 6 | 4940 | nd | 10700 |
| Sens 6 | 9% | nd | 0% |
| Spec 6 | 91% | nd | 94% |
| OR Quart 2 | 2.1 | nd | >1.5 |
| p Value | 0.49 | nd | <0.79 |

|  | At Enrollment |  |  |
|---|---|---|---|
|  | sCr or UO | sCr only | UO only |
| 95% CI of OR Quart2 | 0.25 | nd | >0.071 |
|  | 18 | nd | na |
| OR Quart 3 | 2.8 | nd | >6.0 |
| p Value | 0.32 | nd | <0.19 |
| 95% CI of OR Quart3 | 0.36 | nd | >0.42 |
|  | 22 | nd | na |
| OR Quart 4 | 1.2 | nd | >1.5 |
| p Value | 0.89 | nd | <0.79 |
| 95% CI of OR Quart4 | 0.12 | nd | >0.071 |
|  | 11 | nd | na |

Table 4: Comparison of the maximum marker levels in urine samples collected from Cohort 1 (patients that did not progress beyond RIFLE stage 0) and the maximum values in urine samples collected from subjects between enrollment and 0, 24 hours, and 48 hours prior to reaching stage F in Cohort 2.

**Toll-like receptor 2**

| sCr or UO | 0hr prior to AKI stage |  | 24hr prior to AKI stage |  | 48hr prior to AKI stage |  |
|---|---|---|---|---|---|---|
|  | Cohort 1 | Cohort 2 | Cohort 1 | Cohort 2 | Cohort 1 | Cohort 2 |
| Median | 0.399 | 0.930 | 0.399 | 0.926 | 0.399 | 0.378 |
| Average | 0.675 | 0.919 | 0.675 | 0.874 | 0.675 | 0.671 |
| Stdev | 0.706 | 0.438 | 0.706 | 0.383 | 0.706 | 0.531 |
| p(t-test) |  | 0.37 |  | 0.46 |  | 0.99 |
| Min | 0.0754 | 0.352 | 0.0754 | 0.352 | 0.0754 | 0.352 |
| Max | 3.06 | 1.52 | 3.06 | 1.29 | 3.06 | 1.28 |
| n (Samp) | 22 | 8 | 22 | 8 | 22 | 3 |
| n (Patient) | 22 | 8 | 22 | 8 | 22 | 3 |

| UO only | 0hr prior to AKI stage |  | 24hr prior to AKI stage |  | 48hr prior to AKI stage |  |
|---|---|---|---|---|---|---|
|  | Cohort 1 | Cohort 2 | Cohort 1 | Cohort 2 | Cohort 1 | Cohort 2 |
| Median | 0.389 | 0.930 | 0.389 | 0.926 | 0.389 | 0.378 |
| Average | 0.693 | 0.919 | 0.693 | 0.874 | 0.693 | 0.671 |
| Stdev | 0.703 | 0.438 | 0.703 | 0.383 | 0.703 | 0.531 |
| p(t-test) |  | 0.40 |  | 0.50 |  | 0.96 |
| Min | 0.0754 | 0.352 | 0.0754 | 0.352 | 0.0754 | 0.352 |
| Max | 3.06 | 1.52 | 3.06 | 1.29 | 3.06 | 1.28 |
| n (Samp) | 22 | 8 | 22 | 8 | 22 | 3 |
| n (Patient) | 22 | 8 | 22 | 8 | 22 | 3 |

|  | 0hr prior to AKI stage |  |  | 24hr prior to AKI stage |  |  | 48hr prior to AKI stage |  |  |
|---|---|---|---|---|---|---|---|---|---|
|  | sCr or UO | sCr only | UO only | sCr or UO | sCr only | UO only | sCr or UO | sCr only | UO only |
| AUC | 0.71 | nd | 0.70 | 0.70 | nd | 0.69 | 0.53 | nd | 0.53 |
| SE | 0.11 | nd | 0.12 | 0.12 | nd | 0.12 | 0.18 | nd | 0.18 |
| p | 0.067 | nd | 0.087 | 0.087 | nd | 0.11 | 0.87 | nd | 0.87 |
| nCohort 1 | 22 | nd | 22 | 22 | nd | 22 | 22 | nd | 22 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 8 | nd | 8 | 8 | nd | 8 | 3 | nd | 3 |
| Cutoff 1 | 0.626 | nd | 0.626 | 0.626 | nd | 0.626 | 0.346 | nd | 0.346 |
| Sens 1 | 75% | nd | 75% | 75% | nd | 75% | 100% | nd | 100% |
| Spec 1 | 73% | nd | 68% | 73% | nd | 68% | 36% | nd | 32% |
| Cutoff 2 | 0.360 | nd | 0.375 | 0.360 | nd | 0.375 | 0.346 | nd | 0.346 |
| Sens 2 | 88% | nd | 88% | 88% | nd | 88% | 100% | nd | 100% |
| Spec 2 | 41% | nd | 45% | 41% | nd | 45% | 36% | nd | 32% |
| Cutoff 3 | 0.346 | nd | 0.346 | 0.346 | nd | 0.346 | 0.346 | nd | 0.346 |
| Sens 3 | 100% | nd | 100% | 100% | nd | 100% | 100% | nd | 100% |
| Spec 3 | 36% | nd | 32% | 36% | nd | 32% | 36% | nd | 32% |
| Cutoff 4 | 0.626 | nd | 0.678 | 0.626 | nd | 0.678 | 0.626 | nd | 0.678 |
| Sens 4 | 75% | nd | 62% | 75% | nd | 62% | 33% | nd | 33% |
| Spec 4 | 73% | nd | 73% | 73% | nd | 73% | 73% | nd | 73% |
| Cutoff 5 | 0.708 | nd | 0.838 | 0.708 | nd | 0.838 | 0.708 | nd | 0.838 |
| Sens 5 | 62% | nd | 50% | 62% | nd | 50% | 33% | nd | 33% |
| Spec 5 | 82% | nd | 82% | 82% | nd | 82% | 82% | nd | 82% |
| Cutoff 6 | 1.48 | nd | 1.48 | 1.48 | nd | 1.48 | 1.48 | nd | 1.48 |
| Sens 6 | 12% | nd | 12% | 0% | nd | 0% | 0% | nd | 0% |
| Spec 6 | 91% | nd | 91% | 91% | nd | 91% | 91% | nd | 91% |
| OR Quart 2 | >2.3 | nd | >2.3 | >2.3 | nd | >2.3 | >3.0 | nd | >3.0 |
| p Value | <0.53 | nd | <0.53 | <0.53 | nd | <0.53 | <0.43 | nd | <0.43 |
| 95% CI of OR Quart2 | >0.17 na | nd | >0.17 na | >0.17 na | nd | >0.17 na | >0.20 na | nd | >0.20 na |
| OR Quart 3 | >2.8 | nd | >2.8 | >2.8 | nd | >2.8 | >0 | nd | >0 |
| p Value | <0.45 | nd | <0.45 | <0.45 | nd | <0.45 | <na | nd | <na |
| 95% CI of OR Quart3 | >0.20 na | nd | >0.20 na | >0.20 na | nd | >0.20 na | >na na | nd | >na na |
| OR Quart 4 | >7.0 | nd | >7.0 | >7.0 | nd | >7.0 | >1.0 | nd | >1.0 |
| p Value | <0.13 | nd | <0.13 | <0.13 | nd | <0.13 | <1.0 | nd | <1.0 |
| 95% CI of OR Quart4 | >0.57 na | nd | >0.57 na | >0.57 na | nd | >0.57 na | >0.050 na | nd | >0.050 na |

**Antithrombin-III**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 131 | 243 | 131 | 236 | 131 | 191 |
| Average | 612 | 834 | 612 | 751 | 612 | 657 |
| Stdev | 1420 | 1510 | 1420 | 1530 | 1420 | 1520 |
| p(t-test) | | 0.50 | | 0.68 | | 0.92 |
| Min | 3.56 | 75.0 | 3.56 | 11.5 | 3.56 | 10.2 |
| Max | 6000 | 5660 | 6000 | 5660 | 6000 | 5660 |
| n (Samp) | 106 | 23 | 106 | 22 | 106 | 13 |
| n (Patient) | 106 | 23 | 106 | 22 | 106 | 13 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 157 | 203 | 157 | 203 | 157 | 142 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 644 | 705 | 644 | 697 | 644 | 761 |
| Stdev | 1360 | 1520 | 1360 | 1520 | 1360 | 1740 |
| p(t-test) | | 0.88 | | 0.89 | | 0.79 |
| Min | 3.56 | 75.0 | 3.56 | 11.5 | 3.56 | 10.2 |
| Max | 6000 | 5660 | 6000 | 5660 | 6000 | 5660 |
| n (Samp) | 217 | 13 | 217 | 13 | 217 | 10 |
| n (Patient) | 217 | 13 | 217 | 13 | 217 | 10 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 148 | 276 | 148 | 268 | 148 | 219 |
| Average | 610 | 1150 | 610 | 1040 | 610 | 992 |
| Stdev | 1360 | 1870 | 1360 | 1940 | 1360 | 2060 |
| p(t-test) | | 0.18 | | 0.30 | | 0.49 |
| Min | 3.56 | 54.7 | 3.56 | 54.7 | 3.56 | 36.0 |
| Max | 6000 | 5660 | 6000 | 5660 | 6000 | 5660 |
| n (Samp) | 117 | 14 | 117 | 13 | 117 | 7 |
| n (Patient) | 117 | 14 | 117 | 13 | 117 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.68 | 0.57 | 0.67 | 0.64 | 0.54 | 0.64 | 0.56 | 0.47 | 0.57 |
| SE | 0.066 | 0.085 | 0.083 | 0.068 | 0.084 | 0.086 | 0.087 | 0.095 | 0.12 |
| p | 0.0075 | 0.41 | 0.038 | 0.036 | 0.62 | 0.097 | 0.49 | 0.77 | 0.57 |
| nCohort 1 | 106 | 217 | 117 | 106 | 217 | 117 | 106 | 217 | 117 |
| nCohort 2 | 23 | 13 | 14 | 22 | 13 | 13 | 13 | 10 | 7 |
| Cutoff 1 | 180 | 97.6 | 202 | 180 | 96.5 | 194 | 96.5 | 96.5 | 189 |
| Sens 1 | 74% | 77% | 71% | 73% | 77% | 77% | 77% | 70% | 71% |
| Spec 1 | 62% | 33% | 62% | 62% | 32% | 61% | 40% | 32% | 58% |
| Cutoff 2 | 114 | 96.5 | 114 | 97.6 | 85.9 | 114 | 85.3 | 85.9 | 53.8 |
| Sens 2 | 83% | 85% | 86% | 82% | 85% | 85% | 85% | 80% | 86% |
| Spec 2 | 43% | 32% | 39% | 41% | 27% | 39% | 33% | 27% | 19% |
| Cutoff 3 | 96.5 | 85.9 | 73.2 | 85.3 | 74.3 | 73.2 | 34.6 | 36.0 | 34.6 |
| Sens 3 | 91% | 92% | 93% | 91% | 92% | 92% | 92% | 90% | 100% |
| Spec 3 | 40% | 27% | 26% | 33% | 22% | 26% | 12% | 8% | 10% |
| Cutoff 4 | 266 | 286 | 281 | 266 | 286 | 281 | 266 | 286 | 281 |
| Sens 4 | 48% | 31% | 50% | 45% | 31% | 46% | 31% | 20% | 29% |
| Spec 4 | 71% | 70% | 70% | 71% | 70% | 70% | 71% | 70% | 70% |
| Cutoff 5 | 374 | 594 | 476 | 374 | 594 | 476 | 374 | 594 | 476 |
| Sens 5 | 30% | 23% | 36% | 27% | 23% | 23% | 23% | 20% | 29% |
| Spec 5 | 80% | 80% | 80% | 80% | 80% | 80% | 80% | 80% | 80% |
| Cutoff 6 | 1260 | 1650 | 1260 | 1260 | 1650 | 1260 | 1260 | 1650 | 1260 |
| Sens 6 | 13% | 8% | 21% | 9% | 8% | 15% | 8% | 10% | 14% |
| Spec 6 | 91% | 90% | 91% | 91% | 90% | 91% | 91% | 90% | 91% |
| OR Quart 2 | 5.7 | 4.1 | 0.47 | 5.7 | 1.5 | 0.47 | 1.5 | 1.5 | 0 |
| p Value | 0.12 | 0.21 | 0.54 | 0.12 | 0.66 | 0.54 | 0.67 | 0.65 | na |
| 95% CI of | 0.63 | 0.45 | 0.040 | 0.63 | 0.24 | 0.040 | 0.23 | 0.25 | na |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart2 | 52 | 38 | 5.4 | 52 | 9.3 | 5.4 | 9.7 | 9.5 | na |
| OR Quart 3 | 12 | 5.4 | 3.3 | 12 | 2.6 | 3.5 | 2.7 | 1.5 | 1.6 |
| p Value | 0.022 | 0.13 | 0.16 | 0.022 | 0.26 | 0.15 | 0.26 | 0.65 | 0.64 |
| 95% CI of | 1.4 | 0.61 | 0.62 | 1.4 | 0.49 | 0.64 | 0.48 | 0.25 | 0.24 |
| OR Quart3 | 100 | 48 | 18 | 100 | 14 | 19 | 15 | 9.5 | 10 |
| OR Quart 4 | 9.9 | 3.1 | 2.7 | 8.7 | 1.5 | 2.1 | 1.5 | 1.0 | 1.0 |
| p Value | 0.036 | 0.34 | 0.26 | 0.050 | 0.66 | 0.42 | 0.67 | 0.99 | 1.0 |
| 95% CI of | 1.2 | 0.31 | 0.48 | 1.0 | 0.24 | 0.35 | 0.23 | 0.14 | 0.13 |
| OR Quart4 | 85 | 30 | 15 | 75 | 9.3 | 12 | 9.7 | 7.5 | 7.6 |

**Extracellular matrix protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.536 | 2.38 | 0.536 | 2.06 | 0.536 | 1.49 |
| Average | 0.998 | 5.58 | 0.998 | 5.48 | 0.998 | 3.29 |
| Stdev | 1.23 | 11.8 | 1.23 | 12.1 | 1.23 | 4.20 |
| p(t-test) | | 1.3E-4 | | 2.5E-4 | | 2.5E-5 |
| Min | 0.0273 | 0.327 | 0.0273 | 0.327 | 0.0273 | 0.327 |
| Max | 7.81 | 57.7 | 7.81 | 57.7 | 7.81 | 14.4 |
| n (Samp) | 106 | 23 | 106 | 22 | 106 | 13 |
| n (Patient) | 106 | 23 | 106 | 22 | 106 | 13 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.851 | 1.69 | 0.851 | 1.49 | 0.851 | 1.38 |
| Average | 2.25 | 3.38 | 2.25 | 3.31 | 2.25 | 3.75 |
| Stdev | 10.9 | 4.13 | 10.9 | 4.16 | 10.9 | 4.71 |
| p(t-test) | | 0.71 | | 0.73 | | 0.66 |
| Min | 0.0143 | 0.327 | 0.0143 | 0.327 | 0.0143 | 0.327 |
| Max | 150 | 14.4 | 150 | 14.4 | 150 | 14.4 |
| n (Samp) | 217 | 13 | 217 | 13 | 217 | 10 |
| n (Patient) | 217 | 13 | 217 | 13 | 217 | 10 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.678 | 2.73 | 0.678 | 2.68 | 0.678 | 2.18 |
| Average | 1.11 | 6.94 | 1.11 | 6.95 | 1.11 | 2.57 |
| Stdev | 1.26 | 14.8 | 1.26 | 15.4 | 1.26 | 3.11 |
| p(t-test) | | 4.1E-5 | | 7.3E-5 | | 0.0087 |
| Min | 0.0273 | 0.327 | 0.0273 | 0.327 | 0.0273 | 0.327 |
| Max | 7.81 | 57.7 | 7.81 | 57.7 | 7.81 | 9.15 |
| n (Samp) | 117 | 14 | 117 | 13 | 117 | 7 |
| n (Patient) | 117 | 14 | 117 | 13 | 117 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.83 | 0.71 | 0.80 | 0.82 | 0.69 | 0.79 | 0.74 | 0.67 | 0.66 |
| SE | 0.055 | 0.083 | 0.073 | 0.057 | 0.083 | 0.077 | 0.082 | 0.096 | 0.12 |
| p | 2.1E-9 | 0.013 | 2.7E-5 | 3.2E-8 | 0.020 | 1.5E-4 | 0.0027 | 0.071 | 0.16 |
| nCohort 1 | 106 | 217 | 117 | 106 | 217 | 117 | 106 | 217 | 117 |
| nCohort 2 | 23 | 13 | 14 | 22 | 13 | 13 | 13 | 10 | 7 |
| Cutoff 1 | 1.27 | 1.10 | 2.15 | 1.27 | 1.10 | 1.27 | 0.678 | 0.721 | 0.451 |
| Sens 1 | 74% | 77% | 71% | 73% | 77% | 77% | 77% | 70% | 71% |
| Spec 1 | 77% | 62% | 82% | 77% | 62% | 74% | 56% | 46% | 39% |
| Cutoff 2 | 1.10 | 0.716 | 0.786 | 1.10 | 0.716 | 0.786 | 0.543 | 0.716 | 0.348 |
| Sens 2 | 83% | 85% | 86% | 82% | 85% | 85% | 85% | 80% | 86% |
| Spec 2 | 75% | 46% | 52% | 75% | 46% | 52% | 51% | 46% | 32% |
| Cutoff 3 | 0.678 | 0.547 | 0.348 | 0.678 | 0.547 | 0.348 | 0.453 | 0.547 | 0.324 |
| Sens 3 | 91% | 92% | 93% | 91% | 92% | 92% | 92% | 90% | 100% |
| Spec 3 | 56% | 40% | 32% | 56% | 40% | 32% | 47% | 40% | 26% |
| Cutoff 4 | 1.03 | 1.60 | 1.21 | 1.03 | 1.60 | 1.21 | 1.03 | 1.60 | 1.21 |
| Sens 4 | 83% | 54% | 79% | 82% | 46% | 77% | 62% | 40% | 57% |
| Spec 4 | 71% | 70% | 70% | 71% | 70% | 70% | 71% | 70% | 70% |
| Cutoff 5 | 1.67 | 2.23 | 2.04 | 1.67 | 2.23 | 2.04 | 1.67 | 2.23 | 2.04 |
| Sens 5 | 65% | 38% | 71% | 59% | 31% | 69% | 46% | 40% | 57% |
| Spec 5 | 80% | 80% | 80% | 80% | 80% | 80% | 80% | 80% | 80% |
| Cutoff 6 | 2.51 | 3.26 | 2.56 | 2.51 | 3.26 | 2.56 | 2.51 | 3.26 | 2.56 |
| Sens 6 | 48% | 23% | 64% | 45% | 23% | 62% | 38% | 30% | 43% |
| Spec 6 | 91% | 90% | 91% | 91% | 90% | 91% | 91% | 90% | 91% |
| OR Quart 2 | >3.3 | 2.0 | 2.0 | >3.3 | 2.0 | 2.0 | >3.2 | >4.2 | >3.3 |
| p Value | <0.31 | 0.58 | 0.58 | <0.31 | 0.58 | 0.58 | <0.32 | <0.20 | <0.31 |
| 95% CI of | >0.33 | 0.18 | 0.17 | >0.33 | 0.18 | 0.17 | >0.32 | >0.46 | >0.33 |
| OR Quart2 | na | 23 | 23 | na | 23 | 23 | na | na | na |
| OR Quart 3 | >9.0 | 5.4 | 0.97 | >11 | 6.6 | 1.0 | >3.2 | >2.0 | >0 |
| p Value | <0.047 | 0.13 | 0.98 | <0.031 | 0.086 | 1.0 | <0.32 | <0.57 | <na |
| 95% CI of | >1.0 | 0.61 | 0.058 | >1.2 | 0.77 | 0.060 | >0.32 | >0.18 | >na |
| OR Quart3 | na | 48 | 16 | na | 57 | 17 | na | na | na |
| OR Quart 4 | >21 | 5.3 | 13 | >17 | 4.1 | 12 | >8.8 | >4.2 | >4.6 |
| p Value | <0.0048 | 0.13 | 0.016 | <0.0091 | 0.21 | 0.024 | <0.049 | <0.20 | <0.18 |
| 95% CI of | >2.5 | 0.60 | 1.6 | >2.0 | 0.45 | 1.4 | >1.0 | >0.46 | >0.48 |
| OR Quart4 | na | 47 | 110 | na | 38 | 98 | na | na | na |

**Coagulation factor XIII A and B chains**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.92 | 8.57 | 5.92 | 8.53 | 5.92 | 7.60 |
| Average | 9.60 | 14.5 | 9.60 | 13.1 | 9.60 | 8.49 |
| Stdev | 13.5 | 14.0 | 13.5 | 13.1 | 13.5 | 8.58 |
| p(t-test) | | 0.12 | | 0.27 | | 0.77 |
| Min | 0.000189 | 0.745 | 0.000189 | 0.579 | 0.000189 | 0.0238 |
| Max | 84.2 | 46.4 | 84.2 | 46.4 | 84.2 | 30.6 |
| n (Samp) | 106 | 23 | 106 | 22 | 106 | 13 |
| n (Patient) | 106 | 23 | 106 | 22 | 106 | 13 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.66 | 7.60 | 6.66 | 7.60 | 6.66 | 6.88 |
| Average | 11.3 | 9.44 | 11.3 | 9.25 | 11.3 | 9.20 |
| Stdev | 15.0 | 8.07 | 15.0 | 8.26 | 15.0 | 9.55 |
| p(t-test) | | 0.65 | | 0.62 | | 0.66 |
| Min | 0.000189 | 2.07 | 0.000189 | 0.579 | 0.000189 | 0.0238 |
| Max | 143 | 30.6 | 143 | 30.6 | 143 | 30.6 |
| n (Samp) | 217 | 13 | 217 | 13 | 217 | 10 |
| n (Patient) | 217 | 13 | 217 | 13 | 217 | 10 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.17 | 9.36 | 6.17 | 8.86 | 6.17 | 8.50 |
| Average | 9.98 | 17.0 | 9.98 | 15.1 | 9.98 | 6.64 |
| Stdev | 13.0 | 16.3 | 13.0 | 15.2 | 13.0 | 4.84 |
| p(t-test) | | 0.066 | | 0.19 | | 0.50 |
| Min | 0.000189 | 0.745 | 0.000189 | 0.745 | 0.000189 | 0.952 |
| Max | 84.2 | 46.4 | 84.2 | 46.4 | 84.2 | 14.5 |
| n (Samp) | 117 | 14 | 117 | 13 | 117 | 7 |
| n (Patient) | 117 | 14 | 117 | 13 | 117 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.65 | 0.51 | 0.65 | 0.62 | 0.50 | 0.63 | 0.51 | 0.46 | 0.47 |
| SE | 0.067 | 0.083 | 0.083 | 0.069 | 0.083 | 0.087 | 0.086 | 0.095 | 0.11 |
| p | 0.026 | 0.88 | 0.073 | 0.074 | 0.97 | 0.15 | 0.86 | 0.69 | 0.82 |
| nCohort 1 | 106 | 217 | 117 | 106 | 217 | 117 | 106 | 217 | 117 |
| nCohort 2 | 23 | 13 | 14 | 22 | 13 | 13 | 13 | 10 | 7 |
| Cutoff 1 | 4.76 | 4.19 | 8.42 | 4.76 | 4.19 | 3.61 | 2.04 | 2.07 | 2.94 |
| Sens 1 | 74% | 77% | 71% | 73% | 77% | 77% | 77% | 70% | 71% |
| Spec 1 | 45% | 32% | 63% | 45% | 32% | 37% | 24% | 18% | 32% |
| Cutoff 2 | 3.61 | 3.04 | 2.94 | 3.61 | 2.07 | 2.94 | 1.77 | 2.04 | 2.04 |
| Sens 2 | 83% | 85% | 86% | 82% | 85% | 85% | 85% | 80% | 86% |
| Spec 2 | 40% | 26% | 32% | 40% | 18% | 32% | 22% | 18% | 22% |
| Cutoff 3 | 2.07 | 2.07 | 2.04 | 2.04 | 2.04 | 2.04 | 0.809 | 1.89 | 0.809 |
| Sens 3 | 91% | 92% | 93% | 91% | 92% | 92% | 92% | 90% | 100% |
| Spec 3 | 24% | 18% | 22% | 24% | 18% | 22% | 13% | 16% | 9% |
| Cutoff 4 | 8.90 | 11.6 | 10.0 | 8.90 | 11.6 | 10.0 | 8.90 | 11.6 | 10.0 |
| Sens 4 | 43% | 31% | 43% | 41% | 31% | 38% | 23% | 30% | 14% |
| Spec 4 | 71% | 70% | 70% | 71% | 70% | 70% | 71% | 70% | 70% |
| Cutoff 5 | 16.0 | 18.5 | 16.3 | 16.0 | 18.5 | 16.3 | 16.0 | 18.5 | 16.3 |
| Sens 5 | 30% | 8% | 36% | 27% | 8% | 31% | 15% | 10% | 0% |
| Spec 5 | 80% | 80% | 80% | 80% | 80% | 80% | 80% | 80% | 80% |
| Cutoff 6 | 20.7 | 24.7 | 20.7 | 20.7 | 24.7 | 20.7 | 20.7 | 24.7 | 20.7 |
| Sens 6 | 26% | 8% | 36% | 23% | 8% | 31% | 8% | 10% | 0% |
| Spec 6 | 91% | 90% | 91% | 91% | 90% | 91% | 91% | 90% | 91% |
| OR Quart 2 | 1.4 | 2.0 | 0.97 | 0.72 | 2.7 | 0.97 | 0.22 | 1.5 | 3.2 |
| p Value | 0.69 | 0.42 | 0.97 | 0.69 | 0.25 | 0.97 | 0.18 | 0.65 | 0.32 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| 95% CI of | 0.28 | 0.36 | 0.13 | 0.15 | 0.50 | 0.13 | 0.023 | 0.25 | 0.32 |
| OR Quart2 | 6.7 | 12 | 7.3 | 3.5 | 14 | 7.3 | 2.1 | 9.5 | 33 |
| OR Quart 3 | 2.7 | 2.6 | 2.1 | 2.0 | 1.5 | 2.1 | 1.2 | 0.49 | 1.0 |
| p Value | 0.18 | 0.26 | 0.42 | 0.33 | 0.65 | 0.40 | 0.76 | 0.57 | 1.0 |
| 95% CI of | 0.63 | 0.49 | 0.35 | 0.51 | 0.25 | 0.36 | 0.30 | 0.043 | 0.060 |
| OR Quart3 | 12 | 14 | 12 | 7.5 | 9.5 | 13 | 5.2 | 5.6 | 17 |
| OR Quart 4 | 3.6 | 0.98 | 3.3 | 2.3 | 1.6 | 2.7 | 0.69 | 2.1 | 2.1 |
| p Value | 0.074 | 0.99 | 0.16 | 0.21 | 0.64 | 0.26 | 0.65 | 0.40 | 0.56 |
| 95% CI of | 0.88 | 0.13 | 0.62 | 0.62 | 0.25 | 0.48 | 0.14 | 0.37 | 0.18 |
| OR Quart4 | 15 | 7.2 | 18 | 8.7 | 9.7 | 15 | 3.4 | 12 | 24 |

**Vitronectin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 35.2 | 87.9 | 35.2 | 87.4 | 35.2 | 56.6 |
| Average | 90.6 | 144 | 90.6 | 131 | 90.6 | 136 |
| Stdev | 148 | 175 | 148 | 173 | 148 | 220 |
| p(t-test) |  | 0.13 |  | 0.26 |  | 0.33 |
| Min | 2.17 | 3.33 | 2.17 | 3.33 | 2.17 | 0.119 |
| Max | 750 | 750 | 750 | 750 | 750 | 750 |
| n (Samp) | 106 | 23 | 106 | 22 | 106 | 13 |
| n (Patient) | 106 | 23 | 106 | 22 | 106 | 13 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 45.6 | 86.8 | 45.6 | 86.8 | 45.6 | 71.7 |
| Average | 122 | 149 | 122 | 148 | 122 | 165 |
| Stdev | 181 | 214 | 181 | 214 | 181 | 244 |
| p(t-test) |  | 0.61 |  | 0.61 |  | 0.47 |
| Min | 1.96 | 8.89 | 1.96 | 6.79 | 1.96 | 6.79 |
| Max | 750 | 750 | 750 | 750 | 750 | 750 |
| n (Samp) | 217 | 13 | 217 | 13 | 217 | 10 |
| n (Patient) | 217 | 13 | 217 | 13 | 217 | 10 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 36.5 | 141 | 36.5 | 114 | 36.5 | 101 |
| Average | 87.8 | 160 | 87.8 | 139 | 87.8 | 139 |
| Stdev | 145 | 139 | 145 | 132 | 145 | 171 |
| p(t-test) |  | 0.077 |  | 0.23 |  | 0.37 |
| Min | 2.17 | 3.33 | 2.17 | 3.33 | 2.17 | 0.119 |
| Max | 750 | 462 | 750 | 462 | 750 | 462 |
| n (Samp) | 117 | 14 | 117 | 13 | 117 | 7 |
| n (Patient) | 117 | 14 | 117 | 13 | 117 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.59 | 0.66 | 0.63 | 0.59 | 0.64 | 0.55 | 0.56 | 0.55 |
| SE | 0.067 | 0.085 | 0.083 | 0.069 | 0.085 | 0.087 | 0.087 | 0.096 | 0.12 |
| p | 0.036 | 0.28 | 0.056 | 0.069 | 0.29 | 0.12 | 0.56 | 0.55 | 0.65 |
| nCohort 1 | 106 | 217 | 117 | 106 | 217 | 117 | 106 | 217 | 117 |
| nCohort 2 | 23 | 13 | 14 | 22 | 13 | 13 | 13 | 10 | 7 |
| Cutoff 1 | 48.3 | 49.7 | 50.5 | 48.3 | 49.7 | 24.6 | 23.0 | 49.7 | 23.8 |
| Sens 1 | 74% | 77% | 71% | 73% | 77% | 77% | 77% | 70% | 71% |
| Spec 1 | 65% | 54% | 65% | 65% | 54% | 34% | 29% | 54% | 32% |
| Cutoff 2 | 13.8 | 26.5 | 13.8 | 13.8 | 26.5 | 13.8 | 6.72 | 24.1 | 2.83 |
| Sens 2 | 83% | 85% | 86% | 82% | 85% | 85% | 85% | 80% | 86% |
| Spec 2 | 11% | 29% | 9% | 11% | 29% | 9% | 5% | 22% | 2% |
| Cutoff 3 | 8.89 | 9.92 | 8.64 | 8.64 | 8.64 | 8.64 | 2.83 | 23.0 | 0 |
| Sens 3 | 91% | 92% | 93% | 91% | 92% | 92% | 92% | 90% | 100% |
| Spec 3 | 7% | 6% | 5% | 7% | 5% | 5% | 2% | 20% | 0% |
| Cutoff 4 | 59.7 | 91.0 | 64.8 | 59.7 | 91.0 | 64.8 | 59.7 | 91.0 | 64.8 |
| Sens 4 | 61% | 38% | 64% | 59% | 38% | 62% | 46% | 30% | 57% |
| Spec 4 | 71% | 70% | 70% | 71% | 70% | 70% | 71% | 70% | 70% |
| Cutoff 5 | 96.7 | 148 | 96.7 | 96.7 | 148 | 96.7 | 96.7 | 148 | 96.7 |
| Sens 5 | 48% | 15% | 64% | 45% | 15% | 62% | 31% | 20% | 57% |
| Spec 5 | 80% | 80% | 80% | 80% | 80% | 80% | 80% | 80% | 80% |
| Cutoff 6 | 273 | 430 | 230 | 273 | 430 | 230 | 273 | 430 | 230 |
| Sens 6 | 13% | 15% | 36% | 9% | 15% | 23% | 15% | 20% | 29% |
| Spec 6 | 91% | 90% | 91% | 91% | 90% | 91% | 91% | 90% | 91% |
| OR Quart 2 | 0.17 | 0.48 | 0.30 | 0.17 | 0.48 | 0.30 | 0.62 | 0 | 0.48 |
| p Value | 0.12 | 0.56 | 0.31 | 0.12 | 0.56 | 0.31 | 0.61 | na | 0.56 |
| 95% CI of OR Quart2 | 0.019 | 0.043 | 0.030 | 0.019 | 0.043 | 0.030 | 0.096 | na | 0.042 |
| | 1.6 | 5.5 | 3.1 | 1.6 | 5.5 | 3.1 | 4.0 | na | 5.6 |
| OR Quart 3 | 1.2 | 3.8 | 0.30 | 1.2 | 3.8 | 0.31 | 0.62 | 1.7 | 0 |
| p Value | 0.74 | 0.10 | 0.31 | 0.74 | 0.10 | 0.32 | 0.61 | 0.48 | na |
| 95% CI of OR Quart3 | 0.34 | 0.76 | 0.030 | 0.34 | 0.76 | 0.031 | 0.096 | 0.39 | na |
| | 4.6 | 19 | 3.1 | 4.6 | 19 | 3.2 | 4.0 | 7.5 | na |
| OR Quart 4 | 2.7 | 1.5 | 3.6 | 2.5 | 1.5 | 3.1 | 2.2 | 0.64 | 2.1 |
| p Value | 0.10 | 0.66 | 0.074 | 0.15 | 0.66 | 0.12 | 0.31 | 0.64 | 0.40 |
| 95% CI of OR Quart4 | 0.82 | 0.24 | 0.88 | 0.73 | 0.24 | 0.74 | 0.49 | 0.10 | 0.36 |
| | 8.9 | 9.3 | 15 | 8.2 | 9.3 | 13 | 9.6 | 4.0 | 13 |

**Estradiol**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.69 | 9.74 | 6.69 | 9.74 | 6.69 | 18.2 |
| Average | 11.2 | 16.3 | 11.2 | 16.3 | 11.2 | 19.0 |
| Stdev | 18.3 | 11.1 | 18.3 | 11.1 | 18.3 | 14.8 |
| p(t-test) | | 0.48 | | 0.48 | | 0.47 |
| Min | 0.143 | 4.59 | 0.143 | 4.59 | 0.143 | 4.59 |
| Max | 128 | 34.2 | 128 | 34.2 | 128 | 34.2 |
| n (Samp) | 50 | 7 | 50 | 7 | 50 | 3 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 50 | 7 | 50 | 7 | 50 | 3 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 8.10 | 9.48 | 8.10 | 9.48 | nd | nd |
| Average | 12.3 | 13.0 | 12.3 | 13.0 | nd | nd |
| Stdev | 16.0 | 10.5 | 16.0 | 10.5 | nd | nd |
| p(t-test) | | 0.93 | | 0.93 | nd | nd |
| Min | 0.143 | 4.59 | 0.143 | 4.59 | nd | nd |
| Max | 128 | 28.3 | 128 | 28.3 | nd | nd |
| n (Samp) | 81 | 4 | 81 | 4 | nd | nd |
| n (Patient) | 81 | 4 | 81 | 4 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 6.61 | 14.0 | 6.61 | 14.0 | 6.61 | 18.2 |
| Average | 8.23 | 16.7 | 8.23 | 16.7 | 8.23 | 19.0 |
| Stdev | 6.60 | 13.0 | 6.60 | 13.0 | 6.60 | 14.8 |
| p(t-test) | | 0.030 | | 0.030 | | 0.016 |
| Min | 0.143 | 4.59 | 0.143 | 4.59 | 0.143 | 4.59 |
| Max | 31.6 | 34.2 | 31.6 | 34.2 | 31.6 | 34.2 |
| n (Samp) | 42 | 4 | 42 | 4 | 42 | 3 |
| n (Patient) | 42 | 4 | 42 | 4 | 42 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.73 | 0.58 | 0.73 | 0.73 | 0.58 | 0.73 | 0.72 | nd | 0.74 |
| SE | 0.11 | 0.15 | 0.15 | 0.11 | 0.15 | 0.15 | 0.17 | nd | 0.17 |
| p | 0.042 | 0.60 | 0.12 | 0.042 | 0.60 | 0.12 | 0.20 | nd | 0.16 |
| nCohort 1 | 50 | 81 | 42 | 50 | 81 | 42 | 50 | nd | 42 |
| nCohort 2 | 7 | 4 | 4 | 7 | 4 | 4 | 3 | nd | 3 |
| Cutoff 1 | 9.38 | 9.25 | 9.24 | 9.38 | 9.25 | 9.24 | 4.21 | nd | 3.62 |
| Sens 1 | 71% | 75% | 75% | 71% | 75% | 75% | 100% | nd | 100% |
| Spec 1 | 68% | 57% | 71% | 68% | 57% | 71% | 30% | nd | 29% |
| Cutoff 2 | 9.24 | 4.21 | 3.62 | 9.24 | 4.21 | 3.62 | 4.21 | nd | 3.62 |
| Sens 2 | 86% | 100% | 100% | 86% | 100% | 100% | 100% | nd | 100% |
| Spec 2 | 66% | 22% | 29% | 66% | 22% | 29% | 30% | nd | 29% |
| Cutoff 3 | 4.21 | 4.21 | 3.62 | 4.21 | 4.21 | 3.62 | 4.21 | nd | 3.62 |
| Sens 3 | 100% | 100% | 100% | 100% | 100% | 100% | 100% | nd | 100% |
| Spec 3 | 30% | 22% | 29% | 30% | 22% | 29% | 30% | nd | 29% |
| Cutoff 4 | 9.96 | 11.5 | 9.24 | 9.96 | 11.5 | 9.24 | 9.96 | nd | 9.24 |
| Sens 4 | 43% | 25% | 75% | 43% | 25% | 75% | 67% | nd | 67% |
| Spec 4 | 70% | 70% | 71% | 70% | 70% | 71% | 70% | nd | 71% |
| Cutoff 5 | 12.9 | 14.6 | 11.2 | 12.9 | 14.6 | 11.2 | 12.9 | nd | 11.2 |
| Sens 5 | 43% | 25% | 50% | 43% | 25% | 50% | 67% | nd | 67% |
| Spec 5 | 80% | 80% | 81% | 80% | 80% | 81% | 80% | nd | 81% |
| Cutoff 6 | 18.8 | 24.6 | 14.4 | 18.8 | 24.6 | 14.4 | 18.8 | nd | 14.4 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 6 | 29% | 25% | 50% | 29% | 25% | 50% | 33% | nd | 67% |
| Spec 6 | 90% | 90% | 90% | 90% | 90% | 90% | 90% | nd | 90% |
| OR Quart 2 | >1.1 | 0 | >1.0 | >1.1 | 0 | >1.0 | >1.1 | nd | >1.1 |
| p Value | <0.96 | na | <1.0 | <0.96 | na | <1.0 | <0.96 | nd | <0.95 |
| 95% CI of | >0.061 | na | >0.055 | >0.061 | na | >0.055 | >0.061 | nd | >0.060 |
| OR Quart2 | na | na | na | na | na | na | na | nd | na |
| OR Quart 3 | >3.8 | 2.1 | >1.1 | >3.8 | 2.1 | >1.1 | >0 | nd | >0 |
| p Value | <0.27 | 0.56 | <0.95 | <0.27 | 0.56 | <0.95 | <na | nd | <na |
| 95% CI of | >0.35 | 0.18 | >0.060 | >0.35 | 0.18 | >0.060 | >na | nd | >na |
| OR Quart3 | na | 25 | na | na | 25 | na | na | nd | na |
| OR Quart 4 | >3.5 | 0.95 | >2.2 | >3.5 | 0.95 | >2.2 | >2.2 | nd | >2.2 |
| p Value | <0.30 | 0.97 | <0.54 | <0.30 | 0.97 | <0.54 | <0.55 | nd | <0.54 |
| 95% CI of | >0.32 | 0.056 | >0.17 | >0.32 | 0.056 | >0.17 | >0.17 | nd | >0.17 |
| OR Quart4 | na | 16 | na | na | 16 | na | na | nd | na |

**Progesterone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 22.9 | 39.6 | 22.9 | 39.6 | 22.9 | 53.1 |
| Average | 31.4 | 39.5 | 31.4 | 39.5 | 31.4 | 43.9 |
| Stdev | 27.4 | 24.0 | 27.4 | 24.0 | 27.4 | 20.1 |
| p(t-test) | | 0.46 | | 0.46 | | 0.44 |
| Min | 2.91 | 7.44 | 2.91 | 7.44 | 2.91 | 20.9 |
| Max | 152 | 75.2 | 152 | 75.2 | 152 | 57.8 |
| n (Samp) | 50 | 7 | 50 | 7 | 50 | 3 |
| n (Patient) | 50 | 7 | 50 | 7 | 50 | 3 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 28.1 | 31.1 | 28.1 | 31.1 | nd | nd |
| Average | 39.5 | 31.9 | 39.5 | 31.9 | nd | nd |
| Stdev | 37.0 | 21.7 | 37.0 | 21.7 | nd | nd |
| p(t-test) | | 0.69 | | 0.69 | nd | nd |
| Min | 2.91 | 7.44 | 2.91 | 7.44 | nd | nd |
| Max | 228 | 57.8 | 228 | 57.8 | nd | nd |
| n (Samp) | 81 | 4 | 81 | 4 | nd | nd |
| n (Patient) | 81 | 4 | 81 | 4 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 21.1 | 55.5 | 21.1 | 55.5 | 21.1 | 53.1 |
| Average | 32.2 | 51.7 | 32.2 | 51.7 | 32.2 | 43.9 |
| Stdev | 32.9 | 22.7 | 32.9 | 22.7 | 32.9 | 20.1 |
| p(t-test) | | 0.25 | | 0.25 | | 0.55 |
| Min | 2.91 | 20.9 | 2.91 | 20.9 | 2.91 | 20.9 |
| Max | 152 | 75.2 | 152 | 75.2 | 152 | 57.8 |
| n (Samp) | 42 | 4 | 42 | 4 | 42 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 42 | 4 | 42 | 4 | 42 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.62 | 0.48 | 0.77 | 0.62 | 0.48 | 0.77 | 0.69 | nd | 0.72 |
| SE | 0.12 | 0.15 | 0.14 | 0.12 | 0.15 | 0.14 | 0.18 | nd | 0.17 |
| p | 0.32 | 0.87 | 0.062 | 0.32 | 0.87 | 0.062 | 0.29 | nd | 0.20 |
| nCohort 1 | 50 | 81 | 42 | 50 | 81 | 42 | 50 | nd | 42 |
| nCohort 2 | 7 | 4 | 4 | 7 | 4 | 4 | 3 | nd | 3 |
| Cutoff 1 | 21.1 | 21.1 | 51.6 | 21.1 | 21.1 | 51.6 | 19.3 | nd | 19.3 |
| Sens 1 | 71% | 75% | 75% | 71% | 75% | 75% | 100% | nd | 100% |
| Spec 1 | 48% | 40% | 83% | 48% | 40% | 83% | 44% | nd | 48% |
| Cutoff 2 | 19.3 | 7.23 | 19.3 | 19.3 | 7.23 | 19.3 | 19.3 | nd | 19.3 |
| Sens 2 | 86% | 100% | 100% | 86% | 100% | 100% | 100% | nd | 100% |
| Spec 2 | 44% | 9% | 48% | 44% | 9% | 48% | 44% | nd | 48% |
| Cutoff 3 | 7.23 | 7.23 | 19.3 | 7.23 | 7.23 | 19.3 | 19.3 | nd | 19.3 |
| Sens 3 | 100% | 100% | 100% | 100% | 100% | 100% | 100% | nd | 100% |
| Spec 3 | 10% | 9% | 48% | 10% | 9% | 48% | 44% | nd | 48% |
| Cutoff 4 | 31.6 | 43.9 | 30.0 | 31.6 | 43.9 | 30.0 | 31.6 | nd | 30.0 |
| Sens 4 | 57% | 25% | 75% | 57% | 25% | 75% | 67% | nd | 67% |
| Spec 4 | 70% | 70% | 71% | 70% | 70% | 71% | 70% | nd | 71% |
| Cutoff 5 | 51.6 | 60.2 | 40.1 | 51.6 | 60.2 | 40.1 | 51.6 | nd | 40.1 |
| Sens 5 | 43% | 0% | 75% | 43% | 0% | 75% | 67% | nd | 67% |
| Spec 5 | 80% | 80% | 81% | 80% | 80% | 81% | 80% | nd | 81% |
| Cutoff 6 | 65.3 | 79.5 | 68.1 | 65.3 | 79.5 | 68.1 | 65.3 | nd | 68.1 |
| Sens 6 | 14% | 0% | 25% | 14% | 0% | 25% | 0% | nd | 0% |
| Spec 6 | 90% | 90% | 90% | 90% | 90% | 90% | 90% | nd | 90% |
| OR Quart 2 | 2.2 | 1.0 | >1.0 | 2.2 | 1.0 | >1.0 | >1.1 | nd | >1.1 |
| p Value | 0.55 | 0.97 | <1.0 | 0.55 | 0.97 | <1.0 | <0.96 | nd | <0.95 |
| 95% CI of | 0.17 | 0.061 | >0.055 | 0.17 | 0.061 | >0.055 | >0.061 | nd | >0.060 |
| OR Quart2 | 27 | 18 | na | 27 | 18 | na | na | nd | na |
| OR Quart 3 | 1.0 | 1.0 | >0 | 1.0 | 1.0 | >0 | >0 | nd | >0 |
| p Value | 1.0 | 0.97 | <na | 1.0 | 0.97 | <na | <na | nd | <na |
| 95% CI of | 0.056 | 0.061 | >na | 0.056 | 0.061 | >na | >na | nd | >na |
| OR Quart3 | 18 | 18 | na | 18 | 18 | na | na | nd | na |
| OR Quart 4 | 3.2 | 1.0 | >3.7 | 3.2 | 1.0 | >3.7 | >2.2 | nd | >2.2 |
| p Value | 0.33 | 0.97 | <0.29 | 0.33 | 0.97 | <0.29 | <0.55 | nd | <0.54 |
| 95% CI of | 0.30 | 0.061 | >0.32 | 0.30 | 0.061 | >0.32 | >0.17 | nd | >0.17 |
| OR Quart4 | 36 | 18 | na | 36 | 18 | na | na | nd | na |

T3

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.38 | 3.41 | 3.38 | 3.41 | 3.38 | 3.41 |
| Average | 4.72 | 3.91 | 4.72 | 3.91 | 4.72 | 3.29 |
| Stdev | 4.52 | 2.20 | 4.52 | 2.20 | 4.52 | 2.45 |
| p(t-test) | | 0.65 | | 0.65 | | 0.59 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.000958 | 0.792 | 0.000958 | 0.792 | 0.000958 | 0.792 |
| Max | 20.4 | 6.79 | 20.4 | 6.79 | 20.4 | 5.68 |
| n (Samp) | 50 | 7 | 50 | 7 | 50 | 3 |
| n (Patient) | 50 | 7 | 50 | 7 | 50 | 3 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.06 | 2.51 | 4.06 | 2.51 | nd | nd |
| Average | 5.11 | 2.87 | 5.11 | 2.87 | nd | nd |
| Stdev | 4.20 | 2.09 | 4.20 | 2.09 | nd | nd |
| p(t-test) | | 0.29 | | 0.29 | nd | nd |
| Min | 0.000958 | 0.792 | 0.000958 | 0.792 | nd | nd |
| Max | 20.4 | 5.67 | 20.4 | 5.67 | nd | nd |
| n (Samp) | 81 | 4 | 81 | 4 | nd | nd |
| n (Patient) | 81 | 4 | 81 | 4 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 3.33 | 4.54 | 3.33 | 4.54 | 3.33 | 3.41 |
| Average | 4.28 | 4.17 | 4.28 | 4.17 | 4.28 | 3.29 |
| Stdev | 3.80 | 2.65 | 3.80 | 2.65 | 3.80 | 2.45 |
| p(t-test) | | 0.95 | | 0.95 | | 0.66 |
| Min | 0.00599 | 0.792 | 0.00599 | 0.792 | 0.00599 | 0.792 |
| Max | 19.3 | 6.79 | 19.3 | 6.79 | 19.3 | 5.68 |
| n (Samp) | 42 | 4 | 42 | 4 | 42 | 3 |
| n (Patient) | 42 | 4 | 42 | 4 | 42 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.32 | 0.55 | 0.50 | 0.32 | 0.55 | 0.43 | nd | 0.45 |
| SE | 0.12 | 0.15 | 0.16 | 0.12 | 0.15 | 0.16 | 0.18 | nd | 0.18 |
| p | 0.98 | 0.24 | 0.73 | 0.98 | 0.24 | 0.73 | 0.71 | nd | 0.79 |
| nCohort 1 | 50 | 81 | 42 | 50 | 81 | 42 | 50 | nd | 42 |
| nCohort 2 | 7 | 4 | 4 | 7 | 4 | 4 | 3 | nd | 3 |
| Cutoff 1 | 2.96 | 1.93 | 3.35 | 2.96 | 1.93 | 3.35 | 0.746 | nd | 0.746 |
| Sens 1 | 71% | 75% | 75% | 71% | 75% | 75% | 100% | nd | 100% |
| Spec 1 | 42% | 22% | 52% | 42% | 22% | 52% | 8% | nd | 7% |
| Cutoff 2 | 1.93 | 0.746 | 0.746 | 1.93 | 0.746 | 0.746 | 0.746 | nd | 0.746 |
| Sens 2 | 86% | 100% | 100% | 86% | 100% | 100% | 100% | nd | 100% |
| Spec 2 | 26% | 7% | 7% | 26% | 7% | 7% | 8% | nd | 7% |
| Cutoff 3 | 0.746 | 0.746 | 0.746 | 0.746 | 0.746 | 0.746 | 0.746 | nd | 0.746 |
| Sens 3 | 100% | 100% | 100% | 100% | 100% | 100% | 100% | nd | 100% |
| Spec 3 | 8% | 7% | 7% | 8% | 7% | 7% | 8% | nd | 7% |
| Cutoff 4 | 5.16 | 6.56 | 4.65 | 5.16 | 6.56 | 4.65 | 5.16 | nd | 4.65 |
| Sens 4 | 43% | 0% | 50% | 43% | 0% | 50% | 33% | nd | 33% |
| Spec 4 | 70% | 70% | 71% | 70% | 70% | 71% | 70% | nd | 71% |
| Cutoff 5 | 5.89 | 7.09 | 5.80 | 5.89 | 7.09 | 5.80 | 5.89 | nd | 5.80 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 5 | 14% | 0% | 25% | 14% | 0% | 25% | 0% | nd | 0% |
| Spec 5 | 80% | 80% | 81% | 80% | 80% | 81% | 80% | nd | 81% |
| Cutoff 6 | 7.12 | 9.76 | 7.09 | 7.12 | 9.76 | 7.09 | 7.12 | nd | 7.09 |
| Sens 6 | 0% | 0% | 0% | 0% | 0% | 0% | 0% | nd | 0% |
| Spec 6 | 90% | 90% | 90% | 90% | 90% | 90% | 90% | nd | 90% |
| OR Quart 2 | 2.2 | >1.1 | 0 | 2.2 | >1.1 | 0 | >2.5 | nd | 1.1 |
| p Value | 0.55 | <0.95 | na | 0.55 | <0.95 | na | <0.47 | nd | 0.95 |
| 95% CI of OR Quart2 | 0.17 27 | >0.064 na | na na | 0.17 27 | >0.064 na | na na | >0.20 na | nd nd | 0.060 20 |
| OR Quart 3 | 3.5 | >1.1 | 1.0 | 3.5 | >1.1 | 1.0 | >0 | nd | 0 |
| p Value | 0.30 | <0.95 | 1.0 | 0.30 | <0.95 | 1.0 | <na | nd | na |
| 95% CI of OR Quart3 | 0.32 39 | >0.064 na | 0.055 18 | 0.32 39 | >0.064 na | 0.055 18 | >na na | nd nd | na na |
| OR Quart 4 | 0.93 | >2.3 | 2.0 | 0.93 | >2.3 | 2.0 | >1.2 | nd | 1.1 |
| p Value | 0.96 | <0.51 | 0.59 | 0.96 | <0.51 | 0.59 | <0.92 | nd | 0.95 |
| 95% CI of OR Quart4 | 0.053 16 | >0.19 na | 0.16 26 | 0.053 16 | >0.19 na | 0.16 26 | >0.066 na | nd nd | 0.060 20 |

**T4**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.61 | 8.98 | 4.61 | 8.98 | 4.61 | 10.1 |
| Average | 5.53 | 7.92 | 5.53 | 7.92 | 5.53 | 9.58 |
| Stdev | 5.47 | 5.20 | 5.47 | 5.20 | 5.47 | 6.40 |
| p(t-test) | | 0.28 | | 0.28 | | 0.22 |
| Min | 0.00501 | 1.20 | 0.00501 | 1.20 | 0.00501 | 2.96 |
| Max | 28.5 | 15.7 | 28.5 | 15.7 | 28.5 | 15.7 |
| n (Samp) | 50 | 7 | 50 | 7 | 50 | 3 |
| n (Patient) | 50 | 7 | 50 | 7 | 50 | 3 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 5.32 | 3.83 | 5.32 | 3.83 | nd | nd |
| Average | 6.53 | 4.46 | 6.53 | 4.46 | nd | nd |
| Stdev | 6.33 | 3.33 | 6.33 | 3.33 | nd | nd |
| p(t-test) | | 0.52 | | 0.52 | nd | nd |
| Min | 0.00501 | 1.20 | 0.00501 | 1.20 | nd | nd |
| Max | 36.2 | 8.98 | 36.2 | 8.98 | nd | nd |
| n (Samp) | 81 | 4 | 81 | 4 | nd | nd |
| n (Patient) | 81 | 4 | 81 | 4 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.43 | 10.9 | 4.43 | 10.9 | 4.43 | 10.1 |
| Average | 5.17 | 10.1 | 5.17 | 10.1 | 5.17 | 9.58 |
| Stdev | 4.67 | 5.34 | 4.67 | 5.34 | 4.67 | 6.40 |
| p(t-test) | | 0.050 | | 0.050 | | 0.13 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.00501 | 2.96 | 0.00501 | 2.96 | 0.00501 | 2.96 |
| Max | 16.8 | 15.7 | 16.8 | 15.7 | 16.8 | 15.7 |
| n (Samp) | 42 | 4 | 42 | 4 | 42 | 3 |
| n (Patient) | 42 | 4 | 42 | 4 | 42 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.66 | 0.43 | 0.78 | 0.66 | 0.43 | 0.78 | 0.73 | nd | 0.74 |
| SE | 0.12 | 0.15 | 0.14 | 0.12 | 0.15 | 0.14 | 0.17 | nd | 0.17 |
| p | 0.17 | 0.66 | 0.048 | 0.17 | 0.66 | 0.048 | 0.18 | nd | 0.16 |
| nCohort 1 | 50 | 81 | 42 | 50 | 81 | 42 | 50 | nd | 42 |
| nCohort 2 | 7 | 4 | 4 | 7 | 4 | 4 | 3 | nd | 3 |
| Cutoff 1 | 4.51 | 2.67 | 9.47 | 4.51 | 2.67 | 9.47 | 2.67 | nd | 2.67 |
| Sens 1 | 71% | 75% | 75% | 71% | 75% | 75% | 100% | nd | 100% |
| Spec 1 | 50% | 31% | 86% | 50% | 31% | 86% | 36% | nd | 38% |
| Cutoff 2 | 2.67 | 1.07 | 2.67 | 2.67 | 1.07 | 2.67 | 2.67 | nd | 2.67 |
| Sens 2 | 86% | 100% | 100% | 86% | 100% | 100% | 100% | nd | 100% |
| Spec 2 | 36% | 21% | 38% | 36% | 21% | 38% | 36% | nd | 38% |
| Cutoff 3 | 1.07 | 1.07 | 2.67 | 1.07 | 1.07 | 2.67 | 2.67 | nd | 2.67 |
| Sens 3 | 100% | 100% | 100% | 100% | 100% | 100% | 100% | nd | 100% |
| Spec 3 | 24% | 21% | 38% | 24% | 21% | 38% | 36% | nd | 38% |
| Cutoff 4 | 6.88 | 8.06 | 7.20 | 6.88 | 8.06 | 7.20 | 6.88 | nd | 7.20 |
| Sens 4 | 57% | 25% | 75% | 57% | 25% | 75% | 67% | nd | 67% |
| Spec 4 | 70% | 70% | 71% | 70% | 70% | 71% | 70% | nd | 71% |
| Cutoff 5 | 8.68 | 9.47 | 8.85 | 8.68 | 9.47 | 8.85 | 8.68 | nd | 8.85 |
| Sens 5 | 57% | 0% | 75% | 57% | 0% | 75% | 67% | nd | 67% |
| Spec 5 | 80% | 80% | 81% | 80% | 80% | 81% | 80% | nd | 81% |
| Cutoff 6 | 11.0 | 13.5 | 11.0 | 11.0 | 13.5 | 11.0 | 11.0 | nd | 11.0 |
| Sens 6 | 29% | 0% | 50% | 29% | 0% | 50% | 33% | nd | 33% |
| Spec 6 | 90% | 90% | 90% | 90% | 90% | 90% | 90% | nd | 90% |
| OR Quart 2 | 2.2 | 0 | >1.0 | 2.2 | 0 | >1.0 | >1.1 | nd | >1.1 |
| p Value | 0.55 | na | <1.0 | 0.55 | na | <1.0 | <0.96 | nd | <0.95 |
| 95% CI of OR Quart2 | 0.17 27 | na na | >0.055 na | 0.17 27 | na na | >0.055 na | >0.061 na | nd nd | >0.060 na |
| OR Quart 3 | 0 | 2.2 | >0 | 0 | 2.2 | >0 | >0 | nd | >0 |
| p Value | na | 0.53 | <na | na | 0.53 | <na | <na | nd | <na |
| 95% CI of OR Quart3 | na na | 0.19 26 | >na na | na na | 0.19 26 | >na na | >na na | nd nd | >na na |
| OR Quart 4 | 4.7 | 1.0 | >3.7 | 4.7 | 1.0 | >3.7 | >2.2 | nd | >2.2 |
| p Value | 0.19 | 0.97 | <0.29 | 0.19 | 0.97 | <0.29 | <0.55 | nd | <0.54 |
| 95% CI of OR Quart4 | 0.46 49 | 0.061 18 | >0.32 na | 0.46 49 | 0.061 18 | >0.32 na | >0.17 na | nd nd | >0.17 na |

**Growth/differentiation factor 15**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 26900 | 38600 | 26900 | 38600 | 26900 | 38600 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 71300 | 51100 | 71300 | 51100 | 71300 | 50900 |
| Stdev | 131000 | 35000 | 131000 | 35000 | 131000 | 52900 |
| p(t-test) | | 0.69 | | 0.69 | | 0.79 |
| Min | 641 | 5180 | 641 | 5180 | 641 | 5180 |
| Max | 810000 | 109000 | 810000 | 109000 | 810000 | 109000 |
| n (Samp) | 50 | 7 | 50 | 7 | 50 | 3 |
| n (Patient) | 50 | 7 | 50 | 7 | 50 | 3 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 43100 | 48500 | 43100 | 48500 | nd | nd |
| Average | 83700 | 45500 | 83700 | 45500 | nd | nd |
| Stdev | 117000 | 32700 | 117000 | 32700 | nd | nd |
| p(t-test) | | 0.52 | | 0.52 | nd | nd |
| Min | 641 | 5180 | 641 | 5180 | nd | nd |
| Max | 810000 | 79700 | 810000 | 79700 | nd | nd |
| n (Samp) | 81 | 4 | 81 | 4 | nd | nd |
| n (Patient) | 81 | 4 | 81 | 4 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 20300 | 33400 | 20300 | 33400 | 20300 | 38600 |
| Average | 53100 | 45200 | 53100 | 45200 | 53100 | 50900 |
| Stdev | 80600 | 44700 | 80600 | 44700 | 80600 | 52900 |
| p(t-test) | | 0.85 | | 0.85 | | 0.96 |
| Min | 641 | 5180 | 641 | 5180 | 641 | 5180 |
| Max | 326000 | 109000 | 326000 | 109000 | 326000 | 109000 |
| n (Samp) | 42 | 4 | 42 | 4 | 42 | 3 |
| n (Patient) | 42 | 4 | 42 | 4 | 42 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.59 | 0.45 | 0.55 | 0.59 | 0.45 | 0.55 | 0.51 | nd | 0.56 |
| SE | 0.12 | 0.15 | 0.16 | 0.12 | 0.15 | 0.16 | 0.17 | nd | 0.18 |
| p | 0.45 | 0.76 | 0.73 | 0.45 | 0.76 | 0.73 | 0.97 | nd | 0.76 |
| nCohort 1 | 50 | 81 | 42 | 50 | 81 | 42 | 50 | nd | 42 |
| nCohort 2 | 7 | 4 | 4 | 7 | 4 | 4 | 3 | nd | 3 |
| Cutoff 1 | 33700 | 33700 | 24100 | 33700 | 33700 | 24100 | 5070 | nd | 5170 |
| Sens 1 | 71% | 75% | 75% | 71% | 75% | 75% | 100% | nd | 100% |
| Spec 1 | 58% | 46% | 55% | 58% | 46% | 55% | 10% | nd | 14% |
| Cutoff 2 | 24100 | 5070 | 5170 | 24100 | 5070 | 5170 | 5070 | nd | 5170 |
| Sens 2 | 86% | 100% | 100% | 86% | 100% | 100% | 100% | nd | 100% |
| Spec 2 | 50% | 7% | 14% | 50% | 7% | 14% | 10% | nd | 14% |
| Cutoff 3 | 5070 | 5070 | 5170 | 5070 | 5070 | 5170 | 5070 | nd | 5170 |
| Sens 3 | 100% | 100% | 100% | 100% | 100% | 100% | 100% | nd | 100% |
| Spec 3 | 10% | 7% | 14% | 10% | 7% | 14% | 10% | nd | 14% |
| Cutoff 4 | 51600 | 96200 | 46200 | 51600 | 96200 | 46200 | 51600 | nd | 46200 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 4 | 43% | 0% | 25% | 43% | 0% | 25% | 33% | nd | 33% |
| Spec 4 | 70% | 70% | 71% | 70% | 70% | 71% | 70% | nd | 71% |
| Cutoff 5 | 82100 | 124000 | 60400 | 82100 | 124000 | 60400 | 82100 | nd | 60400 |
| Sens 5 | 14% | 0% | 25% | 14% | 0% | 25% | 33% | nd | 33% |
| Spec 5 | 80% | 80% | 81% | 80% | 80% | 81% | 80% | nd | 81% |
| Cutoff 6 | 205000 | 218000 | 205000 | 205000 | 218000 | 205000 | 205000 | nd | 205000 |
| Sens 6 | 0% | 0% | 0% | 0% | 0% | 0% | 0% | nd | 0% |
| Spec 6 | 90% | 90% | 90% | 90% | 90% | 90% | 90% | nd | 90% |
| OR Quart 2 | 1.0 | >2.3 | 0 | 1.0 | >2.3 | 0 | 0 | nd | 0 |
| p Value | 1.0 | <0.51 | na | 1.0 | <0.51 | na | na | nd | na |
| 95% CI of | 0.056 | >0.19 | na | 0.056 | >0.19 | na | na | nd | na |
| OR Quart2 | 18 | na | na | 18 | na | na | na | nd | na |
| OR Quart 3 | 2.2 | >1.1 | 2.2 | 2.2 | >1.1 | 2.2 | 1.0 | nd | 1.0 |
| p Value | 0.55 | <0.95 | 0.54 | 0.55 | <0.95 | 0.54 | 1.0 | nd | 1.0 |
| 95% CI of | 0.17 | >0.064 | 0.17 | 0.17 | >0.064 | 0.17 | 0.056 | nd | 0.055 |
| OR Quart3 | 27 | na | 29 | 27 | na | 29 | 18 | nd | 18 |
| OR Quart 4 | 3.2 | >1.1 | 0.91 | 3.2 | >1.1 | 0.91 | 0.92 | nd | 0.91 |
| p Value | 0.33 | <0.95 | 0.95 | 0.33 | <0.95 | 0.95 | 0.96 | nd | 0.95 |
| 95% CI of | 0.30 | >0.064 | 0.050 | 0.30 | >0.064 | 0.050 | 0.052 | nd | 0.050 |
| OR Quart4 | 36 | na | 17 | 36 | na | 17 | 16 | nd | 17 |

**Proprotein convertase subtilisin/kexin type 9**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 406 | 920 | 406 | 920 | 406 | 920 |
| Average | 3910 | 1530 | 3910 | 1530 | 3910 | 1530 |
| Stdev | 14600 | 1140 | 14600 | 1140 | 14600 | 1350 |
| p(t-test) | | 0.67 | | 0.67 | | 0.78 |
| Min | 95.1 | 592 | 95.1 | 592 | 95.1 | 592 |
| Max | 96400 | 3260 | 96400 | 3260 | 96400 | 3080 |
| n (Samp) | 50 | 7 | 50 | 7 | 50 | 3 |
| n (Patient) | 50 | 7 | 50 | 7 | 50 | 3 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 461 | 1050 | 461 | 1050 | nd | nd |
| Average | 3410 | 1550 | 3410 | 1550 | nd | nd |
| Stdev | 11700 | 1150 | 11700 | 1150 | nd | nd |
| p(t-test) | | 0.75 | | 0.75 | nd | nd |
| Min | 48.1 | 813 | 48.1 | 813 | nd | nd |
| Max | 96400 | 3260 | 96400 | 3260 | nd | nd |
| n (Samp) | 81 | 4 | 81 | 4 | nd | nd |
| n (Patient) | 81 | 4 | 81 | 4 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 486 | 882 | 486 | 882 | 486 | 920 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 3360 | 1360 | 3360 | 1360 | 3360 | 1530 |
| Stdev | 14800 | 1160 | 14800 | 1160 | 14800 | 1350 |
| p(t-test) | | 0.79 | | 0.79 | | 0.83 |
| Min | 95.1 | 592 | 95.1 | 592 | 95.1 | 592 |
| Max | 96400 | 3080 | 96400 | 3080 | 96400 | 3080 |
| n (Samp) | 42 | 4 | 42 | 4 | 42 | 3 |
| n (Patient) | 42 | 4 | 42 | 4 | 42 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.73 | 0.70 | 0.75 | 0.73 | 0.70 | 0.74 | nd | 0.71 |
| SE | 0.11 | 0.15 | 0.15 | 0.11 | 0.15 | 0.15 | 0.17 | nd | 0.17 |
| p | 0.022 | 0.13 | 0.20 | 0.022 | 0.13 | 0.20 | 0.16 | nd | 0.22 |
| nCohort 1 | 50 | 81 | 42 | 50 | 81 | 42 | 50 | nd | 42 |
| nCohort 2 | 7 | 4 | 4 | 7 | 4 | 4 | 3 | nd | 3 |
| Cutoff 1 | 813 | 844 | 745 | 813 | 844 | 745 | 580 | nd | 580 |
| Sens 1 | 71% | 75% | 75% | 71% | 75% | 75% | 100% | nd | 100% |
| Spec 1 | 70% | 67% | 64% | 70% | 67% | 64% | 62% | nd | 57% |
| Cutoff 2 | 804 | 804 | 580 | 804 | 804 | 580 | 580 | nd | 580 |
| Sens 2 | 86% | 100% | 100% | 86% | 100% | 100% | 100% | nd | 100% |
| Spec 2 | 70% | 65% | 57% | 70% | 65% | 57% | 62% | nd | 57% |
| Cutoff 3 | 580 | 804 | 580 | 580 | 804 | 580 | 580 | nd | 580 |
| Sens 3 | 100% | 100% | 100% | 100% | 100% | 100% | 100% | nd | 100% |
| Spec 3 | 62% | 65% | 57% | 62% | 65% | 57% | 62% | nd | 57% |
| Cutoff 4 | 804 | 1010 | 1060 | 804 | 1010 | 1060 | 804 | nd | 1060 |
| Sens 4 | 86% | 50% | 25% | 86% | 50% | 25% | 67% | nd | 33% |
| Spec 4 | 70% | 70% | 71% | 70% | 70% | 71% | 70% | nd | 71% |
| Cutoff 5 | 1340 | 1950 | 1360 | 1340 | 1950 | 1360 | 1340 | nd | 1360 |
| Sens 5 | 29% | 25% | 25% | 29% | 25% | 25% | 33% | nd | 33% |
| Spec 5 | 80% | 80% | 81% | 80% | 80% | 81% | 80% | nd | 81% |
| Cutoff 6 | 2850 | 6690 | 2850 | 2850 | 6690 | 2850 | 2850 | nd | 2850 |
| Sens 6 | 29% | 0% | 25% | 29% | 0% | 25% | 33% | nd | 33% |
| Spec 6 | 90% | 90% | 90% | 90% | 90% | 90% | 90% | nd | 90% |
| OR Quart 2 | >0 | >0 | >0 | >0 | >0 | >0 | >0 | nd | >0 |
| p Value | <na | <na | <na | <na | <na | <na | <na | nd | <na |
| 95% CI of OR Quart2 | >na na | >na na | >na na | >na na | >na na | >na na | >na na | nd | >na na |
| OR Quart 3 | >5.6 | >3.5 | >4.1 | >5.6 | >3.5 | >4.1 | >2.4 | nd | >2.4 |
| p Value | <0.15 | <0.30 | <0.25 | <0.15 | <0.30 | <0.25 | <0.51 | nd | <0.49 |
| 95% CI of OR Quart3 | >0.54 na | >0.33 na | >0.36 na | >0.54 na | >0.33 na | >0.36 na | >0.19 na | nd | >0.19 na |
| OR Quart 4 | >3.5 | >1.0 | >1.0 | >3.5 | >1.0 | >1.0 | >1.0 | nd | >1.0 |
| p Value | <0.30 | <1.0 | <1.0 | <0.30 | <1.0 | <1.0 | <1.0 | nd | <1.0 |
| 95% CI of OR Quart4 | >0.32 na | >0.059 na | >0.055 na | >0.32 na | >0.059 na | >0.055 na | >0.056 na | nd | >0.055 na |

Table 5: Comparison of marker levels in EDTA samples collected from Cohort 1 (patients that did not progress beyond RIFLE stage 0) and in EDTA samples collected from subjects at 0, 24 hours, and 48 hours prior to reaching stage R, I or F in Cohort 2.

**Stanniocalcin-1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0607 | 0.112 | 0.0607 | 0.0730 | 0.0607 | 0.0406 |
| Average | 0.513 | 0.119 | 0.513 | 0.100 | 0.513 | 0.0584 |
| Stdev | 1.36 | 0.115 | 1.36 | 0.0764 | 1.36 | 0.0379 |
| p(t-test) | | 0.19 | | 0.21 | | 0.46 |
| Min | 0.00673 | 0.000985 | 0.00673 | 0.0150 | 0.00673 | 0.0257 |
| Max | 6.00 | 0.509 | 6.00 | 0.283 | 6.00 | 0.101 |
| n (Samp) | 50 | 21 | 50 | 18 | 50 | 5 |
| n (Patient) | 25 | 21 | 25 | 18 | 25 | 5 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0533 | 0.123 | 0.0533 | 0.138 | 0.0533 | 0.0983 |
| Average | 0.310 | 0.101 | 0.310 | 0.137 | 0.310 | 0.0766 |
| Stdev | 0.990 | 0.0527 | 0.990 | 0.0670 | 0.990 | 0.0411 |
| p(t-test) | | 0.67 | | 0.65 | | 0.60 |
| Min | 0.000985 | 0.0237 | 0.000985 | 0.0441 | 0.000985 | 0.0265 |
| Max | 6.00 | 0.136 | 6.00 | 0.238 | 6.00 | 0.119 |
| n (Samp) | 99 | 4 | 99 | 7 | 99 | 5 |
| n (Patient) | 49 | 4 | 49 | 7 | 49 | 5 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.0701 | 0.127 | 0.0701 | 0.109 | 0.0701 | 0.0983 |
| Average | 0.547 | 0.202 | 0.547 | 0.198 | 0.547 | 0.122 |
| Stdev | 1.36 | 0.366 | 1.36 | 0.295 | 1.36 | 0.104 |
| p(t-test) | | 0.27 | | 0.27 | | 0.41 |
| Min | 0.00830 | 0.000985 | 0.00830 | 0.0150 | 0.00830 | 0.0257 |
| Max | 6.00 | 1.68 | 6.00 | 1.26 | 6.00 | 0.301 |
| n (Samp) | 51 | 20 | 51 | 19 | 51 | 7 |
| n (Patient) | 26 | 20 | 26 | 19 | 26 | 7 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.52 | 0.59 | 0.53 | 0.52 | 0.69 | 0.54 | 0.38 | 0.52 | 0.48 |
| SE | 0.076 | 0.15 | 0.077 | 0.080 | 0.11 | 0.079 | 0.14 | 0.13 | 0.12 |
| p | 0.75 | 0.55 | 0.72 | 0.82 | 0.097 | 0.64 | 0.38 | 0.90 | 0.90 |
| nCohort 1 | 50 | 99 | 51 | 50 | 99 | 51 | 50 | 99 | 51 |
| nCohort 2 | 21 | 4 | 20 | 18 | 7 | 19 | 5 | 5 | 7 |
| Cutoff 1 | 0.0369 | 0.109 | 0.0376 | 0.0441 | 0.109 | 0.0459 | 0.0263 | 0.0388 | 0.0388 |
| Sens 1 | 71% | 75% | 70% | 72% | 71% | 74% | 80% | 80% | 71% |
| Spec 1 | 28% | 69% | 25% | 32% | 69% | 31% | 18% | 37% | 27% |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Cutoff 2 | 0.0263 | 0.0228 | 0.0361 | 0.0354 | 0.0706 | 0.0354 | 0.0263 | 0.0388 | 0.0263 |
| Sens 2 | 81% | 100% | 80% | 83% | 86% | 84% | 80% | 80% | 86% |
| Spec 2 | 18% | 20% | 25% | 26% | 60% | 22% | 18% | 37% | 14% |
| Cutoff 3 | 0.0182 | 0.0228 | 0.0182 | 0.0289 | 0.0414 | 0.0289 | 0.0227 | 0.0263 | 0.0227 |
| Sens 3 | 90% | 100% | 90% | 94% | 100% | 95% | 100% | 100% | 100% |
| Spec 3 | 12% | 20% | 8% | 20% | 39% | 16% | 16% | 23% | 12% |
| Cutoff 4 | 0.121 | 0.121 | 0.128 | 0.121 | 0.121 | 0.128 | 0.121 | 0.121 | 0.128 |
| Sens 4 | 48% | 50% | 50% | 28% | 57% | 37% | 0% | 0% | 43% |
| Spec 4 | 70% | 71% | 71% | 70% | 71% | 71% | 70% | 71% | 71% |
| Cutoff 5 | 0.208 | 0.203 | 0.208 | 0.208 | 0.203 | 0.208 | 0.208 | 0.203 | 0.208 |
| Sens 5 | 14% | 0% | 20% | 11% | 14% | 26% | 0% | 0% | 14% |
| Spec 5 | 80% | 81% | 80% | 80% | 81% | 80% | 80% | 81% | 80% |
| Cutoff 6 | 0.592 | 0.406 | 1.30 | 0.592 | 0.406 | 1.30 | 0.592 | 0.406 | 1.30 |
| Sens 6 | 0% | 0% | 5% | 0% | 0% | 0% | 0% | 0% | 0% |
| Spec 6 | 90% | 91% | 90% | 90% | 91% | 90% | 90% | 91% | 90% |
| OR Quart 2 | 0.48 | 0 | 0.93 | 1.0 | >1.0 | 1.2 | >2.3 | 1.0 | 1.1 |
| p Value | 0.37 | na | 0.93 | 1.0 | <1.0 | 0.77 | <0.51 | 1.0 | 0.94 |
| 95% CI of | 0.095 | na | 0.19 | 0.20 | >0.059 | 0.27 | >0.19 | 0.059 | 0.13 |
| OR Quart2 | 2.4 | na | 4.5 | 4.9 | na | 5.7 | na | 17 | 8.9 |
| OR Quart 3 | 1.5 | 3.1 | 2.1 | 1.8 | >3.4 | 1.4 | >1.1 | 3.3 | 0.46 |
| p Value | 0.56 | 0.34 | 0.33 | 0.45 | <0.30 | 0.70 | <0.96 | 0.32 | 0.55 |
| 95% CI of | 0.37 | 0.30 | 0.48 | 0.40 | >0.33 | 0.29 | >0.061 | 0.32 | 0.037 |
| OR Quart3 | 6.3 | 32 | 9.0 | 7.9 | na | 6.3 | na | 34 | 5.7 |
| OR Quart 4 | 1.2 | 0 | 1.2 | 1.0 | >3.2 | 1.2 | >2.5 | 0 | 1.1 |
| p Value | 0.80 | na | 0.77 | 1.0 | <0.32 | 0.77 | <0.47 | na | 0.94 |
| 95% CI of | 0.29 | na | 0.27 | 0.20 | >0.32 | 0.27 | >0.20 | na | 0.13 |
| OR Quart4 | 5.0 | na | 5.7 | 4.9 | na | 5.7 | na | na | 8.9 |

**Extracellular matrix protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1530 | 1440 | 1530 | 1350 | 1530 | 1400 |
| Average | 1610 | 1540 | 1610 | 1490 | 1610 | 1490 |
| Stdev | 462 | 374 | 462 | 478 | 462 | 437 |
| p(t-test) | | 0.63 | | 0.29 | | 0.45 |
| Min | 779 | 1020 | 779 | 846 | 779 | 1110 |
| Max | 3060 | 2350 | 3060 | 2630 | 3060 | 2570 |
| n (Samp) | 54 | 13 | 54 | 23 | 54 | 9 |
| n (Patient) | 53 | 13 | 53 | 23 | 53 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1490 | 1570 | 1490 | 1930 | 1490 | 2100 |
| Average | 1550 | 1570 | 1550 | 1930 | 1550 | 2030 |
| Stdev | 449 | 462 | 449 | 982 | 449 | 568 |
| p(t-test) | | 0.96 | | 0.24 | | 0.070 |
| Min | 535 | 1240 | 535 | 1240 | 535 | 1440 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Max | 3060 | 1890 | 3060 | 2630 | 3060 | 2570 |
| n (Samp) | 110 | 2 | 110 | 2 | 110 | 3 |
| n (Patient) | 92 | 2 | 92 | 2 | 92 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1490 | 1490 | 1490 | 1450 | 1490 | 1400 |
| Average | 1570 | 1550 | 1570 | 1520 | 1570 | 1490 |
| Stdev | 466 | 368 | 466 | 478 | 466 | 436 |
| p(t-test) | | 0.91 | | 0.67 | | 0.64 |
| Min | 779 | 1020 | 779 | 846 | 779 | 1110 |
| Max | 3060 | 2350 | 3060 | 2630 | 3060 | 2570 |
| n (Samp) | 48 | 12 | 48 | 25 | 48 | 9 |
| n (Patient) | 44 | 12 | 44 | 25 | 44 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | 0.52 | 0.52 | 0.40 | 0.60 | 0.45 | 0.36 | 0.76 | 0.41 |
| SE | 0.091 | 0.21 | 0.095 | 0.072 | 0.21 | 0.072 | 0.11 | 0.16 | 0.11 |
| p | 0.70 | 0.94 | 0.83 | 0.15 | 0.63 | 0.51 | 0.19 | 0.11 | 0.40 |
| nCohort 1 | 54 | 110 | 48 | 54 | 110 | 48 | 54 | 110 | 48 |
| nCohort 2 | 13 | 2 | 12 | 23 | 2 | 25 | 9 | 3 | 9 |
| Cutoff 1 | 1210 | 1230 | 1400 | 1110 | 1230 | 1170 | 1200 | 1430 | 1200 |
| Sens 1 | 77% | 100% | 75% | 83% | 100% | 72% | 78% | 100% | 78% |
| Spec 1 | 17% | 23% | 35% | 17% | 23% | 17% | 17% | 44% | 17% |
| Cutoff 2 | 1180 | 1230 | 1180 | 1110 | 1230 | 1110 | 1110 | 1430 | 1110 |
| Sens 2 | 85% | 100% | 83% | 83% | 100% | 84% | 89% | 100% | 89% |
| Spec 2 | 17% | 23% | 17% | 17% | 23% | 17% | 17% | 44% | 17% |
| Cutoff 3 | 1110 | 1230 | 1110 | 900 | 1230 | 900 | 1080 | 1430 | 1080 |
| Sens 3 | 92% | 100% | 92% | 91% | 100% | 92% | 100% | 100% | 100% |
| Spec 3 | 17% | 23% | 17% | 4% | 23% | 4% | 13% | 44% | 15% |
| Cutoff 4 | 1710 | 1690 | 1660 | 1710 | 1690 | 1660 | 1710 | 1690 | 1660 |
| Sens 4 | 31% | 50% | 33% | 26% | 50% | 32% | 11% | 67% | 11% |
| Spec 4 | 70% | 70% | 71% | 70% | 70% | 71% | 70% | 70% | 71% |
| Cutoff 5 | 1950 | 1890 | 1890 | 1950 | 1890 | 1890 | 1950 | 1890 | 1890 |
| Sens 5 | 15% | 50% | 17% | 17% | 50% | 20% | 11% | 67% | 11% |
| Spec 5 | 83% | 80% | 81% | 83% | 80% | 81% | 83% | 80% | 81% |
| Cutoff 6 | 2270 | 2240 | 2230 | 2270 | 2240 | 2230 | 2270 | 2240 | 2230 |
| Sens 6 | 8% | 0% | 8% | 9% | 50% | 8% | 11% | 33% | 11% |
| Spec 6 | 93% | 90% | 92% | 93% | 90% | 92% | 93% | 90% | 92% |
| OR Quart 2 | 0.43 | 0 | 1.0 | 0.44 | 0 | 0.66 | 1.0 | >1.0 | 3.8 |
| p Value | 0.38 | na | 1.0 | 0.30 | na | 0.56 | 1.0 | <0.98 | 0.27 |
| 95% CI of | 0.068 | na | 0.17 | 0.092 | na | 0.16 | 0.057 | >0.062 | 0.35 |
| OR Quart2 | 2.8 | na | 6.0 | 2.1 | na | 2.6 | 18 | na | 42 |
| OR Quart 3 | 0.70 | 0 | 1.0 | 0.83 | 0 | 0.49 | 3.5 | >0 | 1.1 |
| p Value | 0.67 | na | 1.0 | 0.80 | na | 0.33 | 0.31 | <na | 0.96 |
| 95% CI of | 0.13 | na | 0.17 | 0.21 | na | 0.11 | 0.32 | >na | 0.061 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart3 | 3.7 | na | 6.0 | 3.4 | na | 2.1 | 37 | na | 19 |
| OR Quart 4 | 1.1 | 1.0 | 1.0 | 2.1 | 1.0 | 1.7 | 5.5 | >2.1 | 5.6 |
| p Value | 0.92 | 1.0 | 1.0 | 0.27 | 1.0 | 0.42 | 0.15 | <0.56 | 0.15 |
| 95% CI of | 0.22 | 0.059 | 0.17 | 0.56 | 0.059 | 0.46 | 0.53 | >0.18 | 0.54 |
| OR Quart4 | 5.3 | 17 | 6.0 | 7.8 | 17 | 6.4 | 56 | na | 58 |

**Coagulation factor XIII A and B chains**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 12000 | 10800 | 12000 | 10300 | 12000 | 9150 |
| Average | 13200 | 10600 | 13200 | 10900 | 13200 | 10700 |
| Stdev | 6670 | 5200 | 6670 | 4830 | 6670 | 3840 |
| p(t-test) |  | 0.19 |  | 0.14 |  | 0.27 |
| Min | 881 | 1730 | 881 | 2220 | 881 | 7150 |
| Max | 33300 | 21300 | 33300 | 20400 | 33300 | 18700 |
| n (Samp) | 54 | 13 | 54 | 23 | 54 | 9 |
| n (Patient) | 53 | 13 | 53 | 23 | 53 | 9 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 10900 | 12200 | 10900 | 18400 | 10900 | 11400 |
| Average | 12000 | 12200 | 12000 | 18400 | 12000 | 13400 |
| Stdev | 6080 | 2020 | 6080 | 2740 | 6080 | 4700 |
| p(t-test) |  | 0.96 |  | 0.14 |  | 0.69 |
| Min | 881 | 10800 | 881 | 16500 | 881 | 9970 |
| Max | 33300 | 13600 | 33300 | 20400 | 33300 | 18700 |
| n (Samp) | 110 | 2 | 110 | 2 | 110 | 3 |
| n (Patient) | 92 | 2 | 92 | 2 | 92 | 3 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 10300 | 9890 | 10300 | 10800 | 10300 | 9150 |
| Average | 12000 | 10400 | 12000 | 11300 | 12000 | 10800 |
| Stdev | 6320 | 5350 | 6320 | 4810 | 6320 | 3900 |
| p(t-test) |  | 0.42 |  | 0.62 |  | 0.59 |
| Min | 881 | 1730 | 881 | 2220 | 881 | 7150 |
| Max | 33300 | 21300 | 33300 | 20400 | 33300 | 18700 |
| n (Samp) | 48 | 12 | 48 | 25 | 48 | 9 |
| n (Patient) | 44 | 12 | 44 | 25 | 44 | 9 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| AUC | 0.39 | 0.59 | 0.44 | 0.41 | 0.84 | 0.50 | 0.37 | 0.62 | 0.45 |
| SE | 0.091 | 0.21 | 0.095 | 0.073 | 0.18 | 0.072 | 0.11 | 0.18 | 0.11 |
| p | 0.23 | 0.68 | 0.52 | 0.20 | 0.050 | 1.0 | 0.23 | 0.51 | 0.65 |
| nCohort 1 | 54 | 110 | 48 | 54 | 110 | 48 | 54 | 110 | 48 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| nCohort 2 | 13 | 2 | 12 | 23 | 2 | 25 | 9 | 3 | 9 |
| Cutoff 1 | 8650 | 10800 | 8650 | 7990 | 16200 | 8160 | 7990 | 9800 | 8080 |
| Sens 1 | 77% | 100% | 75% | 74% | 100% | 72% | 78% | 100% | 78% |
| Spec 1 | 22% | 49% | 29% | 19% | 81% | 25% | 19% | 44% | 25% |
| Cutoff 2 | 7410 | 10800 | 7410 | 7000 | 16200 | 6730 | 7150 | 9800 | 7150 |
| Sens 2 | 85% | 100% | 83% | 83% | 100% | 84% | 89% | 100% | 89% |
| Spec 2 | 15% | 49% | 17% | 13% | 81% | 15% | 13% | 44% | 15% |
| Cutoff 3 | 2520 | 10800 | 2320 | 3550 | 16200 | 4370 | 7000 | 9800 | 6730 |
| Sens 3 | 92% | 100% | 92% | 91% | 100% | 92% | 100% | 100% | 100% |
| Spec 3 | 6% | 49% | 4% | 6% | 81% | 6% | 13% | 44% | 15% |
| Cutoff 4 | 15400 | 13900 | 12900 | 15400 | 13900 | 12900 | 15400 | 13900 | 12900 |
| Sens 4 | 15% | 0% | 17% | 17% | 100% | 40% | 11% | 33% | 22% |
| Spec 4 | 70% | 71% | 71% | 70% | 71% | 71% | 70% | 71% | 71% |
| Cutoff 5 | 18300 | 16100 | 15400 | 18300 | 16100 | 15400 | 18300 | 16100 | 15400 |
| Sens 5 | 8% | 0% | 17% | 9% | 100% | 20% | 11% | 33% | 11% |
| Spec 5 | 81% | 80% | 81% | 81% | 80% | 81% | 81% | 80% | 81% |
| Cutoff 6 | 22900 | 21500 | 22900 | 22900 | 21500 | 22900 | 22900 | 21500 | 22900 |
| Sens 6 | 0% | 0% | 0% | 0% | 0% | 0% | 0% | 0% | 0% |
| Spec 6 | 91% | 90% | 92% | 91% | 90% | 92% | 91% | 90% | 92% |
| OR Quart 2 | 1.6 | >1.0 | 2.4 | 1.8 | >0 | 0.86 | 2.1 | >1.0 | 0.50 |
| p Value | 0.63 | <0.98 | 0.37 | 0.41 | <na | 0.82 | 0.55 | <0.98 | 0.59 |
| 95% CI of | 0.23 | >0.062 | 0.36 | 0.43 | >na | 0.22 | 0.17 | >0.062 | 0.040 |
| OR Quart2 | 11 | na | 15 | 8.0 | na | 3.3 | 26 | na | 6.2 |
| OR Quart 3 | 3.1 | >1.0 | 1.6 | 1.4 | >0 | 0.86 | 3.5 | >1.0 | 2.6 |
| p Value | 0.22 | <0.98 | 0.63 | 0.64 | <na | 0.82 | 0.31 | <0.98 | 0.32 |
| 95% CI of | 0.51 | >0.062 | 0.23 | 0.32 | >na | 0.22 | 0.32 | >0.062 | 0.39 |
| OR Quart3 | 19 | na | 11 | 6.4 | na | 3.3 | 37 | na | 17 |
| OR Quart 4 | 1.7 | >0 | 1.6 | 2.9 | >2.2 | 0.86 | 3.8 | >1.0 | 1.1 |
| p Value | 0.58 | <na | 0.63 | 0.14 | <0.54 | 0.82 | 0.28 | <1.0 | 0.94 |
| 95% CI of | 0.25 | >na | 0.23 | 0.70 | >0.18 | 0.22 | 0.34 | >0.060 | 0.13 |
| OR Quart4 | 12 | na | 11 | 12 | na | 3.3 | 41 | na | 8.9 |

**Vitronectin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 120000 | 132000 | 120000 | 104000 | 120000 | 113000 |
| Average | 123000 | 136000 | 123000 | 108000 | 123000 | 121000 |
| Stdev | 32800 | 30700 | 32800 | 42000 | 32800 | 35300 |
| p(t-test) | | 0.19 | | 0.098 | | 0.86 |
| Min | 65900 | 96100 | 65900 | 35100 | 65900 | 84900 |
| Max | 205000 | 192000 | 205000 | 184000 | 205000 | 197000 |
| n (Samp) | 54 | 13 | 54 | 23 | 54 | 9 |
| n (Patient) | 53 | 13 | 53 | 23 | 53 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 119000 | 116000 | 119000 | 99400 | 119000 | 108000 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 120000 | 116000 | 120000 | 99400 | 120000 | 105000 |
| Stdev | 36900 | 576 | 36900 | 6820 | 36900 | 16700 |
| p(t-test) | | 0.88 | | 0.44 | | 0.50 |
| Min | 35100 | 115000 | 35100 | 94600 | 35100 | 87500 |
| Max | 205000 | 116000 | 205000 | 104000 | 205000 | 121000 |
| n (Samp) | 110 | 2 | 110 | 2 | 110 | 3 |
| n (Patient) | 92 | 2 | 92 | 2 | 92 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 119000 | 133000 | 119000 | 104000 | 119000 | 113000 |
| Average | 120000 | 136000 | 120000 | 105000 | 120000 | 120000 |
| Stdev | 30100 | 33800 | 30100 | 43900 | 30100 | 35300 |
| p(t-test) | | 0.12 | | 0.091 | | 0.98 |
| Min | 65900 | 90400 | 65900 | 21800 | 65900 | 84900 |
| Max | 205000 | 192000 | 205000 | 184000 | 205000 | 197000 |
| n (Samp) | 48 | 12 | 48 | 25 | 48 | 9 |
| n (Patient) | 44 | 12 | 44 | 25 | 44 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.48 | 0.65 | 0.39 | 0.33 | 0.40 | 0.47 | 0.37 | 0.46 |
| SE | 0.090 | 0.21 | 0.094 | 0.072 | 0.21 | 0.071 | 0.11 | 0.18 | 0.11 |
| p | 0.13 | 0.93 | 0.11 | 0.13 | 0.42 | 0.16 | 0.76 | 0.46 | 0.73 |
| nCohort 1 | 54 | 110 | 48 | 54 | 110 | 48 | 54 | 110 | 48 |
| nCohort 2 | 13 | 2 | 12 | 23 | 2 | 25 | 9 | 3 | 9 |
| Cutoff 1 | 115000 | 115000 | 115000 | 83800 | 94600 | 78300 | 95000 | 87400 | 95000 |
| Sens 1 | 77% | 100% | 75% | 74% | 100% | 72% | 78% | 100% | 78% |
| Spec 1 | 46% | 48% | 44% | 9% | 29% | 8% | 28% | 18% | 27% |
| Cutoff 2 | 115000 | 115000 | 97900 | 65900 | 94600 | 65900 | 84900 | 87400 | 89000 |
| Sens 2 | 85% | 100% | 83% | 83% | 100% | 80% | 89% | 100% | 89% |
| Spec 2 | 46% | 48% | 29% | 2% | 29% | 2% | 9% | 18% | 12% |
| Cutoff 3 | 97900 | 115000 | 95000 | 40400 | 94600 | 35100 | 83800 | 87400 | 78300 |
| Sens 3 | 92% | 100% | 92% | 91% | 100% | 92% | 100% | 100% | 100% |
| Spec 3 | 30% | 48% | 27% | 0% | 29% | 0% | 9% | 18% | 8% |
| Cutoff 4 | 132000 | 137000 | 128000 | 132000 | 137000 | 128000 | 132000 | 137000 | 128000 |
| Sens 4 | 54% | 0% | 58% | 39% | 0% | 36% | 22% | 0% | 33% |
| Spec 4 | 70% | 70% | 71% | 70% | 70% | 71% | 70% | 70% | 71% |
| Cutoff 5 | 158000 | 153000 | 140000 | 158000 | 153000 | 140000 | 158000 | 153000 | 140000 |
| Sens 5 | 23% | 0% | 33% | 9% | 0% | 32% | 11% | 0% | 22% |
| Spec 5 | 81% | 80% | 81% | 81% | 80% | 81% | 81% | 80% | 81% |
| Cutoff 6 | 173000 | 173000 | 167000 | 173000 | 173000 | 167000 | 173000 | 173000 | 167000 |
| Sens 6 | 23% | 0% | 25% | 4% | 0% | 4% | 11% | 0% | 11% |
| Spec 6 | 91% | 90% | 92% | 91% | 90% | 92% | 91% | 90% | 92% |
| OR Quart 2 | 4.6 | >0 | 1.0 | 0.44 | >0 | 0.17 | 1.0 | >1.1 | 0.50 |
| p Value | 0.20 | <na | 1.0 | 0.30 | <na | 0.046 | 1.0 | <0.96 | 0.59 |
| 95% CI of | 0.46 | >na | 0.12 | 0.092 | >na | 0.030 | 0.12 | >0.064 | 0.040 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| OR Quart2 | 47 | na | 8.2 | 2.1 | na | 0.97 | 8.1 | na | 6.2 |
| OR Quart 3 | 4.6 | >2.2 | 1.6 | 0.62 | >2.2 | 0.53 | 1.6 | >1.1 | 2.6 |
| p Value | 0.20 | <0.54 | 0.63 | 0.52 | <0.54 | 0.36 | 0.63 | <0.96 | 0.32 |
| 95% CI of | 0.46 | >0.18 | 0.23 | 0.14 | >0.18 | 0.13 | 0.23 | >0.064 | 0.39 |
| OR Quart3 | 47 | na | 11 | 2.7 | na | 2.1 | 11 | na | 17 |
| OR Quart 4 | 4.6 | >0 | 3.2 | 2.6 | >0 | 1.7 | 1.1 | >1.1 | 1.1 |
| p Value | 0.20 | <na | 0.21 | 0.16 | <na | 0.41 | 0.94 | <0.96 | 0.94 |
| 95% CI of | 0.46 | >na | 0.52 | 0.70 | >na | 0.47 | 0.13 | >0.064 | 0.13 |
| OR Quart4 | 47 | na | 20 | 9.6 | na | 6.3 | 8.8 | na | 8.9 |

**Estradiol**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.24 | 1.99 | 1.24 | 1.37 | 1.24 | 1.48 |
| Average | 1.80 | 2.66 | 1.80 | 2.75 | 1.80 | 1.61 |
| Stdev | 1.84 | 1.89 | 1.84 | 3.95 | 1.84 | 0.932 |
| p(t-test) |  | 0.14 |  | 0.16 |  | 0.77 |
| Min | 0.513 | 0.979 | 0.513 | 0.288 | 0.513 | 0.541 |
| Max | 10.8 | 7.74 | 10.8 | 15.4 | 10.8 | 3.68 |
| n (Samp) | 53 | 13 | 53 | 22 | 53 | 9 |
| n (Patient) | 52 | 13 | 52 | 22 | 52 | 9 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.40 | 1.38 | 1.40 | 1.00 | 1.40 | 1.48 |
| Average | 2.32 | 1.38 | 2.32 | 1.00 | 2.32 | 1.42 |
| Stdev | 2.86 | 0.194 | 2.86 | 0.514 | 2.86 | 0.529 |
| p(t-test) |  | 0.64 |  | 0.52 |  | 0.59 |
| Min | 0.288 | 1.24 | 0.288 | 0.638 | 0.288 | 0.871 |
| Max | 17.4 | 1.51 | 17.4 | 1.37 | 17.4 | 1.93 |
| n (Samp) | 108 | 2 | 108 | 2 | 108 | 3 |
| n (Patient) | 91 | 2 | 91 | 2 | 91 | 3 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 1.16 | 2.76 | 1.16 | 1.41 | 1.16 | 1.40 |
| Average | 1.97 | 2.97 | 1.97 | 2.86 | 1.97 | 1.55 |
| Stdev | 2.00 | 1.90 | 2.00 | 3.80 | 2.00 | 0.926 |
| p(t-test) |  | 0.13 |  | 0.20 |  | 0.55 |
| Min | 0.513 | 0.979 | 0.513 | 0.288 | 0.513 | 0.541 |
| Max | 10.8 | 7.74 | 10.8 | 15.4 | 10.8 | 3.68 |
| n (Samp) | 47 | 12 | 47 | 24 | 47 | 9 |
| n (Patient) | 43 | 12 | 43 | 24 | 43 | 9 |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.73 | 0.49 | 0.74 | 0.54 | 0.27 | 0.57 | 0.54 | 0.47 | 0.51 |
| SE | 0.086 | 0.21 | 0.088 | 0.074 | 0.21 | 0.073 | 0.11 | 0.17 | 0.11 |
| p | 0.0085 | 0.95 | 0.0053 | 0.55 | 0.27 | 0.34 | 0.69 | 0.87 | 0.92 |
| nCohort 1 | 53 | 108 | 47 | 53 | 108 | 47 | 53 | 108 | 47 |
| nCohort 2 | 13 | 2 | 12 | 22 | 2 | 24 | 9 | 3 | 9 |
| Cutoff 1 | 1.32 | 1.20 | 1.60 | 0.996 | 0.635 | 1.08 | 1.13 | 0.864 | 1.16 |
| Sens 1 | 77% | 100% | 75% | 73% | 100% | 71% | 78% | 100% | 78% |
| Spec 1 | 55% | 43% | 66% | 36% | 8% | 49% | 47% | 19% | 51% |
| Cutoff 2 | 1.20 | 1.20 | 1.32 | 0.864 | 0.635 | 0.864 | 0.552 | 0.864 | 0.552 |
| Sens 2 | 85% | 100% | 83% | 82% | 100% | 83% | 89% | 100% | 89% |
| Spec 2 | 49% | 43% | 57% | 21% | 8% | 23% | 4% | 19% | 4% |
| Cutoff 3 | 1.13 | 1.20 | 1.16 | 0.635 | 0.635 | 0.635 | 0.513 | 0.864 | 0.513 |
| Sens 3 | 92% | 100% | 92% | 91% | 100% | 92% | 100% | 100% | 100% |
| Spec 3 | 47% | 43% | 51% | 8% | 8% | 9% | 2% | 19% | 2% |
| Cutoff 4 | 1.60 | 1.99 | 1.93 | 1.60 | 1.99 | 1.93 | 1.60 | 1.99 | 1.93 |
| Sens 4 | 62% | 0% | 67% | 36% | 0% | 38% | 44% | 0% | 22% |
| Spec 4 | 72% | 70% | 70% | 72% | 70% | 70% | 72% | 70% | 70% |
| Cutoff 5 | 2.00 | 2.58 | 2.34 | 2.00 | 2.58 | 2.34 | 2.00 | 2.58 | 2.34 |
| Sens 5 | 46% | 0% | 58% | 32% | 0% | 33% | 22% | 0% | 11% |
| Spec 5 | 81% | 81% | 81% | 81% | 81% | 81% | 81% | 81% | 81% |
| Cutoff 6 | 3.05 | 4.89 | 4.47 | 3.05 | 4.89 | 4.47 | 3.05 | 4.89 | 4.47 |
| Sens 6 | 31% | 0% | 17% | 18% | 0% | 17% | 11% | 0% | 0% |
| Spec 6 | 91% | 91% | 91% | 91% | 91% | 91% | 91% | 91% | 91% |
| OR Quart 2 | >4.9 | >1.1 | >2.2 | 0.93 | >0 | 0.69 | 0.43 | >2.2 | 0.46 |
| p Value | <0.18 | <0.96 | <0.55 | 0.92 | <na | 0.63 | 0.51 | <0.54 | 0.55 |
| 95% CI of | >0.49 | >0.064 | >0.17 | 0.22 | >na | 0.15 | 0.035 | >0.18 | 0.037 |
| OR Quart2 | na | na | na | 4.0 | na | 3.1 | 5.3 | na | 5.8 |
| OR Quart 3 | >3.7 | >1.0 | >3.5 | 0.93 | >1.0 | 1.5 | 1.6 | >0 | 2.4 |
| p Value | <0.28 | <0.98 | <0.30 | 0.92 | <0.98 | 0.56 | 0.63 | <na | 0.36 |
| 95% CI of | >0.34 | >0.062 | >0.32 | 0.22 | >0.062 | 0.37 | 0.23 | >na | 0.36 |
| OR Quart3 | na | na | na | 4.0 | na | 6.3 | 11 | na | 16 |
| OR Quart 4 | >8.7 | >0 | >12 | 1.5 | >1.1 | 1.9 | 1.5 | >1.1 | 1.0 |
| p Value | <0.059 | <na | <0.030 | 0.56 | <0.96 | 0.36 | 0.68 | <0.96 | 1.0 |
| 95% CI of | >0.92 | >na | >1.3 | 0.38 | >0.064 | 0.47 | 0.21 | >0.064 | 0.12 |
| OR Quart4 | na | na | na | 6.1 | na | 7.8 | 11 | na | 8.3 |

**Progesterone**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.43 | 6.18 | 4.43 | 3.87 | 4.43 | 3.90 |
| Average | 6.21 | 6.58 | 6.21 | 21.9 | 6.21 | 5.65 |
| Stdev | 7.20 | 3.08 | 7.20 | 52.7 | 7.20 | 4.28 |
| p(t-test) | | 0.86 | | 0.036 | | 0.82 |
| Min | 1.35 | 3.40 | 1.35 | 1.65 | 1.35 | 2.45 |
| Max | 46.5 | 12.8 | 46.5 | 194 | 46.5 | 15.8 |
| n (Samp) | 53 | 13 | 53 | 22 | 53 | 9 |
| n (Patient) | 52 | 13 | 52 | 22 | 52 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.44 | 7.13 | 4.44 | 2.48 | 4.44 | 3.68 |
| Average | 10.8 | 7.13 | 10.8 | 2.48 | 10.8 | 7.50 |
| Stdev | 28.5 | 1.01 | 28.5 | 1.17 | 28.5 | 7.16 |
| p(t-test) | | 0.86 | | 0.68 | | 0.84 |
| Min | 1.35 | 6.41 | 1.35 | 1.65 | 1.35 | 3.07 |
| Max | 194 | 7.84 | 194 | 3.31 | 194 | 15.8 |
| n (Samp) | 108 | 2 | 108 | 2 | 108 | 3 |
| n (Patient) | 91 | 2 | 91 | 2 | 91 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 4.43 | 6.12 | 4.43 | 4.23 | 4.43 | 3.90 |
| Average | 5.75 | 6.48 | 5.75 | 20.6 | 5.75 | 4.76 |
| Stdev | 4.91 | 3.20 | 4.91 | 50.5 | 4.91 | 2.28 |
| p(t-test) | | 0.63 | | 0.048 | | 0.56 |
| Min | 1.35 | 3.40 | 1.35 | 1.65 | 1.35 | 2.45 |
| Max | 31.4 | 12.8 | 31.4 | 194 | 31.4 | 9.36 |
| n (Samp) | 47 | 12 | 47 | 24 | 47 | 9 |
| n (Patient) | 43 | 12 | 43 | 24 | 43 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.64 | 0.73 | 0.62 | 0.48 | 0.15 | 0.50 | 0.49 | 0.51 | 0.47 |
| SE | 0.090 | 0.21 | 0.095 | 0.074 | 0.17 | 0.073 | 0.11 | 0.17 | 0.11 |
| p | 0.13 | 0.27 | 0.20 | 0.76 | 0.040 | 0.96 | 0.93 | 0.96 | 0.80 |
| nCohort 1 | 53 | 108 | 47 | 53 | 108 | 47 | 53 | 108 | 47 |
| nCohort 2 | 13 | 2 | 12 | 22 | 2 | 24 | 9 | 3 | 9 |
| Cutoff 1 | 3.73 | 6.37 | 3.73 | 2.60 | 1.35 | 2.91 | 3.32 | 2.91 | 3.41 |
| Sens 1 | 77% | 100% | 75% | 73% | 100% | 71% | 78% | 100% | 78% |
| Spec 1 | 40% | 69% | 40% | 19% | 1% | 28% | 36% | 24% | 36% |
| Cutoff 2 | 3.57 | 6.37 | 3.57 | 2.23 | 1.35 | 2.16 | 3.09 | 2.91 | 3.09 |
| Sens 2 | 85% | 100% | 83% | 82% | 100% | 83% | 89% | 100% | 89% |
| Spec 2 | 36% | 69% | 36% | 13% | 1% | 13% | 30% | 24% | 30% |
| Cutoff 3 | 3.40 | 6.37 | 3.41 | 2.14 | 1.35 | 2.14 | 2.23 | 2.91 | 2.06 |
| Sens 3 | 92% | 100% | 92% | 91% | 100% | 92% | 100% | 100% | 100% |
| Spec 3 | 36% | 69% | 36% | 11% | 1% | 13% | 13% | 24% | 13% |
| Cutoff 4 | 6.37 | 6.61 | 6.28 | 6.37 | 6.61 | 6.28 | 6.37 | 6.61 | 6.28 |
| Sens 4 | 46% | 50% | 42% | 27% | 0% | 29% | 22% | 33% | 22% |
| Spec 4 | 72% | 70% | 70% | 72% | 70% | 70% | 72% | 70% | 70% |
| Cutoff 5 | 7.12 | 8.62 | 7.77 | 7.12 | 8.62 | 7.77 | 7.12 | 8.62 | 7.77 |
| Sens 5 | 38% | 0% | 25% | 27% | 0% | 29% | 22% | 33% | 22% |
| Spec 5 | 81% | 81% | 81% | 81% | 81% | 81% | 81% | 81% | 81% |
| Cutoff 6 | 9.61 | 11.6 | 9.73 | 9.61 | 11.6 | 9.73 | 9.61 | 11.6 | 9.73 |
| Sens 6 | 15% | 0% | 17% | 23% | 0% | 17% | 11% | 33% | 0% |
| Spec 6 | 91% | 91% | 91% | 91% | 91% | 91% | 91% | 91% | 91% |
| OR Quart 2 | >6.7 | >0 | >7.0 | 0.58 | >0 | 0.45 | 0.50 | 0.96 | 0.46 |
| p Value | <0.10 | <na | <0.097 | 0.46 | <na | 0.28 | 0.59 | 0.98 | 0.55 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of OR Quart2 | >0.69 | >na | >0.71 | 0.13 | >na | 0.10 | 0.041 | 0.057 | 0.037 |
| | na | na | na | 2.5 | na | 1.9 | 6.2 | 16 | 5.8 |
| OR Quart 3 | >3.7 | >1.0 | >3.5 | 0.77 | >1.0 | 0.79 | 3.2 | 0 | 3.3 |
| p Value | <0.28 | <0.98 | <0.30 | 0.72 | <0.98 | 0.73 | 0.21 | na | 0.20 |
| 95% CI of OR Quart3 | >0.34 | >0.062 | >0.32 | 0.19 | >0.062 | 0.20 | 0.52 | na | 0.52 |
| | na | na | na | 3.2 | na | 3.1 | 20 | na | 21 |
| OR Quart 4 | >6.7 | >1.0 | >5.1 | 1.4 | >1.1 | 1.1 | 0.50 | 0.96 | 0.46 |
| p Value | <0.10 | <1.0 | <0.17 | 0.64 | <0.96 | 0.89 | 0.59 | 0.98 | 0.55 |
| 95% CI of OR Quart4 | >0.69 | >0.059 | >0.50 | 0.36 | >0.064 | 0.28 | 0.041 | 0.057 | 0.037 |
| | na | na | na | 5.3 | na | 4.3 | 6.2 | 16 | 5.8 |

**T3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.15 | 1.34 | 1.15 | 1.00 | 1.15 | 1.34 |
| Average | 1.25 | 1.36 | 1.25 | 0.902 | 1.25 | 1.14 |
| Stdev | 0.601 | 0.757 | 0.601 | 0.493 | 0.601 | 0.550 |
| p(t-test) | | 0.57 | | 0.019 | | 0.59 |
| Min | 0.302 | 0.356 | 0.302 | 0.000162 | 0.302 | 0.0946 |
| Max | 3.76 | 3.18 | 3.76 | 1.59 | 3.76 | 1.61 |
| n (Samp) | 53 | 13 | 53 | 22 | 53 | 9 |
| n (Patient) | 52 | 13 | 52 | 22 | 52 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.07 | 1.56 | 1.07 | 0.641 | 1.07 | 1.34 |
| Average | 1.13 | 1.56 | 1.13 | 0.641 | 1.13 | 1.42 |
| Stdev | 0.602 | 0.00217 | 0.602 | 0.906 | 0.602 | 0.169 |
| p(t-test) | | 0.31 | | 0.26 | | 0.41 |
| Min | 0.000162 | 1.56 | 0.000162 | 0.000227 | 0.000162 | 1.30 |
| Max | 3.76 | 1.56 | 3.76 | 1.28 | 3.76 | 1.61 |
| n (Samp) | 108 | 2 | 108 | 2 | 108 | 3 |
| n (Patient) | 91 | 2 | 91 | 2 | 91 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1.13 | 1.33 | 1.13 | 0.946 | 1.13 | 1.01 |
| Average | 1.21 | 1.36 | 1.21 | 0.868 | 1.21 | 1.07 |
| Stdev | 0.520 | 0.793 | 0.520 | 0.506 | 0.520 | 0.521 |
| p(t-test) | | 0.42 | | 0.011 | | 0.46 |
| Min | 0.344 | 0.356 | 0.344 | 0.000162 | 0.344 | 0.0946 |
| Max | 2.78 | 3.18 | 2.78 | 1.59 | 2.78 | 1.61 |
| n (Samp) | 47 | 12 | 47 | 24 | 47 | 9 |
| n (Patient) | 43 | 12 | 43 | 24 | 43 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.54 | 0.79 | 0.54 | 0.36 | 0.34 | 0.36 | 0.48 | 0.73 | 0.45 |
| SE | 0.091 | 0.19 | 0.095 | 0.073 | 0.21 | 0.071 | 0.11 | 0.17 | 0.11 |
| p | 0.68 | 0.14 | 0.68 | 0.046 | 0.46 | 0.043 | 0.88 | 0.16 | 0.65 |
| nCohort 1 | 53 | 108 | 47 | 53 | 108 | 47 | 53 | 108 | 47 |
| nCohort 2 | 13 | 2 | 12 | 22 | 2 | 24 | 9 | 3 | 9 |
| Cutoff 1 | 0.725 | 1.54 | 0.725 | 0.551 | 0.000162 | 0.551 | 0.908 | 1.27 | 0.908 |
| Sens 1 | 77% | 100% | 75% | 73% | 100% | 71% | 78% | 100% | 78% |
| Spec 1 | 17% | 79% | 17% | 6% | 1% | 4% | 32% | 68% | 34% |
| Cutoff 2 | 0.695 | 1.54 | 0.695 | 0.449 | 0.000162 | 0.344 | 0.344 | 1.27 | 0.344 |
| Sens 2 | 85% | 100% | 83% | 82% | 100% | 83% | 89% | 100% | 89% |
| Spec 2 | 13% | 79% | 13% | 4% | 1% | 2% | 4% | 68% | 2% |
| Cutoff 3 | 0.618 | 1.54 | 0.618 | 0.000227 | 0.000162 | 0.000162 | 0 | 1.27 | 0 |
| Sens 3 | 92% | 100% | 92% | 91% | 100% | 92% | 100% | 100% | 100% |
| Spec 3 | 8% | 79% | 6% | 0% | 1% | 0% | 0% | 68% | 0% |
| Cutoff 4 | 1.48 | 1.34 | 1.39 | 1.48 | 1.34 | 1.39 | 1.48 | 1.34 | 1.39 |
| Sens 4 | 46% | 100% | 42% | 9% | 0% | 12% | 44% | 33% | 33% |
| Spec 4 | 72% | 70% | 70% | 72% | 70% | 70% | 72% | 70% | 70% |
| Cutoff 5 | 1.68 | 1.57 | 1.66 | 1.68 | 1.57 | 1.66 | 1.68 | 1.57 | 1.66 |
| Sens 5 | 23% | 0% | 42% | 0% | 0% | 0% | 0% | 33% | 0% |
| Spec 5 | 81% | 81% | 81% | 81% | 81% | 81% | 81% | 81% | 81% |
| Cutoff 6 | 1.88 | 1.86 | 1.88 | 1.88 | 1.86 | 1.88 | 1.88 | 1.86 | 1.88 |
| Sens 6 | 23% | 0% | 25% | 0% | 0% | 0% | 0% | 0% | 0% |
| Spec 6 | 91% | 91% | 91% | 91% | 91% | 91% | 91% | 91% | 91% |
| OR Quart 2 | 0 | >0 | 0.18 | 6.2 | >1.1 | 4.0 | 0.67 | >0 | 1.0 |
| p Value | na | <na | 0.15 | 0.038 | <0.96 | 0.080 | 0.68 | <na | 1.0 |
| 95% CI of OR Quart2 | na na | >na na | 0.017 1.8 | 1.1 35 | >0.064 na | 0.85 19 | 0.095 4.7 | >na na | 0.12 8.3 |
| OR Quart 3 | 0.73 | >0 | 0.38 | 1.6 | >0 | 1.0 | 0.62 | >2.1 | 1.6 |
| p Value | 0.69 | <na | 0.32 | 0.63 | <na | 1.0 | 0.63 | <0.56 | 0.62 |
| 95% CI of OR Quart3 | 0.16 3.5 | >na na | 0.058 2.5 | 0.23 11 | >na na | 0.17 5.8 | 0.089 4.3 | >0.18 na | 0.23 12 |
| OR Quart 4 | 0.68 | >2.1 | 1.2 | 8.5 | >1.1 | 7.1 | 0.67 | >1.0 | 1.0 |
| p Value | 0.62 | <0.56 | 0.78 | 0.015 | <0.96 | 0.014 | 0.68 | <1.0 | 1.0 |
| 95% CI of OR Quart4 | 0.15 3.2 | >0.18 na | 0.26 6.1 | 1.5 48 | >0.064 na | 1.5 34 | 0.095 4.7 | >0.059 na | 0.12 8.3 |

**Growth/differentiation factor 15**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 1820 | 2080 | 1820 | 2280 | 1820 | 1820 |
| Average | 2320 | 2240 | 2320 | 3250 | 2320 | 2480 |
| Stdev | 1700 | 1540 | 1700 | 2200 | 1700 | 1550 |
| p(t-test) | | 0.88 | | 0.052 | | 0.79 |
| Min | 271 | 437 | 271 | 1010 | 271 | 733 |
| Max | 7790 | 5190 | 7790 | 8110 | 7790 | 5690 |
| n (Samp) | 53 | 13 | 53 | 22 | 53 | 9 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 52 | 13 | 52 | 22 | 52 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2080 | 1150 | 2080 | 4700 | 2080 | 1010 |
| Average | 2650 | 1150 | 2650 | 4700 | 2650 | 1180 |
| Stdev | 1830 | 744 | 1830 | 3700 | 1830 | 551 |
| p(t-test) | | 0.25 | | 0.12 | | 0.17 |
| Min | 271 | 620 | 271 | 2090 | 271 | 733 |
| Max | 8110 | 1670 | 8110 | 7320 | 8110 | 1800 |
| n (Samp) | 108 | 2 | 108 | 2 | 108 | 3 |
| n (Patient) | 91 | 2 | 91 | 2 | 91 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 2030 | 2630 | 2030 | 2370 | 2030 | 1900 |
| Average | 2360 | 2500 | 2360 | 3430 | 2360 | 2700 |
| Stdev | 1640 | 1520 | 1640 | 2260 | 1640 | 1400 |
| p(t-test) | | 0.80 | | 0.025 | | 0.57 |
| Min | 452 | 437 | 452 | 1010 | 452 | 1400 |
| Max | 7790 | 5190 | 7790 | 8110 | 7790 | 5690 |
| n (Samp) | 47 | 12 | 47 | 24 | 47 | 9 |
| n (Patient) | 43 | 12 | 43 | 24 | 43 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.50 | 0.20 | 0.55 | 0.65 | 0.74 | 0.66 | 0.55 | 0.21 | 0.60 |
| SE | 0.090 | 0.19 | 0.095 | 0.073 | 0.20 | 0.071 | 0.11 | 0.16 | 0.11 |
| p | 0.97 | 0.11 | 0.63 | 0.041 | 0.25 | 0.028 | 0.63 | 0.066 | 0.35 |
| nCohort 1 | 53 | 108 | 47 | 53 | 108 | 47 | 53 | 108 | 47 |
| nCohort 2 | 13 | 2 | 12 | 22 | 2 | 24 | 9 | 3 | 9 |
| Cutoff 1 | 703 | 618 | 1390 | 1930 | 2090 | 2090 | 1570 | 703 | 1780 |
| Sens 1 | 77% | 100% | 75% | 73% | 100% | 71% | 78% | 100% | 78% |
| Spec 1 | 11% | 6% | 30% | 55% | 51% | 57% | 43% | 8% | 45% |
| Cutoff 2 | 620 | 618 | 733 | 1570 | 2090 | 1570 | 1390 | 703 | 1570 |
| Sens 2 | 85% | 100% | 83% | 82% | 100% | 83% | 89% | 100% | 89% |
| Spec 2 | 9% | 6% | 11% | 43% | 51% | 40% | 34% | 8% | 40% |
| Cutoff 3 | 618 | 618 | 620 | 1200 | 2090 | 1190 | 703 | 703 | 1390 |
| Sens 3 | 92% | 100% | 92% | 91% | 100% | 92% | 100% | 100% | 100% |
| Spec 3 | 9% | 6% | 6% | 28% | 51% | 26% | 11% | 8% | 30% |
| Cutoff 4 | 2630 | 3190 | 2660 | 2630 | 3190 | 2660 | 2630 | 3190 | 2660 |
| Sens 4 | 38% | 0% | 42% | 41% | 50% | 46% | 33% | 0% | 44% |
| Spec 4 | 72% | 70% | 70% | 72% | 70% | 70% | 72% | 70% | 70% |
| Cutoff 5 | 3470 | 3980 | 3190 | 3470 | 3980 | 3190 | 3470 | 3980 | 3190 |
| Sens 5 | 15% | 0% | 25% | 32% | 50% | 46% | 33% | 0% | 33% |
| Spec 5 | 81% | 81% | 81% | 81% | 81% | 81% | 81% | 81% | 81% |
| Cutoff 6 | 4990 | 5440 | 4990 | 4990 | 5440 | 4990 | 4990 | 5440 | 4990 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| Sens 6 | 8% | 0% | 8% | 18% | 50% | 21% | 11% | 0% | 11% |
| Spec 6 | 91% | 91% | 91% | 91% | 91% | 91% | 91% | 91% | 91% |
| OR Quart 2 | 0.75 | >0 | 0.26 | 1.3 | >0 | 0.93 | 4.7 | >0 | >7.8 |
| p Value | 0.74 | <na | 0.27 | 0.73 | <na | 0.93 | 0.19 | <na | <0.081 |
| 95% CI of | 0.14 | >na | 0.024 | 0.25 | >na | 0.19 | 0.46 | >na | >0.78 |
| OR Quart2 | 4.0 | na | 2.9 | 7.0 | na | 4.5 | 48 | na | na |
| OR Quart 3 | 0.43 | >1.0 | 0.92 | 2.9 | >1.0 | 2.1 | 1.0 | >1.0 | >1.1 |
| p Value | 0.38 | <0.98 | 0.92 | 0.18 | <0.98 | 0.33 | 1.0 | <0.98 | <0.96 |
| 95% CI of | 0.068 | >0.062 | 0.15 | 0.62 | >0.062 | 0.48 | 0.057 | >0.062 | >0.061 |
| OR Quart3 | 2.8 | na | 5.5 | 14 | na | 9.0 | 18 | na | na |
| OR Quart 4 | 1.1 | >1.1 | 1.8 | 3.6 | >1.0 | 3.2 | 3.2 | >2.2 | >3.8 |
| p Value | 0.92 | <0.96 | 0.48 | 0.100 | <1.0 | 0.11 | 0.34 | <0.52 | <0.27 |
| 95% CI of | 0.22 | >0.064 | 0.35 | 0.78 | >0.059 | 0.76 | 0.30 | >0.19 | >0.35 |
| OR Quart4 | 5.3 | na | 9.7 | 17 | na | 14 | 35 | na | na |

**Proprotein convertase subtilisin/kexin type 9**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 462000 | 539000 | 462000 | 443000 | 462000 | 543000 |
| Average | 513000 | 573000 | 513000 | 456000 | 513000 | 569000 |
| Stdev | 226000 | 191000 | 226000 | 208000 | 226000 | 189000 |
| p(t-test) | | 0.38 | | 0.30 | | 0.48 |
| Min | 76300 | 176000 | 76300 | 102000 | 76300 | 326000 |
| Max | 1100000 | 1000000 | 1100000 | 858000 | 1100000 | 884000 |
| n (Samp) | 54 | 13 | 54 | 23 | 54 | 9 |
| n (Patient) | 53 | 13 | 53 | 23 | 53 | 9 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 491000 | 618000 | 491000 | 321000 | 491000 | 529000 |
| Average | 504000 | 618000 | 504000 | 321000 | 504000 | 466000 |
| Stdev | 213000 | 113000 | 213000 | 124000 | 213000 | 122000 |
| p(t-test) | | 0.45 | | 0.23 | | 0.76 |
| Min | 76300 | 539000 | 76300 | 233000 | 76300 | 326000 |
| Max | 1100000 | 698000 | 1100000 | 409000 | 1100000 | 543000 |
| n (Samp) | 110 | 2 | 110 | 2 | 110 | 3 |
| n (Patient) | 92 | 2 | 92 | 2 | 92 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 466000 | 539000 | 466000 | 443000 | 466000 | 494000 |
| Average | 504000 | 568000 | 504000 | 448000 | 504000 | 564000 |
| Stdev | 211000 | 195000 | 211000 | 216000 | 211000 | 191000 |
| p(t-test) | | 0.34 | | 0.29 | | 0.43 |
| Min | 76300 | 176000 | 76300 | 80700 | 76300 | 326000 |
| Max | 867000 | 1000000 | 867000 | 858000 | 867000 | 884000 |
| n (Samp) | 48 | 12 | 48 | 25 | 48 | 9 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| n (Patient) | 44 | 12 | 44 | 25 | 44 | 9 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.61 | 0.72 | 0.60 | 0.43 | 0.21 | 0.43 | 0.59 | 0.48 | 0.58 |
| SE | 0.091 | 0.21 | 0.095 | 0.073 | 0.19 | 0.072 | 0.11 | 0.17 | 0.11 |
| p | 0.23 | 0.28 | 0.29 | 0.37 | 0.14 | 0.35 | 0.39 | 0.93 | 0.44 |
| nCohort 1 | 54 | 110 | 48 | 54 | 110 | 48 | 54 | 110 | 48 |
| nCohort 2 | 13 | 2 | 12 | 23 | 2 | 25 | 9 | 3 | 9 |
| Cutoff 1 | 497000 | 538000 | 497000 | 262000 | 228000 | 274000 | 491000 | 318000 | 491000 |
| Sens 1 | 77% | 100% | 75% | 74% | 100% | 72% | 78% | 100% | 78% |
| Spec 1 | 56% | 64% | 54% | 11% | 9% | 15% | 56% | 20% | 54% |
| Cutoff 2 | 491000 | 538000 | 491000 | 246000 | 228000 | 248000 | 326000 | 318000 | 326000 |
| Sens 2 | 85% | 100% | 83% | 83% | 100% | 80% | 89% | 100% | 89% |
| Spec 2 | 56% | 64% | 54% | 9% | 9% | 10% | 20% | 20% | 19% |
| Cutoff 3 | 442000 | 538000 | 442000 | 228000 | 228000 | 102000 | 318000 | 318000 | 318000 |
| Sens 3 | 92% | 100% | 92% | 91% | 100% | 92% | 100% | 100% | 100% |
| Spec 3 | 48% | 64% | 48% | 7% | 9% | 2% | 20% | 20% | 19% |
| Cutoff 4 | 642000 | 597000 | 667000 | 642000 | 597000 | 667000 | 642000 | 597000 | 667000 |
| Sens 4 | 23% | 50% | 17% | 17% | 0% | 16% | 33% | 0% | 33% |
| Spec 4 | 70% | 70% | 71% | 70% | 70% | 71% | 70% | 70% | 71% |
| Cutoff 5 | 786000 | 692000 | 698000 | 786000 | 692000 | 698000 | 786000 | 692000 | 698000 |
| Sens 5 | 8% | 50% | 17% | 9% | 0% | 12% | 11% | 0% | 22% |
| Spec 5 | 81% | 80% | 81% | 81% | 80% | 81% | 81% | 80% | 81% |
| Cutoff 6 | 835000 | 826000 | 834000 | 835000 | 826000 | 834000 | 835000 | 826000 | 834000 |
| Sens 6 | 8% | 0% | 8% | 4% | 0% | 4% | 11% | 0% | 11% |
| Spec 6 | 91% | 90% | 92% | 91% | 90% | 92% | 91% | 90% | 92% |
| OR Quart 2 | 2.0 | >0 | 3.5 | 2.3 | >0 | 3.0 | 0 | >2.2 | 0 |
| p Value | 0.59 | <na | 0.30 | 0.25 | <na | 0.14 | na | <0.52 | na |
| 95% CI of | 0.16 | >na | 0.32 | 0.55 | >na | 0.71 | na | >0.19 | na |
| OR Quart2 | 24 | na | 38 | 9.8 | na | 13 | na | na | na |
| OR Quart 3 | 10 | >1.0 | 9.3 | 1.4 | >1.0 | 1.4 | 2.2 | >0 | 2.4 |
| p Value | 0.040 | <0.98 | 0.054 | 0.64 | <0.98 | 0.63 | 0.42 | <na | 0.36 |
| 95% CI of | 1.1 | >0.062 | 0.96 | 0.32 | >0.062 | 0.32 | 0.33 | >na | 0.36 |
| OR Quart3 | 99 | na | 91 | 6.4 | na | 6.5 | 14 | na | 16 |
| OR Quart 4 | 3.2 | >1.0 | 2.2 | 2.3 | >1.0 | 3.0 | 1.5 | >1.1 | 1.5 |
| p Value | 0.34 | <0.98 | 0.55 | 0.25 | <0.98 | 0.14 | 0.68 | <0.96 | 0.69 |
| 95% CI of | 0.30 | >0.062 | 0.17 | 0.55 | >0.062 | 0.71 | 0.21 | >0.064 | 0.21 |
| OR Quart4 | 35 | na | 27 | 9.8 | na | 13 | 11 | na | 11 |

Table 6: Comparison of marker levels in EDTA samples collected from Cohort 1 (patients that did not progress beyond RIFLE stage 0 or R) and in EDTA samples collected from subjects at 0, 24 hours, and 48 hours prior to reaching stage I or F in Cohort 2.

**Toll-like receptor 2**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 0.0733 | 0.0997 | 0.0733 | 0.0983 |
| Average | 1.61 | 0.672 | 1.61 | 0.473 |
| Stdev | 4.33 | 2.04 | 4.33 | 1.45 |
| p(t-test) | | 0.36 | | 0.25 |
| Min | 1.14E-5 | 1.14E-5 | 1.14E-5 | 0.00107 |
| Max | 19.7 | 8.94 | 19.7 | 6.59 |
| n (Samp) | 91 | 19 | 91 | 20 |
| n (Patient) | 45 | 19 | 45 | 20 |

| sCr only | 0hr prior to AKI stage | | 24hr prior to AKI stage | |
|---|---|---|---|---|
| | Cohort 1 | Cohort 2 | Cohort 1 | Cohort 2 |
| Median | 0.0778 | 0.0365 | 0.0778 | 0.141 |
| Average | 1.36 | 0.114 | 1.36 | 0.140 |
| Stdev | 3.86 | 0.142 | 3.86 | 0.0999 |
| p(t-test) | | 0.58 | | 0.59 |
| Min | 1.14E-5 | 0.0276 | 1.14E-5 | 0.0394 |
| Max | 19.7 | 0.279 | 19.7 | 0.239 |
| n (Samp) | 123 | 3 | 123 | 3 |
| n (Patient) | 61 | 3 | 61 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.0770 | 0.0997 | 0.0770 | 0.0925 |
| Average | 1.74 | 0.733 | 1.74 | 0.485 |
| Stdev | 4.48 | 2.15 | 4.48 | 1.49 |
| p(t-test) | | 0.37 | | 0.23 |
| Min | 1.14E-5 | 1.14E-5 | 1.14E-5 | 0.00107 |
| Max | 19.7 | 8.94 | 19.7 | 6.59 |
| n (Samp) | 84 | 17 | 84 | 19 |
| n (Patient) | 42 | 17 | 42 | 19 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.49 | 0.46 | 0.46 | 0.54 | 0.59 | 0.50 |
| SE | 0.074 | 0.17 | 0.078 | 0.073 | 0.18 | 0.074 |
| p | 0.87 | 0.81 | 0.58 | 0.59 | 0.63 | 0.97 |
| nCohort 1 | 91 | 123 | 84 | 91 | 123 | 84 |
| nCohort 2 | 19 | 3 | 17 | 20 | 3 | 19 |
| Cutoff 1 | 0.0247 | 0.0247 | 0.0247 | 0.0380 | 0.0380 | 0.0366 |
| Sens 1 | 74% | 100% | 71% | 70% | 100% | 74% |
| Spec 1 | 31% | 29% | 26% | 41% | 40% | 37% |
| Cutoff 2 | 0 | 0.0247 | 0 | 0.0276 | 0.0380 | 0.0182 |
| Sens 2 | 100% | 100% | 100% | 80% | 100% | 84% |
| Spec 2 | 0% | 29% | 0% | 32% | 40% | 26% |
| Cutoff 3 | 0 | 0.0247 | 0 | 0.00402 | 0.0380 | 0.00107 |
| Sens 3 | 100% | 100% | 100% | 90% | 100% | 95% |
| Spec 3 | 0% | 29% | 0% | 20% | 40% | 12% |

| | 0hr prior to AKI stage | | | 24hr prior to AKI stage | | |
|---|---|---|---|---|---|---|
| | sCr or UO | sCr only | UO only | sCr or UO | sCr only | UO only |
| Cutoff 4 | 0.211 | 0.194 | 0.279 | 0.211 | 0.194 | 0.279 |
| Sens 4 | 26% | 33% | 24% | 30% | 33% | 21% |
| Spec 4 | 70% | 71% | 70% | 70% | 71% | 70% |
| Cutoff 5 | 0.372 | 0.398 | 0.462 | 0.372 | 0.398 | 0.462 |
| Sens 5 | 21% | 0% | 24% | 20% | 0% | 16% |
| Spec 5 | 80% | 80% | 81% | 80% | 80% | 81% |
| Cutoff 6 | 4.99 | 4.75 | 6.65 | 4.99 | 4.75 | 6.65 |
| Sens 6 | 5% | 0% | 6% | 5% | 0% | 0% |
| Spec 6 | 90% | 90% | 90% | 90% | 90% | 90% |
| OR Quart 2 | 1.7 | >1.1 | 1.4 | 1.7 | >1.0 | 1.7 |
| p Value | 0.45 | <0.96 | 0.67 | 0.48 | <1.0 | 0.48 |
| 95% CI of OR Quart2 | 0.43 6.9 | >0.064 na | 0.32 5.8 | 0.37 8.1 | >0.060 na | 0.37 8.2 |
| OR Quart 3 | 1.3 | >2.1 | 1.0 | 3.2 | >2.1 | 2.7 |
| p Value | 0.72 | <0.54 | 0.95 | 0.12 | <0.54 | 0.19 |
| 95% CI of OR Quart3 | 0.31 5.5 | >0.18 na | 0.23 4.7 | 0.75 14 | >0.18 na | 0.61 12 |
| OR Quart 4 | 1.0 | >0 | 1.0 | 1.3 | >0 | 1.3 |
| p Value | 0.96 | <na | 0.95 | 0.72 | <na | 0.73 |
| 95% CI of OR Quart4 | 0.23 4.7 | >na na | 0.23 4.7 | 0.27 6.6 | >na na | 0.27 6.7 |

**Antithrombin-III**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 110000 | 86200 | 110000 | 94500 |
| Average | nd | nd | 115000 | 109000 | 115000 | 97000 |
| Stdev | nd | nd | 36600 | 53700 | 36600 | 35200 |
| p(t-test) | nd | nd | | 0.66 | | 0.21 |
| Min | nd | nd | 36200 | 49300 | 36200 | 32000 |
| Max | nd | nd | 252000 | 206000 | 252000 | 140000 |
| n (Samp) | nd | nd | 112 | 9 | 112 | 7 |
| n (Patient) | nd | nd | 91 | 9 | 91 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 105000 | 86200 | 105000 | 102000 |
| Average | nd | nd | 110000 | 109000 | 110000 | 98400 |
| Stdev | nd | nd | 30700 | 53700 | 30700 | 38400 |
| p(t-test) | nd | nd | | 0.92 | | 0.38 |
| Min | nd | nd | 36200 | 49300 | 36200 | 32000 |
| Max | nd | nd | 186000 | 206000 | 186000 | 140000 |
| n (Samp) | nd | nd | 98 | 9 | 98 | 6 |
| n (Patient) | nd | nd | 76 | 9 | 76 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.41 | nd | 0.43 | 0.39 | nd | 0.44 |
| SE | nd | nd | nd | 0.10 | nd | 0.10 | 0.12 | nd | 0.13 |
| p | nd | nd | nd | 0.40 | nd | 0.51 | 0.34 | nd | 0.62 |
| nCohort 1 | nd | nd | nd | 112 | nd | 98 | 112 | nd | 98 |
| nCohort 2 | nd | nd | nd | 9 | nd | 9 | 7 | nd | 6 |
| Cutoff 1 | nd | nd | nd | 64700 | nd | 64700 | 88100 | nd | 83500 |
| Sens 1 | nd | nd | nd | 78% | nd | 78% | 71% | nd | 83% |
| Spec 1 | nd | nd | nd | 4% | nd | 5% | 23% | nd | 22% |
| Cutoff 2 | nd | nd | nd | 61900 | nd | 61900 | 83500 | nd | 83500 |
| Sens 2 | nd | nd | nd | 89% | nd | 89% | 86% | nd | 83% |
| Spec 2 | nd | nd | nd | 3% | nd | 3% | 21% | nd | 22% |
| Cutoff 3 | nd | nd | nd | 36200 | nd | 36200 | 0 | nd | 0 |
| Sens 3 | nd | nd | nd | 100% | nd | 100% | 100% | nd | 100% |
| Spec 3 | nd | nd | nd | 1% | nd | 1% | 0% | nd | 0% |
| Cutoff 4 | nd | nd | nd | 123000 | nd | 121000 | 123000 | nd | 121000 |
| Sens 4 | nd | nd | nd | 33% | nd | 33% | 29% | nd | 33% |
| Spec 4 | nd | nd | nd | 71% | nd | 70% | 71% | nd | 70% |
| Cutoff 5 | nd | nd | nd | 142000 | nd | 141000 | 142000 | nd | 141000 |
| Sens 5 | nd | nd | nd | 33% | nd | 33% | 0% | nd | 0% |
| Spec 5 | nd | nd | nd | 80% | nd | 82% | 80% | nd | 82% |
| Cutoff 6 | nd | nd | nd | 163000 | nd | 151000 | 163000 | nd | 151000 |
| Sens 6 | nd | nd | nd | 22% | nd | 22% | 0% | nd | 0% |
| Spec 6 | nd | nd | nd | 90% | nd | 91% | 90% | nd | 91% |
| OR Quart 2 | nd | nd | nd | 0 | nd | 0.31 | 1.0 | nd | 2.1 |
| p Value | nd | nd | nd | na | nd | 0.32 | 1.0 | nd | 0.56 |
| 95% CI of OR Quart2 | nd | nd | nd | na | nd | 0.030 | 0.060 | nd | 0.18 |
| | nd | nd | nd | na | nd | 3.2 | 17 | nd | 25 |
| OR Quart 3 | nd | nd | nd | 0.32 | nd | 0.31 | 3.2 | nd | 1.0 |
| p Value | nd | nd | nd | 0.34 | nd | 0.32 | 0.32 | nd | 1.0 |
| 95% CI of OR Quart3 | nd | nd | nd | 0.032 | nd | 0.030 | 0.32 | nd | 0.059 |
| | nd | nd | nd | 3.3 | nd | 3.2 | 33 | nd | 17 |
| OR Quart 4 | nd | nd | nd | 1.9 | nd | 1.5 | 2.1 | nd | 2.1 |
| p Value | nd | nd | nd | 0.42 | nd | 0.65 | 0.54 | nd | 0.56 |
| 95% CI of OR Quart4 | nd | nd | nd | 0.40 | nd | 0.29 | 0.18 | nd | 0.18 |
| | nd | nd | nd | 8.6 | nd | 7.2 | 25 | nd | 25 |

**Extracellular matrix protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1510 | 1350 | 1510 | 1490 |
| Average | nd | nd | 1580 | 1480 | 1580 | 1560 |
| Stdev | nd | nd | 458 | 434 | 458 | 410 |
| p(t-test) | nd | nd | | 0.51 | | 0.87 |
| Min | nd | nd | 535 | 846 | 535 | 945 |
| Max | nd | nd | 3060 | 2280 | 3060 | 2100 |
| n (Samp) | nd | nd | 112 | 9 | 112 | 7 |
| n (Patient) | nd | nd | 91 | 9 | 91 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1470 | 1350 | 1470 | 1450 |
| Average | nd | nd | 1560 | 1480 | 1560 | 1460 |
| Stdev | nd | nd | 468 | 434 | 468 | 364 |
| p(t-test) | nd | nd | | 0.60 | | 0.61 |
| Min | nd | nd | 535 | 846 | 535 | 945 |
| Max | nd | nd | 3060 | 2280 | 3060 | 1990 |
| n (Samp) | nd | nd | 98 | 9 | 98 | 6 |
| n (Patient) | nd | nd | 76 | 9 | 76 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.43 | nd | 0.44 | 0.49 | nd | 0.45 |
| SE | nd | nd | nd | 0.10 | nd | 0.10 | 0.11 | nd | 0.12 |
| p | nd | nd | nd | 0.47 | nd | 0.56 | 0.95 | nd | 0.70 |
| nCohort 1 | nd | nd | nd | 112 | nd | 98 | 112 | nd | 98 |
| nCohort 2 | nd | nd | nd | 9 | nd | 9 | 7 | nd | 6 |
| Cutoff 1 | nd | nd | nd | 1200 | nd | 1200 | 1400 | nd | 1230 |
| Sens 1 | nd | nd | nd | 78% | nd | 78% | 71% | nd | 83% |
| Spec 1 | nd | nd | nd | 18% | nd | 19% | 35% | nd | 21% |
| Cutoff 2 | nd | nd | nd | 1110 | nd | 1110 | 1230 | nd | 1230 |
| Sens 2 | nd | nd | nd | 89% | nd | 89% | 86% | nd | 83% |
| Spec 2 | nd | nd | nd | 14% | nd | 15% | 20% | nd | 21% |
| Cutoff 3 | nd | nd | nd | 779 | nd | 779 | 900 | nd | 900 |
| Sens 3 | nd | nd | nd | 100% | nd | 100% | 100% | nd | 100% |
| Spec 3 | nd | nd | nd | 3% | nd | 3% | 4% | nd | 5% |
| Cutoff 4 | nd | nd | nd | 1720 | nd | 1710 | 1720 | nd | 1710 |
| Sens 4 | nd | nd | nd | 33% | nd | 33% | 29% | nd | 33% |
| Spec 4 | nd | nd | nd | 71% | nd | 70% | 71% | nd | 70% |
| Cutoff 5 | nd | nd | nd | 1950 | nd | 1910 | 1950 | nd | 1910 |
| Sens 5 | nd | nd | nd | 11% | nd | 11% | 29% | nd | 17% |
| Spec 5 | nd | nd | nd | 81% | nd | 81% | 81% | nd | 81% |
| Cutoff 6 | nd | nd | nd | 2240 | nd | 2240 | 2240 | nd | 2240 |
| Sens 6 | nd | nd | nd | 11% | nd | 11% | 0% | nd | 0% |
| Spec 6 | nd | nd | nd | 90% | nd | 91% | 90% | nd | 91% |
| OR Quart 2 | nd | nd | nd | 0.50 | nd | 0 | 0.48 | nd | 2.1 |
| p Value | nd | nd | nd | 0.58 | nd | na | 0.56 | nd | 0.56 |
| 95% CI of | nd | nd | nd | 0.043 | nd | na | 0.041 | nd | 0.18 |
| OR Quart2 | nd | nd | nd | 5.8 | nd | na | 5.6 | nd | 25 |
| OR Quart 3 | nd | nd | nd | 1.6 | nd | 1.0 | 1.0 | nd | 1.0 |
| p Value | nd | nd | nd | 0.62 | nd | 1.0 | 1.0 | nd | 1.0 |
| 95% CI of | nd | nd | nd | 0.25 | nd | 0.18 | 0.13 | nd | 0.059 |
| OR Quart3 | nd | nd | nd | 10 | nd | 5.5 | 7.6 | nd | 17 |
| OR Quart 4 | nd | nd | nd | 1.6 | nd | 1.0 | 1.0 | nd | 2.1 |
| p Value | nd | nd | nd | 0.62 | nd | 0.96 | 0.97 | nd | 0.56 |
| 95% CI of | nd | nd | nd | 0.25 | nd | 0.19 | 0.14 | nd | 0.18 |
| OR Quart4 | nd | nd | nd | 10 | nd | 5.7 | 7.9 | nd | 25 |

**Coagulation factor XIII A and B chains**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 11300 | 10300 | 11300 | 10900 |
| Average | nd | nd | 12100 | 12800 | 12100 | 12700 |
| Stdev | nd | nd | 5970 | 6690 | 5970 | 3900 |
| p(t-test) | nd | nd | | 0.74 | | 0.79 |
| Min | nd | nd | 881 | 3550 | 881 | 8160 |
| Max | nd | nd | 33300 | 24900 | 33300 | 19200 |
| n (Samp) | nd | nd | 112 | 9 | 112 | 7 |
| n (Patient) | nd | nd | 91 | 9 | 91 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 10300 | 10300 | 10300 | 12100 |
| Average | nd | nd | 11400 | 12800 | 11400 | 13100 |
| Stdev | nd | nd | 5710 | 6690 | 5710 | 4070 |
| p(t-test) | nd | nd | | 0.51 | | 0.48 |
| Min | nd | nd | 881 | 3550 | 881 | 8160 |
| Max | nd | nd | 33300 | 24900 | 33300 | 19200 |
| n (Samp) | nd | nd | 98 | 9 | 98 | 6 |
| n (Patient) | nd | nd | 76 | 9 | 76 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.53 | nd | 0.56 | 0.56 | nd | 0.63 |
| SE | nd | nd | nd | 0.10 | nd | 0.10 | 0.12 | nd | 0.13 |
| p | nd | nd | nd | 0.77 | nd | 0.54 | 0.60 | nd | 0.30 |
| nCohort 1 | nd | nd | nd | 112 | nd | 98 | 112 | nd | 98 |
| nCohort 2 | nd | nd | nd | 9 | nd | 9 | 7 | nd | 6 |
| Cutoff 1 | nd | nd | nd | 9230 | nd | 9230 | 10800 | nd | 10800 |
| Sens 1 | nd | nd | nd | 78% | nd | 78% | 71% | nd | 83% |
| Spec 1 | nd | nd | nd | 41% | nd | 46% | 46% | nd | 53% |
| Cutoff 2 | nd | nd | nd | 7830 | nd | 7830 | 9230 | nd | 10800 |
| Sens 2 | nd | nd | nd | 89% | nd | 89% | 86% | nd | 83% |
| Spec 2 | nd | nd | nd | 22% | nd | 23% | 41% | nd | 53% |
| Cutoff 3 | nd | nd | nd | 3130 | nd | 3130 | 8080 | nd | 8080 |
| Sens 3 | nd | nd | nd | 100% | nd | 100% | 100% | nd | 100% |
| Spec 3 | nd | nd | nd | 6% | nd | 6% | 24% | nd | 27% |
| Cutoff 4 | nd | nd | nd | 14000 | nd | 13100 | 14000 | nd | 13100 |
| Sens 4 | nd | nd | nd | 33% | nd | 33% | 29% | nd | 50% |
| Spec 4 | nd | nd | nd | 71% | nd | 70% | 71% | nd | 70% |
| Cutoff 5 | nd | nd | nd | 16500 | nd | 15400 | 16500 | nd | 15400 |
| Sens 5 | nd | nd | nd | 22% | nd | 22% | 14% | nd | 33% |
| Spec 5 | nd | nd | nd | 80% | nd | 81% | 80% | nd | 81% |
| Cutoff 6 | nd | nd | nd | 21300 | nd | 19800 | 21300 | nd | 19800 |
| Sens 6 | nd | nd | nd | 11% | nd | 22% | 0% | nd | 0% |
| Spec 6 | nd | nd | nd | 90% | nd | 91% | 90% | nd | 91% |
| OR Quart 2 | nd | nd | nd | 1.6 | nd | 1.5 | 3.1 | nd | >1.0 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| p Value | nd | nd | nd | 0.64 | nd | 0.67 | 0.34 | nd | <0.98 |
| 95% CI of | nd | nd | nd | 0.24 | nd | 0.23 | 0.30 | nd | >0.062 |
| OR Quart2 | nd | nd | nd | 10 | nd | 9.8 | 32 | nd | na |
| OR Quart 3 | nd | nd | nd | 1.0 | nd | 0.46 | 0.97 | nd | >3.4 |
| p Value | nd | nd | nd | 1.0 | nd | 0.54 | 0.98 | nd | <0.30 |
| 95% CI of | nd | nd | nd | 0.13 | nd | 0.039 | 0.058 | nd | >0.33 |
| OR Quart3 | nd | nd | nd | 7.6 | nd | 5.4 | 16 | nd | na |
| OR Quart 4 | nd | nd | nd | 0.97 | nd | 1.5 | 2.0 | nd | >2.2 |
| p Value | nd | nd | nd | 0.97 | nd | 0.67 | 0.58 | nd | <0.54 |
| 95% CI of | nd | nd | nd | 0.13 | nd | 0.23 | 0.17 | nd | >0.18 |
| OR Quart4 | nd | nd | nd | 7.3 | nd | 9.8 | 23 | nd | na |

**Vitronectin**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 118000 | 111000 | 118000 | 87500 |
| Average | nd | nd | 121000 | 104000 | 121000 | 85900 |
| Stdev | nd | nd | 33500 | 45900 | 33500 | 49400 |
| p(t-test) | nd | nd | | 0.15 | | 0.010 |
| Min | nd | nd | 57400 | 35100 | 57400 | 21800 |
| Max | nd | nd | 205000 | 167000 | 205000 | 182000 |
| n (Samp) | nd | nd | 112 | 9 | 112 | 7 |
| n (Patient) | nd | nd | 91 | 9 | 91 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 118000 | 111000 | 118000 | 77500 |
| Average | nd | nd | 120000 | 104000 | 120000 | 85700 |
| Stdev | nd | nd | 33200 | 45900 | 33200 | 54100 |
| p(t-test) | nd | nd | | 0.18 | | 0.020 |
| Min | nd | nd | 57400 | 35100 | 57400 | 21800 |
| Max | nd | nd | 205000 | 167000 | 205000 | 182000 |
| n (Samp) | nd | nd | 98 | 9 | 98 | 6 |
| n (Patient) | nd | nd | 76 | 9 | 76 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.40 | nd | 0.41 | 0.23 | nd | 0.24 |
| SE | nd | nd | nd | 0.10 | nd | 0.10 | 0.11 | nd | 0.12 |
| p | nd | nd | nd | 0.36 | nd | 0.39 | 0.011 | nd | 0.030 |
| nCohort 1 | nd | nd | nd | 112 | nd | 98 | 112 | nd | 98 |
| nCohort 2 | nd | nd | nd | 9 | nd | 9 | 7 | nd | 6 |
| Cutoff 1 | nd | nd | nd | 83800 | nd | 79600 | 60700 | nd | 57400 |
| Sens 1 | nd | nd | nd | 78% | nd | 78% | 71% | nd | 83% |
| Spec 1 | nd | nd | nd | 12% | nd | 13% | 1% | nd | 1% |
| Cutoff 2 | nd | nd | nd | 35100 | nd | 35100 | 57400 | nd | 57400 |
| Sens 2 | nd | nd | nd | 89% | nd | 89% | 86% | nd | 83% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Spec 2 | nd | nd | nd | 0% | nd | 0% | 1% | nd | 1% |
| Cutoff 3 | nd | nd | nd | 0 | nd | 0 | 0 | nd | 0 |
| Sens 3 | nd | nd | nd | 100% | nd | 100% | 100% | nd | 100% |
| Spec 3 | nd | nd | nd | 0% | nd | 0% | 0% | nd | 0% |
| Cutoff 4 | nd | nd | nd | 134000 | nd | 133000 | 134000 | nd | 133000 |
| Sens 4 | nd | nd | nd | 22% | nd | 22% | 14% | nd | 17% |
| Spec 4 | nd | nd | nd | 71% | nd | 70% | 71% | nd | 70% |
| Cutoff 5 | nd | nd | nd | 150000 | nd | 149000 | 150000 | nd | 149000 |
| Sens 5 | nd | nd | nd | 22% | nd | 22% | 14% | nd | 17% |
| Spec 5 | nd | nd | nd | 80% | nd | 81% | 80% | nd | 81% |
| Cutoff 6 | nd | nd | nd | 172000 | nd | 172000 | 172000 | nd | 172000 |
| Sens 6 | nd | nd | nd | 0% | nd | 0% | 14% | nd | 17% |
| Spec 6 | nd | nd | nd | 90% | nd | 91% | 90% | nd | 91% |
| OR Quart 2 | nd | nd | nd | 1.0 | nd | 1.0 | 0 | nd | 0 |
| p Value | nd | nd | nd | 0.97 | nd | 1.0 | na | nd | na |
| 95% CI of OR Quart2 | nd | nd | nd | 0.14 | nd | 0.13 | na | nd | na |
|  | nd | nd | nd | 7.9 | nd | 7.7 | na | nd | na |
| OR Quart 3 | nd | nd | nd | 0.50 | nd | 0.48 | 2.1 | nd | 1.0 |
| p Value | nd | nd | nd | 0.58 | nd | 0.56 | 0.56 | nd | 1.0 |
| 95% CI of OR Quart3 | nd | nd | nd | 0.043 | nd | 0.041 | 0.18 | nd | 0.059 |
|  | nd | nd | nd | 5.8 | nd | 5.6 | 24 | nd | 17 |
| OR Quart 4 | nd | nd | nd | 2.2 | nd | 2.3 | 4.6 | nd | 4.5 |
| p Value | nd | nd | nd | 0.38 | nd | 0.37 | 0.18 | nd | 0.19 |
| 95% CI of OR Quart4 | nd | nd | nd | 0.38 | nd | 0.38 | 0.49 | nd | 0.47 |
|  | nd | nd | nd | 13 | nd | 14 | 44 | nd | 44 |

**Interleukin-17F**

| sCr or UO | 0hr prior to AKI stage | | 24hr prior to AKI stage | |
|---|---|---|---|---|
|  | Cohort 1 | Cohort 2 | Cohort 1 | Cohort 2 |
| Median | 0.227 | 0.182 | 0.227 | 0.243 |
| Average | 0.375 | 0.203 | 0.375 | 0.250 |
| Stdev | 0.663 | 0.0866 | 0.663 | 0.0981 |
| p(t-test) |  | 0.26 |  | 0.40 |
| Min | 2.43E-6 | 0.0608 | 2.43E-6 | 0.101 |
| Max | 4.55 | 0.381 | 4.55 | 0.468 |
| n (Samp) | 91 | 19 | 91 | 20 |
| n (Patient) | 45 | 19 | 45 | 20 |

| sCr only | 0hr prior to AKI stage | | 24hr prior to AKI stage | |
|---|---|---|---|---|
|  | Cohort 1 | Cohort 2 | Cohort 1 | Cohort 2 |
| Median | 0.227 | 0.225 | 0.227 | 0.259 |
| Average | 0.337 | 0.194 | 0.337 | 0.233 |
| Stdev | 0.575 | 0.0644 | 0.575 | 0.0957 |
| p(t-test) |  | 0.67 |  | 0.75 |
| Min | 2.43E-6 | 0.120 | 2.43E-6 | 0.126 |
| Max | 4.55 | 0.237 | 4.55 | 0.312 |
| n (Samp) | 123 | 3 | 123 | 3 |

| sCr only | 0hr prior to AKI stage | | 24hr prior to AKI stage | |
|---|---|---|---|---|
| | Cohort 1 | Cohort 2 | Cohort 1 | Cohort 2 |
| n (Patient) | 61 | 3 | 61 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.239 | 0.182 | 0.239 | 0.233 |
| Average | 0.394 | 0.206 | 0.394 | 0.250 |
| Stdev | 0.686 | 0.0891 | 0.686 | 0.101 |
| p(t-test) | | 0.26 | | 0.36 |
| Min | 2.43E-6 | 0.0608 | 2.43E-6 | 0.101 |
| Max | 4.55 | 0.381 | 4.55 | 0.468 |
| n (Samp) | 84 | 17 | 84 | 19 |
| n (Patient) | 42 | 17 | 42 | 19 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.37 | 0.38 | 0.36 | 0.51 | 0.50 | 0.48 |
| SE | 0.074 | 0.18 | 0.078 | 0.072 | 0.17 | 0.074 |
| p | 0.091 | 0.50 | 0.072 | 0.93 | 0.99 | 0.79 |
| nCohort 1 | 91 | 123 | 84 | 91 | 123 | 84 |
| nCohort 2 | 19 | 3 | 17 | 20 | 3 | 19 |
| Cutoff 1 | 0.147 | 0.115 | 0.165 | 0.203 | 0.120 | 0.175 |
| Sens 1 | 74% | 100% | 71% | 70% | 100% | 74% |
| Spec 1 | 18% | 11% | 21% | 36% | 12% | 25% |
| Cutoff 2 | 0.120 | 0.115 | 0.131 | 0.170 | 0.120 | 0.169 |
| Sens 2 | 84% | 100% | 82% | 80% | 100% | 84% |
| Spec 2 | 12% | 11% | 12% | 25% | 12% | 24% |
| Cutoff 3 | 0.106 | 0.115 | 0.106 | 0.139 | 0.120 | 0.101 |
| Sens 3 | 95% | 100% | 94% | 90% | 100% | 95% |
| Spec 3 | 9% | 11% | 5% | 16% | 12% | 4% |
| Cutoff 4 | 0.299 | 0.295 | 0.299 | 0.299 | 0.295 | 0.299 |
| Sens 4 | 16% | 0% | 18% | 25% | 33% | 26% |
| Spec 4 | 70% | 71% | 70% | 70% | 71% | 70% |
| Cutoff 5 | 0.339 | 0.337 | 0.356 | 0.339 | 0.337 | 0.356 |
| Sens 5 | 11% | 0% | 12% | 15% | 0% | 16% |
| Spec 5 | 80% | 80% | 81% | 80% | 80% | 81% |
| Cutoff 6 | 0.473 | 0.410 | 0.529 | 0.473 | 0.410 | 0.529 |
| Sens 6 | 0% | 0% | 0% | 0% | 0% | 0% |
| Spec 6 | 90% | 90% | 90% | 90% | 90% | 90% |
| OR Quart 2 | 1.9 | >1.1 | 1.0 | 1.2 | 0 | 0.76 |
| p Value | 0.42 | <0.96 | 0.96 | 0.76 | na | 0.71 |
| 95% CI of | 0.41 | >0.064 | 0.19 | 0.30 | na | 0.18 |
| OR Quart2 | 8.9 | na | 5.7 | 5.3 | na | 3.2 |
| OR Quart 3 | 1.8 | >1.0 | 1.5 | 1.6 | 1.0 | 1.0 |
| p Value | 0.45 | <0.98 | 0.64 | 0.53 | 1.0 | 1.0 |
| 95% CI of | 0.39 | >0.062 | 0.29 | 0.39 | 0.060 | 0.25 |
| OR Quart3 | 8.4 | na | 7.3 | 6.3 | 17 | 4.0 |
| OR Quart 4 | 2.4 | >1.1 | 3.0 | 1.2 | 0.97 | 1.0 |

|            | 0hr prior to AKI stage | | | 24hr prior to AKI stage | | |
|------------|-----------|----------|---------|-----------|----------|---------|
|            | sCr or UO | sCr only | UO only | sCr or UO | sCr only | UO only |
| p Value    | 0.26      | <0.96    | 0.15    | 0.76      | 0.98     | 0.94    |
| 95% CI of  | 0.53      | >0.064   | 0.67    | 0.30      | 0.058    | 0.26    |
| OR Quart4  | 11        | na       | 13      | 5.3       | 16       | 4.2     |

**Interleukin-22**

| sCr or UO  | 0hr prior to AKI stage | | 24hr prior to AKI stage | |
|------------|----------|----------|----------|----------|
|            | Cohort 1 | Cohort 2 | Cohort 1 | Cohort 2 |
| Median     | 0.734    | 0.572    | 0.734    | 0.806    |
| Average    | 1.24     | 0.603    | 1.24     | 0.815    |
| Stdev      | 2.20     | 0.326    | 2.20     | 0.460    |
| p(t-test)  |          | 0.21     |          | 0.39     |
| Min        | 4.00E-6  | 0.117    | 4.00E-6  | 0.195    |
| Max        | 15.3     | 1.52     | 15.3     | 1.85     |
| n (Samp)   | 91       | 19       | 91       | 20       |
| n (Patient)| 45       | 19       | 45       | 20       |

| sCr only   | 0hr prior to AKI stage | | 24hr prior to AKI stage | |
|------------|----------|----------|----------|----------|
|            | Cohort 1 | Cohort 2 | Cohort 1 | Cohort 2 |
| Median     | 0.709    | 0.591    | 0.709    | 0.829    |
| Average    | 1.11     | 0.570    | 1.11     | 0.703    |
| Stdev      | 1.91     | 0.228    | 1.91     | 0.382    |
| p(t-test)  |          | 0.63     |          | 0.72     |
| Min        | 4.00E-6  | 0.332    | 4.00E-6  | 0.274    |
| Max        | 15.3     | 0.787    | 15.3     | 1.01     |
| n (Samp)   | 123      | 3        | 123      | 3        |
| n (Patient)| 61       | 3        | 61       | 3        |

| UO only    | 0hr prior to AKI stage | | 24hr prior to AKI stage | |
|------------|----------|----------|----------|----------|
|            | Cohort 1 | Cohort 2 | Cohort 1 | Cohort 2 |
| Median     | 0.764    | 0.572    | 0.764    | 0.783    |
| Average    | 1.31     | 0.608    | 1.31     | 0.814    |
| Stdev      | 2.27     | 0.336    | 2.27     | 0.472    |
| p(t-test)  |          | 0.21     |          | 0.35     |
| Min        | 4.00E-6  | 0.117    | 4.00E-6  | 0.195    |
| Max        | 15.3     | 1.52     | 15.3     | 1.85     |
| n (Samp)   | 84       | 17       | 84       | 19       |
| n (Patient)| 42       | 17       | 42       | 19       |

|          | 0hr prior to AKI stage | | | 24hr prior to AKI stage | | |
|----------|-----------|----------|---------|-----------|----------|---------|
|          | sCr or UO | sCr only | UO only | sCr or UO | sCr only | UO only |
| AUC      | 0.33      | 0.34     | 0.30    | 0.50      | 0.48     | 0.48    |
| SE       | 0.073     | 0.17     | 0.076   | 0.072     | 0.17     | 0.074   |
| p        | 0.019     | 0.35     | 0.0091  | 0.97      | 0.91     | 0.74    |
| nCohort 1| 91        | 123      | 84      | 91        | 123      | 84      |
| nCohort 2| 19        | 3        | 17      | 20        | 3        | 19      |
| Cutoff 1 | 0.370     | 0.286    | 0.423   | 0.492     | 0.219    | 0.489   |

| | 0hr prior to AKI stage | | | 24hr prior to AKI stage | | |
|---|---|---|---|---|---|---|
| | sCr or UO | sCr only | UO only | sCr or UO | sCr only | UO only |
| Sens 1 | 74% | 100% | 71% | 70% | 100% | 74% |
| Spec 1 | 15% | 10% | 13% | 24% | 8% | 21% |
| Cutoff 2 | 0.335 | 0.286 | 0.369 | 0.469 | 0.219 | 0.369 |
| Sens 2 | 84% | 100% | 82% | 80% | 100% | 84% |
| Spec 2 | 10% | 10% | 12% | 22% | 8% | 12% |
| Cutoff 3 | 0.219 | 0.286 | 0.209 | 0.219 | 0.219 | 0.195 |
| Sens 3 | 95% | 100% | 94% | 90% | 100% | 95% |
| Spec 3 | 8% | 10% | 4% | 8% | 8% | 2% |
| Cutoff 4 | 0.953 | 0.920 | 0.953 | 0.953 | 0.920 | 0.953 |
| Sens 4 | 11% | 0% | 12% | 30% | 33% | 32% |
| Spec 4 | 70% | 71% | 70% | 70% | 71% | 70% |
| Cutoff 5 | 1.13 | 1.10 | 1.14 | 1.13 | 1.10 | 1.14 |
| Sens 5 | 11% | 0% | 12% | 15% | 0% | 16% |
| Spec 5 | 80% | 80% | 81% | 80% | 80% | 81% |
| Cutoff 6 | 1.60 | 1.52 | 1.63 | 1.60 | 1.52 | 1.63 |
| Sens 6 | 0% | 0% | 0% | 10% | 0% | 11% |
| Spec 6 | 90% | 90% | 90% | 90% | 90% | 90% |
| OR Quart 2 | 2.3 | >1.1 | 1.6 | 1.3 | 1.0 | 1.0 |
| p Value | 0.37 | <0.96 | 0.61 | 0.74 | 0.98 | 1.0 |
| 95% CI of | 0.38 | >0.064 | 0.25 | 0.33 | 0.062 | 0.25 |
| OR Quart2 | 14 | na | 11 | 4.7 | 17 | 4.0 |
| OR Quart 3 | 2.8 | >1.0 | 3.0 | 0.77 | 0 | 0.35 |
| p Value | 0.24 | <0.98 | 0.22 | 0.72 | na | 0.24 |
| 95% CI of | 0.50 | >0.062 | 0.52 | 0.18 | na | 0.061 |
| OR Quart3 | 16 | na | 17 | 3.2 | na | 2.0 |
| OR Quart 4 | 5.5 | >1.1 | 4.7 | 1.0 | 1.0 | 1.6 |
| p Value | 0.045 | <0.96 | 0.073 | 0.95 | 0.98 | 0.46 |
| 95% CI of | 1.0 | >0.064 | 0.86 | 0.27 | 0.062 | 0.44 |
| OR Quart4 | 29 | na | 25 | 4.1 | 17 | 6.0 |

**T3**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.10 | 0.984 | 1.10 | 0.551 |
| Average | nd | nd | 1.18 | 0.988 | 1.18 | 0.616 |
| Stdev | nd | nd | 0.593 | 0.550 | 0.593 | 0.543 |
| p(t-test) | nd | nd | | 0.36 | | 0.016 |
| Min | nd | nd | 0.0946 | 0.000162 | 0.0946 | 0.000162 |
| Max | nd | nd | 3.76 | 1.59 | 3.76 | 1.34 |
| n (Samp) | nd | nd | 110 | 9 | 110 | 7 |
| n (Patient) | nd | nd | 90 | 9 | 90 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 1.07 | 0.984 | 1.07 | 0.528 |
| Average | nd | nd | 1.14 | 0.988 | 1.14 | 0.502 |
| Stdev | nd | nd | 0.554 | 0.550 | 0.554 | 0.495 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| p(t-test) | nd | nd | | 0.44 | | 0.0071 |
| Min | nd | nd | 0.0946 | 0.000162 | 0.0946 | 0.000162 |
| Max | nd | nd | 3.18 | 1.59 | 3.18 | 1.34 |
| n (Samp) | nd | nd | 96 | 9 | 96 | 6 |
| n (Patient) | nd | nd | 75 | 9 | 75 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.44 | nd | 0.45 | 0.23 | nd | 0.17 |
| SE | nd | nd | nd | 0.10 | nd | 0.10 | 0.11 | nd | 0.10 |
| p | nd | nd | nd | 0.54 | nd | 0.63 | 0.012 | nd | 0.0014 |
| nCohort 1 | nd | nd | nd | 110 | nd | 96 | 110 | nd | 96 |
| nCohort 2 | nd | nd | nd | 9 | nd | 9 | 7 | nd | 6 |
| Cutoff 1 | nd | nd | nd | 0.510 | nd | 0.510 | 0.500 | nd | 0.000162 |
| Sens 1 | nd | nd | nd | 78% | nd | 78% | 71% | nd | 83% |
| Spec 1 | nd | nd | nd | 10% | nd | 10% | 8% | nd | 0% |
| Cutoff 2 | nd | nd | nd | 0.424 | nd | 0.424 | 0.000162 | nd | 0.000162 |
| Sens 2 | nd | nd | nd | 89% | nd | 89% | 86% | nd | 83% |
| Spec 2 | nd | nd | nd | 6% | nd | 6% | 0% | nd | 0% |
| Cutoff 3 | nd | nd | nd | 0 | nd | 0 | 0 | nd | 0 |
| Sens 3 | nd | nd | nd | 100% | nd | 100% | 100% | nd | 100% |
| Spec 3 | nd | nd | nd | 0% | nd | 0% | 0% | nd | 0% |
| Cutoff 4 | nd | nd | nd | 1.39 | nd | 1.34 | 1.39 | nd | 1.34 |
| Sens 4 | nd | nd | nd | 33% | nd | 33% | 0% | nd | 17% |
| Spec 4 | nd | nd | nd | 70% | nd | 71% | 70% | nd | 71% |
| Cutoff 5 | nd | nd | nd | 1.60 | nd | 1.60 | 1.60 | nd | 1.60 |
| Sens 5 | nd | nd | nd | 0% | nd | 0% | 0% | nd | 0% |
| Spec 5 | nd | nd | nd | 80% | nd | 80% | 80% | nd | 80% |
| Cutoff 6 | nd | nd | nd | 1.86 | nd | 1.86 | 1.86 | nd | 1.86 |
| Sens 6 | nd | nd | nd | 0% | nd | 0% | 0% | nd | 0% |
| Spec 6 | nd | nd | nd | 90% | nd | 91% | 90% | nd | 91% |
| OR Quart 2 | nd | nd | nd | 1.0 | nd | 0.32 | >2.2 | nd | >1.1 |
| p Value | nd | nd | nd | 1.0 | nd | 0.34 | <0.52 | nd | <0.96 |
| 95% CI of OR Quart2 | nd | nd | nd | 0.13 7.6 | nd | 0.031 3.3 | >0.19 na | nd | >0.064 na |
| OR Quart 3 | nd | nd | nd | 1.0 | nd | 0.67 | >0 | nd | >0 |
| p Value | nd | nd | nd | 1.0 | nd | 0.67 | <na | nd | <na |
| 95% CI of OR Quart3 | nd | nd | nd | 0.13 7.6 | nd | 0.10 4.4 | >na na | nd | >na na |
| OR Quart 4 | nd | nd | nd | 1.6 | nd | 1.0 | >6.2 | nd | >6.5 |
| p Value | nd | nd | nd | 0.61 | nd | 0.96 | <0.10 | nd | <0.099 |
| 95% CI of OR Quart4 | nd | nd | nd | 0.25 10 | nd | 0.19 5.7 | >0.68 na | nd | >0.70 na |

T4

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 2.57 | 1.79 | 2.57 | 1.37 |
| Average | nd | nd | 3.16 | 2.20 | 3.16 | 1.46 |
| Stdev | nd | nd | 2.79 | 1.92 | 2.79 | 1.15 |
| p(t-test) | nd | nd | | 0.31 | | 0.11 |
| Min | nd | nd | 0.000136 | 0.000327 | 0.000136 | 0.000136 |
| Max | nd | nd | 22.2 | 4.97 | 22.2 | 3.13 |
| n (Samp) | nd | nd | 110 | 9 | 110 | 7 |
| n (Patient) | nd | nd | 90 | 9 | 90 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 2.53 | 1.79 | 2.53 | 1.54 |
| Average | nd | nd | 3.01 | 2.20 | 3.01 | 1.51 |
| Stdev | nd | nd | 2.71 | 1.92 | 2.71 | 1.25 |
| p(t-test) | nd | nd | | 0.38 | | 0.18 |
| Min | nd | nd | 0.000136 | 0.000327 | 0.000136 | 0.000136 |
| Max | nd | nd | 22.2 | 4.97 | 22.2 | 3.13 |
| n (Samp) | nd | nd | 96 | 9 | 96 | 6 |
| n (Patient) | nd | nd | 75 | 9 | 75 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.39 | nd | 0.41 | 0.26 | nd | 0.29 |
| SE | nd | nd | nd | 0.10 | nd | 0.10 | 0.11 | nd | 0.12 |
| p | nd | nd | nd | 0.30 | nd | 0.36 | 0.033 | nd | 0.092 |
| nCohort 1 | nd | nd | nd | 110 | nd | 96 | 110 | nd | 96 |
| nCohort 2 | nd | nd | nd | 9 | nd | 9 | 7 | nd | 6 |
| Cutoff 1 | nd | nd | nd | 0.256 | nd | 0.256 | 1.13 | nd | 0.196 |
| Sens 1 | nd | nd | nd | 78% | nd | 78% | 71% | nd | 83% |
| Spec 1 | nd | nd | nd | 5% | nd | 6% | 18% | nd | 5% |
| Cutoff 2 | nd | nd | nd | 0.000136 | nd | 0.000136 | 0.196 | nd | 0.196 |
| Sens 2 | nd | nd | nd | 100% | nd | 100% | 86% | nd | 83% |
| Spec 2 | nd | nd | nd | 1% | nd | 1% | 5% | nd | 5% |
| Cutoff 3 | nd | nd | nd | 0.000136 | nd | 0.000136 | 0 | nd | 0 |
| Sens 3 | nd | nd | nd | 100% | nd | 100% | 100% | nd | 100% |
| Spec 3 | nd | nd | nd | 1% | nd | 1% | 0% | nd | 0% |
| Cutoff 4 | nd | nd | nd | 3.66 | nd | 3.57 | 3.66 | nd | 3.57 |
| Sens 4 | nd | nd | nd | 33% | nd | 33% | 0% | nd | 0% |
| Spec 4 | nd | nd | nd | 70% | nd | 71% | 70% | nd | 71% |
| Cutoff 5 | nd | nd | nd | 4.47 | nd | 4.44 | 4.47 | nd | 4.44 |
| Sens 5 | nd | nd | nd | 11% | nd | 11% | 0% | nd | 0% |
| Spec 5 | nd | nd | nd | 80% | nd | 80% | 80% | nd | 80% |
| Cutoff 6 | nd | nd | nd | 6.18 | nd | 5.67 | 6.18 | nd | 5.67 |
| Sens 6 | nd | nd | nd | 0% | nd | 0% | 0% | nd | 0% |
| Spec 6 | nd | nd | nd | 90% | nd | 91% | 90% | nd | 91% |
| OR Quart 2 | nd | nd | nd | 1.0 | nd | 1.0 | >2.2 | nd | >2.3 |
| p Value | nd | nd | nd | 1.0 | nd | 0.97 | <0.52 | nd | <0.52 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| 95% CI of | nd | nd | nd | 0.13 | nd | 0.14 | >0.19 | nd | >0.19 |
| OR Quart2 | nd | nd | nd | 7.6 | nd | 8.0 | na | nd | na |
| OR Quart 3 | nd | nd | nd | 1.0 | nd | 1.0 | >2.2 | nd | >2.2 |
| p Value | nd | nd | nd | 1.0 | nd | 0.97 | <0.52 | nd | <0.54 |
| 95% CI of | nd | nd | nd | 0.13 | nd | 0.14 | >0.19 | nd | >0.18 |
| OR Quart3 | nd | nd | nd | 7.6 | nd | 8.0 | na | nd | na |
| OR Quart 4 | nd | nd | nd | 1.6 | nd | 1.6 | >3.5 | nd | >2.3 |
| p Value | nd | nd | nd | 0.61 | nd | 0.61 | <0.29 | nd | <0.52 |
| 95% CI of | nd | nd | nd | 0.25 | nd | 0.25 | >0.34 | nd | >0.19 |
| OR Quart4 | nd | nd | nd | 10 | nd | 11 | na | nd | na |

**Proprotein convertase subtilisin/kexin type 9**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 505000 | 291000 | 505000 | 345000 |
| Average | nd | nd | 523000 | 342000 | 523000 | 330000 |
| Stdev | nd | nd | 203000 | 173000 | 203000 | 167000 |
| p(t-test) | nd | nd | | 0.010 | | 0.015 |
| Min | nd | nd | 76300 | 102000 | 76300 | 80700 |
| Max | nd | nd | 1100000 | 596000 | 1100000 | 529000 |
| n (Samp) | nd | nd | 112 | 9 | 112 | 7 |
| n (Patient) | nd | nd | 91 | 9 | 91 | 7 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | nd | nd | 496000 | 291000 | 496000 | 303000 |
| Average | nd | nd | 518000 | 342000 | 518000 | 297000 |
| Stdev | nd | nd | 199000 | 173000 | 199000 | 156000 |
| p(t-test) | nd | nd | | 0.012 | | 0.0090 |
| Min | nd | nd | 76300 | 102000 | 76300 | 80700 |
| Max | nd | nd | 1000000 | 596000 | 1000000 | 511000 |
| n (Samp) | nd | nd | 98 | 9 | 98 | 6 |
| n (Patient) | nd | nd | 76 | 9 | 76 | 6 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | nd | nd | nd | 0.25 | nd | 0.26 | 0.24 | nd | 0.20 |
| SE | nd | nd | nd | 0.097 | nd | 0.098 | 0.11 | nd | 0.11 |
| p | nd | nd | nd | 0.0093 | nd | 0.013 | 0.018 | nd | 0.0064 |
| nCohort 1 | nd | nd | nd | 112 | nd | 98 | 112 | nd | 98 |
| nCohort 2 | nd | nd | nd | 9 | nd | 9 | 7 | nd | 6 |
| Cutoff 1 | nd | nd | nd | 261000 | nd | 261000 | 246000 | nd | 176000 |
| Sens 1 | nd | nd | nd | 78% | nd | 78% | 71% | nd | 83% |
| Spec 1 | nd | nd | nd | 10% | nd | 11% | 9% | nd | 2% |
| Cutoff 2 | nd | nd | nd | 102000 | nd | 102000 | 176000 | nd | 176000 |
| Sens 2 | nd | nd | nd | 89% | nd | 89% | 86% | nd | 83% |
| Spec 2 | nd | nd | nd | 1% | nd | 1% | 3% | nd | 2% |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Cutoff 3 | nd | nd | nd | 76300 | nd | 76300 | 76300 | nd | 76300 |
| Sens 3 | nd | nd | nd | 100% | nd | 100% | 100% | nd | 100% |
| Spec 3 | nd | nd | nd | 1% | nd | 1% | 1% | nd | 1% |
| Cutoff 4 | nd | nd | nd | 620000 | nd | 620000 | 620000 | nd | 620000 |
| Sens 4 | nd | nd | nd | 0% | nd | 0% | 0% | nd | 0% |
| Spec 4 | nd | nd | nd | 71% | nd | 70% | 71% | nd | 70% |
| Cutoff 5 | nd | nd | nd | 698000 | nd | 698000 | 698000 | nd | 698000 |
| Sens 5 | nd | nd | nd | 0% | nd | 0% | 0% | nd | 0% |
| Spec 5 | nd | nd | nd | 80% | nd | 81% | 80% | nd | 81% |
| Cutoff 6 | nd | nd | nd | 826000 | nd | 826000 | 826000 | nd | 826000 |
| Sens 6 | nd | nd | nd | 0% | nd | 0% | 0% | nd | 0% |
| Spec 6 | nd | nd | nd | 90% | nd | 91% | 90% | nd | 91% |
| OR Quart 2 | nd | nd | nd | >2.2 | nd | >2.2 | >2.1 | nd | >1.0 |
| p Value | nd | nd | nd | <0.53 | nd | <0.54 | <0.54 | nd | <0.98 |
| 95% CI of OR Quart2 | nd | nd | nd | >0.19 na | nd | >0.18 na | >0.18 na | nd | >0.062 na |
| OR Quart 3 | nd | nd | nd | >2.2 | nd | >2.2 | >1.0 | nd | >2.2 |
| p Value | nd | nd | nd | <0.53 | nd | <0.54 | <0.98 | nd | <0.54 |
| 95% CI of OR Quart3 | nd | nd | nd | >0.19 na | nd | >0.18 na | >0.062 na | nd | >0.18 na |
| OR Quart 4 | nd | nd | nd | >6.2 | nd | >6.4 | >4.8 | nd | >3.4 |
| p Value | nd | nd | nd | <0.11 | nd | <0.10 | <0.17 | nd | <0.30 |
| 95% CI of OR Quart4 | nd | nd | nd | >0.68 na | nd | >0.70 na | >0.50 na | nd | >0.33 na |

Table 7: Comparison of marker levels in EDTA samples collected within 12 hours of reaching stage R from Cohort 1 (patients that reached, but did not progress beyond, RIFLE stage R) and from Cohort 2 (patients that reached RIFLE stage I or F).

**Estradiol**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 1.42 | 3.82 | nd | nd | 1.82 | 1.84 |
| Average | 2.39 | 4.90 | nd | nd | 2.74 | 5.46 |
| Stdev | 1.91 | 5.70 | nd | nd | 2.04 | 8.00 |
| p(t-test) |  | 0.074 | nd | nd |  | 0.19 |
| Min | 0.642 | 0.774 | nd | nd | 0.642 | 0.774 |
| Max | 7.74 | 17.4 | nd | nd | 7.74 | 17.4 |
| n (Samp) | 23 | 7 | nd | nd | 18 | 4 |
| n (Patient) | 23 | 7 | nd | nd | 18 | 4 |

|  | At Enrollment | | |
|---|---|---|---|
|  | sCr or UO | sCr only | UO only |
| AUC | 0.64 | nd | 0.46 |
| SE | 0.13 | nd | 0.17 |
| p | 0.27 | nd | 0.80 |

|  | At Enrollment | | |
|---|---|---|---|
|  | sCr or UO | sCr only | UO only |
| nCohort 1 | 23 | nd | 18 |
| nCohort 2 | 7 | nd | 4 |
| Cutoff 1 | 1.99 | nd | 0.979 |
| Sens 1 | 71% | nd | 75% |
| Spec 1 | 65% | nd | 17% |
| Cutoff 2 | 1.08 | nd | 0.732 |
| Sens 2 | 86% | nd | 100% |
| Spec 2 | 22% | nd | 11% |
| Cutoff 3 | 0.732 | nd | 0.732 |
| Sens 3 | 100% | nd | 100% |
| Spec 3 | 13% | nd | 11% |
| Cutoff 4 | 3.22 | nd | 3.24 |
| Sens 4 | 57% | nd | 25% |
| Spec 4 | 74% | nd | 72% |
| Cutoff 5 | 3.48 | nd | 4.66 |
| Sens 5 | 57% | nd | 25% |
| Spec 5 | 83% | nd | 83% |
| Cutoff 6 | 4.89 | nd | 6.27 |
| Sens 6 | 14% | nd | 25% |
| Spec 6 | 91% | nd | 94% |
| OR Quart 2 | 0 | nd | 1.2 |
| p Value | na | nd | 0.89 |
| 95% CI of | na | nd | 0.058 |
| OR Quart2 | na | nd | 27 |
| OR Quart 3 | 0.42 | nd | 0 |
| p Value | 0.52 | nd | na |
| 95% CI of | 0.029 | nd | na |
| OR Quart3 | 6.1 | nd | na |
| OR Quart 4 | 2.5 | nd | 3.3 |
| p Value | 0.40 | nd | 0.40 |
| 95% CI of | 0.29 | nd | 0.20 |
| OR Quart4 | 21 | nd | 55 |

**Growth/differentiation factor 15**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 2080 | 2160 | nd | nd | 2600 | 1500 |
| Average | 2420 | 3510 | nd | nd | 2610 | 2340 |
| Stdev | 1510 | 2640 | nd | nd | 1580 | 2240 |
| p(t-test) |  | 0.17 | nd | nd |  | 0.77 |
| Min | 437 | 750 | nd | nd | 437 | 750 |
| Max | 6010 | 7320 | nd | nd | 6010 | 5600 |
| n (Samp) | 23 | 7 | nd | nd | 18 | 4 |
| n (Patient) | 23 | 7 | nd | nd | 18 | 4 |

|  | At Enrollment | | |
|---|---|---|---|
|  | sCr or UO | sCr only | UO only |

|  | At Enrollment | | |
|---|---|---|---|
|  | sCr or UO | sCr only | UO only |
| AUC | 0.60 | nd | 0.40 |
| SE | 0.13 | nd | 0.17 |
| p | 0.45 | nd | 0.56 |
| nCohort 1 | 23 | nd | 18 |
| nCohort 2 | 7 | nd | 4 |
| Cutoff 1 | 1740 | nd | 829 |
| Sens 1 | 71% | nd | 75% |
| Spec 1 | 43% | nd | 17% |
| Cutoff 2 | 829 | nd | 660 |
| Sens 2 | 86% | nd | 100% |
| Spec 2 | 17% | nd | 11% |
| Cutoff 3 | 660 | nd | 660 |
| Sens 3 | 100% | nd | 100% |
| Spec 3 | 13% | nd | 11% |
| Cutoff 4 | 3120 | nd | 3120 |
| Sens 4 | 43% | nd | 25% |
| Spec 4 | 74% | nd | 72% |
| Cutoff 5 | 3530 | nd | 3920 |
| Sens 5 | 43% | nd | 25% |
| Spec 5 | 83% | nd | 83% |
| Cutoff 6 | 4700 | nd | 5190 |
| Sens 6 | 43% | nd | 25% |
| Spec 6 | 91% | nd | 94% |
| OR Quart 2 | 0.36 | nd | 0 |
| p Value | 0.45 | nd | na |
| 95% CI of | 0.025 | nd | na |
| OR Quart2 | 5.1 | nd | na |
| OR Quart 3 | 0.42 | nd | 1.0 |
| p Value | 0.52 | nd | 1.0 |
| 95% CI of | 0.029 | nd | 0.048 |
| OR Quart3 | 6.1 | nd | 21 |
| OR Quart 4 | 1.5 | nd | 3.3 |
| p Value | 0.72 | nd | 0.40 |
| 95% CI of | 0.17 | nd | 0.20 |
| OR Quart4 | 13 | nd | 55 |

**Proprotein convertase subtilisin/kexin type 9**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 522000 | 282000 | nd | nd | 523000 | 351000 |
| Average | 520000 | 321000 | nd | nd | 526000 | 334000 |
| Stdev | 195000 | 190000 | nd | nd | 210000 | 169000 |
| p(t-test) |  | 0.025 | nd | nd |  | 0.10 |
| Min | 176000 | 80700 | nd | nd | 176000 | 123000 |
| Max | 1000000 | 580000 | nd | nd | 1000000 | 511000 |
| n (Samp) | 23 | 7 | nd | nd | 18 | 4 |
| n (Patient) | 23 | 7 | nd | nd | 18 | 4 |

| | At Enrollment | | |
|---|---|---|---|
| | sCr or UO | sCr only | UO only |
| AUC | 0.24 | nd | 0.19 |
| SE | 0.11 | nd | 0.14 |
| p | 0.021 | nd | 0.030 |
| nCohort 1 | 23 | nd | 18 |
| nCohort 2 | 7 | nd | 4 |
| Cutoff 1 | 210000 | nd | 210000 |
| Sens 1 | 71% | nd | 75% |
| Spec 1 | 13% | nd | 17% |
| Cutoff 2 | 80700 | nd | 0 |
| Sens 2 | 86% | nd | 100% |
| Spec 2 | 0% | nd | 0% |
| Cutoff 3 | 0 | nd | 0 |
| Sens 3 | 100% | nd | 100% |
| Spec 3 | 0% | nd | 0% |
| Cutoff 4 | 620000 | nd | 604000 |
| Sens 4 | 0% | nd | 0% |
| Spec 4 | 74% | nd | 72% |
| Cutoff 5 | 654000 | nd | 654000 |
| Sens 5 | 0% | nd | 0% |
| Spec 5 | 83% | nd | 83% |
| Cutoff 6 | 764000 | nd | 786000 |
| Sens 6 | 0% | nd | 0% |
| Spec 6 | 91% | nd | 94% |
| OR Quart 2 | 1.2 | nd | >1.5 |
| p Value | 0.92 | nd | <0.79 |
| 95% CI of | 0.059 | nd | >0.071 |
| OR Quart2 | 23 | nd | na |
| OR Quart 3 | 1.0 | nd | >1.2 |
| p Value | 1.0 | nd | <0.91 |
| 95% CI of | 0.052 | nd | >0.059 |
| OR Quart3 | 19 | nd | na |
| OR Quart 4 | 9.3 | nd | >4.0 |
| p Value | 0.089 | nd | <0.33 |
| 95% CI of | 0.71 | nd | >0.25 |
| OR Quart4 | 120 | nd | na |

Table 8: Comparison of the maximum marker levels in EDTA samples collected from Cohort 1 (patients that did not progress beyond RIFLE stage 0) and the maximum values in EDTA samples collected from subjects between enrollment and 0, 24 hours, and 48 hours prior to reaching stage F in Cohort 2.

**Toll-like receptor 2**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Mcdian | 0.0902 | 0.0961 | 0.0902 | 0.0648 | 0.0902 | 0.0925 |
| Average | 1.96 | 0.141 | 1.96 | 0.121 | 1.96 | 0.0768 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Stdev | 5.50 | 0.168 | 5.50 | 0.162 | 5.50 | 0.0649 |
| p(t-test) | | 0.36 | | 0.36 | | 0.56 |
| Min | 0.00107 | 0.00551 | 0.00107 | 0.00551 | 0.00107 | 0.00551 |
| Max | 19.7 | 0.531 | 19.7 | 0.496 | 19.7 | 0.132 |
| n (Samp) | 25 | 8 | 25 | 8 | 25 | 3 |
| n (Patient) | 25 | 8 | 25 | 8 | 25 | 3 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.112 | 0.0961 | 0.112 | 0.0648 | 0.112 | 0.0925 |
| Average | 2.20 | 0.141 | 2.20 | 0.121 | 2.20 | 0.0768 |
| Stdev | 5.52 | 0.168 | 5.52 | 0.162 | 5.52 | 0.0649 |
| p(t-test) | | 0.30 | | 0.30 | | 0.52 |
| Min | 0.00107 | 0.00551 | 0.00107 | 0.00551 | 0.00107 | 0.00551 |
| Max | 19.7 | 0.531 | 19.7 | 0.496 | 19.7 | 0.132 |
| n (Samp) | 26 | 8 | 26 | 8 | 26 | 3 |
| n (Patient) | 26 | 8 | 26 | 8 | 26 | 3 |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.47 | nd | 0.44 | 0.41 | nd | 0.38 | 0.39 | nd | 0.35 |
| SE | 0.12 | nd | 0.12 | 0.12 | nd | 0.12 | 0.18 | nd | 0.18 |
| p | 0.80 | nd | 0.60 | 0.47 | nd | 0.31 | 0.54 | nd | 0.40 |
| nCohort 1 | 25 | nd | 26 | 25 | nd | 26 | 25 | nd | 26 |
| nCohort 2 | 8 | nd | 8 | 8 | nd | 8 | 3 | nd | 3 |
| Cutoff 1 | 0.0380 | nd | 0.0380 | 0.0366 | nd | 0.0366 | 0.00402 | nd | 0.00107 |
| Sens 1 | 75% | nd | 75% | 75% | nd | 75% | 100% | nd | 100% |
| Spec 1 | 28% | nd | 27% | 28% | nd | 27% | 8% | nd | 4% |
| Cutoff 2 | 0.0366 | nd | 0.0366 | 0.0182 | nd | 0.0182 | 0.00402 | nd | 0.00107 |
| Sens 2 | 88% | nd | 88% | 88% | nd | 88% | 100% | nd | 100% |
| Spec 2 | 28% | nd | 27% | 16% | nd | 12% | 8% | nd | 4% |
| Cutoff 3 | 0.00402 | nd | 0.00107 | 0.00402 | nd | 0.00107 | 0.00402 | nd | 0.00107 |
| Sens 3 | 100% | nd | 100% | 100% | nd | 100% | 100% | nd | 100% |
| Spec 3 | 8% | nd | 4% | 8% | nd | 4% | 8% | nd | 4% |
| Cutoff 4 | 0.214 | nd | 0.292 | 0.214 | nd | 0.292 | 0.214 | nd | 0.292 |
| Sens 4 | 12% | nd | 12% | 12% | nd | 12% | 0% | nd | 0% |
| Spec 4 | 72% | nd | 73% | 72% | nd | 73% | 72% | nd | 73% |
| Cutoff 5 | 0.316 | nd | 0.372 | 0.316 | nd | 0.372 | 0.316 | nd | 0.372 |
| Sens 5 | 12% | nd | 12% | 12% | nd | 12% | 0% | nd | 0% |
| Spec 5 | 80% | nd | 81% | 80% | nd | 81% | 80% | nd | 81% |
| Cutoff 6 | 6.65 | nd | 8.08 | 6.65 | nd | 8.08 | 6.65 | nd | 8.08 |
| Sens 6 | 0% | nd | 0% | 0% | nd | 0% | 0% | nd | 0% |
| Spec 6 | 92% | nd | 92% | 92% | nd | 92% | 92% | nd | 92% |
| OR Quart 2 | 8.0 | nd | 2.7 | 4.8 | nd | 2.7 | >2.8 | nd | >1.3 |
| p Value | 0.10 | nd | 0.46 | 0.22 | nd | 0.46 | <0.45 | nd | <0.85 |
| 95% CI of OR Quart2 | 0.66 97 | nd | 0.19 37 | 0.38 60 | nd | 0.19 37 | >0.20 na | nd | >0.069 na |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| OR Quart 3 | 2.7 | nd | 6.4 | 2.7 | nd | 4.0 | >0 | nd | >1.3 |
| p Value | 0.46 | nd | 0.14 | 0.46 | nd | 0.28 | <na | nd | <0.85 |
| 95% CI of | 0.19 | nd | 0.55 | 0.19 | nd | 0.33 | >na | nd | >0.069 |
| OR Quart3 | 37 | nd | 75 | 37 | nd | 49 | na | nd | na |
| OR Quart 4 | 1.1 | nd | 1.1 | 2.7 | nd | 2.7 | >1.2 | nd | >1.3 |
| p Value | 0.93 | nd | 0.93 | 0.46 | nd | 0.46 | <0.92 | nd | <0.85 |
| 95% CI of | 0.060 | nd | 0.060 | 0.19 | nd | 0.19 | >0.059 | nd | >0.069 |
| OR Quart4 | 22 | nd | 22 | 37 | nd | 37 | na | nd | na |

**Antithrombin-III**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 110000 | 49300 | 110000 | 49300 |
| Average | 119000 | 49300 | 119000 | 49300 |
| Stdev | 41300 | 17300 | 41300 | 17300 |
| p(t-test) | | 0.0054 | | 0.0054 |
| Min | 61300 | 32000 | 61300 | 32000 |
| Max | 252000 | 66600 | 252000 | 66600 |
| n (Samp) | 53 | 3 | 53 | 3 |
| n (Patient) | 53 | 3 | 53 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 103000 | 49300 | 103000 | 49300 |
| Average | 114000 | 49300 | 114000 | 49300 |
| Stdev | 33700 | 17300 | 33700 | 17300 |
| p(t-test) | | 0.0022 | | 0.0022 |
| Min | 61300 | 32000 | 61300 | 32000 |
| Max | 186000 | 66600 | 186000 | 66600 |
| n (Samp) | 44 | 3 | 44 | 3 |
| n (Patient) | 44 | 3 | 44 | 3 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.013 | nd | 0.015 | 0.013 | nd | 0.015 |
| SE | 0.046 | nd | 0.050 | 0.046 | nd | 0.050 |
| p | <1.0E-5 | nd | <1.0E-5 | <1.0E-5 | nd | <1.0E-5 |
| nCohort 1 | 53 | nd | 44 | 53 | nd | 44 |
| nCohort 2 | 3 | nd | 3 | 3 | nd | 3 |
| Cutoff 1 | 0 | nd | 0 | 0 | nd | 0 |
| Sens 1 | 100% | nd | 100% | 100% | nd | 100% |
| Spec 1 | 0% | nd | 0% | 0% | nd | 0% |
| Cutoff 2 | 0 | nd | 0 | 0 | nd | 0 |
| Sens 2 | 100% | nd | 100% | 100% | nd | 100% |
| Spec 2 | 0% | nd | 0% | 0% | nd | 0% |
| Cutoff 3 | 0 | nd | 0 | 0 | nd | 0 |
| Sens 3 | 100% | nd | 100% | 100% | nd | 100% |

|  | 0hr prior to AKI stage | | | 24hr prior to AKI stage | | |
|---|---|---|---|---|---|---|
|  | sCr or UO | sCr only | UO only | sCr or UO | sCr only | UO only |
| Spec 3 | 0% | nd | 0% | 0% | nd | 0% |
| Cutoff 4 | 130000 | nd | 130000 | 130000 | nd | 130000 |
| Sens 4 | 0% | nd | 0% | 0% | nd | 0% |
| Spec 4 | 72% | nd | 70% | 72% | nd | 70% |
| Cutoff 5 | 147000 | nd | 145000 | 147000 | nd | 145000 |
| Sens 5 | 0% | nd | 0% | 0% | nd | 0% |
| Spec 5 | 81% | nd | 82% | 81% | nd | 82% |
| Cutoff 6 | 177000 | nd | 166000 | 177000 | nd | 166000 |
| Sens 6 | 0% | nd | 0% | 0% | nd | 0% |
| Spec 6 | 91% | nd | 91% | 91% | nd | 91% |
| OR Quart 2 | >0 | nd | >0 | >0 | nd | >0 |
| p Value | <na | nd | <na | <na | nd | <na |
| 95% CI of | >na | nd | >na | >na | nd | >na |
| OR Quart2 | na | nd | na | na | nd | na |
| OR Quart 3 | >0 | nd | >0 | >0 | nd | >0 |
| p Value | <na | nd | <na | <na | nd | <na |
| 95% CI of | >na | nd | >na | >na | nd | >na |
| OR Quart3 | na | nd | na | na | nd | na |
| OR Quart 4 | >3.8 | nd | >4.5 | >3.8 | nd | >4.5 |
| p Value | <0.27 | nd | <0.23 | <0.27 | nd | <0.23 |
| 95% CI of | >0.35 | nd | >0.39 | >0.35 | nd | >0.39 |
| OR Quart4 | na | nd | na | na | nd | na |

**Extracellular matrix protein 1**

| sCr or UO | 0hr prior to AKI stage | | 24hr prior to AKI stage | |
|---|---|---|---|---|
|  | Cohort 1 | Cohort 2 | Cohort 1 | Cohort 2 |
| Median | 1530 | 1450 | 1530 | 1450 |
| Average | 1610 | 1340 | 1610 | 1340 |
| Stdev | 465 | 447 | 465 | 447 |
| p(t-test) |  | 0.32 |  | 0.32 |
| Min | 779 | 846 | 779 | 846 |
| Max | 3060 | 1720 | 3060 | 1720 |
| n (Samp) | 53 | 3 | 53 | 3 |
| n (Patient) | 53 | 3 | 53 | 3 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 1510 | 1450 | 1510 | 1450 |
| Average | 1590 | 1340 | 1590 | 1340 |
| Stdev | 483 | 447 | 483 | 447 |
| p(t-test) |  | 0.39 |  | 0.39 |
| Min | 779 | 846 | 779 | 846 |
| Max | 3060 | 1720 | 3060 | 1720 |
| n (Samp) | 44 | 3 | 44 | 3 |
| n (Patient) | 44 | 3 | 44 | 3 |

|  | 0hr prior to AKI stage | 24hr prior to AKI stage |
|---|---|---|
|  |  |  |

| | sCr or UO | sCr only | UO only | sCr or UO | sCr only | UO only |
|---|---|---|---|---|---|---|
| AUC | 0.36 | nd | 0.38 | 0.36 | nd | 0.38 |
| SE | 0.18 | nd | 0.18 | 0.18 | nd | 0.18 |
| p | 0.43 | nd | 0.51 | 0.43 | nd | 0.51 |
| nCohort 1 | 53 | nd | 44 | 53 | nd | 44 |
| nCohort 2 | 3 | nd | 3 | 3 | nd | 3 |
| Cutoff 1 | 779 | nd | 779 | 779 | nd | 779 |
| Sens 1 | 100% | nd | 100% | 100% | nd | 100% |
| Spec 1 | 2% | nd | 2% | 2% | nd | 2% |
| Cutoff 2 | 779 | nd | 779 | 779 | nd | 779 |
| Sens 2 | 100% | nd | 100% | 100% | nd | 100% |
| Spec 2 | 2% | nd | 2% | 2% | nd | 2% |
| Cutoff 3 | 779 | nd | 779 | 779 | nd | 779 |
| Sens 3 | 100% | nd | 100% | 100% | nd | 100% |
| Spec 3 | 2% | nd | 2% | 2% | nd | 2% |
| Cutoff 4 | 1720 | nd | 1710 | 1720 | nd | 1710 |
| Sens 4 | 0% | nd | 33% | 0% | nd | 33% |
| Spec 4 | 74% | nd | 70% | 74% | nd | 70% |
| Cutoff 5 | 1950 | nd | 1910 | 1950 | nd | 1910 |
| Sens 5 | 0% | nd | 0% | 0% | nd | 0% |
| Spec 5 | 83% | nd | 82% | 83% | nd | 82% |
| Cutoff 6 | 2270 | nd | 2230 | 2270 | nd | 2230 |
| Sens 6 | 0% | nd | 0% | 0% | nd | 0% |
| Spec 6 | 92% | nd | 91% | 92% | nd | 91% |
| OR Quart 2 | >1.1 | nd | >1.1 | >1.1 | nd | >1.1 |
| p Value | <0.96 | nd | <0.95 | <0.96 | nd | <0.95 |
| 95% CI of OR Quart2 | >0.061 na | nd | >0.061 na | >0.061 na | nd | >0.061 na |
| OR Quart 3 | >1.1 | nd | >1.1 | >1.1 | nd | >1.1 |
| p Value | <0.96 | nd | <0.95 | <0.96 | nd | <0.95 |
| 95% CI of OR Quart3 | >0.061 na | nd | >0.061 na | >0.061 na | nd | >0.061 na |
| OR Quart 4 | >1.1 | nd | >1.2 | >1.1 | nd | >1.2 |
| p Value | <0.96 | nd | <0.90 | <0.96 | nd | <0.90 |
| 95% CI of OR Quart4 | >0.061 na | nd | >0.066 na | >0.061 na | nd | >0.066 na |

**Coagulation factor XIII A and B chains**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 12200 | 13200 | 12200 | 13200 |
| Average | 13300 | 10500 | 13300 | 10500 |
| Stdev | 6710 | 6040 | 6710 | 6040 |
| p(t-test) | | 0.48 | | 0.48 |
| Min | 881 | 3550 | 881 | 3550 |
| Max | 33300 | 14700 | 33300 | 14700 |
| n (Samp) | 53 | 3 | 53 | 3 |
| n (Patient) | 53 | 3 | 53 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 10500 | 13200 | 10500 | 13200 |
| Average | 12300 | 10500 | 12300 | 10500 |
| Stdev | 6450 | 6040 | 6450 | 6040 |
| p(t-test) | | 0.63 | | 0.63 |
| Min | 881 | 3550 | 881 | 3550 |
| Max | 33300 | 14700 | 33300 | 14700 |
| n (Samp) | 44 | 3 | 44 | 3 |
| n (Patient) | 44 | 3 | 44 | 3 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.43 | nd | 0.49 | 0.43 | nd | 0.49 |
| SE | 0.18 | nd | 0.17 | 0.18 | nd | 0.17 |
| p | 0.71 | nd | 0.97 | 0.71 | nd | 0.97 |
| nCohort 1 | 53 | nd | 44 | 53 | nd | 44 |
| nCohort 2 | 3 | nd | 3 | 3 | nd | 3 |
| Cutoff 1 | 2520 | nd | 2320 | 2520 | nd | 2320 |
| Sens 1 | 100% | nd | 100% | 100% | nd | 100% |
| Spec 1 | 6% | nd | 5% | 6% | nd | 5% |
| Cutoff 2 | 2520 | nd | 2320 | 2520 | nd | 2320 |
| Sens 2 | 100% | nd | 100% | 100% | nd | 100% |
| Spec 2 | 6% | nd | 5% | 6% | nd | 5% |
| Cutoff 3 | 2520 | nd | 2320 | 2520 | nd | 2320 |
| Sens 3 | 100% | nd | 100% | 100% | nd | 100% |
| Spec 3 | 6% | nd | 5% | 6% | nd | 5% |
| Cutoff 4 | 15700 | nd | 13600 | 15700 | nd | 13600 |
| Sens 4 | 0% | nd | 33% | 0% | nd | 33% |
| Spec 4 | 72% | nd | 70% | 72% | nd | 70% |
| Cutoff 5 | 18300 | nd | 16100 | 18300 | nd | 16100 |
| Sens 5 | 0% | nd | 0% | 0% | nd | 0% |
| Spec 5 | 81% | nd | 82% | 81% | nd | 82% |
| Cutoff 6 | 22900 | nd | 22900 | 22900 | nd | 22900 |
| Sens 6 | 0% | nd | 0% | 0% | nd | 0% |
| Spec 6 | 91% | nd | 91% | 91% | nd | 91% |
| OR Quart 2 | >2.3 | nd | 1.0 | >2.3 | nd | 1.0 |
| p Value | <0.51 | nd | 1.0 | <0.51 | nd | 1.0 |
| 95% CI of | >0.19 | nd | 0.055 | >0.19 | nd | 0.055 |
| OR Quart2 | na | nd | 18 | na | nd | 18 |
| OR Quart 3 | >0 | nd | 0 | >0 | nd | 0 |
| p Value | <na | nd | na | <na | nd | na |
| 95% CI of | >na | nd | na | >na | nd | na |
| OR Quart3 | na | nd | na | na | nd | na |
| OR Quart 4 | >1.1 | nd | 1.1 | >1.1 | nd | 1.1 |
| p Value | <0.96 | nd | 0.95 | <0.96 | nd | 0.95 |
| 95% CI of | >0.061 | nd | 0.060 | >0.061 | nd | 0.060 |
| OR Quart4 | na | nd | 20 | na | nd | 20 |

**Vitronectin**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 120000 | 35100 | 120000 | 35100 |
| Average | 123000 | 32400 | 123000 | 32400 |
| Stdev | 33100 | 9630 | 33100 | 9630 |
| p(t-test) | | 1.9E-5 | | 1.9E-5 |
| Min | 65900 | 21800 | 65900 | 21800 |
| Max | 205000 | 40400 | 205000 | 40400 |
| n (Samp) | 53 | 3 | 53 | 3 |
| n (Patient) | 53 | 3 | 53 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 120000 | 35100 | 120000 | 35100 |
| Average | 121000 | 32400 | 121000 | 32400 |
| Stdev | 31100 | 9630 | 31100 | 9630 |
| p(t-test) | | 1.4E-5 | | 1.4E-5 |
| Min | 65900 | 21800 | 65900 | 21800 |
| Max | 205000 | 40400 | 205000 | 40400 |
| n (Samp) | 44 | 3 | 44 | 3 |
| n (Patient) | 44 | 3 | 44 | 3 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0 | nd | 0 | 0 | nd | 0 |
| SE | <0.001 | nd | <0.001 | <0.001 | nd | <0.001 |
| p | <1.0E-5 | nd | <1.0E-5 | <1.0E-5 | nd | <1.0E-5 |
| nCohort 1 | 53 | nd | 44 | 53 | nd | 44 |
| nCohort 2 | 3 | nd | 3 | 3 | nd | 3 |
| Cutoff 1 | 0 | nd | 0 | 0 | nd | 0 |
| Sens 1 | 100% | nd | 100% | 100% | nd | 100% |
| Spec 1 | 0% | nd | 0% | 0% | nd | 0% |
| Cutoff 2 | 0 | nd | 0 | 0 | nd | 0 |
| Sens 2 | 100% | nd | 100% | 100% | nd | 100% |
| Spec 2 | 0% | nd | 0% | 0% | nd | 0% |
| Cutoff 3 | 0 | nd | 0 | 0 | nd | 0 |
| Sens 3 | 100% | nd | 100% | 100% | nd | 100% |
| Spec 3 | 0% | nd | 0% | 0% | nd | 0% |
| Cutoff 4 | 133000 | nd | 131000 | 133000 | nd | 131000 |
| Sens 4 | 0% | nd | 0% | 0% | nd | 0% |
| Spec 4 | 72% | nd | 70% | 72% | nd | 70% |
| Cutoff 5 | 158000 | nd | 157000 | 158000 | nd | 157000 |
| Sens 5 | 0% | nd | 0% | 0% | nd | 0% |
| Spec 5 | 81% | nd | 82% | 81% | nd | 82% |
| Cutoff 6 | 173000 | nd | 167000 | 173000 | nd | 167000 |
| Sens 6 | 0% | nd | 0% | 0% | nd | 0% |
| Spec 6 | 91% | nd | 91% | 91% | nd | 91% |
| OR Quart 2 | >0 | nd | >0 | >0 | nd | >0 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| p Value | <na | nd | <na | <na | nd | <na |
| 95% CI of | >na | nd | >na | >na | nd | >na |
| OR Quart2 | na | nd | na | na | nd | na |
| OR Quart 3 | >0 | nd | >0 | >0 | nd | >0 |
| p Value | <na | nd | <na | <na | nd | <na |
| 95% CI of | >na | nd | >na | >na | nd | >na |
| OR Quart3 | na | nd | na | na | nd | na |
| OR Quart 4 | >3.8 | nd | >4.5 | >3.8 | nd | >4.5 |
| p Value | <0.27 | nd | <0.23 | <0.27 | nd | <0.23 |
| 95% CI of | >0.35 | nd | >0.39 | >0.35 | nd | >0.39 |
| OR Quart4 | na | nd | na | na | nd | na |

**Estradiol**

| sCr or UO | 0hr prior to AKI stage | | 24hr prior to AKI stage | |
|---|---|---|---|---|
|  | Cohort 1 | Cohort 2 | Cohort 1 | Cohort 2 |
| Median | 1.28 | 13.6 | 1.28 | 13.6 |
| Average | 1.82 | 11.2 | 1.82 | 11.2 |
| Stdev | 1.85 | 5.81 | 1.85 | 5.81 |
| p(t-test) |  | 1.0E-9 |  | 1.0E-9 |
| Min | 0.552 | 4.57 | 0.552 | 4.57 |
| Max | 10.8 | 15.4 | 10.8 | 15.4 |
| n (Samp) | 52 | 3 | 52 | 3 |
| n (Patient) | 52 | 3 | 52 | 3 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 1.16 | 13.6 | 1.16 | 13.6 |
| Average | 2.00 | 11.2 | 2.00 | 11.2 |
| Stdev | 2.05 | 5.81 | 2.05 | 5.81 |
| p(t-test) |  | 5.3E-8 |  | 5.3E-8 |
| Min | 0.552 | 4.57 | 0.552 | 4.57 |
| Max | 10.8 | 15.4 | 10.8 | 15.4 |
| n (Samp) | 43 | 3 | 43 | 3 |
| n (Patient) | 43 | 3 | 43 | 3 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| AUC | 0.97 | nd | 0.97 | 0.97 | nd | 0.97 |
| SE | 0.065 | nd | 0.071 | 0.065 | nd | 0.071 |
| p | 2.5E-13 | nd | 4.3E-11 | 2.5E-13 | nd | 4.3E-11 |
| nCohort 1 | 52 | nd | 43 | 52 | nd | 43 |
| nCohort 2 | 3 | nd | 3 | 3 | nd | 3 |
| Cutoff 1 | 3.57 | nd | 4.47 | 3.57 | nd | 4.47 |
| Sens 1 | 100% | nd | 100% | 100% | nd | 100% |
| Spec 1 | 92% | nd | 91% | 92% | nd | 91% |
| Cutoff 2 | 3.57 | nd | 4.47 | 3.57 | nd | 4.47 |
| Sens 2 | 100% | nd | 100% | 100% | nd | 100% |

|  | 0hr prior to AKI stage | | | 24hr prior to AKI stage | | |
|---|---|---|---|---|---|---|
|  | sCr or UO | sCr only | UO only | sCr or UO | sCr only | UO only |
| Spec 2 | 92% | nd | 91% | 92% | nd | 91% |
| Cutoff 3 | 3.57 | nd | 4.47 | 3.57 | nd | 4.47 |
| Sens 3 | 100% | nd | 100% | 100% | nd | 100% |
| Spec 3 | 92% | nd | 91% | 92% | nd | 91% |
| Cutoff 4 | 1.60 | nd | 1.96 | 1.60 | nd | 1.96 |
| Sens 4 | 100% | nd | 100% | 100% | nd | 100% |
| Spec 4 | 71% | nd | 72% | 71% | nd | 72% |
| Cutoff 5 | 2.00 | nd | 2.34 | 2.00 | nd | 2.34 |
| Sens 5 | 100% | nd | 100% | 100% | nd | 100% |
| Spec 5 | 81% | nd | 81% | 81% | nd | 81% |
| Cutoff 6 | 3.05 | nd | 4.47 | 3.05 | nd | 4.47 |
| Sens 6 | 100% | nd | 100% | 100% | nd | 100% |
| Spec 6 | 90% | nd | 91% | 90% | nd | 91% |
| OR Quart 2 | >0 | nd | >0 | >0 | nd | >0 |
| p Value | <na | nd | <na | <na | nd | <na |
| 95% CI of | >na | nd | >na | >na | nd | >na |
| OR Quart2 | na | nd | na | na | nd | na |
| OR Quart 3 | >0 | nd | >0 | >0 | nd | >0 |
| p Value | <na | nd | <na | <na | nd | <na |
| 95% CI of | >na | nd | >na | >na | nd | >na |
| OR Quart3 | na | nd | na | na | nd | na |
| OR Quart 4 | >3.5 | nd | >3.7 | >3.5 | nd | >3.7 |
| p Value | <0.30 | nd | <0.29 | <0.30 | nd | <0.29 |
| 95% CI of | >0.32 | nd | >0.32 | >0.32 | nd | >0.32 |
| OR Quart4 | na | nd | na | na | nd | na |

**Progesterone**

| sCr or UO | 0hr prior to AKI stage | | 24hr prior to AKI stage | |
|---|---|---|---|---|
|  | Cohort 1 | Cohort 2 | Cohort 1 | Cohort 2 |
| Median | 4.44 | 31.8 | 4.44 | 31.8 |
| Average | 6.27 | 77.0 | 6.27 | 77.0 |
| Stdev | 7.25 | 102 | 7.25 | 102 |
| p(t-test) |  | 6.7E-7 |  | 6.7E-7 |
| Min | 1.35 | 5.07 | 1.35 | 5.07 |
| Max | 46.5 | 194 | 46.5 | 194 |
| n (Samp) | 52 | 3 | 52 | 3 |
| n (Patient) | 52 | 3 | 52 | 3 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 4.43 | 31.8 | 4.43 | 31.8 |
| Average | 5.83 | 77.0 | 5.83 | 77.0 |
| Stdev | 5.07 | 102 | 5.07 | 102 |
| p(t-test) |  | 3.2E-6 |  | 3.2E-6 |
| Min | 1.35 | 5.07 | 1.35 | 5.07 |
| Max | 31.4 | 194 | 31.4 | 194 |
| n (Samp) | 43 | 3 | 43 | 3 |

| UO only | 0hr prior to AKI stage | | 24hr prior to AKI stage | |
|---|---|---|---|---|
| | Cohort 1 | Cohort 2 | Cohort 1 | Cohort 2 |
| n (Patient) | 43 | 3 | 43 | 3 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.86 | nd | 0.86 | 0.86 | nd | 0.86 |
| SE | 0.14 | nd | 0.14 | 0.14 | nd | 0.14 |
| p | 0.0097 | nd | 0.0094 | 0.0097 | nd | 0.0094 |
| nCohort 1 | 52 | nd | 43 | 52 | nd | 43 |
| nCohort 2 | 3 | nd | 3 | 3 | nd | 3 |
| Cutoff 1 | 4.97 | nd | 4.97 | 4.97 | nd | 4.97 |
| Sens 1 | 100% | nd | 100% | 100% | nd | 100% |
| Spec 1 | 60% | nd | 58% | 60% | nd | 58% |
| Cutoff 2 | 4.97 | nd | 4.97 | 4.97 | nd | 4.97 |
| Sens 2 | 100% | nd | 100% | 100% | nd | 100% |
| Spec 2 | 60% | nd | 58% | 60% | nd | 58% |
| Cutoff 3 | 4.97 | nd | 4.97 | 4.97 | nd | 4.97 |
| Sens 3 | 100% | nd | 100% | 100% | nd | 100% |
| Spec 3 | 60% | nd | 58% | 60% | nd | 58% |
| Cutoff 4 | 6.37 | nd | 6.28 | 6.37 | nd | 6.28 |
| Sens 4 | 67% | nd | 67% | 67% | nd | 67% |
| Spec 4 | 71% | nd | 72% | 71% | nd | 72% |
| Cutoff 5 | 7.12 | nd | 7.77 | 7.12 | nd | 7.77 |
| Sens 5 | 67% | nd | 67% | 67% | nd | 67% |
| Spec 5 | 81% | nd | 81% | 81% | nd | 81% |
| Cutoff 6 | 9.61 | nd | 9.73 | 9.61 | nd | 9.73 |
| Sens 6 | 67% | nd | 67% | 67% | nd | 67% |
| Spec 6 | 90% | nd | 91% | 90% | nd | 91% |
| OR Quart 2 | >0 | nd | >0 | >0 | nd | >0 |
| p Value | <na | nd | <na | <na | nd | <na |
| 95% CI of | >na | nd | >na | >na | nd | >na |
| OR Quart2 | na | nd | na | na | nd | na |
| OR Quart 3 | >1.0 | nd | >1.1 | >1.0 | nd | >1.1 |
| p Value | <1.0 | nd | <0.95 | <1.0 | nd | <0.95 |
| 95% CI of | >0.056 | nd | >0.060 | >0.056 | nd | >0.060 |
| OR Quart3 | na | nd | na | na | nd | na |
| OR Quart 4 | >2.2 | nd | >2.2 | >2.2 | nd | >2.2 |
| p Value | <0.55 | nd | <0.54 | <0.55 | nd | <0.54 |
| 95% CI of | >0.17 | nd | >0.17 | >0.17 | nd | >0.17 |
| OR Quart4 | na | nd | na | na | nd | na |

T3

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 1.16 | 0.000162 | 1.16 | 0.000162 |
| Average | 1.26 | 0.425 | 1.26 | 0.425 |
| Stdev | 0.601 | 0.736 | 0.601 | 0.736 |
| p(t-test) | | 0.024 | | 0.024 |

| sCr or UO | 0hr prior to AKI stage | | 24hr prior to AKI stage | |
|---|---|---|---|---|
| | Cohort 1 | Cohort 2 | Cohort 1 | Cohort 2 |
| Min | 0.302 | 0.000162 | 0.302 | 0.000162 |
| Max | 3.76 | 1.27 | 3.76 | 1.27 |
| n (Samp) | 52 | 3 | 52 | 3 |
| n (Patient) | 52 | 3 | 52 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Mcdian | 1.14 | 0.000162 | 1.14 | 0.000162 |
| Average | 1.23 | 0.425 | 1.23 | 0.425 |
| Stdev | 0.529 | 0.736 | 0.529 | 0.736 |
| p(t-test) | | 0.017 | | 0.017 |
| Min | 0.344 | 0.000162 | 0.344 | 0.000162 |
| Max | 2.78 | 1.27 | 2.78 | 1.27 |
| n (Samp) | 43 | 3 | 43 | 3 |
| n (Patient) | 43 | 3 | 43 | 3 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.21 | nd | 0.22 | 0.21 | nd | 0.22 |
| SE | 0.16 | nd | 0.16 | 0.16 | nd | 0.16 |
| p | 0.071 | nd | 0.092 | 0.071 | nd | 0.092 |
| nCohort 1 | 52 | nd | 43 | 52 | nd | 43 |
| nCohort 2 | 3 | nd | 3 | 3 | nd | 3 |
| Cutoff 1 | 0 | nd | 0 | 0 | nd | 0 |
| Sens 1 | 100% | nd | 100% | 100% | nd | 100% |
| Spec 1 | 0% | nd | 0% | 0% | nd | 0% |
| Cutoff 2 | 0 | nd | 0 | 0 | nd | 0 |
| Sens 2 | 100% | nd | 100% | 100% | nd | 100% |
| Spec 2 | 0% | nd | 0% | 0% | nd | 0% |
| Cutoff 3 | 0 | nd | 0 | 0 | nd | 0 |
| Sens 3 | 100% | nd | 100% | 100% | nd | 100% |
| Spec 3 | 0% | nd | 0% | 0% | nd | 0% |
| Cutoff 4 | 1.48 | nd | 1.48 | 1.48 | nd | 1.48 |
| Sens 4 | 0% | nd | 0% | 0% | nd | 0% |
| Spcc 4 | 71% | nd | 72% | 71% | nd | 72% |
| Cutoff 5 | 1.68 | nd | 1.70 | 1.68 | nd | 1.70 |
| Sens 5 | 0% | nd | 0% | 0% | nd | 0% |
| Spec 5 | 81% | nd | 81% | 81% | nd | 81% |
| Cutoff 6 | 1.88 | nd | 1.88 | 1.88 | nd | 1.88 |
| Sens 6 | 0% | nd | 0% | 0% | nd | 0% |
| Spec 6 | 90% | nd | 91% | 90% | nd | 91% |
| OR Quart 2 | >1.1 | nd | >1.2 | >1.1 | nd | >1.2 |
| p Value | <0.96 | nd | <0.90 | <0.96 | nd | <0.90 |
| 95% CI of OR Quart2 | >0.061 na | nd | >0.066 na | >0.061 na | nd | >0.066 na |
| OR Quart 3 | >0 | nd | >0 | >0 | nd | >0 |
| p Value | <na | nd | <na | <na | nd | <na |

|  | 0hr prior to AKI stage | | | 24hr prior to AKI stage | | |
|---|---|---|---|---|---|---|
|  | sCr or UO | sCr only | UO only | sCr or UO | sCr only | UO only |
| 95% CI of | >na | nd | >na | >na | nd | >na |
| OR Quart3 | na | nd | na | na | nd | na |
| OR Quart 4 | >2.5 | nd | >2.7 | >2.5 | nd | >2.7 |
| p Value | <0.47 | nd | <0.45 | <0.47 | nd | <0.45 |
| 95% CI of | >0.20 | nd | >0.21 | >0.20 | nd | >0.21 |
| OR Quart4 | na | nd | na | na | nd | na |

**T4**

| sCr or UO | 0hr prior to AKI stage | | 24hr prior to AKI stage | |
|---|---|---|---|---|
|  | Cohort 1 | Cohort 2 | Cohort 1 | Cohort 2 |
| Median | 2.49 | 0.291 | 2.49 | 0.291 |
| Average | 3.05 | 0.572 | 3.05 | 0.572 |
| Stdev | 2.39 | 0.752 | 2.39 | 0.752 |
| p(t-test) |  | 0.082 |  | 0.082 |
| Min | 0.000136 | 0.000136 | 0.000136 | 0.000136 |
| Max | 11.7 | 1.42 | 11.7 | 1.42 |
| n (Samp) | 52 | 3 | 52 | 3 |
| n (Patient) | 52 | 3 | 52 | 3 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 2.39 | 0.291 | 2.39 | 0.291 |
| Average | 2.71 | 0.572 | 2.71 | 0.572 |
| Stdev | 1.92 | 0.752 | 1.92 | 0.752 |
| p(t-test) |  | 0.064 |  | 0.064 |
| Min | 0.000136 | 0.000136 | 0.000136 | 0.000136 |
| Max | 7.48 | 1.42 | 7.48 | 1.42 |
| n (Samp) | 43 | 3 | 43 | 3 |
| n (Patient) | 43 | 3 | 43 | 3 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| AUC | 0.12 | nd | 0.13 | 0.12 | nd | 0.13 |
| SE | 0.13 | nd | 0.13 | 0.13 | nd | 0.13 |
| p | 0.0033 | nd | 0.0055 | 0.0033 | nd | 0.0055 |
| nCohort 1 | 52 | nd | 43 | 52 | nd | 43 |
| nCohort 2 | 3 | nd | 3 | 3 | nd | 3 |
| Cutoff 1 | 0 | nd | 0 | 0 | nd | 0 |
| Sens 1 | 100% | nd | 100% | 100% | nd | 100% |
| Spec 1 | 0% | nd | 0% | 0% | nd | 0% |
| Cutoff 2 | 0 | nd | 0 | 0 | nd | 0 |
| Sens 2 | 100% | nd | 100% | 100% | nd | 100% |
| Spec 2 | 0% | nd | 0% | 0% | nd | 0% |
| Cutoff 3 | 0 | nd | 0 | 0 | nd | 0 |
| Sens 3 | 100% | nd | 100% | 100% | nd | 100% |
| Spec 3 | 0% | nd | 0% | 0% | nd | 0% |
| Cutoff 4 | 3.92 | nd | 3.34 | 3.92 | nd | 3.34 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| Sens 4 | 0% | nd | 0% | 0% | nd | 0% |
| Spec 4 | 71% | nd | 72% | 71% | nd | 72% |
| Cutoff 5 | 4.33 | nd | 4.05 | 4.33 | nd | 4.05 |
| Sens 5 | 0% | nd | 0% | 0% | nd | 0% |
| Spec 5 | 81% | nd | 81% | 81% | nd | 81% |
| Cutoff 6 | 6.68 | nd | 5.01 | 6.68 | nd | 5.01 |
| Sens 6 | 0% | nd | 0% | 0% | nd | 0% |
| Spec 6 | 90% | nd | 91% | 90% | nd | 91% |
| OR Quart 2 | >0 | nd | >0 | >0 | nd | >0 |
| p Value | <na | nd | <na | <na | nd | <na |
| 95% CI of | >na | nd | >na | >na | nd | >na |
| OR Quart2 | na | nd | na | na | nd | na |
| OR Quart 3 | >1.1 | nd | >1.1 | >1.1 | nd | >1.1 |
| p Value | <0.96 | nd | <0.95 | <0.96 | nd | <0.95 |
| 95% CI of | >0.061 | nd | >0.061 | >0.061 | nd | >0.061 |
| OR Quart3 | na | nd | na | na | nd | na |
| OR Quart 4 | >2.5 | nd | >2.7 | >2.5 | nd | >2.7 |
| p Value | <0.47 | nd | <0.45 | <0.47 | nd | <0.45 |
| 95% CI of | >0.20 | nd | >0.21 | >0.20 | nd | >0.21 |
| OR Quart4 | na | nd | na | na | nd | na |

**Growth/differentiation factor 15**

| sCr or UO | 0hr prior to AKI stage | | 24hr prior to AKI stage | |
|---|---|---|---|---|
|  | Cohort 1 | Cohort 2 | Cohort 1 | Cohort 2 |
| Median | 1810 | 8090 | 1810 | 8090 |
| Average | 2270 | 7840 | 2270 | 7840 |
| Stdev | 1680 | 450 | 1680 | 450 |
| p(t-test) |  | 5.8E-7 |  | 5.8E-7 |
| Min | 271 | 7320 | 271 | 7320 |
| Max | 7790 | 8110 | 7790 | 8110 |
| n (Samp) | 52 | 3 | 52 | 3 |
| n (Patient) | 52 | 3 | 52 | 3 |

| UO only | 0hr prior to AKI stage | | 24hr prior to AKI stage | |
|---|---|---|---|---|
|  | Cohort 1 | Cohort 2 | Cohort 1 | Cohort 2 |
| Median | 2030 | 8090 | 2030 | 8090 |
| Average | 2380 | 7840 | 2380 | 7840 |
| Stdev | 1640 | 450 | 1640 | 450 |
| p(t-test) |  | 8.8E-7 |  | 8.8E-7 |
| Min | 452 | 7320 | 452 | 7320 |
| Max | 7790 | 8110 | 7790 | 8110 |
| n (Samp) | 43 | 3 | 43 | 3 |
| n (Patient) | 43 | 3 | 43 | 3 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| AUC | 0.99 | nd | 0.99 | 0.99 | nd | 0.99 |

|  | 0hr prior to AKI stage |  |  | 24hr prior to AKI stage |  |  |
|---|---|---|---|---|---|---|
|  | sCr or UO | sCr only | UO only | sCr or UO | sCr only | UO only |
| SE | 0.033 | nd | 0.036 | 0.033 | nd | 0.036 |
| p | <1.0E-5 | nd | <1.0E-5 | <1.0E-5 | nd | <1.0E-5 |
| nCohort 1 | 52 | nd | 43 | 52 | nd | 43 |
| nCohort 2 | 3 | nd | 3 | 3 | nd | 3 |
| Cutoff 1 | 6800 | nd | 6800 | 6800 | nd | 6800 |
| Sens 1 | 100% | nd | 100% | 100% | nd | 100% |
| Spec 1 | 98% | nd | 98% | 98% | nd | 98% |
| Cutoff 2 | 6800 | nd | 6800 | 6800 | nd | 6800 |
| Sens 2 | 100% | nd | 100% | 100% | nd | 100% |
| Spec 2 | 98% | nd | 98% | 98% | nd | 98% |
| Cutoff 3 | 6800 | nd | 6800 | 6800 | nd | 6800 |
| Sens 3 | 100% | nd | 100% | 100% | nd | 100% |
| Spec 3 | 98% | nd | 98% | 98% | nd | 98% |
| Cutoff 4 | 2480 | nd | 2740 | 2480 | nd | 2740 |
| Sens 4 | 100% | nd | 100% | 100% | nd | 100% |
| Spec 4 | 71% | nd | 72% | 71% | nd | 72% |
| Cutoff 5 | 3190 | nd | 3190 | 3190 | nd | 3190 |
| Sens 5 | 100% | nd | 100% | 100% | nd | 100% |
| Spec 5 | 81% | nd | 81% | 81% | nd | 81% |
| Cutoff 6 | 4990 | nd | 4990 | 4990 | nd | 4990 |
| Sens 6 | 100% | nd | 100% | 100% | nd | 100% |
| Spec 6 | 90% | nd | 91% | 90% | nd | 91% |
| OR Quart 2 | >0 | nd | >0 | >0 | nd | >0 |
| p Value | <na | nd | <na | <na | nd | <na |
| 95% CI of | >na | nd | >na | >na | nd | >na |
| OR Quart2 | na | nd | na | na | nd | na |
| OR Quart 3 | >0 | nd | >0 | >0 | nd | >0 |
| p Value | <na | nd | <na | <na | nd | <na |
| 95% CI of | >na | nd | >na | >na | nd | >na |
| OR Quart3 | na | nd | na | na | nd | na |
| OR Quart 4 | >3.5 | nd | >3.7 | >3.5 | nd | >3.7 |
| p Value | <0.30 | nd | <0.29 | <0.30 | nd | <0.29 |
| 95% CI of | >0.32 | nd | >0.32 | >0.32 | nd | >0.32 |
| OR Quart4 | na | nd | na | na | nd | na |

**Proprotein convertase subtilisin/kexin type 9**

| sCr or UO | 0hr prior to AKI stage |  | 24hr prior to AKI stage |  |
|---|---|---|---|---|
|  | Cohort 1 | Cohort 2 | Cohort 1 | Cohort 2 |
| Median | 468000 | 102000 | 468000 | 102000 |
| Average | 515000 | 158000 | 515000 | 158000 |
| Stdev | 228000 | 115000 | 228000 | 115000 |
| p(t-test) |  | 0.0099 |  | 0.0099 |
| Min | 76300 | 80700 | 76300 | 80700 |
| Max | 1100000 | 291000 | 1100000 | 291000 |
| n (Samp) | 53 | 3 | 53 | 3 |
| n (Patient) | 53 | 3 | 53 | 3 |

| UO only | 0hr prior to AKI stage | | 24hr prior to AKI stage | |
|---|---|---|---|---|
| | Cohort 1 | Cohort 2 | Cohort 1 | Cohort 2 |
| Median | 484000 | 102000 | 484000 | 102000 |
| Average | 514000 | 158000 | 514000 | 158000 |
| Stdev | 215000 | 115000 | 215000 | 115000 |
| p(t-test) | | 0.0070 | | 0.0070 |
| Min | 76300 | 80700 | 76300 | 80700 |
| Max | 867000 | 291000 | 867000 | 291000 |
| n (Samp) | 44 | 3 | 44 | 3 |
| n (Patient) | 44 | 3 | 44 | 3 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.050 | nd | 0.061 | 0.050 | nd | 0.061 |
| SE | 0.089 | nd | 0.097 | 0.089 | nd | 0.097 |
| p | 4.5E-7 | nd | 6.3E-6 | 4.5E-7 | nd | 6.3E-6 |
| nCohort 1 | 53 | nd | 44 | 53 | nd | 44 |
| nCohort 2 | 3 | nd | 3 | 3 | nd | 3 |
| Cutoff 1 | 76300 | nd | 76300 | 76300 | nd | 76300 |
| Sens 1 | 100% | nd | 100% | 100% | nd | 100% |
| Spec 1 | 2% | nd | 2% | 2% | nd | 2% |
| Cutoff 2 | 76300 | nd | 76300 | 76300 | nd | 76300 |
| Sens 2 | 100% | nd | 100% | 100% | nd | 100% |
| Spec 2 | 2% | nd | 2% | 2% | nd | 2% |
| Cutoff 3 | 76300 | nd | 76300 | 76300 | nd | 76300 |
| Sens 3 | 100% | nd | 100% | 100% | nd | 100% |
| Spec 3 | 2% | nd | 2% | 2% | nd | 2% |
| Cutoff 4 | 667000 | nd | 668000 | 667000 | nd | 668000 |
| Sens 4 | 0% | nd | 0% | 0% | nd | 0% |
| Spec 4 | 72% | nd | 70% | 72% | nd | 70% |
| Cutoff 5 | 786000 | nd | 786000 | 786000 | nd | 786000 |
| Sens 5 | 0% | nd | 0% | 0% | nd | 0% |
| Spec 5 | 81% | nd | 82% | 81% | nd | 82% |
| Cutoff 6 | 835000 | nd | 834000 | 835000 | nd | 834000 |
| Sens 6 | 0% | nd | 0% | 0% | nd | 0% |
| Spec 6 | 91% | nd | 91% | 91% | nd | 91% |
| OR Quart 2 | >0 | nd | >0 | >0 | nd | >0 |
| p Value | <na | nd | <na | <na | nd | <na |
| 95% CI of OR Quart2 | >na | nd | >na | >na | nd | >na |
| | na | nd | na | na | nd | na |
| OR Quart 3 | >0 | nd | >0 | >0 | nd | >0 |
| p Value | <na | nd | <na | <na | nd | <na |
| 95% CI of OR Quart3 | >na | nd | >na | >na | nd | >na |
| | na | nd | na | na | nd | na |
| OR Quart 4 | >3.8 | nd | >4.5 | >3.8 | nd | >4.5 |
| p Value | <0.27 | nd | <0.23 | <0.27 | nd | <0.23 |
| 95% CI of OR Quart4 | >0.35 | nd | >0.39 | >0.35 | nd | >0.39 |
| | na | nd | na | na | nd | na |

Table 9: Comparison of marker levels in urine samples collected from Cohort 1 (patients that did not progress beyond RIFLE stage 0, R, or I) and in urine samples collected from Cohort 2 (subjects who progress to RIFLE stage F) at 0, 24 hours, and 48 hours prior to the subject reaching RIFLE stage I.

**Toll-like receptor 2**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 0.345 | 0.577 | 0.345 | 0.926 |
| Average | 0.512 | 0.679 | 0.512 | 0.838 |
| Stdev | 0.525 | 0.473 | 0.525 | 0.447 |
| p(t-test) | | 0.45 | | 0.089 |
| Min | 0.0538 | 0.206 | 0.0538 | 0.0621 |
| Max | 3.06 | 1.52 | 3.06 | 1.29 |
| n (Samp) | 116 | 6 | 116 | 8 |
| n (Patient) | 56 | 6 | 56 | 8 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.345 | 0.577 | 0.345 | 0.926 |
| Average | 0.526 | 0.679 | 0.526 | 0.838 |
| Stdev | 0.544 | 0.473 | 0.544 | 0.447 |
| p(t-test) | | 0.50 | | 0.12 |
| Min | 0.0538 | 0.206 | 0.0538 | 0.0621 |
| Max | 3.06 | 1.52 | 3.06 | 1.29 |
| n (Samp) | 106 | 6 | 106 | 8 |
| n (Patient) | 51 | 6 | 51 | 8 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.67 | nd | 0.67 | 0.73 | nd | 0.72 |
| SE | 0.12 | nd | 0.12 | 0.10 | nd | 0.11 |
| p | 0.17 | nd | 0.18 | 0.031 | nd | 0.038 |
| nCohort 1 | 116 | nd | 106 | 116 | nd | 106 |
| nCohort 2 | 6 | nd | 6 | 8 | nd | 8 |
| Cutoff 1 | 0.366 | nd | 0.366 | 0.641 | nd | 0.641 |
| Sens 1 | 83% | nd | 83% | 75% | nd | 75% |
| Spec 1 | 54% | nd | 55% | 78% | nd | 77% |
| Cutoff 2 | 0.366 | nd | 0.366 | 0.375 | nd | 0.375 |
| Sens 2 | 83% | nd | 83% | 88% | nd | 88% |
| Spec 2 | 54% | nd | 55% | 56% | nd | 57% |
| Cutoff 3 | 0.200 | nd | 0.200 | 0.0592 | nd | 0.0592 |
| Sens 3 | 100% | nd | 100% | 100% | nd | 100% |
| Spec 3 | 25% | nd | 25% | 2% | nd | 2% |
| Cutoff 4 | 0.492 | nd | 0.492 | 0.492 | nd | 0.492 |
| Sens 4 | 50% | nd | 50% | 75% | nd | 75% |
| Spec 4 | 71% | nd | 71% | 71% | nd | 71% |
| Cutoff 5 | 0.668 | nd | 0.708 | 0.668 | nd | 0.708 |

| | 0hr prior to AKI stage | | | 24hr prior to AKI stage | | |
|---|---|---|---|---|---|---|
| | sCr or UO | sCr only | UO only | sCr or UO | sCr only | UO only |
| Sens 5 | 50% | nd | 50% | 62% | nd | 62% |
| Spec 5 | 80% | nd | 80% | 80% | nd | 80% |
| Cutoff 6 | 1.09 | nd | 1.43 | 1.09 | nd | 1.43 |
| Sens 6 | 17% | nd | 17% | 38% | nd | 0% |
| Spec 6 | 91% | nd | 91% | 91% | nd | 91% |
| OR Quart 2 | 0 | nd | 0 | 0 | nd | 0 |
| p Value | na | nd | na | na | nd | na |
| 95% CI of OR Quart2 | na | nd | na | na | nd | na |
| | na | nd | na | na | nd | na |
| OR Quart 3 | 2.1 | nd | 2.1 | 1.0 | nd | 2.1 |
| p Value | 0.56 | nd | 0.56 | 1.0 | nd | 0.56 |
| 95% CI of OR Quart3 | 0.18 | nd | 0.18 | 0.060 | nd | 0.18 |
| | 24 | nd | 24 | 17 | nd | 24 |
| OR Quart 4 | 3.1 | nd | 3.2 | 7.2 | nd | 5.6 |
| p Value | 0.34 | nd | 0.32 | 0.076 | nd | 0.13 |
| 95% CI of OR Quart4 | 0.30 | nd | 0.32 | 0.81 | nd | 0.61 |
| | 32 | nd | 33 | 64 | nd | 52 |

**Antithrombin-III**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 107 | 187 | 107 | 203 | 107 | 27.9 |
| Average | 358 | 509 | 358 | 877 | 358 | 176 |
| Stdev | 946 | 593 | 946 | 1850 | 946 | 370 |
| p(t-test) | | 0.61 | | 0.041 | | 0.64 |
| Min | 0.0182 | 50.2 | 0.0182 | 11.5 | 0.0182 | 0.347 |
| Max | 6000 | 1840 | 6000 | 5660 | 6000 | 930 |
| n (Samp) | 688 | 10 | 688 | 15 | 688 | 6 |
| n (Patient) | 283 | 10 | 283 | 15 | 283 | 6 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 108 | 151 | 108 | 98.8 | 108 | 75.6 |
| Average | 365 | 151 | 365 | 222 | 365 | 273 |
| Stdev | 958 | 45.1 | 958 | 290 | 958 | 440 |
| p(t-test) | | 0.75 | | 0.67 | | 0.85 |
| Min | 0.0182 | 119 | 0.0182 | 11.5 | 0.0182 | 8.92 |
| Max | 6000 | 183 | 6000 | 908 | 6000 | 930 |
| n (Samp) | 696 | 2 | 696 | 8 | 696 | 4 |
| n (Patient) | 288 | 2 | 288 | 8 | 288 | 4 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 103 | 351 | 103 | 219 | nd | nd |
| Average | 355 | 648 | 355 | 1140 | nd | nd |
| Stdev | 939 | 692 | 939 | 2120 | nd | nd |
| p(t-test) | | 0.44 | | 0.0078 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Min | 0.0182 | 49.1 | 0.0182 | 14.7 | nd | nd |
| Max | 6000 | 1840 | 6000 | 5660 | nd | nd |
| n (Samp) | 699 | 6 | 699 | 11 | nd | nd |
| n (Patient) | 273 | 6 | 273 | 11 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.70 | 0.61 | 0.75 | 0.62 | 0.53 | 0.65 | 0.25 | 0.43 | nd |
| SE | 0.094 | 0.21 | 0.12 | 0.078 | 0.10 | 0.091 | 0.12 | 0.15 | nd |
| p | 0.034 | 0.60 | 0.030 | 0.11 | 0.78 | 0.11 | 0.033 | 0.63 | nd |
| nCohort 1 | 688 | 696 | 699 | 688 | 696 | 699 | 688 | 696 | nd |
| nCohort 2 | 10 | 2 | 6 | 15 | 8 | 11 | 6 | 4 | nd |
| Cutoff 1 | 181 | 119 | 184 | 96.5 | 86.6 | 114 | 8.57 | 58.2 | nd |
| Sens 1 | 70% | 100% | 83% | 73% | 75% | 73% | 83% | 75% | nd |
| Spec 1 | 69% | 54% | 69% | 47% | 42% | 54% | 2% | 30% | nd |
| Cutoff 2 | 119 | 119 | 184 | 78.6 | 74.9 | 74.9 | 8.57 | 8.57 | nd |
| Sens 2 | 80% | 100% | 83% | 80% | 88% | 82% | 83% | 100% | nd |
| Spec 2 | 55% | 54% | 69% | 39% | 37% | 37% | 2% | 2% | nd |
| Cutoff 3 | 80.8 | 119 | 49.0 | 32.9 | 11.5 | 54.4 | 0.0182 | 8.57 | nd |
| Sens 3 | 90% | 100% | 100% | 93% | 100% | 91% | 100% | 100% | nd |
| Spec 3 | 40% | 54% | 23% | 16% | 3% | 27% | 0% | 2% | nd |
| Cutoff 4 | 189 | 190 | 188 | 189 | 190 | 188 | 189 | 190 | nd |
| Sens 4 | 50% | 0% | 67% | 60% | 38% | 64% | 17% | 25% | nd |
| Spec 4 | 70% | 70% | 70% | 70% | 70% | 70% | 70% | 70% | nd |
| Cutoff 5 | 280 | 281 | 279 | 280 | 281 | 279 | 280 | 281 | nd |
| Sens 5 | 40% | 0% | 67% | 27% | 25% | 27% | 17% | 25% | nd |
| Spec 5 | 80% | 80% | 80% | 80% | 80% | 80% | 80% | 80% | nd |
| Cutoff 6 | 594 | 596 | 594 | 594 | 596 | 594 | 594 | 596 | nd |
| Sens 6 | 30% | 0% | 33% | 13% | 12% | 18% | 17% | 25% | nd |
| Spec 6 | 90% | 90% | 90% | 90% | 90% | 90% | 90% | 90% | nd |
| OR Quart 2 | 0.99 | >0 | 0 | 1.5 | 4.1 | 2.0 | 0 | 0 | nd |
| p Value | 1.00 | <na | na | 0.66 | 0.21 | 0.57 | na | na | nd |
| 95% CI of OR Quart2 | 0.062 16 | >na na | na na | 0.25 9.1 | 0.45 37 | 0.18 22 | na na | na na | nd |
| OR Quart 3 | 4.1 | >2.0 | 1.0 | 2.0 | 1.0 | 3.0 | 1.0 | 2.0 | nd |
| p Value | 0.21 | <0.57 | 1.0 | 0.42 | 1.0 | 0.34 | 1.0 | 0.57 | nd |
| 95% CI of OR Quart3 | 0.45 37 | >0.18 na | 0.062 16 | 0.36 11 | 0.062 16 | 0.31 29 | 0.062 16 | 0.18 22 | nd |
| OR Quart 4 | 4.0 | >0 | 4.0 | 3.1 | 2.0 | 5.1 | 4.1 | 1.0 | nd |
| p Value | 0.21 | <na | 0.21 | 0.18 | 0.57 | 0.14 | 0.21 | 1.0 | nd |
| 95% CI of OR Quart4 | 0.45 37 | >na na | 0.45 37 | 0.61 15 | 0.18 22 | 0.59 44 | 0.45 37 | 0.062 16 | nd |

**Extracellular matrix protein 1**

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.492 | 1.88 | 0.492 | 1.69 | 0.492 | 0.178 |

| sCr or UO | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 1.62 | 4.82 | 1.62 | 2.05 | 1.62 | 1.18 |
| Stdev | 9.60 | 5.81 | 9.60 | 2.23 | 9.60 | 2.37 |
| p(t-test) | | 0.29 | | 0.86 | | 0.91 |
| Min | 3.61E-6 | 0.129 | 3.61E-6 | 0.124 | 3.61E-6 | 0.0117 |
| Max | 150 | 15.7 | 150 | 9.15 | 150 | 6.00 |
| n (Samp) | 689 | 10 | 689 | 15 | 689 | 6 |
| n (Patient) | 283 | 10 | 283 | 15 | 283 | 6 |

| sCr only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.507 | 8.39 | 0.507 | 0.924 | 0.507 | 0.641 |
| Average | 1.73 | 8.39 | 1.73 | 0.953 | 1.73 | 1.89 |
| Stdev | 9.79 | 8.49 | 9.79 | 0.692 | 9.79 | 2.75 |
| p(t-test) | | 0.34 | | 0.82 | | 0.97 |
| Min | 3.61E-6 | 2.38 | 3.61E-6 | 0.128 | 3.61E-6 | 0.272 |
| Max | 150 | 14.4 | 150 | 1.95 | 150 | 6.00 |
| n (Samp) | 697 | 2 | 697 | 8 | 697 | 4 |
| n (Patient) | 288 | 2 | 288 | 8 | 288 | 4 |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Median | 0.508 | 4.07 | 0.508 | 2.18 | nd | nd |
| Average | 1.62 | 5.38 | 1.62 | 2.32 | nd | nd |
| Stdev | 9.53 | 5.59 | 9.53 | 2.55 | nd | nd |
| p(t-test) | | 0.34 | | 0.81 | nd | nd |
| Min | 3.61E-6 | 0.315 | 3.61E-6 | 0.00419 | nd | nd |
| Max | 150 | 15.7 | 150 | 9.15 | nd | nd |
| n (Samp) | 699 | 6 | 699 | 11 | nd | nd |
| n (Patient) | 273 | 6 | 273 | 11 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.75 | 0.94 | 0.84 | 0.70 | 0.58 | 0.70 | 0.35 | 0.61 | nd |
| SE | 0.090 | 0.12 | 0.10 | 0.077 | 0.11 | 0.089 | 0.12 | 0.15 | nd |
| p | 0.0050 | 1.3E-4 | 7.9E-4 | 0.0098 | 0.46 | 0.023 | 0.21 | 0.48 | nd |
| nCohort 1 | 689 | 697 | 699 | 689 | 697 | 699 | 689 | 697 | nd |
| nCohort 2 | 10 | 2 | 6 | 15 | 8 | 11 | 6 | 4 | nd |
| Cutoff 1 | 1.10 | 2.38 | 1.37 | 0.794 | 0.324 | 0.794 | 0.0290 | 0.560 | nd |
| Sens 1 | 70% | 100% | 83% | 73% | 75% | 73% | 83% | 75% | nd |
| Spec 1 | 71% | 89% | 78% | 61% | 39% | 61% | 4% | 53% | nd |
| Cutoff 2 | 0.547 | 2.38 | 1.37 | 0.285 | 0.275 | 0.361 | 0.0290 | 0.271 | nd |
| Sens 2 | 80% | 100% | 83% | 80% | 88% | 82% | 83% | 100% | nd |
| Spec 2 | 53% | 89% | 78% | 36% | 35% | 42% | 4% | 34% | nd |
| Cutoff 3 | 0.453 | 2.38 | 0.312 | 0.127 | 0.127 | 0.275 | 0.00904 | 0.271 | nd |
| Sens 3 | 90% | 100% | 100% | 93% | 100% | 91% | 100% | 100% | nd |
| Spec 3 | 48% | 89% | 39% | 17% | 17% | 35% | 1% | 34% | nd |
| Cutoff 4 | 1.09 | 1.14 | 1.10 | 1.09 | 1.14 | 1.10 | 1.09 | 1.14 | nd |

|  | 0hr prior to AKI stage | | | 24hr prior to AKI stage | | | 48hr prior to AKI stage | | |
|---|---|---|---|---|---|---|---|---|---|
|  | sCr or UO | sCr only | UO only | sCr or UO | sCr only | UO only | sCr or UO | sCr only | UO only |
| Sens 4 | 70% | 100% | 83% | 67% | 38% | 64% | 17% | 25% | nd |
| Spec 4 | 70% | 70% | 70% | 70% | 70% | 70% | 70% | 70% | nd |
| Cutoff 5 | 1.47 | 1.57 | 1.49 | 1.47 | 1.57 | 1.49 | 1.47 | 1.57 | nd |
| Sens 5 | 50% | 100% | 67% | 53% | 25% | 55% | 17% | 25% | nd |
| Spec 5 | 80% | 80% | 80% | 80% | 80% | 80% | 80% | 80% | nd |
| Cutoff 6 | 2.44 | 2.48 | 2.44 | 2.44 | 2.48 | 2.44 | 2.44 | 2.48 | nd |
| Sens 6 | 40% | 50% | 67% | 33% | 0% | 45% | 17% | 25% | nd |
| Spec 6 | 90% | 90% | 90% | 90% | 90% | 90% | 90% | 90% | nd |
| OR Quart 2 | 0.99 | >0 | >1.0 | 1.0 | 2.0 | 2.0 | 1.0 | >1.0 | nd |
| p Value | 1.00 | <na | <1.00 | 1.0 | 0.57 | 0.57 | 1.0 | <1.00 | nd |
| 95% CI of OR Quart2 | 0.062 | >na | >0.062 | 0.14 | 0.18 | 0.18 | 0.062 | >0.062 | nd |
|  | 16 | na | na | 7.2 | 22 | 22 | 16 | na | nd |
| OR Quart 3 | 2.0 | >0 | >0 | 0.50 | 2.0 | 1.0 | 1.0 | >2.0 | nd |
| p Value | 0.57 | <na | <na | 0.57 | 0.57 | 1.0 | 1.0 | <0.57 | nd |
| 95% CI of OR Quart3 | 0.18 | >na | >na | 0.045 | 0.18 | 0.062 | 0.062 | >0.18 | nd |
|  | 22 | na | na | 5.5 | 22 | 16 | 16 | na | nd |
| OR Quart 4 | 6.1 | >2.0 | >5.1 | 5.2 | 3.0 | 7.2 | 3.1 | >1.0 | nd |
| p Value | 0.094 | <0.57 | <0.14 | 0.034 | 0.34 | 0.066 | 0.34 | <1.0 | nd |
| 95% CI of OR Quart4 | 0.73 | >0.18 | >0.59 | 1.1 | 0.31 | 0.88 | 0.31 | >0.062 | nd |
|  | 52 | na | na | 24 | 29 | 59 | 30 | na | nd |

**Vitronectin**

| sCr or UO | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 31.7 | 22.5 | 31.7 | 50.9 | 31.7 | 10.3 |
| Average | 74.2 | 84.5 | 74.2 | 70.1 | 74.2 | 133 |
| Stdev | 132 | 114 | 132 | 70.9 | 132 | 302 |
| p(t-test) |  | 0.81 |  | 0.90 |  | 0.29 |
| Min | 0.0795 | 0.119 | 0.0795 | 3.33 | 0.0795 | 0.237 |
| Max | 750 | 317 | 750 | 236 | 750 | 750 |
| n (Samp) | 689 | 10 | 689 | 15 | 689 | 6 |
| n (Patient) | 283 | 10 | 283 | 15 | 283 | 6 |

| sCr only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 31.9 | 16.7 | 31.9 | 40.8 | 31.9 | 22.5 |
| Average | 74.8 | 16.7 | 74.8 | 43.8 | 74.8 | 200 |
| Stdev | 132 | 9.45 | 132 | 33.5 | 132 | 367 |
| p(t-test) |  | 0.53 |  | 0.51 |  | 0.062 |
| Min | 0.0795 | 10.0 | 0.0795 | 5.44 | 0.0795 | 4.15 |
| Max | 750 | 23.4 | 750 | 101 | 750 | 750 |
| n (Samp) | 697 | 2 | 697 | 8 | 697 | 4 |
| n (Patient) | 288 | 2 | 288 | 8 | 288 | 4 |

| UO only | 0hr prior to AKI stage | | 24hr prior to AKI stage | | 48hr prior to AKI stage | |
|---|---|---|---|---|---|---|
|  | Cohort 1 | Cohort 2 | Cohort 1 | Cohort 2 | Cohort 1 | Cohort 2 |
| Median | 31.0 | 136 | 31.0 | 52.9 | nd | nd |

| UO only | 0hr prior to AKI stage | | 24hr prior to AKI stage | | 48hr prior to AKI stage | |
|---|---|---|---|---|---|---|
| | Cohort 1 | Cohort 2 | Cohort 1 | Cohort 2 | Cohort 1 | Cohort 2 |
| Average | 73.4 | 146 | 73.4 | 83.2 | nd | nd |
| Stdev | 133 | 151 | 133 | 78.0 | nd | nd |
| p(t-test) | | 0.18 | | 0.81 | nd | nd |
| Min | 0.0795 | 5.54 | 0.0795 | 2.03E-5 | nd | nd |
| Max | 750 | 317 | 750 | 236 | nd | nd |
| n (Samp) | 699 | 6 | 699 | 11 | nd | nd |
| n (Patient) | 273 | 6 | 273 | 11 | nd | nd |

| | 0hr prior to AKI stage | | | 24hr prior to AKI stage | | | 48hr prior to AKI stage | | |
|---|---|---|---|---|---|---|---|---|---|
| | sCr or UO | sCr only | UO only | sCr or UO | sCr only | UO only | sCr or UO | sCr only | UO only |
| AUC | 0.48 | 0.27 | 0.55 | 0.55 | 0.51 | 0.59 | 0.29 | 0.45 | nd |
| SE | 0.093 | 0.20 | 0.12 | 0.077 | 0.10 | 0.091 | 0.12 | 0.15 | nd |
| p | 0.85 | 0.27 | 0.71 | 0.54 | 0.93 | 0.33 | 0.079 | 0.73 | nd |
| nCohort 1 | 689 | 697 | 699 | 689 | 697 | 699 | 689 | 697 | nd |
| nCohort 2 | 10 | 2 | 6 | 15 | 8 | 11 | 6 | 4 | nd |
| Cutoff 1 | 12.1 | 9.97 | 11.1 | 17.9 | 23.0 | 26.7 | 3.97 | 22.2 | nd |
| Sens 1 | 70% | 100% | 83% | 73% | 75% | 73% | 83% | 75% | nd |
| Spec 1 | 19% | 16% | 18% | 31% | 39% | 46% | 4% | 37% | nd |
| Cutoff 2 | 11.1 | 9.97 | 11.1 | 14.1 | 8.80 | 14.1 | 3.97 | 4.09 | nd |
| Sens 2 | 80% | 100% | 83% | 80% | 88% | 82% | 83% | 100% | nd |
| Spec 2 | 18% | 16% | 18% | 23% | 14% | 23% | 4% | 5% | nd |
| Cutoff 3 | 9.97 | 9.97 | 5.44 | 5.37 | 5.37 | 3.16 | 0.112 | 4.09 | nd |
| Sens 3 | 90% | 100% | 100% | 93% | 100% | 91% | 100% | 100% | nd |
| Spec 3 | 16% | 16% | 7% | 7% | 7% | 3% | 0% | 5% | nd |
| Cutoff 4 | 53.0 | 53.9 | 51.5 | 53.0 | 53.9 | 51.5 | 53.0 | 53.9 | nd |
| Sens 4 | 40% | 0% | 50% | 40% | 50% | 55% | 17% | 25% | nd |
| Spec 4 | 70% | 70% | 70% | 70% | 70% | 70% | 70% | 70% | nd |
| Cutoff 5 | 76.6 | 79.8 | 76.2 | 76.6 | 79.8 | 76.2 | 76.6 | 79.8 | nd |
| Sens 5 | 40% | 0% | 50% | 33% | 12% | 45% | 17% | 25% | nd |
| Spec 5 | 80% | 80% | 80% | 80% | 80% | 80% | 80% | 80% | nd |
| Cutoff 6 | 169 | 173 | 166 | 169 | 173 | 166 | 169 | 173 | nd |
| Sens 6 | 20% | 0% | 50% | 7% | 0% | 18% | 17% | 25% | nd |
| Spec 6 | 90% | 90% | 90% | 90% | 90% | 90% | 90% | 90% | nd |
| OR Quart 2 | 0 | >0 | 0 | 0.75 | 1.0 | 0.33 | 0 | 0 | nd |
| p Value | na | <na | na | 0.70 | 1.0 | 0.34 | na | na | nd |
| 95% CI of | na | >na | na | 0.16 | 0.14 | 0.034 | na | na | nd |
| OR Quart2 | na | na | na | 3.4 | 7.2 | 3.2 | na | na | nd |
| OR Quart 3 | 0.49 | >1.0 | 0 | 0.49 | 1.0 | 0.66 | 2.0 | 2.0 | nd |
| p Value | 0.42 | <1.00 | na | 0.42 | 1.0 | 0.65 | 0.57 | 0.57 | nd |
| 95% CI of | 0.089 | >0.062 | na | 0.089 | 0.14 | 0.11 | 0.18 | 0.18 | nd |
| OR Quart3 | 2.7 | na | na | 2.7 | 7.2 | 4.0 | 22 | 23 | nd |
| OR Quart 4 | 1.0 | >1.0 | 0.99 | 1.5 | 0.99 | 1.7 | 3.1 | 1.0 | nd |
| p Value | 0.99 | <0.99 | 0.99 | 0.52 | 1.00 | 0.48 | 0.34 | 1.00 | nd |
| 95% CI of | 0.25 | >0.063 | 0.20 | 0.42 | 0.14 | 0.39 | 0.31 | 0.062 | nd |
| OR Quart4 | 4.1 | na | 5.0 | 5.5 | 7.1 | 7.1 | 30 | 16 | nd |

**Interleukin-22**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 1.78E-5 | 1.78E-5 | 1.78E-5 | 1.78E-5 |
| Average | 0.00124 | 0.0167 | 0.00124 | 0.0305 |
| Stdev | 0.00488 | 0.0295 | 0.00488 | 0.0826 |
| p(t-test) | | 4.8E-6 | | 1.4E-4 |
| Min | 4.41E-6 | 4.41E-6 | 4.41E-6 | 4.41E-6 |
| Max | 0.0367 | 0.0725 | 0.0367 | 0.235 |
| n (Samp) | 116 | 6 | 116 | 8 |
| n (Patient) | 56 | 6 | 56 | 8 |

| UO only | 0hr prior to AKI stage | | 24hr prior to AKI stage | |
|---|---|---|---|---|
| | Cohort 1 | Cohort 2 | Cohort 1 | Cohort 2 |
| Median | 1.78E-5 | 1.78E-5 | 1.78E-5 | 1.78E-5 |
| Average | 0.00117 | 0.0167 | 0.00117 | 0.0305 |
| Stdev | 0.00494 | 0.0295 | 0.00494 | 0.0826 |
| p(t-test) | | 9.0E-6 | | 2.5E-4 |
| Min | 4.41E-6 | 4.41E-6 | 4.41E-6 | 4.41E-6 |
| Max | 0.0367 | 0.0725 | 0.0367 | 0.235 |
| n (Samp) | 106 | 6 | 106 | 8 |
| n (Patient) | 51 | 6 | 51 | 8 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.71 | nd | 0.72 | 0.67 | nd | 0.68 |
| SE | 0.12 | nd | 0.12 | 0.11 | nd | 0.11 |
| p | 0.083 | nd | 0.072 | 0.12 | nd | 0.100 |
| nCohort 1 | 116 | nd | 106 | 116 | nd | 106 |
| nCohort 2 | 6 | nd | 6 | 8 | nd | 8 |
| Cutoff 1 | 4.41E-6 | nd | 4.41E-6 | 4.41E-6 | nd | 4.41E-6 |
| Sens 1 | 83% | nd | 83% | 75% | nd | 75% |
| Spec 1 | 46% | nd | 47% | 46% | nd | 47% |
| Cutoff 2 | 4.41E-6 | nd | 4.41E-6 | 0 | nd | 0 |
| Sens 2 | 83% | nd | 83% | 100% | nd | 100% |
| Spec 2 | 46% | nd | 47% | 0% | nd | 0% |
| Cutoff 3 | 0 | nd | 0 | 0 | nd | 0 |
| Sens 3 | 100% | nd | 100% | 100% | nd | 100% |
| Spec 3 | 0% | nd | 0% | 0% | nd | 0% |
| Cutoff 4 | 1.78E-5 | nd | 1.78E-5 | 1.78E-5 | nd | 1.78E-5 |
| Sens 4 | 33% | nd | 33% | 38% | nd | 38% |
| Spec 4 | 91% | nd | 92% | 91% | nd | 92% |
| Cutoff 5 | 1.78E-5 | nd | 1.78E-5 | 1.78E-5 | nd | 1.78E-5 |
| Sens 5 | 33% | nd | 33% | 38% | nd | 38% |
| Spec 5 | 91% | nd | 92% | 91% | nd | 92% |
| Cutoff 6 | 1.78E-5 | nd | 1.78E-5 | 1.78E-5 | nd | 1.78E-5 |
| Sens 6 | 33% | nd | 33% | 38% | nd | 38% |
| Spec 6 | 91% | nd | 92% | 91% | nd | 92% |
| OR Quart 2 | >3.2 | nd | >2.2 | >6.0 | nd | >4.5 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| p Value | <0.32 | nd | <0.54 | <0.11 | nd | <0.19 |
| 95% CI of | >0.32 | nd | >0.18 | >0.65 | nd | >0.47 |
| OR Quart2 | na | nd | na | na | nd | na |
| OR Quart 3 | >1.0 | nd | >2.2 | >0 | nd | >1.0 |
| p Value | <0.98 | nd | <0.54 | <na | nd | <0.98 |
| 95% CI of | >0.062 | nd | >0.18 | >na | nd | >0.062 |
| OR Quart3 | na | nd | na | na | nd | na |
| OR Quart 4 | >2.1 | nd | >2.2 | >3.3 | nd | >3.2 |
| p Value | <0.56 | nd | <0.54 | <0.31 | nd | <0.32 |
| 95% CI of | >0.18 | nd | >0.18 | >0.33 | nd | >0.32 |
| OR Quart4 | na | nd | na | na | nd | na |

**Estradiol**

| sCr or UO | 24hr prior to AKI stage | |
|---|---|---|
|  | Cohort 1 | Cohort 2 |
| Median | 7.09 | 9.71 |
| Average | 10.6 | 14.3 |
| Stdev | 13.7 | 10.7 |
| p(t-test) |  | 0.51 |
| Min | 0.143 | 4.59 |
| Max | 128 | 34.2 |
| n (Samp) | 122 | 6 |
| n (Patient) | 96 | 6 |

| sCr only | 24hr prior to AKI stage | |
|---|---|---|
|  | Cohort 1 | Cohort 2 |
| Median | 7.11 | 9.69 |
| Average | 10.8 | 15.8 |
| Stdev | 13.6 | 10.9 |
| p(t-test) |  | 0.53 |
| Min | 0.143 | 9.28 |
| Max | 128 | 28.3 |
| n (Samp) | 126 | 3 |
| n (Patient) | 99 | 3 |

| UO only | 24hr prior to AKI stage | |
|---|---|---|
|  | Cohort 1 | Cohort 2 |
| Median | 7.05 | 14.0 |
| Average | 9.65 | 16.7 |
| Stdev | 8.73 | 13.0 |
| p(t-test) |  | 0.12 |
| Min | 0.143 | 4.59 |
| Max | 55.8 | 34.2 |
| n (Samp) | 104 | 4 |
| n (Patient) | 82 | 4 |

|  | 24hr prior to AKI stage | | |
|---|---|---|---|
|  | sCr or UO | sCr only | UO only |
| AUC | 0.68 | 0.74 | 0.70 |
| SE | 0.12 | 0.17 | 0.15 |
| p | 0.15 | 0.15 | 0.19 |
| nCohort 1 | 122 | 126 | 104 |
| nCohort 2 | 6 | 3 | 4 |
| Cutoff 1 | 9.25 | 9.25 | 9.47 |
| Sens 1 | 83% | 100% | 75% |
| Spec 1 | 64% | 63% | 67% |
| Cutoff 2 | 9.25 | 9.25 | 4.53 |
| Sens 2 | 83% | 100% | 100% |
| Spec 2 | 64% | 63% | 27% |
| Cutoff 3 | 4.53 | 9.25 | 4.53 |
| Sens 3 | 100% | 100% | 100% |
| Spec 3 | 28% | 63% | 27% |
| Cutoff 4 | 11.0 | 11.0 | 10.7 |
| Sens 4 | 33% | 33% | 50% |
| Spec 4 | 70% | 71% | 70% |
| Cutoff 5 | 13.3 | 13.9 | 12.9 |
| Sens 5 | 33% | 33% | 50% |
| Spec 5 | 80% | 80% | 81% |
| Cutoff 6 | 22.4 | 23.2 | 22.4 |
| Sens 6 | 17% | 33% | 25% |
| Spec 6 | 90% | 90% | 90% |
| OR Quart 2 | >1.0 | >0 | >1.0 |
| p Value | <0.98 | <na | <0.98 |
| 95% CI of | >0.062 | >na | >0.062 |
| OR Quart2 | na | na | na |
| OR Quart 3 | >3.3 | >2.1 | >1.0 |
| p Value | <0.31 | <0.54 | <0.98 |
| 95% CI of | >0.33 | >0.18 | >0.062 |
| OR Quart3 | na | na | na |
| OR Quart 4 | >2.1 | >1.0 | >2.2 |
| p Value | <0.54 | <1.0 | <0.54 |
| 95% CI of | >0.18 | >0.060 | >0.18 |
| OR Quart4 | na | na | na |

**Progesterone**

| sCr or UO | 24hr prior to AKI stage | |
|---|---|---|
|  | Cohort 1 | Cohort 2 |
| Median | 23.3 | 46.4 |
| Average | 36.3 | 42.3 |
| Stdev | 36.0 | 25.0 |
| p(t-test) |  | 0.68 |
| Min | 2.91 | 7.44 |
| Max | 228 | 75.2 |
| n (Samp) | 122 | 6 |
| n (Patient) | 96 | 6 |

| sCr only | 24hr prior to AKI stage | |
|---|---|---|
| | Cohort 1 | Cohort 2 |
| Median | 23.3 | 22.6 |
| Average | 36.5 | 23.2 |
| Stdev | 35.7 | 16.1 |
| p(t-test) | | 0.52 |
| Min | 2.91 | 7.44 |
| Max | 228 | 39.6 |
| n (Samp) | 126 | 3 |
| n (Patient) | 99 | 3 |

| UO only | 24hr prior to AKI stage | |
|---|---|---|
| | Cohort 1 | Cohort 2 |
| Median | 23.5 | 55.5 |
| Average | 37.4 | 51.7 |
| Stdev | 37.9 | 22.7 |
| p(t-test) | | 0.45 |
| Min | 2.91 | 20.9 |
| Max | 228 | 75.2 |
| n (Samp) | 104 | 4 |
| n (Patient) | 82 | 4 |

| | 24hr prior to AKI stage | | |
|---|---|---|---|
| | sCr or UO | sCr only | UO only |
| AUC | 0.62 | 0.42 | 0.73 |
| SE | 0.13 | 0.17 | 0.15 |
| p | 0.34 | 0.66 | 0.12 |
| nCohort 1 | 122 | 126 | 104 |
| nCohort 2 | 6 | 3 | 4 |
| Cutoff 1 | 20.3 | 7.23 | 52.2 |
| Sens 1 | 83% | 100% | 75% |
| Spec 1 | 43% | 10% | 78% |
| Cutoff 2 | 20.3 | 7.23 | 20.3 |
| Sens 2 | 83% | 100% | 100% |
| Spec 2 | 43% | 10% | 44% |
| Cutoff 3 | 7.23 | 7.23 | 20.3 |
| Sens 3 | 100% | 100% | 100% |
| Spec 3 | 11% | 10% | 44% |
| Cutoff 4 | 37.3 | 40.0 | 39.6 |
| Sens 4 | 67% | 0% | 75% |
| Spec 4 | 70% | 71% | 70% |
| Cutoff 5 | 57.6 | 57.6 | 56.1 |
| Sens 5 | 33% | 0% | 50% |
| Spec 5 | 80% | 80% | 81% |
| Cutoff 6 | 79.5 | 79.5 | 81.6 |
| Sens 6 | 0% | 0% | 0% |
| Spec 6 | 90% | 90% | 90% |
| OR Quart 2 | 1.0 | >1.1 | >1.0 |
| p Value | 1.0 | <0.97 | <0.98 |

| | 24hr prior to AKI stage | | |
|---|---|---|---|
| | sCr or UO | sCr only | UO only |
| 95% CI of | 0.060 | >0.064 | >0.062 |
| OR Quart2 | 17 | na | na |
| OR Quart 3 | 1.0 | >1.1 | >0 |
| p Value | 1.0 | <0.97 | <na |
| 95% CI of | 0.060 | >0.064 | >na |
| OR Quart3 | 17 | na | na |
| OR Quart 4 | 3.2 | >1.1 | >3.4 |
| p Value | 0.32 | <0.97 | <0.31 |
| 95% CI of | 0.32 | >0.064 | >0.33 |
| OR Quart4 | 33 | na | na |

**T4**

| sCr or UO | 24hr prior to AKI stage | |
|---|---|---|
| | Cohort 1 | Cohort 2 |
| Median | 4.41 | 6.49 |
| Average | 5.17 | 6.50 |
| Stdev | 5.65 | 4.33 |
| p(t-test) | | 0.57 |
| Min | 0.00501 | 1.20 |
| Max | 36.2 | 11.8 |
| n (Samp) | 122 | 6 |
| n (Patient) | 96 | 6 |

| sCr only | 24hr prior to AKI stage | |
|---|---|---|
| | Cohort 1 | Cohort 2 |
| Median | 4.43 | 4.69 |
| Average | 5.34 | 4.96 |
| Stdev | 5.68 | 3.89 |
| p(t-test) | | 0.91 |
| Min | 0.00501 | 1.20 |
| Max | 36.2 | 8.98 |
| n (Samp) | 126 | 3 |
| n (Patient) | 99 | 3 |

| UO only | 24hr prior to AKI stage | |
|---|---|---|
| | Cohort 1 | Cohort 2 |
| Median | 4.38 | 7.03 |
| Average | 5.20 | 7.21 |
| Stdev | 5.56 | 4.38 |
| p(t-test) | | 0.48 |
| Min | 0.00501 | 2.96 |
| Max | 36.2 | 11.8 |
| n (Samp) | 104 | 4 |
| n (Patient) | 82 | 4 |

|  | 24hr prior to AKI stage | | |
|---|---|---|---|
|  | sCr or UO | sCr only | UO only |
| AUC | 0.63 | 0.55 | 0.66 |
| SE | 0.13 | 0.17 | 0.15 |
| p | 0.30 | 0.76 | 0.30 |
| nCohort 1 | 122 | 126 | 104 |
| nCohort 2 | 6 | 3 | 4 |
| Cutoff 1 | 2.67 | 1.19 | 3.96 |
| Sens 1 | 83% | 100% | 75% |
| Spec 1 | 39% | 27% | 48% |
| Cutoff 2 | 2.67 | 1.19 | 2.67 |
| Sens 2 | 83% | 100% | 100% |
| Spec 2 | 39% | 27% | 39% |
| Cutoff 3 | 1.19 | 1.19 | 2.67 |
| Sens 3 | 100% | 100% | 100% |
| Spec 3 | 28% | 27% | 39% |
| Cutoff 4 | 6.44 | 6.52 | 6.52 |
| Sens 4 | 50% | 33% | 50% |
| Spec 4 | 70% | 71% | 70% |
| Cutoff 5 | 8.06 | 8.39 | 8.39 |
| Sens 5 | 50% | 33% | 50% |
| Spec 5 | 80% | 80% | 81% |
| Cutoff 6 | 11.7 | 12.5 | 12.5 |
| Sens 6 | 17% | 0% | 0% |
| Spec 6 | 90% | 90% | 90% |
| OR Quart 2 | >3.3 | >1.0 | >2.2 |
| p Value | <0.31 | <0.98 | <0.54 |
| 95% CI of | >0.33 | >0.062 | >0.18 |
| OR Quart2 | na | na | na |
| OR Quart 3 | >0 | >1.0 | >0 |
| p Value | <na | <0.98 | <na |
| 95% CI of | >na | >0.062 | >na |
| OR Quart3 | na | na | na |
| OR Quart 4 | >3.3 | >1.0 | >2.2 |
| p Value | <0.31 | <1.0 | <0.54 |
| 95% CI of | >0.33 | >0.060 | >0.18 |
| OR Quart4 | na | na | na |

Table 10: Comparison of marker levels in EDTA samples collected from Cohort 1 (patients that did not progress beyond RIFLE stage 0, R, or I) and in EDTA samples collected from Cohort 2 (subjects who progress to RIFLE stage F) at 0, 24 hours, and 48 hours prior to the subject reaching RIFLE stage I.

**Toll-like receptor 2**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 0.0770 | 0.0681 | 0.0770 | 0.0648 |

| sCr or UO | 0hr prior to AKI stage | | 24hr prior to AKI stage | |
| --- | --- | --- | --- | --- |
| | Cohort 1 | Cohort 2 | Cohort 1 | Cohort 2 |
| Average | 1.44 | 0.137 | 1.44 | 0.121 |
| Stdev | 3.96 | 0.199 | 3.96 | 0.162 |
| p(t-test) | | 0.43 | | 0.35 |
| Min | 1.14E-5 | 1.14E-5 | 1.14E-5 | 0.00551 |
| Max | 19.7 | 0.531 | 19.7 | 0.496 |
| n (Samp) | 116 | 6 | 116 | 8 |
| n (Patient) | 58 | 6 | 58 | 8 |

| UO only | 0hr prior to AKI stage | | 24hr prior to AKI stage | |
| --- | --- | --- | --- | --- |
| | Cohort 1 | Cohort 2 | Cohort 1 | Cohort 2 |
| Median | 0.0863 | 0.0681 | 0.0863 | 0.0648 |
| Average | 1.57 | 0.137 | 1.57 | 0.121 |
| Stdev | 4.12 | 0.199 | 4.12 | 0.162 |
| p(t-test) | | 0.40 | | 0.33 |
| Min | 1.14E-5 | 1.14E-5 | 1.14E-5 | 0.00551 |
| Max | 19.7 | 0.531 | 19.7 | 0.496 |
| n (Samp) | 106 | 6 | 106 | 8 |
| n (Patient) | 53 | 6 | 53 | 8 |

| | 0hr prior to AKI stage | | | 24hr prior to AKI stage | | |
| --- | --- | --- | --- | --- | --- | --- |
| | sCr or UO | sCr only | UO only | sCr or UO | sCr only | UO only |
| AUC | 0.45 | nd | 0.43 | 0.48 | nd | 0.46 |
| SE | 0.12 | nd | 0.13 | 0.11 | nd | 0.11 |
| p | 0.66 | nd | 0.56 | 0.83 | nd | 0.69 |
| nCohort 1 | 116 | nd | 106 | 116 | nd | 106 |
| nCohort 2 | 6 | nd | 6 | 8 | nd | 8 |
| Cutoff 1 | 0.0247 | nd | 0.0247 | 0.0366 | nd | 0.0366 |
| Sens 1 | 83% | nd | 83% | 75% | nd | 75% |
| Spec 1 | 29% | nd | 26% | 39% | nd | 37% |
| Cutoff 2 | 0.0247 | nd | 0.0247 | 0.0182 | nd | 0.0182 |
| Sens 2 | 83% | nd | 83% | 88% | nd | 88% |
| Spec 2 | 29% | nd | 26% | 28% | nd | 25% |
| Cutoff 3 | 0 | nd | 0 | 0.00402 | nd | 0.00402 |
| Sens 3 | 100% | nd | 100% | 100% | nd | 100% |
| Spec 3 | 0% | nd | 0% | 20% | nd | 18% |
| Cutoff 4 | 0.239 | nd | 0.279 | 0.239 | nd | 0.279 |
| Sens 4 | 17% | nd | 17% | 12% | nd | 12% |
| Spec 4 | 71% | nd | 71% | 71% | nd | 71% |
| Cutoff 5 | 0.389 | nd | 0.462 | 0.389 | nd | 0.462 |
| Sens 5 | 17% | nd | 17% | 12% | nd | 12% |
| Spec 5 | 80% | nd | 80% | 80% | nd | 80% |
| Cutoff 6 | 4.99 | nd | 6.59 | 4.99 | nd | 6.59 |
| Sens 6 | 0% | nd | 0% | 0% | nd | 0% |
| Spec 6 | 91% | nd | 91% | 91% | nd | 91% |
| OR Quart 2 | 2.1 | nd | 2.1 | 3.2 | nd | 3.4 |
| p Value | 0.54 | nd | 0.56 | 0.32 | nd | 0.31 |
| 95% CI of | 0.18 | nd | 0.18 | 0.32 | nd | 0.33 |

|  | 0hr prior to AKI stage | | | 24hr prior to AKI stage | | |
|---|---|---|---|---|---|---|
|  | sCr or UO | sCr only | UO only | sCr or UO | sCr only | UO only |
| OR Quart2 | 25 | nd | 24 | 33 | nd | 34 |
| OR Quart 3 | 2.1 | nd | 2.1 | 3.2 | nd | 3.2 |
| p Value | 0.56 | nd | 0.56 | 0.32 | nd | 0.32 |
| 95% CI of | 0.18 | nd | 0.18 | 0.32 | nd | 0.32 |
| OR Quart3 | 24 | nd | 24 | 33 | nd | 33 |
| OR Quart 4 | 1.0 | nd | 1.0 | 1.0 | nd | 1.0 |
| p Value | 0.98 | nd | 1.0 | 1.0 | nd | 0.98 |
| 95% CI of | 0.062 | nd | 0.059 | 0.060 | nd | 0.062 |
| OR Quart4 | 17 | nd | 17 | 17 | nd | 17 |

**Antithrombin-III**

| sCr or UO | 24hr prior to AKI stage | |
|---|---|---|
|  | Cohort 1 | Cohort 2 |
| Median | 109000 | 58000 |
| Average | 114000 | 58000 |
| Stdev | 36600 | 12200 |
| p(t-test) |  | 0.032 |
| Min | 36200 | 49300 |
| Max | 252000 | 66600 |
| n (Samp) | 128 | 2 |
| n (Patient) | 105 | 2 |

| UO only | 24hr prior to AKI stage | |
|---|---|---|
|  | Cohort 1 | Cohort 2 |
| Median | 105000 | 58000 |
| Average | 111000 | 58000 |
| Stdev | 31800 | 12200 |
| p(t-test) |  | 0.022 |
| Min | 36200 | 49300 |
| Max | 206000 | 66600 |
| n (Samp) | 112 | 2 |
| n (Patient) | 89 | 2 |

|  | 24hr prior to AKI stage | | |
|---|---|---|---|
|  | sCr or UO | sCr only | UO only |
| AUC | 0.027 | nd | 0.031 |
| SE | 0.081 | nd | 0.087 |
| p | 6.2E-9 | nd | 6.5E-8 |
| nCohort 1 | 128 | nd | 112 |
| nCohort 2 | 2 | nd | 2 |
| Cutoff 1 | 36200 | nd | 36200 |
| Sens 1 | 100% | nd | 100% |
| Spec 1 | 1% | nd | 1% |
| Cutoff 2 | 36200 | nd | 36200 |
| Sens 2 | 100% | nd | 100% |
| Spec 2 | 1% | nd | 1% |
| Cutoff 3 | 36200 | nd | 36200 |

| | 24hr prior to AKI stage | | |
|---|---|---|---|
| | sCr or UO | sCr only | UO only |
| Sens 3 | 100% | nd | 100% |
| Spec 3 | 1% | nd | 1% |
| Cutoff 4 | 123000 | nd | 121000 |
| Sens 4 | 0% | nd | 0% |
| Spec 4 | 70% | nd | 71% |
| Cutoff 5 | 142000 | nd | 141000 |
| Sens 5 | 0% | nd | 0% |
| Spec 5 | 80% | nd | 81% |
| Cutoff 6 | 164000 | nd | 151000 |
| Sens 6 | 0% | nd | 0% |
| Spec 6 | 91% | nd | 90% |
| OR Quart 2 | >0 | nd | >0 |
| p Value | <na | nd | <na |
| 95% CI of | >na | nd | >na |
| OR Quart2 | na | nd | na |
| OR Quart 3 | >0 | nd | >0 |
| p Value | <na | nd | <na |
| 95% CI of | >na | nd | >na |
| OR Quart3 | na | nd | na |
| OR Quart 4 | >2.2 | nd | >2.2 |
| p Value | <0.53 | nd | <0.52 |
| 95% CI of | >0.19 | nd | >0.19 |
| OR Quart4 | na | nd | na |

**Extracellular matrix protein 1**

| sCr or UO | 24hr prior to AKI stage | |
|---|---|---|
| | Cohort 1 | Cohort 2 |
| Median | 1500 | 1150 |
| Average | 1580 | 1150 |
| Stdev | 449 | 424 |
| p(t-test) | | 0.18 |
| Min | 535 | 846 |
| Max | 3060 | 1450 |
| n (Samp) | 128 | 2 |
| n (Patient) | 105 | 2 |

| UO only | 24hr prior to AKI stage | |
|---|---|---|
| | Cohort 1 | Cohort 2 |
| Median | 1460 | 1150 |
| Average | 1560 | 1150 |
| Stdev | 459 | 424 |
| p(t-test) | | 0.21 |
| Min | 535 | 846 |
| Max | 3060 | 1450 |
| n (Samp) | 112 | 2 |
| n (Patient) | 89 | 2 |

|  | 24hr prior to AKI stage | | |
|---|---|---|---|
|  | sCr or UO | sCr only | UO only |
| AUC | 0.23 | nd | 0.25 |
| SE | 0.20 | nd | 0.20 |
| p | 0.18 | nd | 0.23 |
| nCohort 1 | 128 | nd | 112 |
| nCohort 2 | 2 | nd | 2 |
| Cutoff 1 | 779 | nd | 779 |
| Sens 1 | 100% | nd | 100% |
| Spec 1 | 2% | nd | 3% |
| Cutoff 2 | 779 | nd | 779 |
| Sens 2 | 100% | nd | 100% |
| Spec 2 | 2% | nd | 3% |
| Cutoff 3 | 779 | nd | 779 |
| Sens 3 | 100% | nd | 100% |
| Spec 3 | 2% | nd | 3% |
| Cutoff 4 | 1720 | nd | 1720 |
| Sens 4 | 0% | nd | 0% |
| Spec 4 | 70% | nd | 71% |
| Cutoff 5 | 1950 | nd | 1910 |
| Sens 5 | 0% | nd | 0% |
| Spec 5 | 81% | nd | 80% |
| Cutoff 6 | 2240 | nd | 2230 |
| Sens 6 | 0% | nd | 0% |
| Spec 6 | 91% | nd | 90% |
| OR Quart 2 | >0 | nd | >0 |
| p Value | <na | nd | <na |
| 95% CI of OR Quart2 | >na na | nd | >na na |
| OR Quart 3 | >1.0 | nd | >1.0 |
| p Value | <0.98 | nd | <0.98 |
| 95% CI of OR Quart3 | >0.062 na | nd | >0.062 na |
| OR Quart 4 | >1.1 | nd | >1.1 |
| p Value | <0.97 | nd | <0.96 |
| 95% CI of OR Quart4 | >0.064 na | nd | >0.064 na |

**Vitronectin**

| sCr or UO | 24hr prior to AKI stage | |
|---|---|---|
|  | Cohort 1 | Cohort 2 |
| Median | 118000 | 37800 |
| Average | 119000 | 37800 |
| Stdev | 34000 | 3730 |
| p(t-test) |  | 9.4E-4 |
| Min | 57400 | 35100 |
| Max | 205000 | 40400 |
| n (Samp) | 128 | 2 |
| n (Patient) | 105 | 2 |

| UO only | 24hr prior to AKI stage | |
|---|---|---|
| | Cohort 1 | Cohort 2 |
| Median | 118000 | 37800 |
| Average | 118000 | 37800 |
| Stdev | 34100 | 3730 |
| p(t-test) | | 0.0012 |
| Min | 57400 | 35100 |
| Max | 205000 | 40400 |
| n (Samp) | 112 | 2 |
| n (Patient) | 89 | 2 |

| | 24hr prior to AKI stage | | |
|---|---|---|---|
| | sCr or UO | sCr only | UO only |
| AUC | 0 | nd | 0 |
| SE | <0.001 | nd | <0.001 |
| p | <1.0E-5 | nd | <1.0E-5 |
| nCohort 1 | 128 | nd | 112 |
| nCohort 2 | 2 | nd | 2 |
| Cutoff 1 | 0 | nd | 0 |
| Sens 1 | 100% | nd | 100% |
| Spec 1 | 0% | nd | 0% |
| Cutoff 2 | 0 | nd | 0 |
| Sens 2 | 100% | nd | 100% |
| Spec 2 | 0% | nd | 0% |
| Cutoff 3 | 0 | nd | 0 |
| Sens 3 | 100% | nd | 100% |
| Spec 3 | 0% | nd | 0% |
| Cutoff 4 | 133000 | nd | 132000 |
| Sens 4 | 0% | nd | 0% |
| Spec 4 | 70% | nd | 71% |
| Cutoff 5 | 150000 | nd | 149000 |
| Sens 5 | 0% | nd | 0% |
| Spec 5 | 80% | nd | 80% |
| Cutoff 6 | 172000 | nd | 167000 |
| Sens 6 | 0% | nd | 0% |
| Spec 6 | 91% | nd | 90% |
| OR Quart 2 | >0 | nd | >0 |
| p Value | <na | nd | <na |
| 95% CI of OR Quart2 | >na na | nd | >na na |
| OR Quart 3 | >0 | nd | >0 |
| p Value | <na | nd | <na |
| 95% CI of OR Quart3 | >na na | nd | >na na |
| OR Quart 4 | >2.2 | nd | >2.2 |
| p Value | <0.53 | nd | <0.52 |
| 95% CI of OR Quart4 | >0.19 na | nd | >0.19 na |

**Interleukin-22**

| sCr or UO | 0hr prior to AKI stage | | 24hr prior to AKI stage | |
|---|---|---|---|---|
| | Cohort 1 | Cohort 2 | Cohort 1 | Cohort 2 |
| Median | 0.708 | 0.577 | 0.708 | 0.919 |
| Average | 1.12 | 0.584 | 1.12 | 0.861 |
| Stdev | 1.96 | 0.359 | 1.96 | 0.545 |
| p(t-test) | | 0.50 | | 0.71 |
| Min | 4.00E-6 | 0.117 | 4.00E-6 | 0.209 |
| Max | 15.3 | 1.17 | 15.3 | 1.78 |
| n (Samp) | 116 | 6 | 116 | 8 |
| n (Patient) | 58 | 6 | 58 | 8 |

| UO only | 0hr prior to AKI stage | | 24hr prior to AKI stage | |
|---|---|---|---|---|
| | Cohort 1 | Cohort 2 | Cohort 1 | Cohort 2 |
| Median | 0.731 | 0.577 | 0.731 | 0.919 |
| Average | 1.18 | 0.584 | 1.18 | 0.861 |
| Stdev | 2.04 | 0.359 | 2.04 | 0.545 |
| p(t-test) | | 0.48 | | 0.66 |
| Min | 4.00E-6 | 0.117 | 4.00E-6 | 0.209 |
| Max | 15.3 | 1.17 | 15.3 | 1.78 |
| n (Samp) | 106 | 6 | 106 | 8 |
| n (Patient) | 53 | 6 | 53 | 8 |

| | 0hr prior to AKI stage | | | 24hr prior to AKI stage | | |
|---|---|---|---|---|---|---|
| | sCr or UO | sCr only | UO only | sCr or UO | sCr only | UO only |
| AUC | 0.35 | nd | 0.33 | 0.54 | nd | 0.53 |
| SE | 0.12 | nd | 0.12 | 0.11 | nd | 0.11 |
| p | 0.24 | nd | 0.18 | 0.71 | nd | 0.81 |
| nCohort 1 | 116 | nd | 106 | 116 | nd | 106 |
| nCohort 2 | 6 | nd | 6 | 8 | nd | 8 |
| Cutoff 1 | 0.335 | nd | 0.335 | 0.413 | nd | 0.413 |
| Sens 1 | 83% | nd | 83% | 75% | nd | 75% |
| Spec 1 | 10% | nd | 8% | 16% | nd | 13% |
| Cutoff 2 | 0.335 | nd | 0.335 | 0.274 | nd | 0.274 |
| Sens 2 | 83% | nd | 83% | 88% | nd | 88% |
| Spec 2 | 10% | nd | 8% | 8% | nd | 5% |
| Cutoff 3 | 0.0453 | nd | 4.00E-6 | 0.195 | nd | 0.195 |
| Sens 3 | 100% | nd | 100% | 100% | nd | 100% |
| Spec 3 | 2% | nd | 1% | 4% | nd | 3% |
| Cutoff 4 | 0.881 | nd | 0.893 | 0.881 | nd | 0.893 |
| Sens 4 | 17% | nd | 17% | 62% | nd | 62% |
| Spec 4 | 71% | nd | 71% | 71% | nd | 71% |
| Cutoff 5 | 1.07 | nd | 1.07 | 1.07 | nd | 1.07 |
| Sens 5 | 17% | nd | 17% | 25% | nd | 25% |
| Spec 5 | 80% | nd | 80% | 80% | nd | 80% |
| Cutoff 6 | 1.52 | nd | 1.60 | 1.52 | nd | 1.60 |
| Sens 6 | 0% | nd | 0% | 12% | nd | 12% |
| Spec 6 | 91% | nd | 91% | 91% | nd | 91% |
| OR Quart 2 | 1.0 | nd | 1.0 | 0 | nd | 0 |
| p Value | 0.98 | nd | 1.0 | na | nd | na |

|  | 0hr prior to AKI stage | | | 24hr prior to AKI stage | | |
|---|---|---|---|---|---|---|
|  | sCr or UO | sCr only | UO only | sCr or UO | sCr only | UO only |
| 95% CI of | 0.062 | nd | 0.059 | na | nd | na |
| OR Quart2 | 17 | nd | 17 | na | nd | na |
| OR Quart 3 | 2.1 | nd | 2.1 | 0.64 | nd | 0.64 |
| p Value | 0.56 | nd | 0.56 | 0.64 | nd | 0.64 |
| 95% CI of | 0.18 | nd | 0.18 | 0.100 | nd | 0.099 |
| OR Quart3 | 24 | nd | 24 | 4.1 | nd | 4.2 |
| OR Quart 4 | 2.1 | nd | 2.1 | 1.0 | nd | 0.96 |
| p Value | 0.54 | nd | 0.56 | 1.0 | nd | 0.96 |
| 95% CI of | 0.18 | nd | 0.18 | 0.19 | nd | 0.18 |
| OR Quart4 | 25 | nd | 24 | 5.4 | nd | 5.2 |

**Estradiol**

| sCr or UO | 24hr prior to AKI stage | |
|---|---|---|
|  | Cohort 1 | Cohort 2 |
| Median | 1.39 | 14.5 |
| Average | 2.06 | 14.5 |
| Stdev | 2.20 | 1.29 |
| p(t-test) |  | 7.9E-13 |
| Min | 0.288 | 13.6 |
| Max | 17.4 | 15.4 |
| n (Samp) | 126 | 2 |
| n (Patient) | 104 | 2 |

| UO only | 24hr prior to AKI stage | |
|---|---|---|
|  | Cohort 1 | Cohort 2 |
| Median | 1.39 | 14.5 |
| Average | 2.16 | 14.5 |
| Stdev | 2.33 | 1.29 |
| p(t-test) |  | 2.2E-11 |
| Min | 0.288 | 13.6 |
| Max | 17.4 | 15.4 |
| n (Samp) | 110 | 2 |
| n (Patient) | 88 | 2 |

|  | 24hr prior to AKI stage | | |
|---|---|---|---|
|  | sCr or UO | sCr only | UO only |
| AUC | 0.99 | nd | 0.99 |
| SE | 0.044 | nd | 0.048 |
| p | <1.0E-5 | nd | <1.0E-5 |
| nCohort 1 | 126 | nd | 110 |
| nCohort 2 | 2 | nd | 2 |
| Cutoff 1 | 10.8 | nd | 10.8 |
| Sens 1 | 100% | nd | 100% |
| Spec 1 | 99% | nd | 99% |
| Cutoff 2 | 10.8 | nd | 10.8 |
| Sens 2 | 100% | nd | 100% |
| Spec 2 | 99% | nd | 99% |

|  | 24hr prior to AKI stage | | |
|---|---|---|---|
|  | sCr or UO | sCr only | UO only |
| Cutoff 3 | 10.8 | nd | 10.8 |
| Sens 3 | 100% | nd | 100% |
| Spec 3 | 99% | nd | 99% |
| Cutoff 4 | 1.96 | nd | 2.01 |
| Sens 4 | 100% | nd | 100% |
| Spec 4 | 71% | nd | 70% |
| Cutoff 5 | 2.49 | nd | 2.94 |
| Sens 5 | 100% | nd | 100% |
| Spec 5 | 80% | nd | 80% |
| Cutoff 6 | 4.07 | nd | 4.47 |
| Sens 6 | 100% | nd | 100% |
| Spec 6 | 90% | nd | 90% |
| OR Quart 2 | >0 | nd | >0 |
| p Value | <na | nd | <na |
| 95% CI of | >na | nd | >na |
| OR Quart2 | na | nd | na |
| OR Quart 3 | >0 | nd | >0 |
| p Value | <na | nd | <na |
| 95% CI of | >na | nd | >na |
| OR Quart3 | na | nd | na |
| OR Quart 4 | >2.1 | nd | >2.2 |
| p Value | <0.54 | nd | <0.54 |
| 95% CI of | >0.18 | nd | >0.18 |
| OR Quart4 | na | nd | na |

**Progesterone**

| sCr or UO | 24hr prior to AKI stage | |
|---|---|---|
|  | Cohort 1 | Cohort 2 |
| Median | 4.44 | 113 |
| Average | 8.39 | 113 |
| Stdev | 20.6 | 115 |
| p(t-test) |  | 2.6E-9 |
| Min | 1.35 | 31.8 |
| Max | 172 | 194 |
| n (Samp) | 126 | 2 |
| n (Patient) | 104 | 2 |

| UO only | 24hr prior to AKI stage | |
|---|---|---|
|  | Cohort 1 | Cohort 2 |
| Median | 4.41 | 113 |
| Average | 8.46 | 113 |
| Stdev | 21.7 | 115 |
| p(t-test) |  | 2.0E-8 |
| Min | 1.35 | 31.8 |
| Max | 172 | 194 |
| n (Samp) | 110 | 2 |
| n (Patient) | 88 | 2 |

|  | 24hr prior to AKI stage | | |
|---|---|---|---|
|  | sCr or UO | sCr only | UO only |
| AUC | 0.99 | nd | 0.99 |
| SE | 0.054 | nd | 0.048 |
| p | <1.0E-5 | nd | <1.0E-5 |
| nCohort 1 | 126 | nd | 110 |
| nCohort 2 | 2 | nd | 2 |
| Cutoff 1 | 31.4 | nd | 31.4 |
| Sens 1 | 100% | nd | 100% |
| Spec 1 | 98% | nd | 98% |
| Cutoff 2 | 31.4 | nd | 31.4 |
| Sens 2 | 100% | nd | 100% |
| Spec 2 | 98% | nd | 98% |
| Cutoff 3 | 31.4 | nd | 31.4 |
| Sens 3 | 100% | nd | 100% |
| Spec 3 | 98% | nd | 98% |
| Cutoff 4 | 6.56 | nd | 6.51 |
| Sens 4 | 100% | nd | 100% |
| Spec 4 | 71% | nd | 70% |
| Cutoff 5 | 8.03 | nd | 8.03 |
| Sens 5 | 100% | nd | 100% |
| Spec 5 | 80% | nd | 80% |
| Cutoff 6 | 10.4 | nd | 10.4 |
| Sens 6 | 100% | nd | 100% |
| Spec 6 | 90% | nd | 90% |
| OR Quart 2 | >0 | nd | >0 |
| p Value | <na | nd | <na |
| 95% CI of | >na | nd | >na |
| OR Quart2 | na | nd | na |
| OR Quart 3 | >0 | nd | >0 |
| p Value | <na | nd | <na |
| 95% CI of | >na | nd | >na |
| OR Quart3 | na | nd | na |
| OR Quart 4 | >2.1 | nd | >2.2 |
| p Value | <0.54 | nd | <0.54 |
| 95% CI of | >0.18 | nd | >0.18 |
| OR Quart4 | na | nd | na |

**T4**

| sCr or UO | 24hr prior to AKI stage | |
|---|---|---|
|  | Cohort 1 | Cohort 2 |
| Median | 2.57 | 0.858 |
| Average | 3.10 | 0.858 |
| Stdev | 2.71 | 0.801 |
| p(t-test) |  | 0.25 |
| Min | 0.000136 | 0.291 |
| Max | 22.2 | 1.42 |
| n (Samp) | 126 | 2 |

| sCr or UO | 24hr prior to AKI stage | |
|---|---|---|
| | Cohort 1 | Cohort 2 |
| n (Patient) | 104 | 2 |

| UO only | 24hr prior to AKI stage | |
|---|---|---|
| | Cohort 1 | Cohort 2 |
| Median | 2.53 | 0.858 |
| Average | 2.96 | 0.858 |
| Stdev | 2.65 | 0.801 |
| p(t-test) | | 0.27 |
| Min | 0.000136 | 0.291 |
| Max | 22.2 | 1.42 |
| n (Samp) | 110 | 2 |
| n (Patient) | 88 | 2 |

| | 24hr prior to AKI stage | | |
|---|---|---|---|
| | sCr or UO | sCr only | UO only |
| AUC | 0.16 | nd | 0.17 |
| SE | 0.18 | nd | 0.18 |
| p | 0.052 | nd | 0.064 |
| nCohort 1 | 126 | nd | 110 |
| nCohort 2 | 2 | nd | 2 |
| Cutoff 1 | 0.256 | nd | 0.256 |
| Sens 1 | 100% | nd | 100% |
| Spec 1 | 7% | nd | 8% |
| Cutoff 2 | 0.256 | nd | 0.256 |
| Sens 2 | 100% | nd | 100% |
| Spec 2 | 7% | nd | 8% |
| Cutoff 3 | 0.256 | nd | 0.256 |
| Sens 3 | 100% | nd | 100% |
| Spec 3 | 7% | nd | 8% |
| Cutoff 4 | 3.68 | nd | 3.39 |
| Sens 4 | 0% | nd | 0% |
| Spec 4 | 71% | nd | 70% |
| Cutoff 5 | 4.47 | nd | 4.31 |
| Sens 5 | 0% | nd | 0% |
| Spec 5 | 80% | nd | 80% |
| Cutoff 6 | 6.18 | nd | 5.44 |
| Sens 6 | 0% | nd | 0% |
| Spec 6 | 90% | nd | 90% |
| OR Quart 2 | >0 | nd | >0 |
| p Value | <na | nd | <na |
| 95% CI of OR Quart2 | >na na | nd | >na na |
| OR Quart 3 | >1.0 | nd | >1.0 |
| p Value | <0.98 | nd | <0.98 |
| 95% CI of OR Quart3 | >0.062 na | nd | >0.062 na |
| OR Quart 4 | >1.0 | nd | >1.0 |

|  | 24hr prior to AKI stage | | |
|---|---|---|---|
|  | sCr or UO | sCr only | UO only |
| p Value | <0.98 | nd | <0.98 |
| 95% CI of | >0.062 | nd | >0.062 |
| OR Quart4 | na | nd | na |

**Growth/differentiation factor 15**

| sCr or UO | 24hr prior to AKI stage | |
|---|---|---|
|  | Cohort 1 | Cohort 2 |
| Median | 2050 | 8100 |
| Average | 2510 | 8100 |
| Stdev | 1680 | 16.4 |
| p(t-test) |  | 7.1E-6 |
| Min | 271 | 8090 |
| Max | 7790 | 8110 |
| n (Samp) | 126 | 2 |
| n (Patient) | 104 | 2 |

| UO only | 24hr prior to AKI stage | |
|---|---|---|
|  | Cohort 1 | Cohort 2 |
| Median | 2090 | 8100 |
| Average | 2620 | 8100 |
| Stdev | 1680 | 16.4 |
| p(t-test) |  | 1.2E-5 |
| Min | 437 | 8090 |
| Max | 7790 | 8110 |
| n (Samp) | 110 | 2 |
| n (Patient) | 88 | 2 |

|  | 24hr prior to AKI stage | | |
|---|---|---|---|
|  | sCr or UO | sCr only | UO only |
| AUC | 1.0 | nd | 1.0 |
| SE | 0 | nd | 0 |
| p | <1.0E-5 | nd | <1.0E-5 |
| nCohort 1 | 126 | nd | 110 |
| nCohort 2 | 2 | nd | 2 |
| Cutoff 1 | 7790 | nd | 7790 |
| Sens 1 | 100% | nd | 100% |
| Spec 1 | 100% | nd | 100% |
| Cutoff 2 | 7790 | nd | 7790 |
| Sens 2 | 100% | nd | 100% |
| Spec 2 | 100% | nd | 100% |
| Cutoff 3 | 7790 | nd | 7790 |
| Sens 3 | 100% | nd | 100% |
| Spec 3 | 100% | nd | 100% |
| Cutoff 4 | 2840 | nd | 3100 |
| Sens 4 | 100% | nd | 100% |
| Spec 4 | 71% | nd | 70% |
| Cutoff 5 | 3750 | nd | 3880 |

|  | 24hr prior to AKI stage | | |
|  | sCr or UO | sCr only | UO only |
| --- | --- | --- | --- |
| Sens 5 | 100% | nd | 100% |
| Spec 5 | 80% | nd | 80% |
| Cutoff 6 | 5190 | nd | 5190 |
| Sens 6 | 100% | nd | 100% |
| Spec 6 | 90% | nd | 90% |
| OR Quart 2 | >0 | nd | >0 |
| p Value | <na | nd | <na |
| 95% CI of | >na | nd | >na |
| OR Quart2 | na | nd | na |
| OR Quart 3 | >0 | nd | >0 |
| p Value | <na | nd | <na |
| 95% CI of | >na | nd | >na |
| OR Quart3 | na | nd | na |
| OR Quart 4 | >2.1 | nd | >2.2 |
| p Value | <0.54 | nd | <0.54 |
| 95% CI of | >0.18 | nd | >0.18 |
| OR Quart4 | na | nd | na |

**Proprotein convertase subtilisin/kexin type 9**

| sCr or UO | 24hr prior to AKI stage | |
|  | Cohort 1 | Cohort 2 |
| --- | --- | --- |
| Median | 493000 | 197000 |
| Average | 505000 | 197000 |
| Stdev | 202000 | 133000 |
| p(t-test) |  | 0.034 |
| Min | 76300 | 102000 |
| Max | 1100000 | 291000 |
| n (Samp) | 128 | 2 |
| n (Patient) | 105 | 2 |

| UO only | 24hr prior to AKI stage | |
|  | Cohort 1 | Cohort 2 |
| --- | --- | --- |
| Median | 493000 | 197000 |
| Average | 502000 | 197000 |
| Stdev | 197000 | 133000 |
| p(t-test) |  | 0.032 |
| Min | 76300 | 102000 |
| Max | 1000000 | 291000 |
| n (Samp) | 112 | 2 |
| n (Patient) | 89 | 2 |

|  | 24hr prior to AKI stage | | |
|  | sCr or UO | sCr only | UO only |
| --- | --- | --- | --- |
| AUC | 0.074 | nd | 0.080 |
| SE | 0.13 | nd | 0.13 |
| p | 9.8E-4 | nd | 0.0017 |
| nCohort 1 | 128 | nd | 112 |

|  | 24hr prior to AKI stage | | |
|  | sCr or UO | sCr only | UO only |
|---|---|---|---|
| nCohort 2 | 2 | nd | 2 |
| Cutoff 1 | 76300 | nd | 76300 |
| Sens 1 | 100% | nd | 100% |
| Spec 1 | 1% | nd | 1% |
| Cutoff 2 | 76300 | nd | 76300 |
| Sens 2 | 100% | nd | 100% |
| Spec 2 | 1% | nd | 1% |
| Cutoff 3 | 76300 | nd | 76300 |
| Sens 3 | 100% | nd | 100% |
| Spec 3 | 1% | nd | 1% |
| Cutoff 4 | 598000 | nd | 598000 |
| Sens 4 | 0% | nd | 0% |
| Spec 4 | 70% | nd | 71% |
| Cutoff 5 | 676000 | nd | 676000 |
| Sens 5 | 0% | nd | 0% |
| Spec 5 | 80% | nd | 80% |
| Cutoff 6 | 820000 | nd | 814000 |
| Sens 6 | 0% | nd | 0% |
| Spec 6 | 91% | nd | 90% |
| OR Quart 2 | >0 | nd | >0 |
| p Value | <na | nd | <na |
| 95% CI of | >na | nd | >na |
| OR Quart2 | na | nd | na |
| OR Quart 3 | >0 | nd | >0 |
| p Value | <na | nd | <na |
| 95% CI of | >na | nd | >na |
| OR Quart3 | na | nd | na |
| OR Quart 4 | >2.2 | nd | >2.2 |
| p Value | <0.53 | nd | <0.52 |
| 95% CI of | >0.19 | nd | >0.19 |
| OR Quart4 | na | nd | na |

Table 11: Comparison of marker levels in enroll urine samples collected from Cohort 1 (patients that did not progress beyond RIFLE stage 0 or R within 48hrs) and in enroll urine samples collected from Cohort 2 (subjects reaching RIFLE stage I or F within 48hrs). Enroll samples from patients already at RIFLE stage I or F were included in Cohort 2.

**Antithrombin-III**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 87.3 | 174 | 97.6 | 172 | 89.5 | 164 |
| Average | 317 | 874 | 434 | 707 | 322 | 975 |
| Stdev | 931 | 1630 | 1120 | 1680 | 935 | 1730 |
| p(t-test) |  | 0.0015 |  | 0.43 |  | 5.3E-4 |
| Min | 0.0182 | 0.347 | 0.0182 | 14.7 | 0.0182 | 0.347 |

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Max | 6000 | 6000 | 6000 | 6000 | 6000 | 6000 |
| n (Samp) | 190 | 54 | 227 | 12 | 188 | 47 |
| n (Patient) | 190 | 54 | 227 | 12 | 188 | 47 |

|  | At Enrollment | | |
|---|---|---|---|
|  | sCr or UO | sCr only | UO only |
| AUC | 0.65 | 0.63 | 0.63 |
| SE | 0.045 | 0.088 | 0.048 |
| p | 8.7E-4 | 0.13 | 0.0064 |
| nCohort 1 | 190 | 227 | 188 |
| nCohort 2 | 54 | 12 | 47 |
| Cutoff 1 | 89.6 | 114 | 84.9 |
| Sens 1 | 70% | 75% | 70% |
| Spec 1 | 52% | 56% | 48% |
| Cutoff 2 | 56.5 | 56.5 | 54.2 |
| Sens 2 | 81% | 83% | 81% |
| Spec 2 | 35% | 32% | 31% |
| Cutoff 3 | 42.4 | 54.2 | 21.8 |
| Sens 3 | 91% | 92% | 91% |
| Spec 3 | 24% | 30% | 13% |
| Cutoff 4 | 152 | 172 | 158 |
| Sens 4 | 57% | 50% | 51% |
| Spec 4 | 70% | 70% | 70% |
| Cutoff 5 | 223 | 280 | 231 |
| Sens 5 | 37% | 33% | 36% |
| Spec 5 | 80% | 80% | 80% |
| Cutoff 6 | 435 | 600 | 472 |
| Sens 6 | 24% | 17% | 28% |
| Spec 6 | 90% | 90% | 90% |
| OR Quart 2 | 2.5 | 2.0 | 2.2 |
| p Value | 0.087 | 0.58 | 0.14 |
| 95% CI of | 0.88 | 0.18 | 0.77 |
| OR Quart2 | 7.0 | 23 | 6.4 |
| OR Quart 3 | 2.7 | 4.1 | 2.0 |
| p Value | 0.057 | 0.21 | 0.21 |
| 95% CI of | 0.97 | 0.45 | 0.68 |
| OR Quart3 | 7.7 | 38 | 5.8 |
| OR Quart 4 | 4.8 | 5.3 | 3.8 |
| p Value | 0.0020 | 0.13 | 0.0096 |
| 95% CI of | 1.8 | 0.60 | 1.4 |
| OR Quart4 | 13 | 47 | 10 |

**Extracellular matrix protein 1**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 0.346 | 1.26 | 0.392 | 1.26 | 0.371 | 1.12 |
| Average | 0.816 | 3.58 | 1.34 | 3.19 | 0.923 | 3.64 |

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Stdev | 1.24 | 8.33 | 4.18 | 5.16 | 1.59 | 8.75 |
| p(t-test) | | 1.5E-5 | | 0.14 | | 7.9E-5 |
| Min | 0.000528 | 0.00419 | 0.000528 | 0.00419 | 0.000528 | 0.00419 |
| Max | 10.9 | 57.7 | 57.7 | 14.4 | 14.4 | 57.7 |
| n (Samp) | 190 | 54 | 227 | 12 | 188 | 47 |
| n (Patient) | 190 | 54 | 227 | 12 | 188 | 47 |

| | At Enrollment | | |
|---|---|---|---|
| | sCr or UO | sCr only | UO only |
| AUC | 0.69 | 0.67 | 0.66 |
| SE | 0.043 | 0.088 | 0.047 |
| p | 1.2E-5 | 0.055 | 4.6E-4 |
| nCohort 1 | 190 | 227 | 188 |
| nCohort 2 | 54 | 12 | 47 |
| Cutoff 1 | 0.411 | 0.689 | 0.396 |
| Sens 1 | 70% | 75% | 70% |
| Spec 1 | 56% | 61% | 54% |
| Cutoff 2 | 0.238 | 0.362 | 0.182 |
| Sens 2 | 81% | 83% | 81% |
| Spec 2 | 41% | 47% | 36% |
| Cutoff 3 | 0.0936 | 0.348 | 0.0837 |
| Sens 3 | 91% | 92% | 91% |
| Spec 3 | 19% | 47% | 18% |
| Cutoff 4 | 0.857 | 1.08 | 0.995 |
| Sens 4 | 56% | 58% | 51% |
| Spec 4 | 70% | 70% | 70% |
| Cutoff 5 | 1.28 | 1.82 | 1.48 |
| Sens 5 | 50% | 25% | 45% |
| Spec 5 | 80% | 80% | 80% |
| Cutoff 6 | 2.15 | 2.82 | 2.21 |
| Sens 6 | 35% | 17% | 38% |
| Spec 6 | 90% | 90% | 90% |
| OR Quart 2 | 1.3 | 2.0 | 1.3 |
| p Value | 0.59 | 0.58 | 0.62 |
| 95% CI of | 0.46 | 0.18 | 0.45 |
| OR Quart2 | 3.8 | 23 | 3.8 |
| OR Quart 3 | 1.9 | 3.1 | 1.5 |
| p Value | 0.22 | 0.34 | 0.46 |
| 95% CI of | 0.69 | 0.31 | 0.52 |
| OR Quart3 | 5.2 | 30 | 4.2 |
| OR Quart 4 | 5.7 | 6.4 | 4.0 |
| p Value | 2.6E-4 | 0.089 | 0.0042 |
| 95% CI of | 2.2 | 0.75 | 1.6 |
| OR Quart4 | 15 | 55 | 10 |

## Coagulation factor XIII A and B chains

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 3.06 | 5.13 | 3.48 | 3.47 | 3.06 | 6.21 |
| Average | 6.14 | 15.5 | 7.81 | 16.7 | 6.13 | 17.1 |
| Stdev | 8.24 | 29.5 | 13.2 | 44.7 | 8.20 | 31.3 |
| p(t-test) |  | 1.4E-4 |  | 0.062 |  | 2.9E-5 |
| Min | 0.000120 | 0.000145 | 0.000120 | 0.000455 | 0.000120 | 0.000145 |
| Max | 58.0 | 158 | 143 | 158 | 58.0 | 158 |
| n (Samp) | 190 | 54 | 227 | 12 | 188 | 47 |
| n (Patient) | 190 | 54 | 227 | 12 | 188 | 47 |

|  | At Enrollment | | |
|---|---|---|---|
|  | sCr or UO | sCr only | UO only |
| AUC | 0.61 | 0.49 | 0.62 |
| SE | 0.045 | 0.086 | 0.048 |
| p | 0.014 | 0.88 | 0.014 |
| nCohort 1 | 190 | 227 | 188 |
| nCohort 2 | 54 | 12 | 47 |
| Cutoff 1 | 2.52 | 1.89 | 2.73 |
| Sens 1 | 70% | 75% | 70% |
| Spec 1 | 45% | 34% | 47% |
| Cutoff 2 | 0.976 | 0.956 | 0.956 |
| Sens 2 | 81% | 83% | 81% |
| Spec 2 | 23% | 21% | 22% |
| Cutoff 3 | 0.000443 | 0.701 | 0.000327 |
| Sens 3 | 91% | 92% | 91% |
| Spec 3 | 5% | 19% | 4% |
| Cutoff 4 | 6.03 | 7.34 | 5.99 |
| Sens 4 | 46% | 25% | 51% |
| Spec 4 | 70% | 70% | 70% |
| Cutoff 5 | 8.96 | 11.3 | 8.96 |
| Sens 5 | 37% | 17% | 40% |
| Spec 5 | 80% | 80% | 80% |
| Cutoff 6 | 18.1 | 20.9 | 18.1 |
| Sens 6 | 24% | 8% | 28% |
| Spec 6 | 90% | 90% | 90% |
| OR Quart 2 | 0.69 | 2.1 | 0.40 |
| p Value | 0.46 | 0.41 | 0.11 |
| 95% CI of | 0.26 | 0.36 | 0.13 |
| OR Quart2 | 1.8 | 12 | 1.2 |
| OR Quart 3 | 1.5 | 1.5 | 1.1 |
| p Value | 0.38 | 0.65 | 0.85 |
| 95% CI of | 0.62 | 0.25 | 0.44 |
| OR Quart3 | 3.6 | 9.5 | 2.7 |
| OR Quart 4 | 2.2 | 1.6 | 2.0 |
| p Value | 0.064 | 0.64 | 0.10 |
| 95% CI of | 0.95 | 0.25 | 0.86 |

| | At Enrollment | | |
|---|---|---|---|
| | sCr or UO | sCr only | UO only |
| OR Quart4 | 5.2 | 9.7 | 4.8 |

**Vitronectin**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 23.0 | 47.1 | 25.8 | 29.5 | 23.0 | 49.4 |
| Average | 47.8 | 108 | 60.5 | 89.7 | 48.4 | 118 |
| Stdev | 92.8 | 154 | 109 | 177 | 93.4 | 162 |
| p(t-test) | | 4.1E-4 | | 0.38 | | 1.3E-4 |
| Min | 0.550 | 2.03E-5 | 0.237 | 2.03E-5 | 0.550 | 2.03E-5 |
| Max | 750 | 641 | 750 | 641 | 750 | 641 |
| n (Samp) | 190 | 54 | 227 | 12 | 188 | 47 |
| n (Patient) | 190 | 54 | 227 | 12 | 188 | 47 |

| | At Enrollment | | |
|---|---|---|---|
| | sCr or UO | sCr only | UO only |
| AUC | 0.66 | 0.52 | 0.68 |
| SE | 0.044 | 0.087 | 0.046 |
| p | 1.9E-4 | 0.78 | 7.8E-5 |
| nCohort 1 | 190 | 227 | 188 |
| nCohort 2 | 54 | 12 | 47 |
| Cutoff 1 | 24.8 | 14.1 | 28.4 |
| Sens 1 | 70% | 75% | 70% |
| Spec 1 | 54% | 30% | 60% |
| Cutoff 2 | 16.0 | 8.80 | 18.5 |
| Sens 2 | 81% | 83% | 81% |
| Spec 2 | 37% | 19% | 44% |
| Cutoff 3 | 7.43 | 6.91 | 7.43 |
| Sens 3 | 91% | 92% | 91% |
| Spec 3 | 19% | 15% | 19% |
| Cutoff 4 | 37.7 | 45.7 | 37.7 |
| Sens 4 | 54% | 33% | 57% |
| Spec 4 | 70% | 70% | 70% |
| Cutoff 5 | 50.6 | 64.3 | 53.9 |
| Sens 5 | 46% | 33% | 47% |
| Spec 5 | 80% | 80% | 80% |
| Cutoff 6 | 98.1 | 138 | 98.1 |
| Sens 6 | 28% | 8% | 32% |
| Spec 6 | 90% | 90% | 90% |
| OR Quart 2 | 1.3 | 0.98 | 1.4 |
| p Value | 0.61 | 0.98 | 0.59 |
| 95% CI of | 0.47 | 0.19 | 0.44 |
| OR Quart2 | 3.6 | 5.1 | 4.2 |
| OR Quart 3 | 1.6 | 0.64 | 2.0 |
| p Value | 0.33 | 0.64 | 0.21 |
| 95% CI of | 0.61 | 0.10 | 0.68 |
| OR Quart3 | 4.3 | 4.0 | 5.8 |

| | At Enrollment | | |
|---|---|---|---|
| | sCr or UO | sCr only | UO only |
| OR Quart 4 | 4.3 | 1.3 | 5.2 |
| p Value | 0.0016 | 0.71 | 0.0013 |
| 95% CI of | 1.7 | 0.29 | 1.9 |
| OR Quart4 | 11 | 6.2 | 14 |

**T3**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 3.33 | 3.97 | 3.39 | 2.60 | 3.37 | 3.41 |
| Average | 4.66 | 4.18 | 4.66 | 3.40 | 4.67 | 3.96 |
| Stdev | 4.35 | 2.48 | 4.21 | 1.99 | 3.90 | 2.60 |
| p(t-test) | | 0.76 | | 0.61 | | 0.64 |
| Min | 0.000958 | 0.792 | 0.000958 | 1.94 | 0.569 | 0.792 |
| Max | 20.4 | 8.37 | 20.4 | 5.67 | 19.3 | 8.37 |
| n (Samp) | 50 | 8 | 55 | 3 | 42 | 7 |
| n (Patient) | 50 | 8 | 55 | 3 | 42 | 7 |

| | At Enrollment | | |
|---|---|---|---|
| | sCr or UO | sCr only | UO only |
| AUC | 0.53 | 0.44 | 0.49 |
| SE | 0.11 | 0.18 | 0.12 |
| p | 0.77 | 0.72 | 0.91 |
| nCohort 1 | 50 | 55 | 42 |
| nCohort 2 | 8 | 3 | 7 |
| Cutoff 1 | 2.59 | 1.87 | 2.59 |
| Sens 1 | 75% | 100% | 71% |
| Spec 1 | 38% | 25% | 33% |
| Cutoff 2 | 1.87 | 1.87 | 1.78 |
| Sens 2 | 88% | 100% | 86% |
| Spec 2 | 26% | 25% | 21% |
| Cutoff 3 | 0.757 | 1.87 | 0.757 |
| Sens 3 | 100% | 100% | 100% |
| Spec 3 | 8% | 25% | 7% |
| Cutoff 4 | 5.32 | 5.68 | 5.68 |
| Sens 4 | 38% | 0% | 29% |
| Spec 4 | 70% | 71% | 71% |
| Cutoff 5 | 6.52 | 6.52 | 6.56 |
| Sens 5 | 12% | 0% | 14% |
| Spec 5 | 80% | 80% | 81% |
| Cutoff 6 | 7.57 | 8.37 | 7.57 |
| Sens 6 | 12% | 0% | 14% |
| Spec 6 | 90% | 91% | 90% |
| OR Quart 2 | 2.0 | >1.2 | 1.1 |
| p Value | 0.59 | <0.92 | 0.93 |
| 95% CI of | 0.16 | >0.065 | 0.13 |
| OR Quart2 | 25 | na | 9.3 |
| OR Quart 3 | 3.5 | >2.3 | 0.50 |

|  | At Enrollment | | |
|---|---|---|---|
|  | sCr or UO | sCr only | UO only |
| p Value | 0.30 | <0.51 | 0.59 |
| 95% CI of | 0.32 | >0.19 | 0.039 |
| OR Quart3 | 39 | na | 6.4 |
| OR Quart 4 | 2.0 | >0 | 1.1 |
| p Value | 0.59 | <na | 0.93 |
| 95% CI of | 0.16 | >na | 0.13 |
| OR Quart4 | 25 | na | 9.3 |

T4

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 4.41 | 2.99 | 4.36 | 1.20 | 4.21 | 3.02 |
| Average | 5.22 | 3.79 | 5.18 | 2.11 | 5.50 | 4.17 |
| Stdev | 4.86 | 3.20 | 4.72 | 2.27 | 5.20 | 3.26 |
| p(t-test) |  | 0.43 |  | 0.27 |  | 0.52 |
| Min | 0.00501 | 0.443 | 0.00501 | 0.443 | 0.00501 | 0.443 |
| Max | 18.8 | 10.1 | 18.8 | 4.69 | 18.8 | 10.1 |
| n (Samp) | 50 | 8 | 55 | 3 | 42 | 7 |
| n (Patient) | 50 | 8 | 55 | 3 | 42 | 7 |

|  | At Enrollment | | |
|---|---|---|---|
|  | sCr or UO | sCr only | UO only |
| AUC | 0.44 | 0.33 | 0.47 |
| SE | 0.11 | 0.18 | 0.12 |
| p | 0.60 | 0.34 | 0.78 |
| nCohort 1 | 50 | 55 | 42 |
| nCohort 2 | 8 | 3 | 7 |
| Cutoff 1 | 1.20 | 0.428 | 2.39 |
| Sens 1 | 75% | 100% | 71% |
| Spec 1 | 26% | 18% | 33% |
| Cutoff 2 | 1.01 | 0.428 | 1.20 |
| Sens 2 | 88% | 100% | 86% |
| Spec 2 | 26% | 18% | 29% |
| Cutoff 3 | 0.428 | 0.428 | 0.428 |
| Sens 3 | 100% | 100% | 100% |
| Spec 3 | 20% | 18% | 21% |
| Cutoff 4 | 5.62 | 6.00 | 6.52 |
| Sens 4 | 25% | 0% | 14% |
| Spec 4 | 70% | 71% | 71% |
| Cutoff 5 | 8.06 | 8.06 | 8.89 |
| Sens 5 | 12% | 0% | 14% |
| Spec 5 | 80% | 80% | 81% |
| Cutoff 6 | 12.8 | 12.8 | 13.5 |
| Sens 6 | 0% | 0% | 0% |
| Spec 6 | 90% | 91% | 90% |
| OR Quart 2 | 0.50 | >1.2 | 2.4 |
| p Value | 0.59 | <0.92 | 0.50 |

|  | At Enrollment | | |
|---|---|---|---|
|  | sCr or UO | sCr only | UO only |
| 95% CI of OR Quart2 | 0.040 | >0.065 | 0.19 |
|  | 6.2 | na | 31 |
| OR Quart 3 | 2.4 | >1.1 | 4.0 |
| p Value | 0.37 | <0.96 | 0.26 |
| 95% CI of OR Quart3 | 0.36 | >0.061 | 0.35 |
|  | 15 | na | 45 |
| OR Quart 4 | 0.50 | >1.2 | 1.1 |
| p Value | 0.59 | <0.92 | 0.95 |
| 95% CI of OR Quart4 | 0.040 | >0.065 | 0.061 |
|  | 6.2 | na | 20 |

**Proprotein convertase subtilisin/kexin type 9**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 379 | 1180 | 443 | 3180 | 379 | 1430 |
| Average | 1220 | 1770 | 1240 | 2420 | 1100 | 1900 |
| Stdev | 2340 | 1190 | 2250 | 1390 | 1980 | 1220 |
| p(t-test) |  | 0.52 |  | 0.37 |  | 0.31 |
| Min | 70.6 | 521 | 70.6 | 813 | 70.6 | 521 |
| Max | 11400 | 3260 | 11400 | 3260 | 11000 | 3260 |
| n (Samp) | 50 | 8 | 55 | 3 | 42 | 7 |
| n (Patient) | 50 | 8 | 55 | 3 | 42 | 7 |

|  | At Enrollment | | |
|---|---|---|---|
|  | sCr or UO | sCr only | UO only |
| AUC | 0.80 | 0.84 | 0.81 |
| SE | 0.097 | 0.15 | 0.10 |
| p | 0.0019 | 0.022 | 0.0023 |
| nCohort 1 | 50 | 55 | 42 |
| nCohort 2 | 8 | 3 | 7 |
| Cutoff 1 | 897 | 804 | 919 |
| Sens 1 | 75% | 100% | 71% |
| Spec 1 | 76% | 69% | 76% |
| Cutoff 2 | 804 | 804 | 897 |
| Sens 2 | 88% | 100% | 86% |
| Spec 2 | 74% | 69% | 76% |
| Cutoff 3 | 479 | 804 | 479 |
| Sens 3 | 100% | 100% | 100% |
| Spec 3 | 60% | 69% | 60% |
| Cutoff 4 | 686 | 897 | 745 |
| Sens 4 | 88% | 67% | 86% |
| Spec 4 | 70% | 71% | 71% |
| Cutoff 5 | 1260 | 1300 | 1260 |
| Sens 5 | 50% | 67% | 57% |
| Spec 5 | 80% | 80% | 81% |
| Cutoff 6 | 3040 | 3080 | 3040 |
| Sens 6 | 38% | 67% | 43% |

|  | At Enrollment | | |
|---|---|---|---|
|  | sCr or UO | sCr only | UO only |
| Spec 6 | 90% | 91% | 90% |
| OR Quart 2 | >0 | >0 | >0 |
| p Value | <na | <na | <na |
| 95% CI of | >na | >na | >na |
| OR Quart2 | na | na | na |
| OR Quart 3 | >5.6 | >1.1 | >4.0 |
| p Value | <0.15 | <0.96 | <0.26 |
| 95% CI of | >0.54 | >0.061 | >0.35 |
| OR Quart3 | na | na | na |
| OR Quart 4 | >5.1 | >2.2 | >5.3 |
| p Value | <0.17 | <0.55 | <0.16 |
| 95% CI of | >0.50 | >0.17 | >0.51 |
| OR Quart4 | na | na | na |

Table 12: Comparison of marker levels in enroll EDTA samples collected from Cohort 1 (patients that did not progress beyond RIFLE stage 0 or R within 48hrs) and in enroll EDTA samples collected from Cohort 2 (subjects reaching RIFLE stage I or F within 48hrs). Enroll samples from patients already at stage I or F were included in Cohort 2.

**Antithrombin-III**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 107000 | 86000 | nd | nd | 105000 | 86000 |
| Average | 112000 | 87900 | nd | nd | 110000 | 87900 |
| Stdev | 37400 | 34600 | nd | nd | 35300 | 34600 |
| p(t-test) | | 0.066 | nd | nd | | 0.085 |
| Min | 36200 | 32000 | nd | nd | 36200 | 32000 |
| Max | 210000 | 146000 | nd | nd | 186000 | 146000 |
| n (Samp) | 47 | 10 | nd | nd | 41 | 10 |
| n (Patient) | 47 | 10 | nd | nd | 41 | 10 |

| | At Enrollment | | |
|---|---|---|---|
| | sCr or UO | sCr only | UO only |
| AUC | 0.32 | nd | 0.33 |
| SE | 0.10 | nd | 0.10 |
| p | 0.072 | nd | 0.094 |
| nCohort 1 | 47 | nd | 41 |
| nCohort 2 | 10 | nd | 10 |
| Cutoff 1 | 81300 | nd | 81300 |
| Sens 1 | 70% | nd | 70% |
| Spec 1 | 23% | nd | 24% |
| Cutoff 2 | 49300 | nd | 49300 |
| Sens 2 | 80% | nd | 80% |
| Spec 2 | 2% | nd | 2% |
| Cutoff 3 | 36200 | nd | 36200 |
| Sens 3 | 90% | nd | 90% |

|  | At Enrollment |  |  |
|---|---|---|---|
|  | sCr or UO | sCr only | UO only |
| Spec 3 | 2% | nd | 2% |
| Cutoff 4 | 120000 | nd | 117000 |
| Sens 4 | 20% | nd | 20% |
| Spec 4 | 70% | nd | 71% |
| Cutoff 5 | 147000 | nd | 145000 |
| Sens 5 | 0% | nd | 10% |
| Spec 5 | 81% | nd | 80% |
| Cutoff 6 | 164000 | nd | 163000 |
| Sens 6 | 0% | nd | 0% |
| Spec 6 | 91% | nd | 90% |
| OR Quart 2 | 0.50 | nd | 0.46 |
| p Value | 0.59 | nd | 0.55 |
| 95% CI of | 0.040 | nd | 0.036 |
| OR Quart2 | 6.2 | nd | 5.8 |
| OR Quart 3 | 2.6 | nd | 2.4 |
| p Value | 0.32 | nd | 0.36 |
| 95% CI of | 0.39 | nd | 0.36 |
| OR Quart3 | 17 | nd | 17 |
| OR Quart 4 | 1.8 | nd | 1.8 |
| p Value | 0.57 | nd | 0.55 |
| 95% CI of | 0.25 | nd | 0.25 |
| OR Quart4 | 13 | nd | 13 |

**Extracellular matrix protein 1**

|  | sCr or UO |  | sCr only |  | UO only |  |
|---|---|---|---|---|---|---|
|  | Cohort 1 | Cohort 2 | Cohort 1 | Cohort 2 | Cohort 1 | Cohort 2 |
| Median | 1430 | 1470 | nd | nd | 1420 | 1470 |
| Average | 1520 | 1500 | nd | nd | 1500 | 1500 |
| Stdev | 461 | 341 | nd | nd | 470 | 341 |
| p(t-test) |  | 0.91 | nd | nd |  | 1.00 |
| Min | 535 | 945 | nd | nd | 535 | 945 |
| Max | 2570 | 1990 | nd | nd | 2570 | 1990 |
| n (Samp) | 47 | 10 | nd | nd | 41 | 10 |
| n (Patient) | 47 | 10 | nd | nd | 41 | 10 |

|  | At Enrollment |  |  |
|---|---|---|---|
|  | sCr or UO | sCr only | UO only |
| AUC | 0.51 | nd | 0.52 |
| SE | 0.10 | nd | 0.10 |
| p | 0.95 | nd | 0.85 |
| nCohort 1 | 47 | nd | 41 |
| nCohort 2 | 10 | nd | 10 |
| Cutoff 1 | 1310 | nd | 1310 |
| Sens 1 | 70% | nd | 70% |
| Spec 1 | 43% | nd | 46% |
| Cutoff 2 | 1170 | nd | 1170 |
| Sens 2 | 80% | nd | 80% |

|  | At Enrollment | | |
|---|---|---|---|
|  | sCr or UO | sCr only | UO only |
| Spec 2 | 23% | nd | 24% |
| Cutoff 3 | 1110 | nd | 1110 |
| Sens 3 | 90% | nd | 90% |
| Spec 3 | 21% | nd | 22% |
| Cutoff 4 | 1710 | nd | 1710 |
| Sens 4 | 40% | nd | 40% |
| Spec 4 | 70% | nd | 71% |
| Cutoff 5 | 1950 | nd | 1840 |
| Sens 5 | 10% | nd | 20% |
| Spec 5 | 81% | nd | 80% |
| Cutoff 6 | 2240 | nd | 2150 |
| Sens 6 | 0% | nd | 0% |
| Spec 6 | 91% | nd | 90% |
| OR Quart 2 | 0.28 | nd | 0.91 |
| p Value | 0.30 | nd | 0.93 |
| 95% CI of | 0.026 | nd | 0.11 |
| OR Quart2 | 3.1 | nd | 7.7 |
| OR Quart 3 | 1.0 | nd | 1.5 |
| p Value | 1.0 | nd | 0.69 |
| 95% CI of | 0.16 | nd | 0.20 |
| OR Quart3 | 6.1 | nd | 11 |
| OR Quart 4 | 0.92 | nd | 1.5 |
| p Value | 0.92 | nd | 0.69 |
| 95% CI of | 0.15 | nd | 0.20 |
| OR Quart4 | 5.5 | nd | 11 |

**Coagulation factor XIII A and B chains**

|  | sCr or UO | | sCr only | | UO only | |
|---|---|---|---|---|---|---|
|  | Cohort 1 | Cohort 2 | Cohort 1 | Cohort 2 | Cohort 1 | Cohort 2 |
| Median | 11700 | 10000 | nd | nd | 11200 | 10000 |
| Average | 12400 | 11400 | nd | nd | 11800 | 11400 |
| Stdev | 6890 | 5310 | nd | nd | 6580 | 5310 |
| p(t-test) |  | 0.67 | nd | nd |  | 0.84 |
| Min | 881 | 3550 | nd | nd | 881 | 3550 |
| Max | 33300 | 21300 | nd | nd | 33300 | 21300 |
| n (Samp) | 47 | 10 | nd | nd | 41 | 10 |
| n (Patient) | 47 | 10 | nd | nd | 41 | 10 |

|  | At Enrollment | | |
|---|---|---|---|
|  | sCr or UO | sCr only | UO only |
| AUC | 0.46 | nd | 0.49 |
| SE | 0.10 | nd | 0.10 |
| p | 0.69 | nd | 0.89 |
| nCohort 1 | 47 | nd | 41 |
| nCohort 2 | 10 | nd | 10 |
| Cutoff 1 | 9230 | nd | 9230 |
| Sens 1 | 70% | nd | 70% |

|  | At Enrollment |  |  |
|--|--|--|--|
|  | sCr or UO | sCr only | UO only |
| Spec 1 | 38% | nd | 41% |
| Cutoff 2 | 7990 | nd | 7990 |
| Sens 2 | 80% | nd | 80% |
| Spec 2 | 28% | nd | 29% |
| Cutoff 3 | 7150 | nd | 7150 |
| Sens 3 | 90% | nd | 90% |
| Spec 3 | 23% | nd | 24% |
| Cutoff 4 | 14000 | nd | 13500 |
| Sens 4 | 20% | nd | 20% |
| Spec 4 | 70% | nd | 71% |
| Cutoff 5 | 16500 | nd | 16200 |
| Sens 5 | 20% | nd | 20% |
| Spec 5 | 81% | nd | 80% |
| Cutoff 6 | 22500 | nd | 19800 |
| Sens 6 | 0% | nd | 10% |
| Spec 6 | 91% | nd | 90% |
| OR Quart 2 | 0.50 | nd | 1.0 |
| p Value | 0.59 | nd | 1.0 |
| 95% CI of | 0.040 | nd | 0.12 |
| OR Quart2 | 6.2 | nd | 8.4 |
| OR Quart 3 | 3.6 | nd | 2.4 |
| p Value | 0.17 | nd | 0.36 |
| 95% CI of | 0.57 | nd | 0.36 |
| OR Quart3 | 23 | nd | 17 |
| OR Quart 4 | 1.1 | nd | 1.1 |
| p Value | 0.94 | nd | 0.93 |
| 95% CI of | 0.13 | nd | 0.13 |
| OR Quart4 | 8.9 | nd | 9.3 |

**Vitronectin**

|  | sCr or UO |  | sCr only |  | UO only |  |
|--|--|--|--|--|--|--|
|  | Cohort 1 | Cohort 2 | Cohort 1 | Cohort 2 | Cohort 1 | Cohort 2 |
| Median | 113000 | 87600 | nd | nd | 112000 | 87600 |
| Average | 114000 | 83300 | nd | nd | 112000 | 83300 |
| Stdev | 31400 | 39800 | nd | nd | 31600 | 39800 |
| p(t-test) |  | 0.011 | nd | nd |  | 0.018 |
| Min | 57400 | 21800 | nd | nd | 57400 | 21800 |
| Max | 177000 | 153000 | nd | nd | 177000 | 153000 |
| n (Samp) | 47 | 10 | nd | nd | 41 | 10 |
| n (Patient) | 47 | 10 | nd | nd | 41 | 10 |

|  | At Enrollment |  |  |
|--|--|--|--|
|  | sCr or UO | sCr only | UO only |
| AUC | 0.27 | nd | 0.29 |
| SE | 0.097 | nd | 0.099 |
| p | 0.020 | nd | 0.030 |
| nCohort 1 | 47 | nd | 41 |

| | At Enrollment | | |
|---|---|---|---|
| | sCr or UO | sCr only | UO only |
| nCohort 2 | 10 | nd | 10 |
| Cutoff 1 | 60700 | nd | 60700 |
| Sens 1 | 70% | nd | 70% |
| Spec 1 | 4% | nd | 5% |
| Cutoff 2 | 60500 | nd | 60500 |
| Sens 2 | 80% | nd | 80% |
| Spec 2 | 4% | nd | 5% |
| Cutoff 3 | 21800 | nd | 21800 |
| Sens 3 | 90% | nd | 90% |
| Spec 3 | 0% | nd | 0% |
| Cutoff 4 | 125000 | nd | 123000 |
| Sens 4 | 10% | nd | 10% |
| Spec 4 | 70% | nd | 71% |
| Cutoff 5 | 145000 | nd | 143000 |
| Sens 5 | 10% | nd | 10% |
| Spec 5 | 81% | nd | 80% |
| Cutoff 6 | 158000 | nd | 154000 |
| Sens 6 | 0% | nd | 0% |
| Spec 6 | 91% | nd | 90% |
| OR Quart 2 | 2.3 | nd | 2.2 |
| p Value | 0.51 | nd | 0.55 |
| 95% CI of | 0.19 | nd | 0.17 |
| OR Quart2 | 29 | nd | 28 |
| OR Quart 3 | 2.3 | nd | 3.6 |
| p Value | 0.51 | nd | 0.30 |
| 95% CI of | 0.19 | nd | 0.32 |
| OR Quart3 | 29 | nd | 40 |
| OR Quart 4 | 7.8 | nd | 6.0 |
| p Value | 0.081 | nd | 0.14 |
| 95% CI of | 0.78 | nd | 0.56 |
| OR Quart4 | 78 | nd | 64 |

**T3**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 0.979 | 0.578 | nd | nd | 0.946 | 0.578 |
| Average | 1.03 | 0.674 | nd | nd | 1.01 | 0.674 |
| Stdev | 0.631 | 0.531 | nd | nd | 0.650 | 0.531 |
| p(t-test) | | 0.11 | nd | nd | | 0.14 |
| Min | 0.000227 | 0.000162 | nd | nd | 0.000227 | 0.000162 |
| Max | 4.25 | 1.59 | nd | nd | 4.25 | 1.59 |
| n (Samp) | 47 | 10 | nd | nd | 41 | 10 |
| n (Patient) | 47 | 10 | nd | nd | 41 | 10 |

| | At Enrollment | | |
|---|---|---|---|
| | sCr or UO | sCr only | UO only |
| AUC | 0.30 | nd | 0.30 |

| | At Enrollment | | |
|---|---|---|---|
| | sCr or UO | sCr only | UO only |
| SE | 0.099 | nd | 0.10 |
| p | 0.039 | nd | 0.046 |
| nCohort 1 | 47 | nd | 41 |
| nCohort 2 | 10 | nd | 10 |
| Cutoff 1 | 0.500 | nd | 0.500 |
| Sens 1 | 70% | nd | 70% |
| Spec 1 | 15% | nd | 15% |
| Cutoff 2 | 0.424 | nd | 0.424 |
| Sens 2 | 80% | nd | 80% |
| Spec 2 | 11% | nd | 10% |
| Cutoff 3 | 0 | nd | 0 |
| Sens 3 | 100% | nd | 100% |
| Spec 3 | 0% | nd | 0% |
| Cutoff 4 | 1.18 | nd | 1.17 |
| Sens 4 | 20% | nd | 20% |
| Spec 4 | 70% | nd | 71% |
| Cutoff 5 | 1.32 | nd | 1.27 |
| Sens 5 | 20% | nd | 20% |
| Spec 5 | 81% | nd | 80% |
| Cutoff 6 | 1.60 | nd | 1.54 |
| Sens 6 | 0% | nd | 10% |
| Spec 6 | 91% | nd | 90% |
| OR Quart 2 | 0.50 | nd | 0.46 |
| p Value | 0.59 | nd | 0.55 |
| 95% CI of | 0.040 | nd | 0.036 |
| OR Quart2 | 6.2 | nd | 5.8 |
| OR Quart 3 | 0 | nd | 0.46 |
| p Value | na | nd | 0.55 |
| 95% CI of | na | nd | 0.036 |
| OR Quart3 | na | nd | 5.8 |
| OR Quart 4 | 6.5 | nd | 5.5 |
| p Value | 0.044 | nd | 0.076 |
| 95% CI of | 1.1 | nd | 0.84 |
| OR Quart4 | 40 | nd | 36 |

T4

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 2.39 | 0.996 | nd | nd | 2.35 | 0.996 |
| Average | 2.84 | 1.46 | nd | nd | 2.68 | 1.46 |
| Stdev | 2.12 | 1.62 | nd | nd | 1.92 | 1.62 |
| p(t-test) | | 0.059 | nd | nd | | 0.070 |
| Min | 0.0578 | 0.000136 | nd | nd | 0.0578 | 0.000136 |
| Max | 8.62 | 4.15 | nd | nd | 7.82 | 4.15 |
| n (Samp) | 47 | 10 | nd | nd | 41 | 10 |
| n (Patient) | 47 | 10 | nd | nd | 41 | 10 |

| | At Enrollment | | |
|---|---|---|---|
| | sCr or UO | sCr only | UO only |
| AUC | 0.29 | nd | 0.30 |
| SE | 0.099 | nd | 0.10 |
| p | 0.034 | nd | 0.052 |
| nCohort 1 | 47 | nd | 41 |
| nCohort 2 | 10 | nd | 10 |
| Cutoff 1 | 0.196 | nd | 0.196 |
| Sens 1 | 70% | nd | 70% |
| Spec 1 | 6% | nd | 7% |
| Cutoff 2 | 0.000136 | nd | 0.000136 |
| Sens 2 | 90% | nd | 90% |
| Spec 2 | 0% | nd | 0% |
| Cutoff 3 | 0.000136 | nd | 0.000136 |
| Sens 3 | 90% | nd | 90% |
| Spec 3 | 0% | nd | 0% |
| Cutoff 4 | 3.39 | nd | 3.32 |
| Sens 4 | 20% | nd | 20% |
| Spec 4 | 70% | nd | 71% |
| Cutoff 5 | 4.35 | nd | 3.97 |
| Sens 5 | 0% | nd | 10% |
| Spec 5 | 81% | nd | 80% |
| Cutoff 6 | 6.06 | nd | 5.44 |
| Sens 6 | 0% | nd | 0% |
| Spec 6 | 91% | nd | 90% |
| OR Quart 2 | 0.50 | nd | 0.46 |
| p Value | 0.59 | nd | 0.55 |
| 95% CI of | 0.040 | nd | 0.036 |
| OR Quart2 | 6.2 | nd | 5.8 |
| OR Quart 3 | 1.1 | nd | 1.0 |
| p Value | 0.94 | nd | 1.0 |
| 95% CI of | 0.13 | nd | 0.12 |
| OR Quart3 | 8.9 | nd | 8.4 |
| OR Quart 4 | 3.6 | nd | 3.9 |
| p Value | 0.17 | nd | 0.16 |
| 95% CI of | 0.57 | nd | 0.59 |
| OR Quart4 | 23 | nd | 26 |

**Proprotein convertase subtilisin/kexin type 9**

| | sCr or UO | | sCr only | | UO only | |
|---|---|---|---|---|---|---|
| | Cohort 1 | Cohort 2 | Cohort 1 | Cohort 2 | Cohort 1 | Cohort 2 |
| Median | 443000 | 261000 | nd | nd | 443000 | 261000 |
| Average | 478000 | 265000 | nd | nd | 481000 | 265000 |
| Stdev | 164000 | 159000 | nd | nd | 171000 | 159000 |
| p(t-test) | | 4.5E-4 | nd | nd | | 6.7E-4 |
| Min | 182000 | 80700 | nd | nd | 182000 | 80700 |
| Max | 858000 | 596000 | nd | nd | 858000 | 596000 |
| n (Samp) | 47 | 10 | nd | nd | 41 | 10 |
| n (Patient) | 47 | 10 | nd | nd | 41 | 10 |

| | At Enrollment | | |
|---|---|---|---|
| | sCr or UO | sCr only | UO only |
| AUC | 0.17 | nd | 0.17 |
| SE | 0.083 | nd | 0.084 |
| p | 5.3E-5 | nd | 7.2E-5 |
| nCohort 1 | 47 | nd | 41 |
| nCohort 2 | 10 | nd | 10 |
| Cutoff 1 | 123000 | nd | 123000 |
| Sens 1 | 70% | nd | 70% |
| Spec 1 | 0% | nd | 0% |
| Cutoff 2 | 102000 | nd | 102000 |
| Sens 2 | 80% | nd | 80% |
| Spec 2 | 0% | nd | 0% |
| Cutoff 3 | 80700 | nd | 80700 |
| Sens 3 | 90% | nd | 90% |
| Spec 3 | 0% | nd | 0% |
| Cutoff 4 | 538000 | nd | 580000 |
| Sens 4 | 10% | nd | 10% |
| Spec 4 | 70% | nd | 71% |
| Cutoff 5 | 654000 | nd | 667000 |
| Sens 5 | 0% | nd | 0% |
| Spec 5 | 81% | nd | 80% |
| Cutoff 6 | 699000 | nd | 699000 |
| Sens 6 | 0% | nd | 0% |
| Spec 6 | 91% | nd | 90% |
| OR Quart 2 | 0 | nd | 0 |
| p Value | na | nd | na |
| 95% CI of | na | nd | na |
| OR Quart2 | na | nd | na |
| OR Quart 3 | 2.3 | nd | 2.2 |
| p Value | 0.51 | nd | 0.55 |
| 95% CI of | 0.19 | nd | 0.17 |
| OR Quart3 | 29 | nd | 28 |
| OR Quart 4 | 14 | nd | 17 |
| p Value | 0.023 | nd | 0.018 |
| 95% CI of | 1.4 | nd | 1.6 |
| OR Quart4 | 140 | nd | 170 |

While the invention has been described and exemplified in sufficient detail for those skilled in this art to make and use it, various alternatives, modifications, and improvements should be apparent without departing from the spirit and scope of the invention. The examples provided herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

It will be readily apparent to a person skilled in the art that varying substitutions and modifications may be made to the invention disclosed herein without departing from the scope and spirit of the invention.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms.

The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

Other embodiments are set forth within the following claims.

The present invention is further characterized by the following items:

1. A method for evaluating renal status in a subject, comprising:

performing one or more assays configured to detect one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 on a body fluid sample obtained from the subject to provide an assay result; and

correlating the assay result(s) to the renal status of the subject, wherein said correlation step comprises correlating the assay result(s) to one or more of diagnosis, risk stratification, prognosis, classifying and monitoring of the renal status of the subject.

2. A method according to item 1, wherein said correlation step comprises correlating the assay result(s) to prognosis of the renal status of the subject.

3. A method according to item 1, wherein said correlating step comprises assigning a likelihood of one or more future changes in renal status to the subject based on the assay result(s).

4. A method according to item 3, wherein said one or more future changes in renal status comprise one or more of a future injury to renal function, future reduced renal function, future improvement in renal function, and future acute renal failure (ARF).

5. A method according to one of items 1-4, wherein said assay results comprise at least 2, 3, 4, or 5 of:

a measured concentration of Stanniocalcin-1, a measured concentration of Antithrombin-III, a measured concentration of Toll-like receptor 2, a measured concentration of Triiodothyronine (T3), a measured concentration of Thyroxine (T4), a measured concentration of Extracellular matrix protein 1, a measured concentration of Coagulation factor XIII A chain, a measured concentration of Coagulation factor XIII B chain, a measured concentration of Interleukin-17F, a measured concentration of Interleukin-22, a measured concentration of Vitronectin, a measured concentration of Progesterone, Estradiol, Growth/differentiation factor 15, and a measured concentration of Proprotein convertase subtilisin/kexin type 9.

6. A method according to one of items 1-5, wherein a plurality of assay results are combined using a function that converts the plurality of assay results into a single composite result.

7. A method according to item 3, wherein said one or more future changes in renal status comprise a clinical outcome related to a renal injury suffered by the subject.

8. A method according to item 3, wherein the likelihood of one or more future changes in renal status is that an event of interest is more or less likely to occur within 30 days of the time at which the body fluid sample is obtained from the subject.

9. A method according to item 8, wherein the likelihood of one or more future changes in renal status is that an event of interest is more or less likely to occur within a period selected from the group consisting of 21 days, 14 days, 7 days, 5 days, 96 hours, 72 hours, 48 hours, 36 hours, 24 hours, and 12 hours.

10. A method according to one of items 1-5, wherein the subject is selected for evaluation of renal status based on the pre-existence in the subject of one or more known risk factors for prerenal, intrinsic renal, or postrenal ARF.

11. A method according to one of items 1-5, wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

12. A method according to one of items 1-5, wherein said correlating step comprises assessing whether or not renal function is improving or worsening in a subject who has suffered from an injury to renal function, reduced renal function, or ARF based on the assay result(s).

13. A method according to one of items 1-5, wherein said method is a method of assigning a risk of the future occurrence or nonoccurrence of an injury to renal function in said subject.

14. A method according to one of items 1-5, wherein said method is a method of assigning a risk of the future occurrence or nonoccurrence of reduced renal function in said subject.

15. A method according to one of items 1-5, wherein said method is a method of assigning a risk of the future occurrence or nonoccurrence of a need for dialysis in said subject.

16. A method according to one of items 1-5, wherein said method is a method of assigning a risk of the future occurrence or nonoccurrence of acute renal failure in said subject.

17. A method according to one of items 1-5, wherein said method is a method of assigning a risk of the future occurrence or nonoccurrence of a need for renal replacement therapy in said subject.

18. A method according to one of items 1-5, wherein said method is a method of assigning a risk of the future occurrence or nonoccurrence of a need for renal transplantation in said subject.

19. A method according to one of items 1-5, wherein said one or more future changes in renal status comprise one or more of a future injury to renal function, future reduced renal function, future improvement in renal function, and future acute renal failure (ARF) within 72 hours of the time at which the body fluid sample is obtained.

20. A method according to one of items 1-5, wherein said one or more future changes in renal status comprise one or more of a future injury to renal function, future reduced renal function, future improvement in renal function, and future acute renal failure (ARF) within 48 hours of the time at which the body fluid sample is obtained.

21. A method according to one of items 1-5, wherein said one or more future changes in renal status comprise one or more of a future injury to renal function, future reduced renal function, future improvement in renal function, and future acute renal failure (ARF) within 24 hours of the time at which the body fluid sample is obtained.

22. A method according to one of items 1-5, wherein the subject is in RIFLE stage 0 or R.

23. A method according to item 22, wherein the subject is in RIFLE stage 0, and said correlating step comprises assigning a likelihood that the subject will reach RIFLE stage R, I or F within 72 hours.

24. A method according to item 23, wherein the subject is in RIFLE stage 0, and said correlating step comprises assigning a likelihood that the subject will reach RIFLE stage I or F within 72 hours.

25. A method according to item 23, wherein the subject is in RIFLE stage 0, and said correlating step comprises assigning a likelihood that the subject will reach RIFLE stage F within 72 hours.

26. A method according to item 22, wherein the subject is in RIFLE stage 0 or R, and said correlating step comprises assigning a likelihood that the subject will reach RIFLE stage I or F within 72 hours.

27. A method according to item 26, wherein the subject is in RIFLE stage 0 or R, and said correlating step comprises assigning a likelihood that the subject will reach RIFLE stage F within 72 hours.

28. A method according to item 22, wherein the subject is in RIFLE stage R, and said correlating step comprises assigning a likelihood that the subject will reach RIFLE stage I or F within 72 hours.

29. A method according to item 28, wherein the subject is in RIFLE stage R, and said correlating step comprises assigning a likelihood that the subject will reach RIFLE stage F within 72 hours.

30. A method according to one of items 1-5, wherein the subject is in RIFLE stage 0, R, or I, and said correlating step comprises assigning a likelihood that the subject will reach RIFLE stage F within 72 hours.

31. A method according to item 30, wherein the subject is in RIFLE stage I, and said correlating step comprises assigning a likelihood that the subject will reach RIFLE stage F within 72 hours.

32. A method according to item 23, wherein said correlating step comprises assigning a likelihood that the subject will reach RIFLE stage R, I or F within 48 hours.

33. A method according to item 24, wherein said correlating step comprises assigning a likelihood that the subject will reach RIFLE stage I or F within 48 hours.

34. A method according to item 25, wherein said correlating step comprises assigning a likelihood that the subject will reach RIFLE stage F within 48 hours.

35. A method according to item 26, wherein said correlating step comprises assigning a likelihood that the subject will reach RIFLE stage I or F within 48 hours.

36. A method according to item 27, wherein said correlating step comprises assigning a likelihood that the subject will reach RIFLE stage F within 48 hours.

37. A method according to item 28, wherein said correlating step comprises assigning a likelihood that the subject will reach RIFLE stage I or F within 48 hours.

38. A method according to item 29, wherein said correlating step comprises assigning a likelihood that the subject will reach RIFLE stage F within 48 hours.

39. A method according to item 30, wherein said correlating step comprises assigning a likelihood that the subject will reach RIFLE stage F within 48 hours.

40. A method according to item 31, wherein said correlating step comprises assigning a likelihood that the subject will reach RIFLE stage F within 48 hours.

41. A method according to item 23, wherein said correlating step comprises assigning a likelihood that the subject will reach RIFLE stage R, I or F within 24 hours.

42. A method according to item 24, wherein said correlating step comprises assigning a likelihood that the subject will reach RIFLE stage I or F within 24 hours.

43. A method according to item 25, wherein said correlating step comprises assigning a likelihood that the subject will reach RIFLE stage F within 24 hours.

44. A method according to item 26, wherein said correlating step comprises assigning a likelihood that the subject will reach RIFLE stage I or F within 24 hours.

45. A method according to item 27, wherein said correlating step comprises assigning a likelihood that the subject will reach RIFLE stage F within 24 hours.

46. A method according to item 28, wherein said correlating step comprises assigning a likelihood that the subject will reach RIFLE stage I or F within 24 hours.

47. A method according to item 29, wherein said correlating step comprises assigning a likelihood that the subject will reach RIFLE stage F within 24 hours.

48. A method according to item 30, wherein said correlating step comprises assigning a likelihood that the subject will reach RIFLE stage F within 24 hours.

49. A method according to item 31, wherein said correlating step comprises assigning a likelihood that the subject will reach RIFLE stage F within 24 hours.

50. A method according to one of items 1-5, wherein the subject is not in acute renal failure.

51. A method according to one of items 1-5, wherein the subject has not experienced a 1.5-fold or greater increase in serum creatinine over a baseline value determined prior to the time at which the body fluid sample is obtained.

52. A method according to one of items 1-5, wherein the subject has a urine output of at least 0.5 ml/kg/hr over the 6 hours preceding the time at which the body fluid sample is obtained.

53. A method according to one of items 1-5, wherein the subject has not experienced an increase of 0.3 mg/dL or greater in serum creatinine over a baseline value determined prior to the time at which the body fluid sample is obtained.

54. A method according to one of items 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained.

55. A method according to one of items 1-5, wherein the subject has not experienced a 1.5-fold or greater increase in serum creatinine over a baseline value determined prior to the time at which the body fluid sample is obtained.

56. A method according to one of items 1-5, wherein the subject has a urine output of at least 0.5 ml/kg/hr over the 6 hours preceding the time at which the body fluid sample is obtained.

57. A method according to one of items 1-5, wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained.

58. A method according to one of items 1-5, wherein said correlating step comprises assigning one or more of: a likelihood that within 72 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

59. A method according to item 58, wherein said correlating step comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

60. A method according to item 58, wherein said correlating step comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine.

61. A method according to item 58, wherein said correlating step comprises assigning a likelihood that within 72 hours the subject will experience a 1.5-fold or greater increase in serum creatinine.

62. A method according to item 58, wherein said correlating step comprises assigning a likelihood that within 72 hours the subject will have a urine output of less than 0.5 ml/kg/hr over a 6 hour period.

63. A method according to item 58, wherein said correlating step comprises assigning a likelihood that within 72 hours the subject will experience an increase of 0.3 mg/dL or greater in serum creatinine.

64. A method according to item 58, wherein said correlating step comprises assigning a likelihood that within 48 hours the subject will experience a 1.5-fold or greater increase in serum creatinine.

65. A method according to item 58, wherein said correlating step comprises assigning a likelihood that within 48 hours the subject will have a urine output of less than 0.5 ml/kg/hr over a 6 hour period.

66. A method according to item 58, wherein said correlating step comprises assigning a likelihood that within 48 hours the subject will experience an increase of 0.3 mg/dL or greater in serum creatinine.

67. A method according to item 58, wherein said correlating step comprises assigning a likelihood that within 24 hours the subject will experience a 1.5-fold or greater increase in serum creatinine.

68. A method according to item 58, wherein said correlating step comprises assigning a likelihood that within 24 hours the subject will have a urine output of less than 0.5 ml/kg/hr over a 6 hour period.

69. A method according to item 58, wherein said correlating step comprises assigning a likelihood that within 24 hours the subject will experience an increase of 0.3 mg/dL or greater in serum creatinine.

70. A method according to one of items 1-5, wherein the subject has not experienced a 2-fold or greater increase in serum creatinine over a baseline value determined prior to the time at which the body fluid sample is obtained.

71. A method according to one of items 1-5, wherein the subject has a urine output of at least 0.5 ml/kg/hr over the 12 hours preceding the time at which the body fluid sample is obtained.

72. A method according to one of items 1-5, wherein the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained.

73. A method according to one of items 1-5, wherein the subject has not experienced a 3-fold or greater increase in serum creatinine over a baseline value determined prior to the time at which the body fluid sample is obtained.

74. A method according to one of items 1-5, wherein the subject has a urine output of at least 0.3 ml/kg/hr over the 24 hours preceding the time at which the body fluid sample is obtained, or anuria over the 12 hours preceding the time at which the body fluid sample is obtained.

75. A method according to one of items 1-5, wherein the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained.

76. A method according to one of items 1-5, wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine.

77. A method according to item 76, wherein said correlating step comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine.

78. A method according to item 76, wherein said correlating step comprises assigning one or more of: a likelihood that within 24 hours the subject will (i) experience a 2-fold or greater increase in serum creatinine, or (ii) have a urine output of less than 0.5 ml/kg/hr over a 6 hour period.

79. A method according to item 76, wherein said correlating step comprises assigning a likelihood that within 72 hours the subject will experience a 2-fold or greater increase in serum creatinine.

80. A method according to item 76, wherein said correlating step comprises assigning a likelihood that within 72 hours the subject will have a urine output of less than 0.5 ml/kg/hr over a 6 hour period.

81. A method according to item 76, wherein said correlating step comprises assigning a likelihood that within 48 hours the subject will experience a 2-fold or greater increase in serum creatinine.

82. A method according to item 76, wherein said correlating step comprises assigning a likelihood that within 48 hours the subject will have a urine output of less than 0.5 ml/kg/hr over a 6 hour period.

83. A method according to item 76, wherein said correlating step comprises assigning a likelihood that within 24 hours the subject will experience a 2-fold or greater increase in serum creatinine.

84. A method according to item 76, wherein said correlating step comprises assigning a likelihood that within 24 hours the subject will have a urine output of less than 0.5 ml/kg/hr over a 6 hour period.

85. A method according to one of items 1-5, wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period.

86. A method according to item 85, wherein said correlating step comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period.

87. A method according to item 85, wherein said correlating step comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period.

88. A method according to item 85, wherein said correlating step comprises assigning a likelihood that within 72 hours the subject will experience a 3-fold or greater increase in serum creatinine.

89. A method according to item 85, wherein said correlating step comprises assigning a likelihood that within 72 hours the subject will have a urine output of less than 0.3 ml/kg/hr over a 24 hour period or anuria over a 12 hour period.

90. A method according to item 85, wherein said correlating step comprises assigning a likelihood that within 48 hours the subject will experience a 3-fold or greater increase in serum creatinine.

91. A method according to item 85, wherein said correlating step comprises assigning a likelihood that within 48 hours the subject will have a urine output of less than 0.3 ml/kg/hr over a 24 hour period or anuria over a 12 hour period.

92. A method according to item 85, wherein said correlating step comprises assigning a likelihood that within 24 hours the subject will experience a 3-fold or greater increase in serum creatinine.

93. A method according to item 85, wherein said correlating step comprises assigning a likelihood that within 24 hours the subject will have a urine output of less than 0.3 ml/kg/hr over a 24 hour period or anuria over a 12 hour period.

94. A method according to one of items 1-98, wherein the body fluid sample is a urine sample.

95. A method according to one of items 1-94, wherein said method comprises performing assays that detect one, two or three, or more of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9.

96. Measurement of one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 for the risk stratification, prognosis, classifying and/or monitoring of renal injury.

97. Measurement of one or more biomarkers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 for the risk stratification, prognosis, classifying and/or monitoring of acute renal injury.

98. A kit, comprising:

reagents for performing one or more assays configured to detect one or more kidney injury markers selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9.

99. A kit according to item 98, wherein said reagents comprise one or more binding reagents, each of which specifically binds one of said of kidney injury markers.

100. A kit according to item 99, wherein a plurality of binding reagents are contained in a single assay device.

101. A kit according to item 99, wherein at least one of said assays is configured as a sandwich binding assay.

102. A kit according to item 99, wherein at least one of said assays is configured as a competitive binding assay.

103. A kit according to one of items 98-102, wherein said one or more assays comprise assays that detect one, two or three, or more of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9.

104. A method for evaluating biomarker levels in a body fluid sample, comprising:

obtaining a urine sample from a subject selected for evaluation based on a determination that the subject is at risk of a future or current acute renal injury; and
performing a plurality of analyte binding assays configured to detect a plurality of biomarkers, one or more of which is selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9 by introducing the urine sample obtained from the subject into an assay instrument which (i) contacts a plurality of reagents which specifically bind for detection the plurality of biomarkers with the urine sample, and (ii) generates one or more assay results indicative of binding of each biomarker which is assayed to a respective specific binding reagent in the plurality of reagents, wherein the subject is selected for evaluation based on a determination that the subject is in need of diagnosis, risk stratification, staging, prognosis, classifying or monitoring of the renal status of the subject.

105. A method according to item 104, wherein the subject is selected for evaluation based on a determination that the subject is at risk of a future acute renal injury.

106. A method according to item 105, wherein the subject is selected for evaluation based on a determination that the subject is at risk of a future injury to renal function, future reduced renal function, future improvement in renal function, and future acute renal failure (ARF).

107. A method according to item 105, wherein the subject is selected for evaluation based on a determination that

# EP 3 282 257 A1

the subject is at risk of a future acute renal injury within 30 days of the time at which the urine sample is obtained from the subject.

108. A method according to item 107, wherein the subject is selected for evaluation based on a determination that the subject is at risk of a future acute renal injury within a period selected from the group consisting of 21 days, 14 days, 7 days, 5 days, 96 hours, 72 hours, 48 hours, 36 hours, 24 hours, and 12 hours.

109. A method according to item 104, wherein the subject is selected based on the pre-existence in the subject of one or more known risk factors for prerenal, intrinsic renal, or postrenal ARF.

110. A method according to item 104, wherein the subject is selected for evaluation based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin.

111. A method according to item 104, wherein the plurality of assays are immunoassays performed by (i) introducing the urine sample into an assay device comprising a plurality of antibodies, at least one of which binds to each biomarker which is assayed, and (ii) generating an assay result indicative of binding of each biomarker to its respective antibody.

112. A method according to item 104, wherein the subject is selected for evaluation based on a determination that the subject is at risk of one or more future changes in renal status selected from the group consisting of a future injury to renal function, future reduced renal function, future improvement in renal function, and future acute renal failure (ARF) within 72 hours of the time at which the urine sample is obtained.

113. A method according to item 104, wherein the subject is selected for evaluation based on a determination that the subject is at risk of one or more future changes in renal status selected from the group consisting of a future injury to renal function, future reduced renal function, future improvement in renal function, and future acute renal failure (ARF) within 48 hours of the time at which the urine sample is obtained.

114. A method according to item 104, wherein the subject is selected for evaluation based on a determination that the subject is at risk of one or more future changes in renal status selected from the group consisting of a future injury to renal function, future reduced renal function, future improvement in renal function, and future acute renal failure (ARF) within 24 hours of the time at which the urine sample is obtained.

115. A method according to item 104, wherein the subject is in RIFLE stage 0 or R.

116. A method according to item 104, wherein the subject is in RIFLE stage 0, R, or I.

117. A method according to item 104, wherein at least one assay result is a measured concentration of Stanniocalcin-1, a measured concentration of Antithrombin-III, a measured concentration of Toll-like receptor 2, a measured concentration of Triiodothyronine (T3), a measured concentration of Thyroxine (T4), a measured concentration of Extracellular matrix protein 1, a measured concentration of Coagulation factor XIII A chain, a measured concentration of Coagulation factor XIII B chain, a measured concentration of Interleukin-17F, a measured concentration of Interleukin-22, a measured concentration of Vitronectin, a measured concentration of Progesterone, Estradiol, Growth/differentiation factor 15, and a measured concentration of Proprotein convertase subtilisin/kexin type 9.

118. A system for evaluating biomarker levels, comprising:

a plurality of reagents which specifically bind for detection the plurality of biomarkers, one or more of which is selected from the group consisting of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, Growth/differentiation factor 15, and Proprotein convertase subtilisin/kexin type 9;

179

an assay instrument configured to receive a urine sample and contact the plurality of reagents with the urine sample and to generate one or more assay results indicative of binding of each biomarker which is assayed to a respective specific binding reagent in the plurality of reagents.

119. A system according to item 118, wherein the reagents comprise a plurality of antibodies, at least one of which binds to each of the biomarkers which are assayed.

120. A system according to item 119, wherein assay instrument comprises an assay device and an assay device reader, wherein the plurality of antibodies are immobilized at a plurality of predetermined locations within the assay device, wherein the assay device is configured to receive the urine sample such that the urine sample contacts the plurality of predetermined locations, and wherein the assay device reader interrogates the plurality of predetermined locations to generate the assay results.

**Claims**

1. A method for evaluating renal status in a subject, comprising:

    performing one or more assays configured to detect Growth/differentiation factor 15, and optionally further one or more biomarkers of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, and Proprotein convertase subtilisin/kexin type 9 on a body fluid sample obtained from the subject to provide an assay result; and
    correlating the assay result(s) to the renal status of the subject, wherein said correlation step comprises correlating the assay result(s) to one or more of diagnosis, risk stratification, prognosis, classifying and monitoring of the renal status of the subject.

2. A method according to claim 1, wherein said correlation step comprises correlating the assay result(s) to prognosis of the renal status of the subject.

3. A method according to claim 1, wherein said correlating step comprises assigning a likelihood of one or more future changes in renal status to the subject based on the assay result(s).

4. A method according to claim 3, wherein said one or more future changes in renal status comprise one or more of a future injury to renal function, future reduced renal function, future improvement in renal function, and future acute renal failure (ARF).

5. A method according to one of claims 1-4, wherein said assay results comprise a measured concentration of Growth/differentiation factor 15, and optionally further at least 1, 2, 3, 4, or 5 of:

    a measured concentration of Stanniocalcin-1, a measured concentration of Antithrombin-III, a measured concentration of Toll-like receptor 2, a measured concentration of Triiodothyronine (T3), a measured concentration of Thyroxine (T4), a measured concentration of Extracellular matrix protein 1, a measured concentration of Coagulation factor XIII A chain, a measured concentration of Coagulation factor XIII B chain, a measured concentration of Interleukin-17F, a measured concentration of Interleukin-22, a measured concentration of Vitronectin, a measured concentration of Progesterone, Estradiol, and a measured concentration of Proprotein convertase subtilisin/kexin type 9.

6. A method according to one of claims 1-5, wherein a plurality of assay results are combined using a function that converts the plurality of assay results into a single composite result.

7. A method according to claim 3, wherein said one or more future changes in renal status comprise a clinical outcome related to a renal injury suffered by the subject, or wherein the likelihood of one or more future changes in renal status is that an event of interest is more or less likely to occur within 30 days of the time at which the body fluid sample is obtained from the subject,
wherein the likelihood of one or more future changes in renal status preferably is that an event of interest is more or less likely to occur within a period selected from the group consisting of 21 days, 14 days, 7 days, 5 days, 96 hours, 72 hours, 48 hours, 36 hours, 24 hours, and 12 hours.

8. A method according to one of claims 1-5, wherein the subject is selected for evaluation of renal status based on the pre-existence in the subject of one or more known risk factors for prerenal, intrinsic renal, or postrenal ARF, or wherein the subject is selected for evaluation of renal status based on an existing diagnosis of one or more of congestive heart failure, preeclampsia, eclampsia, diabetes mellitus, hypertension, coronary artery disease, proteinuria, renal insufficiency, glomerular filtration below the normal range, cirrhosis, serum creatinine above the normal range, sepsis, injury to renal function, reduced renal function, or ARF, or based on undergoing or having undergone major vascular surgery, coronary artery bypass, or other cardiac surgery, or based on exposure to NSAIDs, cyclosporines, tacrolimus, aminoglycosides, foscarnet, ethylene glycol, hemoglobin, myoglobin, ifosfamide, heavy metals, methotrexate, radiopaque contrast agents, or streptozotocin, or
wherein said correlating step comprises assessing whether or not renal function is improving or worsening in a subject who has suffered from an injury to renal function, reduced renal function, or ARF based on the assay result(s), or
wherein said method is a method of assigning a risk of the future occurrence or nonoccurrence of an injury to renal function in said subject, or
wherein said method is a method of assigning a risk of the future occurrence or nonoccurrence of reduced renal function in said subject, or
wherein said method is a method of assigning a risk of the future occurrence or nonoccurrence of a need for dialysis in said subject, or
wherein said method is a method of assigning a risk of the future occurrence or nonoccurrence of acute renal failure in said subject, or
wherein said method is a method of assigning a risk of the future occurrence or nonoccurrence of a need for renal replacement therapy in said subject, or
wherein said method is a method of assigning a risk of the future occurrence or nonoccurrence of a need for renal transplantation in said subject, or
wherein said one or more future changes in renal status comprise one or more of a future injury to renal function, future reduced renal function, future improvement in renal function, and future acute renal failure (ARF) within 72 hours of the time at which the body fluid sample is obtained, or
wherein said one or more future changes in renal status comprise one or more of a future injury to renal function, future reduced renal function, future improvement in renal function, and future acute renal failure (ARF) within 48 hours of the time at which the body fluid sample is obtained, or
wherein said one or more future changes in renal status comprise one or more of a future injury to renal function, future reduced renal function, future improvement in renal function, and future acute renal failure (ARF) within 24 hours of the time at which the body fluid sample is obtained.

9. A method according to one of claims 1-5, wherein the subject is in RIFLE stage 0 or R,
wherein the subject preferably is in RIFLE stage 0 or R, and said correlating step comprises assigning a likelihood that the subject will reach RIFLE stage I or F within 72, 48 or 24 hours,
wherein the subject preferably is in RIFLE stage 0 or R, and said correlating step comprises assigning a likelihood that the subject will reach RIFLE stage F within 72, 48 or 24 hours, or
wherein the subject preferably is in RIFLE stage R, and said correlating step comprises assigning a likelihood that the subject will reach RIFLE stage I or F within 72, 48 or 24 hours,
wherein the subject preferably is in RIFLE stage R, and said correlating step comprises assigning a likelihood that the subject will reach RIFLE stage F within 72, 48 or 24 hours, or
wherein the subject preferably is in RIFLE stage 0, and said correlating step comprises assigning a likelihood that the subject will reach RIFLE stage R, I or F within 72, 48 or 24 hours,
wherein the subject preferably is in RIFLE stage 0, and said correlating step comprises assigning a likelihood that the subject will reach RIFLE stage I or F within 72, 48 or 24 hours, or
wherein the subject preferably is in RIFLE stage 0, and said correlating step comprises assigning a likelihood that the subject will reach RIFLE stage F within 72, 48 or 24 hours, or
wherein the subject is in RIFLE stage 0, R, or I, and said correlating step comprises assigning a likelihood that the subject will reach RIFLE stage F within 72, 48 or 24 hours,
wherein the subject preferably is in RIFLE stage I, and said correlating step comprises assigning a likelihood that the subject will reach RIFLE stage F within 72, 48 or 24 hours.

10. A method according to one of claims 1-5, wherein the subject is not in acute renal failure, or
wherein the subject has not experienced a 1.5-fold or greater increase in serum creatinine over a baseline value determined prior to the time at which the body fluid sample is obtained, or
wherein the subject has a urine output of at least 0.5 ml/kg/hr over the 6 hours preceding the time at which the body fluid sample is obtained, or
wherein the subject has not experienced an increase of 0.3 mg/dL or greater in serum creatinine over a baseline value determined prior to the time at which the body fluid sample is obtained, or
wherein the subject (i) has not experienced a 1.5-fold or greater increase in serum creatinine over a baseline value determined prior to the time at which the body fluid sample is obtained, (ii) has a urine output of at least 0.5 ml/kg/hr over the 6 hours preceding the time at which the body fluid sample is obtained, and (iii) has not experienced an increase of 0.3 mg/dL or greater in serum creatinine over a baseline value determined prior to the time at which the body fluid sample is obtained, or

wherein the subject has not experienced a 1.5-fold or greater increase in serum creatinine over a baseline value determined prior to the time at which the body fluid sample is obtained, or

wherein the subject has a urine output of at least 0.5 ml/kg/hr over the 6 hours preceding the time at which the body fluid sample is obtained, or

wherein the subject (i) has not experienced a 1.5-fold or greater increase in serum creatinine over a baseline value determined prior to the time at which the body fluid sample is obtained, (ii) has a urine output of at least 0.5 ml/kg/hr over the 12 hours preceding the time at which the body fluid sample is obtained, and (iii) has not experienced an increase of 0.3 mg/dL or greater in serum creatinine over a baseline value determined prior to the time at which the body fluid sample is obtained, or

wherein said correlating step comprises assigning one or more of: a likelihood that within 72 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine,

wherein said correlating step preferably comprises assigning one or more of: a likelihood that within 48 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine, or

wherein said correlating step preferably comprises assigning one or more of: a likelihood that within 24 hours the subject will (i) experience a 1.5-fold or greater increase in serum creatinine (ii) have a urine output of less than 0.5 ml/kg/hr over a 6 hour period, or (iii) experience an increase of 0.3 mg/dL or greater in serum creatinine, or

wherein said correlating step preferably comprises assigning a likelihood that within 72 hours the subject will experience a 1.5-fold or greater increase in serum creatinine, or wherein said correlating step preferably comprises assigning a likelihood that within 72 hours the subject will have a urine output of less than 0.5 ml/kg/hr over a 6 hour period, or

wherein said correlating step preferably comprises assigning a likelihood that within 72 hours the subject will experience an increase of 0.3 mg/dL or greater in serum creatinine, or

wherein said correlating step preferably comprises assigning a likelihood that within 48 hours the subject will experience a 1.5-fold or greater increase in serum creatinine, or wherein said correlating step preferably comprises assigning a likelihood that within 48 hours the subject will have a urine output of less than 0.5 ml/kg/hr over a 6 hour period, or

wherein said correlating step preferably comprises assigning a likelihood that within 48 hours the subject will experience an increase of 0.3 mg/dL or greater in serum creatinine, or

wherein said correlating step preferably comprises assigning a likelihood that within 24 hours the subject will experience a 1.5-fold or greater increase in serum creatinine, or wherein said correlating step preferably comprises assigning a likelihood that within 24 hours the subject will have a urine output of less than 0.5 ml/kg/hr over a 6 hour period, or

wherein said correlating step preferably comprises assigning a likelihood that within 24 hours the subject will experience an increase of 0.3 mg/dL or greater in serum creatinine, or

wherein the subject has not experienced a 2-fold or greater increase in serum creatinine over a baseline value determined prior to the time at which the body fluid sample is obtained, or

wherein the subject has a urine output of at least 0.5 ml/kg/hr over the 12 hours preceding the time at which the body fluid sample is obtained, or

wherein the subject (i) has not experienced a 2-fold or greater increase in serum creatinine over a baseline value determined prior to the time at which the body fluid sample is obtained, (ii) has a urine output of at least 0.5 ml/kg/hr over the 2 hours preceding the time at which the body fluid sample is obtained, and (iii) has not experienced an increase of 0.3 mg/dL or greater in serum creatinine over a baseline value determined prior to the time at which the body fluid sample is obtained, or

wherein the subject has not experienced a 3-fold or greater increase in serum creatinine over a baseline value determined prior to the time at which the body fluid sample is obtained, or

wherein the subject has a urine output of at least 0.3 ml/kg/hr over the 24 hours preceding the time at which the body fluid sample is obtained, or anuria over the 12 hours preceding the time at which the body fluid sample is obtained, or

wherein the subject (i) has not experienced a 3-fold or greater increase in serum creatinine over a baseline value determined prior to the time at which the body fluid sample is obtained, (ii) has a urine output of at least 0.3 ml/kg/hr over the 24 hours preceding the time at which the body fluid sample is obtained, or anuria over the 12 hours preceding the time at which the body fluid sample is obtained, and (iii) has not experienced an increase of 0.3 mg/dL or greater in serum creatinine over a baseline value determined prior to the time at which the body fluid sample is obtained, or

wherein said correlating step comprises assigning one or more of: a likelihood that within 72 hours the subject will (i) experience a 2-fold or greater increase in serum creatinine (ii) have a urine output of less than 0.5 ml/kg/hr over a 12 hour period, or (iii) experience an increase of 0.3 mg/dL or greater in serum creatinine,

wherein said correlating step preferably comprises assigning one or more of: a likelihood that within 48 hours the subject will (i) experience a 2-fold or greater increase in serum creatinine (ii) have a urine output of less than 0.5 ml/kg/hr over a 6 hour period, or (iii) experience an increase of 0.3 mg/dL or greater in serum creatinine, or

wherein said correlating step preferably comprises assigning one or more of: a likelihood that within 24 hours the subject will (i) experience a 2-fold or greater increase in serum creatinine, or (ii) have a urine output of less than 0.5 ml/kg/hr over a 6 hour period, or wherein said correlating step preferably comprises assigning a likelihood that within 72 hours the subject will experience a 2-fold or greater increase in serum creatinine, or wherein said correlating step preferably comprises assigning a likelihood that within 72 hours the subject will have a urine output of less than 0.5 ml/kg/hr over a 6 hour period, or

wherein said correlating step preferably comprises assigning a likelihood that within 48 hours the subject will experience a 2-fold or greater increase in serum creatinine, or wherein said correlating step preferably comprises assigning a likelihood that within 48 hours the subject will have a urine output of less than 0.5 ml/kg/hr over a 6 hour period, or wherein said correlating step preferably comprises assigning a likelihood that within 24 hours the subject will experience a 2-fold or greater increase in serum creatinine, or wherein said correlating step preferably comprises assigning a likelihood that within 24 hours the subject will have a urine output of less than 0.5 ml/kg/hr over a 6 hour period, or wherein said correlating step comprises assigning one or more of: a likelihood that within 72 hours the subject will (i) experience a 3-fold or greater increase in serum creatinine, or (ii) have a urine output of less than 0.3 ml/kg/hr over a 24 hour period or anuria over a 12 hour period,

wherein said correlating step preferably comprises assigning one or more of: a likelihood that within 48 hours the subject will (i) experience a 3-fold or greater increase in serum creatinine, or (ii) have a urine output of less than 0.3 ml/kg/hr over a 24 hour period or anuria over a 12 hour period, or

wherein said correlating step preferably comprises assigning one or more of: a likelihood that within 24 hours the subject will (i) experience a 3-fold or greater increase in serum creatinine, or (ii) have a urine output of less than 0.3 ml/kg/hr over a 24 hour period or anuria over a 12 hour period, or

wherein said correlating step preferably comprises assigning a likelihood that within 72 hours the subject will experience a 3-fold or greater increase in serum creatinine, or wherein said correlating step preferably comprises assigning a likelihood that within 72 hours the subject will have a urine output of less than 0.3 ml/kg/hr over a 24 hour period or anuria over a 12 hour period, or

wherein said correlating step preferably comprises assigning a likelihood that within 48 hours the subject will experience a 3-fold or greater increase in serum creatinine, or wherein said correlating step preferably comprises assigning a likelihood that within 48 hours the subject will have a urine output of less than 0.3 ml/kg/hr over a 24 hour period or anuria over a 12 hour period, or

wherein said correlating step preferably comprises assigning a likelihood that within 24 hours the subject will experience a 3-fold or greater increase in serum creatinine, or wherein said correlating step preferably comprises assigning a likelihood that within 24 hours the subject will have a urine output of less than 0.3 ml/kg/hr over a 24 hour period or anuria over a 12 hour period.

11. A method according to one of claims 1-10, wherein the body fluid sample is a urine sample.

12. A method according to one of claims 1-11, wherein said method comprises performing assays that detect Growth/differentiation factor 15, and optionally further one, two or three, or more of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, and Proprotein convertase subtilisin/kexin type 9.

13. Use of Growth/differentiation factor 15, and optionally further one or more biomarkers of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, and Proprotein convertase subtilisin/kexin type 9 for the risk stratification, prognosis, classifying and/or monitoring of renal injury or

use of Growth/differentiation factor 15, and optionally further one or more biomarkers of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, , and Proprotein convertase subtilisin/kexin type 9 for the risk stratification, prognosis, classifying and/or monitoring of acute renal injury.

14. A kit, comprising:

reagents for performing one or more assays configured to detect Growth/differentiation factor 15, and optionally further one or more kidney injury markers of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, and Proprotein convertase subtilisin/kexin type 9;

wherein said reagents preferably comprise one or more binding reagents, each of which specifically binds one of said of kidney injury markers;

wherein a plurality of binding reagents are preferably contained in a single assay device,

or

wherein at least one of said assays is preferably configured as a sandwich binding assay;

or

wherein at least one of said assays is preferably configured as a competitive binding assay;

wherein said one or more assays preferably comprise assays that detect Growth/differentiation factor 15, and optionally further one, two or three, or more of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, and Proprotein convertase subtilisin/kexin type 9.

15. A system for evaluating biomarker levels, comprising:

a plurality of reagents which specifically bind for detection the plurality of biomarkers, one or more of which is selected from Growth/differentiation factor 15, and optionally further of Stanniocalcin-1, Antithrombin-III, Toll-like receptor 2, Triiodothyronine (T3), Thyroxine (T4), Extracellular matrix protein 1, Coagulation factor XIII A chain, Coagulation factor XIII B chain, Interleukin-17F, Interleukin-22, Vitronectin, Progesterone, Estradiol, and Proprotein convertase subtilisin/kexin type 9;

an assay instrument configured to receive a urine sample and contact the plurality of reagents with the urine sample and to generate one or more assay results indicative of binding of each biomarker which is assayed to a respective specific binding reagent in the plurality of reagents,

wherein the reagents preferably comprise a plurality of antibodies, at least one of which binds to each of the biomarkers which are assayed,

wherein assay instrument preferably comprises an assay device and an assay device reader, wherein the plurality of antibodies are immobilized at a plurality of predetermined locations within the assay device, wherein the assay device is configured to receive the urine sample such that the urine sample contacts the plurality of predetermined locations, and wherein the assay device reader interrogates the plurality of predetermined locations to generate the assay results.

**Europäisches Patentamt**
**European Patent Office**
**Office européen des brevets**

# EUROPEAN SEARCH REPORT

**Application Number**

EP 17 19 0074

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2010/048670 A1 (ST VINCENTS HOSP SYDNEY [AU]; BREIT SAMUEL NORBERT [AU]; BROWN DAVID A) 6 May 2010 (2010-05-06) | 1-5,7,8, 10,12-15 | INV. G01N33/68 |
| Y | * the whole document * <br> * in particular Examples 1 and 4-6. * | 6,9,11 | |
| | ----- | | |
| X | EP 2 336 784 A1 (ROCHE DIAGNOSTICS GMBH [DE]; HOFFMANN LA ROCHE [CH]) 22 June 2011 (2011-06-22) | 1-5,7,8, 10,12-15 | |
| Y | * abstract * <br> * paragraphs [0006] - [0011], [0016], [0017], [0021], [0022], [0030], [0035], [0044]; example 1 * | 6,9,11 | |
| | ----- | | |
| Y | US 2011/195429 A1 (ANDERBERG JOSEPH [US] ET AL) 11 August 2011 (2011-08-11) <br> * abstract * <br> * paragraphs [0008], [0024], [0035], [0123]; examples 5,6 * | 6,9,11 | |
| | ----- | | |
| A | Publishing Blackwell ET AL: "Publishing Ltd Protein biomarkers associated with acute renal failure and chronic kidney disease", <br> European Journal of Clinical Investigation Journal Compilation Blackwell Eur J Clin Invest, <br> 1 January 2006 (2006-01-01), pages 753-763, XP55098668, <br> Retrieved from the Internet: <br> URL:http://onlinelibrary.wiley.com/store/1 0.1111/j.1365-2362.2006.01729.x/asset/j.13 65-2362.2006.01729.x.pdf?v=1&t=hqxw8hun&s= f312bbc9e40495d2fe10c345fb2690a9a4e077ed [retrieved on 2014-01-27] <br> * the whole document * | 1-15 | TECHNICAL FIELDS SEARCHED (IPC) <br><br> G01N |
| | ----- | | |

The present search report has been drawn up for all claims

| Place of search | Date of completion of the search | Examiner |
|---|---|---|
| Munich | 21 November 2017 | Thumb, Werner |

EPO FORM 1503 03.82 (P04C01)

## ANNEX TO THE EUROPEAN SEARCH REPORT
## ON EUROPEAN PATENT APPLICATION NO.

EP 17 19 0074

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2017

| Patent document cited in search report | | Publication date | Patent family member(s) | | Publication date |
|---|---|---|---|---|---|
| WO 2010048670 | A1 | 06-05-2010 | CA | 2740632 A1 | 06-05-2010 |
| | | | CN | 102203619 A | 28-09-2011 |
| | | | EP | 2350669 A1 | 03-08-2011 |
| | | | ES | 2434996 T3 | 18-12-2013 |
| | | | HK | 1154652 A1 | 25-04-2014 |
| | | | JP | 5444363 B2 | 19-03-2014 |
| | | | JP | 2012507012 A | 22-03-2012 |
| | | | US | 2012107420 A1 | 03-05-2012 |
| | | | US | 2014205684 A1 | 24-07-2014 |
| | | | US | 2017023587 A1 | 26-01-2017 |
| | | | WO | 2010048670 A1 | 06-05-2010 |
| EP 2336784 | A1 | 22-06-2011 | CA | 2778873 A1 | 23-06-2011 |
| | | | CN | 102652261 A | 29-08-2012 |
| | | | EP | 2336784 A1 | 22-06-2011 |
| | | | EP | 3246708 A2 | 22-11-2017 |
| | | | JP | 5719379 B2 | 20-05-2015 |
| | | | JP | 2013514528 A | 25-04-2013 |
| | | | US | 2012252035 A1 | 04-10-2012 |
| | | | US | 2016109464 A1 | 21-04-2016 |
| | | | WO | 2011073382 A1 | 23-06-2011 |
| US 2011195429 | A1 | 11-08-2011 | AU | 2009285550 A1 | 04-03-2010 |
| | | | CA | 2735587 A1 | 04-03-2010 |
| | | | CN | 102187220 A | 14-09-2011 |
| | | | CN | 105067819 A | 18-11-2015 |
| | | | EP | 2324355 A1 | 25-05-2011 |
| | | | EP | 2743702 A2 | 18-06-2014 |
| | | | HK | 1153268 A1 | 22-08-2014 |
| | | | HK | 1198391 A1 | 17-04-2015 |
| | | | HK | 1213320 A1 | 30-06-2016 |
| | | | JP | 6126186 B2 | 10-05-2017 |
| | | | JP | 2012501456 A | 19-01-2012 |
| | | | JP | 2016048242 A | 07-04-2016 |
| | | | KR | 20110073471 A | 29-06-2011 |
| | | | MX | 341926 B | 07-09-2016 |
| | | | NZ | 591437 A | 26-07-2013 |
| | | | NZ | 610356 A | 27-03-2015 |
| | | | US | 2011195429 A1 | 11-08-2011 |
| | | | WO | 2010025424 A1 | 04-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61562778 A
- US 61562802 B
- US 61562813 B
- US 61562817 B
- US 61562824 B
- US 61562829 B
- US 61562872 B
- US 61562879 B
- US 61562883 B
- US 61562885 B
- US 61562916 B
- US 61562943 B
- US 61562947 B
- US 61562951 B
- US 6143576 A
- US 6113855 A
- US 6019944 A
- US 5985579 A
- US 5947124 A
- US 5939272 A
- US 5922615 A
- US 5885527 A
- US 5851776 A
- US 5824799 A
- US 5679526 A
- US 5525524 A
- US 5480792 A
- US 5631171 A
- US 5955377 A
- US 5571698 A, Ladner
- US 6057098 A

### Non-patent literature cited in the description

- Harrison's Principles of Internal Medicine. McGraw Hill, 1741-1830
- Current Medical Diagnosis & Treatment. McGraw Hill, 2008, 785-815
- Merck Manual
- **PRAUGHT ; SHLIPAK.** *Curr Opin Nephrol Hypertens,* 2005, vol. 14, 265-270
- **CHERTOW et al.** *J Am Soc Nephrol,* 2005, vol. 16, 3365-3370
- **LASSNIGG.** *J Am Soc Nephrol,* 2004, vol. 15, 1597-1605
- **BELLOMO et al.** *Crit Care.,* 2004, vol. 8 (4), R204-12
- **KELLUM.** *Crit. Care Med.,* 2008, vol. 36, 141-45
- **RICCI et al.** *Kidney Int.,* 2008, vol. 73, 538-546
- **MEHTA et al.** *Crit. Care,* 2007, vol. 11, R31
- **MCCOLLOUGH et al.** *Rev Cardiovasc Med.,* 2006, vol. 7 (4), 177-197
- **THAKAR et al.** *J. Am. Soc. Nephrol.,* 2005, vol. 16, 162-68
- **MEHRAN et al.** *J. Am. Coll. Cardiol.,* 2004, vol. 44, 1393-99
- **WIJEYSUNDERA et al.** *JAMA,* 2007, vol. 297, 1801-9
- **GOLDSTEIN ; CHAWLA.** *Clin. J. Am. Soc. Nephrol.,* 2010, vol. 5, 943-49
- **CHAWLA et al.** *Kidney Intl.,* 2005, vol. 68, 2274-80
- The Immunoassay Handbook. Stockton Press, 1994
- Fundamental Immunology. Raven Press, 1993
- **WILSON.** *J. Immunol. Methods,* 1994, vol. 175, 267-273
- **YARMUSH.** *J. Biochem. Biophys. Methods,* 1992, vol. 25, 85-97
- **WARD et al.** *Nature,* 1989, vol. 341, 544-546
- **VAN ERP et al.** *J. Immunoassay,* 1991, vol. 12, 425-43
- **NELSON ; GRISWOLD.** *Comput. Methods Programs Biomed.,* 1988, vol. 27, 65-8
- **CWIRLA et al.** *Proc. Natl. Acad. Sci. USA,* 1990, vol. 87, 6378-82
- **DEVLIN et al.** *Science,* 1990, vol. 249, 404-6
- **SCOTT ; SMITH.** *Science,* 1990, vol. 249, 386-88
- **FISCHER et al.** *Intensive Care Med.,* 2003, vol. 29, 1043-51
- **BAGSHAW et al.** Nephrol. Dial. Transplant. 2008, vol. 23, 1203-1210
- Merck Manual of Diagnosis and Therapy. Merck Research Laboratories, 1999
- **HANLEY, J. A. ; MCNEIL, B.J.** The meaning and use of the area under a receiver operating characteristic (ROC) curve. *Radiology,* 1982, vol. 143, 29-36

SEQUENCE LISTING

<110> ASTUTE MEDICAL, INC.

<120> METHODS AND COMPOSITIONS FOR DIAGNOSIS AND PROGNOSIS OF RENAL INJURY AND RENAL FAILURE

<130> BLG15076PCTEPD1

<140> PCT/US2012/066152
<141> 2012-11-20

<150> 61/562,778
<151> 2011-11-22

<150> 61/562,802
<151> 2011-11-22

<150> 61/562,813
<151> 2011-11-22

<150> 61/562,817
<151> 2011-11-22

<150> 61/562,824
<151> 2011-11-22

<150> 61/562,829
<151> 2011-11-22

<150> 61/562,872
<151> 2011-11-22

<150> 61/562,879
<151> 2011-11-22

<150> 61/562,883
<151> 2011-11-22

<150> 61/562,885
<151> 2011-11-22

<150> 61/562,916
<151> 2011-11-22

<150> 61/562,943
<151> 2011-11-22

<150> 61/562,947
<151> 2011-11-22

<150> 61/562,951
<151> 2011-11-22

<160> 16

<170> PatentIn version 3.5

<210> 1
<211> 247
<212> PRT
<213> Homo sapiens

<400> 1

Met Leu Gln Asn Ser Ala Val Leu Leu Val Leu Val Ile Ser Ala Ser
1               5                   10                  15

Ala Thr His Glu Ala Glu Gln Asn Asp Ser Val Ser Pro Arg Lys Ser
            20                  25                  30

Arg Val Ala Ala Gln Asn Ser Ala Glu Val Val Arg Cys Leu Asn Ser
            35                  40                  45

Ala Leu Gln Val Gly Cys Gly Ala Phe Ala Cys Leu Glu Asn Ser Thr
        50                  55                  60

Cys Asp Thr Asp Gly Met Tyr Asp Ile Cys Lys Ser Phe Leu Tyr Ser
65                  70                  75                  80

Ala Ala Lys Phe Asp Thr Gln Gly Lys Ala Phe Val Lys Glu Ser Leu
            85                  90                  95

Lys Cys Ile Ala Asn Gly Val Thr Ser Lys Val Phe Leu Ala Ile Arg
            100                 105                 110

Arg Cys Ser Thr Phe Gln Arg Met Ile Ala Glu Val Gln Glu Glu Cys
        115                 120                 125

Tyr Ser Lys Leu Asn Val Cys Ser Ile Ala Lys Arg Asn Pro Glu Ala
    130                 135                 140

Ile Thr Glu Val Val Gln Leu Pro Asn His Phe Ser Asn Arg Tyr Tyr
145                 150                 155                 160

Asn Arg Leu Val Arg Ser Leu Leu Glu Cys Asp Glu Asp Thr Val Ser
            165                 170                 175

Thr Ile Arg Asp Ser Leu Met Glu Lys Ile Gly Pro Asn Met Ala Ser
            180                 185                 190

Leu Phe His Ile Leu Gln Thr Asp His Cys Ala Gln Thr His Pro Arg
        195                 200                 205

Ala Asp Phe Asn Arg Arg Arg Thr Asn Glu Pro Gln Lys Leu Lys Val
        210                 215                 220

Leu Leu Arg Asn Leu Arg Gly Glu Glu Asp Ser Pro Ser His Ile Lys
225                 230                 235                 240

Arg Thr Ser His Glu Ser Ala
                245

```
<210>  2
<211>  692
<212>  PRT
<213>  Homo sapiens

<400>  2
Met Gly Thr Val Ser Ser Arg Arg Ser Trp Trp Pro Leu Pro Leu Leu
1               5                   10                  15


Leu Leu Leu Leu Leu Leu Leu Gly Pro Ala Gly Ala Arg Ala Gln Glu
            20                  25                  30


Asp Glu Asp Gly Asp Tyr Glu Glu Leu Val Leu Ala Leu Arg Ser Glu
        35                  40                  45


Glu Asp Gly Leu Ala Glu Ala Pro Glu His Gly Thr Thr Ala Thr Phe
        50                  55                  60


His Arg Cys Ala Lys Asp Pro Trp Arg Leu Pro Gly Thr Tyr Val Val
65                  70                  75                  80


Val Leu Lys Glu Glu Thr His Leu Ser Gln Ser Glu Arg Thr Ala Arg
                85                  90                  95


Arg Leu Gln Ala Gln Ala Ala Arg Arg Gly Tyr Leu Thr Lys Ile Leu
                100                 105                 110


His Val Phe His Gly Leu Leu Pro Gly Phe Leu Val Lys Met Ser Gly
            115                 120                 125


Asp Leu Leu Glu Leu Ala Leu Lys Leu Pro His Val Asp Tyr Ile Glu
        130                 135                 140


Glu Asp Ser Ser Val Phe Ala Gln Ser Ile Pro Trp Asn Leu Glu Arg
145                 150                 155                 160


Ile Thr Pro Pro Arg Tyr Arg Ala Asp Glu Tyr Gln Pro Pro Asp Gly
                165                 170                 175


Gly Ser Leu Val Glu Val Tyr Leu Leu Asp Thr Ser Ile Gln Ser Asp
            180                 185                 190


His Arg Glu Ile Glu Gly Arg Val Met Val Thr Asp Phe Glu Asn Val
            195                 200                 205


Pro Glu Glu Asp Gly Thr Arg Phe His Arg Gln Ala Ser Lys Cys Asp
        210                 215                 220
```

```
Ser His Gly Thr His Leu Ala Gly Val Val Ser Gly Arg Asp Ala Gly
225                 230                 235                 240

Val Ala Lys Gly Ala Ser Met Arg Ser Leu Arg Val Leu Asn Cys Gln
            245                 250                 255

Gly Lys Gly Thr Val Ser Gly Thr Leu Ile Gly Leu Glu Phe Ile Arg
            260                 265                 270

Lys Ser Gln Leu Val Gln Pro Val Gly Pro Leu Val Val Leu Leu Pro
            275                 280                 285

Leu Ala Gly Gly Tyr Ser Arg Val Leu Asn Ala Ala Cys Gln Arg Leu
            290                 295                 300

Ala Arg Ala Gly Val Val Leu Val Thr Ala Ala Gly Asn Phe Arg Asp
305                 310                 315                 320

Asp Ala Cys Leu Tyr Ser Pro Ala Ser Ala Pro Glu Val Ile Thr Val
            325                 330                 335

Gly Ala Thr Asn Ala Gln Asp Gln Pro Val Thr Leu Gly Thr Leu Gly
            340                 345                 350

Thr Asn Phe Gly Arg Cys Val Asp Leu Phe Ala Pro Gly Glu Asp Ile
            355                 360                 365

Ile Gly Ala Ser Ser Asp Cys Ser Thr Cys Phe Val Ser Gln Ser Gly
    370                 375                 380

Thr Ser Gln Ala Ala Ala His Val Ala Gly Ile Ala Ala Met Met Leu
385                 390                 395                 400

Ser Ala Glu Pro Glu Leu Thr Leu Ala Glu Leu Arg Gln Arg Leu Ile
            405                 410                 415

His Phe Ser Ala Lys Asp Val Ile Asn Glu Ala Trp Phe Pro Glu Asp
            420                 425                 430

Gln Arg Val Leu Thr Pro Asn Leu Val Ala Ala Leu Pro Pro Ser Thr
            435                 440                 445

His Gly Ala Gly Trp Gln Leu Phe Cys Arg Thr Val Trp Ser Ala His
            450                 455                 460

Ser Gly Pro Thr Arg Met Ala Thr Ala Val Ala Arg Cys Ala Pro Asp
465                 470                 475                 480
```

```
Glu Glu Leu Leu Ser Cys Ser Ser Phe Ser Arg Ser Gly Lys Arg Arg
                485                 490                 495

Gly Glu Arg Met Glu Ala Gln Gly Gly Lys Leu Val Cys Arg Ala His
            500                 505                 510

Asn Ala Phe Gly Gly Glu Gly Val Tyr Ala Ile Ala Arg Cys Cys Leu
            515                 520                 525

Leu Pro Gln Ala Asn Cys Ser Val His Thr Ala Pro Pro Ala Glu Ala
        530                 535                 540

Ser Met Gly Thr Arg Val His Cys His Gln Gln Gly His Val Leu Thr
545                 550                 555                 560

Gly Cys Ser Ser His Trp Glu Val Glu Asp Leu Gly Thr His Lys Pro
                565                 570                 575

Pro Val Leu Arg Pro Arg Gly Gln Pro Asn Gln Cys Val Gly His Arg
            580                 585                 590

Glu Ala Ser Ile His Ala Ser Cys Cys His Ala Pro Gly Leu Glu Cys
            595                 600                 605

Lys Val Lys Glu His Gly Ile Pro Ala Pro Gln Glu Gln Val Thr Val
        610                 615                 620

Ala Cys Glu Glu Gly Trp Thr Leu Thr Gly Cys Ser Ala Leu Pro Gly
625                 630                 635                 640

Thr Ser His Val Leu Gly Ala Tyr Ala Val Asp Asn Thr Cys Val Val
                645                 650                 655

Arg Ser Arg Asp Val Ser Thr Thr Gly Ser Thr Ser Glu Gly Ala Val
            660                 665                 670

Thr Ala Val Ala Ile Cys Cys Arg Ser Arg His Leu Ala Gln Ala Ser
            675                 680                 685

Gln Glu Leu Gln
        690
```

```
<210> 3
<211> 5
<212> PRT
<213> Homo sapiens
```

```
<400> 3
Met Ser Pro Trp Lys
1               5


<210> 4
<211> 33
<212> PRT
<213> Homo sapiens

<400> 4
Gly Arg Thr Ser Leu Val Pro Pro Ala Thr Ala Ala Pro Ala Leu Cys
1               5               10              15


His Arg Val Gly His His Arg Leu Leu Pro Thr Trp Leu Ala Leu Gln
            20              25              30


Pro


<210> 5
<211> 179
<212> PRT
<213> Homo sapiens

<400> 5
Met Ala Ala Leu Gln Lys Ser Val Ser Ser Phe Leu Met Gly Thr Leu
1               5               10              15


Ala Thr Ser Cys Leu Leu Leu Leu Ala Leu Leu Val Gln Gly Gly Ala
            20              25              30


Ala Ala Pro Ile Ser Ser His Cys Arg Leu Asp Lys Ser Asn Phe Gln
            35              40              45


Gln Pro Tyr Ile Thr Asn Arg Thr Phe Met Leu Ala Lys Glu Ala Ser
    50              55              60


Leu Ala Asp Asn Asn Thr Asp Val Arg Leu Ile Gly Glu Lys Leu Phe
65              70              75              80


His Gly Val Ser Met Ser Glu Arg Cys Tyr Leu Met Lys Gln Val Leu
                85              90              95


Asn Phe Thr Leu Glu Glu Val Leu Phe Pro Gln Ser Asp Arg Phe Gln
            100             105             110


Pro Tyr Met Gln Glu Val Val Pro Phe Leu Ala Arg Leu Ser Asn Arg
            115             120             125


Leu Ser Thr Cys His Ile Glu Gly Asp Asp Leu His Ile Gln Arg Asn
        130             135             140
```

Val Gln Lys Leu Lys Asp Thr Val Lys Lys Leu Gly Glu Ser Gly Glu
145                 150                 155                 160

Ile Lys Ala Ile Gly Glu Leu Asp Leu Leu Phe Met Ser Leu Arg Asn
                165                 170                 175

Ala Cys Ile

<210> 6
<211> 732
<212> PRT
<213> Homo sapiens

<400> 6
Met Ser Glu Thr Ser Arg Thr Ala Phe Gly Gly Arg Arg Ala Val Pro
1               5                   10                  15

Pro Asn Asn Ser Asn Ala Ala Glu Asp Asp Leu Pro Thr Val Glu Leu
                20                  25                  30

Gln Gly Val Val Pro Arg Gly Val Asn Leu Gln Glu Phe Leu Asn Val
                35                  40                  45

Thr Ser Val His Leu Phe Lys Glu Arg Trp Asp Thr Asn Lys Val Asp
                50                  55                  60

His His Thr Asp Lys Tyr Glu Asn Asn Lys Leu Ile Val Arg Arg Gly
65                  70                  75                  80

Gln Ser Phe Tyr Val Gln Ile Asp Phe Ser Arg Pro Tyr Asp Pro Arg
                85                  90                  95

Arg Asp Leu Phe Arg Val Glu Tyr Val Ile Gly Arg Tyr Pro Gln Glu
                100                 105                 110

Asn Lys Gly Thr Tyr Ile Pro Val Pro Ile Val Ser Glu Leu Gln Ser
                115                 120                 125

Gly Lys Trp Gly Ala Lys Ile Val Met Arg Glu Asp Arg Ser Val Arg
                130                 135                 140

Leu Ser Ile Gln Ser Ser Pro Lys Cys Ile Val Gly Lys Phe Arg Met
145                 150                 155                 160

Tyr Val Ala Val Trp Thr Pro Tyr Gly Val Leu Arg Thr Ser Arg Asn
                165                 170                 175

187

```
Pro Glu Thr Asp Thr Tyr Ile Leu Phe Asn Pro Trp Cys Glu Asp Asp
        180             185             190

Ala Val Tyr Leu Asp Asn Glu Lys Glu Arg Glu Glu Tyr Val Leu Asn
        195             200             205

Asp Ile Gly Val Ile Phe Tyr Gly Glu Val Asn Asp Ile Lys Thr Arg
        210             215             220

Ser Trp Ser Tyr Gly Gln Phe Glu Asp Gly Ile Leu Asp Thr Cys Leu
225             230             235             240

Tyr Val Met Asp Arg Ala Gln Met Asp Leu Ser Gly Arg Gly Asn Pro
            245             250             255

Ile Lys Val Ser Arg Val Gly Ser Ala Met Val Asn Ala Lys Asp Asp
            260             265             270

Glu Gly Val Leu Val Gly Ser Trp Asp Asn Ile Tyr Ala Tyr Gly Val
            275             280             285

Pro Pro Ser Ala Trp Thr Gly Ser Val Asp Ile Leu Leu Glu Tyr Arg
        290             295             300

Ser Ser Glu Asn Pro Val Arg Tyr Gly Gln Cys Trp Val Phe Ala Gly
305             310             315             320

Val Phe Asn Thr Phe Leu Arg Cys Leu Gly Ile Pro Ala Arg Ile Val
            325             330             335

Thr Asn Tyr Phe Ser Ala His Asp Asn Asp Ala Asn Leu Gln Met Asp
            340             345             350

Ile Phe Leu Glu Glu Asp Gly Asn Val Asn Ser Lys Leu Thr Lys Asp
        355             360             365

Ser Val Trp Asn Tyr His Cys Trp Asn Glu Ala Trp Met Thr Arg Pro
370             375             380

Asp Leu Pro Val Gly Phe Gly Gly Trp Gln Ala Val Asp Ser Thr Pro
385             390             395             400

Gln Glu Asn Ser Asp Gly Met Tyr Arg Cys Gly Pro Ala Ser Val Gln
            405             410             415

Ala Ile Lys His Gly His Val Cys Phe Gln Phe Asp Ala Pro Phe Val
            420             425             430
```

188

```
Phe Ala Glu Val Asn Ser Asp Leu Ile Tyr Ile Thr Ala Lys Lys Asp
        435                 440             445

Gly Thr His Val Val Glu Asn Val Asp Ala Thr His Ile Gly Lys Leu
        450                 455             460

Ile Val Thr Lys Gln Ile Gly Gly Asp Gly Met Met Asp Ile Thr Asp
465                 470             475                 480

Thr Tyr Lys Phe Gln Glu Gly Gln Glu Glu Glu Arg Leu Ala Leu Glu
                485             490                 495

Thr Ala Leu Met Tyr Gly Ala Lys Lys Pro Leu Asn Thr Glu Gly Val
            500             505             510

Met Lys Ser Arg Ser Asn Val Asp Met Asp Phe Glu Val Glu Asn Ala
        515             520             525

Val Leu Gly Lys Asp Phe Lys Leu Ser Ile Thr Phe Arg Asn Asn Ser
    530             535             540

His Asn Arg Tyr Thr Ile Thr Ala Tyr Leu Ser Ala Asn Ile Thr Phe
545             550             555                 560

Tyr Thr Gly Val Pro Lys Ala Glu Phe Lys Lys Glu Thr Phe Asp Val
            565             570             575

Thr Leu Glu Pro Leu Ser Phe Lys Lys Glu Ala Val Leu Ile Gln Ala
            580             585             590

Gly Glu Tyr Met Gly Gln Leu Leu Glu Gln Ala Ser Leu His Phe Phe
        595             600             605

Val Thr Ala Arg Ile Asn Glu Thr Arg Asp Val Leu Ala Lys Gln Lys
    610             615             620

Ser Thr Val Leu Thr Ile Pro Glu Ile Ile Ile Lys Val Arg Gly Thr
625             630             635                 640

Gln Val Val Gly Ser Asp Met Thr Val Thr Val Gln Phe Thr Asn Pro
            645             650             655

Leu Lys Glu Thr Leu Arg Asn Val Trp Val His Leu Asp Gly Pro Gly
        660             665             670

Val Thr Arg Pro Met Lys Lys Met Phe Arg Glu Ile Arg Pro Asn Ser
```

```
              675                    680                    685

     Thr Val Gln Trp Glu Glu Val Cys Arg Pro Trp Val Ser Gly His Arg
         690                    695                    700

     Lys Leu Ile Ala Ser Met Ser Ser Asp Ser Leu Arg His Val Tyr Gly
     705                    710                    715                    720

     Glu Leu Asp Val Gln Ile Gln Arg Arg Pro Ser Met
                         725                    730


     <210> 7
     <211> 784
     <212> PRT
     <213> Homo sapiens

     <400> 7
     Met Pro His Thr Leu Trp Met Val Trp Val Leu Gly Val Ile Ile Ser
     1               5                   10                  15

     Leu Ser Lys Glu Glu Ser Ser Asn Gln Ala Ser Leu Ser Cys Asp Arg
                 20                  25                  30

     Asn Gly Ile Cys Lys Gly Ser Ser Gly Ser Leu Asn Ser Ile Pro Ser
             35                  40                  45

     Gly Leu Thr Glu Ala Val Lys Ser Leu Asp Leu Ser Asn Asn Arg Ile
         50                  55                  60

     Thr Tyr Ile Ser Asn Ser Asp Leu Gln Arg Cys Val Asn Leu Gln Ala
     65                  70                  75                  80

     Leu Val Leu Thr Ser Asn Gly Ile Asn Thr Ile Glu Glu Asp Ser Phe
                 85                  90                  95

     Ser Ser Leu Gly Ser Leu Glu His Leu Asp Leu Ser Tyr Asn Tyr Leu
                 100                 105                 110

     Ser Asn Leu Ser Ser Ser Trp Phe Lys Pro Leu Ser Ser Leu Thr Phe
                 115                 120                 125

     Leu Asn Leu Leu Gly Asn Pro Tyr Lys Thr Leu Gly Glu Thr Ser Leu
                 130                 135                 140

     Phe Ser His Leu Thr Lys Leu Gln Ile Leu Arg Val Gly Asn Met Asp
     145                 150                 155                 160

     Thr Phe Thr Lys Ile Gln Arg Lys Asp Phe Ala Gly Leu Thr Phe Leu
                 165                 170                 175
```

```
Glu Glu Leu Glu Ile Asp Ala Ser Asp Leu Gln Ser Tyr Glu Pro Lys
            180                 185                 190

Ser Leu Lys Ser Ile Gln Asn Val Ser His Leu Ile Leu His Met Lys
            195                 200                 205

Gln His Ile Leu Leu Leu Glu Ile Phe Val Asp Val Thr Ser Ser Val
            210                 215                 220

Glu Cys Leu Glu Leu Arg Asp Thr Asp Leu Asp Thr Phe His Phe Ser
225                 230                 235                 240

Glu Leu Ser Thr Gly Glu Thr Asn Ser Leu Ile Lys Lys Phe Thr Phe
                245                 250                 255

Arg Asn Val Lys Ile Thr Asp Glu Ser Leu Phe Gln Val Met Lys Leu
            260                 265                 270

Leu Asn Gln Ile Ser Gly Leu Leu Glu Leu Glu Phe Asp Asp Cys Thr
            275                 280                 285

Leu Asn Gly Val Gly Asn Phe Arg Ala Ser Asp Asn Asp Arg Val Ile
            290                 295                 300

Asp Pro Gly Lys Val Glu Thr Leu Thr Ile Arg Arg Leu His Ile Pro
305                 310                 315                 320

Arg Phe Tyr Leu Phe Tyr Asp Leu Ser Thr Leu Tyr Ser Leu Thr Glu
                325                 330                 335

Arg Val Lys Arg Ile Thr Val Glu Asn Ser Lys Val Phe Leu Val Pro
            340                 345                 350

Cys Leu Leu Ser Gln His Leu Lys Ser Leu Glu Tyr Leu Asp Leu Ser
            355                 360                 365

Glu Asn Leu Met Val Glu Glu Tyr Leu Lys Asn Ser Ala Cys Glu Asp
            370                 375                 380

Ala Trp Pro Ser Leu Gln Thr Leu Ile Leu Arg Gln Asn His Leu Ala
385                 390                 395                 400

Ser Leu Glu Lys Thr Gly Glu Thr Leu Leu Thr Leu Lys Asn Leu Thr
                405                 410                 415

Asn Ile Asp Ile Ser Lys Asn Ser Phe His Ser Met Pro Glu Thr Cys
```

```
                420                        425                        430

        Gln Trp Pro Glu Lys Met Lys Tyr Leu Asn Leu Ser Ser Thr Arg Ile
                435                 440                 445

        His Ser Val Thr Gly Cys Ile Pro Lys Thr Leu Glu Ile Leu Asp Val
                450                 455                 460

        Ser Asn Asn Asn Leu Asn Leu Phe Ser Leu Asn Leu Pro Gln Leu Lys
        465                 470                 475                 480

        Glu Leu Tyr Ile Ser Arg Asn Lys Leu Met Thr Leu Pro Asp Ala Ser
                        485                 490                 495

        Leu Leu Pro Met Leu Leu Val Leu Lys Ile Ser Arg Asn Ala Ile Thr
                    500                 505                 510

        Thr Phe Ser Lys Glu Gln Leu Asp Ser Phe His Thr Leu Lys Thr Leu
                    515                 520                 525

        Glu Ala Gly Gly Asn Asn Phe Ile Cys Ser Cys Glu Phe Leu Ser Phe
                530                 535                 540

        Thr Gln Glu Gln Gln Ala Leu Ala Lys Val Leu Ile Asp Trp Pro Ala
        545                 550                 555                 560

        Asn Tyr Leu Cys Asp Ser Pro Ser His Val Arg Gly Gln Gln Val Gln
                        565                 570                 575

        Asp Val Arg Leu Ser Val Ser Glu Cys His Arg Thr Ala Leu Val Ser
                    580                 585                 590

        Gly Met Cys Cys Ala Leu Phe Leu Leu Ile Leu Leu Thr Gly Val Leu
                    595                 600                 605

        Cys His Arg Phe His Gly Leu Trp Tyr Met Lys Met Met Trp Ala Trp
                610                 615                 620

        Leu Gln Ala Lys Arg Lys Pro Arg Lys Ala Pro Ser Arg Asn Ile Cys
        625                 630                 635                 640

        Tyr Asp Ala Phe Val Ser Tyr Ser Glu Arg Asp Ala Tyr Trp Val Glu
                        645                 650                 655

        Asn Leu Met Val Gln Glu Leu Glu Asn Phe Asn Pro Pro Phe Lys Leu
                    660                 665                 670
```

192

```
Cys Leu His Lys Arg Asp Phe Ile Pro Gly Lys Trp Ile Ile Asp Asn
        675             680             685

Ile Ile Asp Ser Ile Glu Lys Ser His Lys Thr Val Phe Val Leu Ser
        690             695             700

Glu Asn Phe Val Lys Ser Glu Trp Cys Lys Tyr Glu Leu Asp Phe Ser
705             710             715                 720

His Phe Arg Leu Phe Asp Glu Asn Asn Asp Ala Ala Ile Leu Ile Leu
                725             730             735

Leu Glu Pro Ile Glu Lys Lys Ala Ile Pro Gln Arg Phe Cys Lys Leu
            740             745             750

Arg Lys Ile Met Asn Thr Lys Thr Tyr Leu Glu Trp Pro Met Asp Glu
        755             760             765

Ala Gln Arg Glu Gly Phe Trp Val Asn Leu Arg Ala Ala Ile Lys Ser
    770             775             780
```

<210> 8
<211> 464
<212> PRT
<213> Homo sapiens

<400> 8
```
Met Tyr Ser Asn Val Ile Gly Thr Val Thr Ser Gly Lys Arg Lys Val
1               5               10              15

Tyr Leu Leu Ser Leu Leu Leu Ile Gly Phe Trp Asp Cys Val Thr Cys
            20              25              30

His Gly Ser Pro Val Asp Ile Cys Thr Ala Lys Pro Arg Asp Ile Pro
            35              40              45

Met Asn Pro Met Cys Ile Tyr Arg Ser Pro Glu Lys Lys Ala Thr Glu
        50              55              60

Asp Glu Gly Ser Glu Gln Lys Ile Pro Glu Ala Thr Asn Arg Arg Val
65              70              75              80

Trp Glu Leu Ser Lys Ala Asn Ser Arg Phe Ala Thr Thr Phe Tyr Gln
            85              90              95

His Leu Ala Asp Ser Lys Asn Asp Asn Asp Asn Ile Phe Leu Ser Pro
            100             105             110

Leu Ser Ile Ser Thr Ala Phe Ala Met Thr Lys Leu Gly Ala Cys Asn
```

                115                        120                        125


Asp Thr Leu Gln Gln Leu Met Glu Val Phe Lys Phe Asp Thr Ile Ser
    130                 135                 140

Glu Lys Thr Ser Asp Gln Ile His Phe Phe Phe Ala Lys Leu Asn Cys
145                 150                 155                     160

Arg Leu Tyr Arg Lys Ala Asn Lys Ser Ser Lys Leu Val Ser Ala Asn
                165                 170                 175

Arg Leu Phe Gly Asp Lys Ser Leu Thr Phe Asn Glu Thr Tyr Gln Asp
                180                 185                 190

Ile Ser Glu Leu Val Tyr Gly Ala Lys Leu Gln Pro Leu Asp Phe Lys
        195                 200                 205

Glu Asn Ala Glu Gln Ser Arg Ala Ala Ile Asn Lys Trp Val Ser Asn
    210                 215                 220

Lys Thr Glu Gly Arg Ile Thr Asp Val Ile Pro Ser Glu Ala Ile Asn
225                 230                 235                     240

Glu Leu Thr Val Leu Val Leu Val Asn Thr Ile Tyr Phe Lys Gly Leu
                245                 250                 255

Trp Lys Ser Lys Phe Ser Pro Glu Asn Thr Arg Lys Glu Leu Phe Tyr
        260                 265                 270

Lys Ala Asp Gly Glu Ser Cys Ser Ala Ser Met Met Tyr Gln Glu Gly
        275                 280                 285

Lys Phe Arg Tyr Arg Arg Val Ala Glu Gly Thr Gln Val Leu Glu Leu
    290                 295                 300

Pro Phe Lys Gly Asp Asp Ile Thr Met Val Leu Ile Leu Pro Lys Pro
305                 310                 315                     320

Glu Lys Ser Leu Ala Lys Val Glu Lys Glu Leu Thr Pro Glu Val Leu
                325                 330                 335

Gln Glu Trp Leu Asp Glu Leu Glu Glu Met Met Leu Val Val His Met
        340                 345                 350

Pro Arg Phe Arg Ile Glu Asp Gly Phe Ser Leu Lys Glu Gln Leu Gln
        355                 360                 365

```
Asp Met Gly Leu Val Asp Leu Phe Ser Pro Glu Lys Ser Lys Leu Pro
    370             375             380

Gly Ile Val Ala Glu Gly Arg Asp Asp Leu Tyr Val Ser Asp Ala Phe
385             390             395             400

His Lys Ala Phe Leu Glu Val Asn Glu Glu Gly Ser Glu Ala Ala Ala
            405             410             415

Ser Thr Ala Val Val Ile Ala Gly Arg Ser Leu Asn Pro Asn Arg Val
        420             425             430

Thr Phe Lys Ala Asn Arg Pro Phe Leu Val Phe Ile Arg Glu Val Pro
        435             440             445

Leu Asn Thr Ile Ile Phe Met Gly Arg Val Ala Asn Pro Cys Val Lys
    450             455             460
```

```
<210> 9
<211> 478
<212> PRT
<213> Homo sapiens

<400> 9
Met Ala Pro Leu Arg Pro Leu Leu Ile Leu Ala Leu Leu Ala Trp Val
1               5               10              15

Ala Leu Ala Asp Gln Glu Ser Cys Lys Gly Arg Cys Thr Glu Gly Phe
        20              25              30

Asn Val Asp Lys Lys Cys Gln Cys Asp Glu Leu Cys Ser Tyr Tyr Gln
        35              40              45

Ser Cys Cys Thr Asp Tyr Thr Ala Glu Cys Lys Pro Gln Val Thr Arg
    50              55              60

Gly Asp Val Phe Thr Met Pro Glu Asp Glu Tyr Thr Val Tyr Asp Asp
65              70              75              80

Gly Glu Glu Lys Asn Asn Ala Thr Val His Glu Gln Val Gly Gly Pro
            85              90              95

Ser Leu Thr Ser Asp Leu Gln Ala Gln Ser Lys Gly Asn Pro Glu Gln
            100             105             110

Thr Pro Val Leu Lys Pro Glu Glu Glu Ala Pro Ala Pro Glu Val Gly
        115             120             125

Ala Ser Lys Pro Glu Gly Ile Asp Ser Arg Pro Glu Thr Leu His Pro
```

```
                130                      135                         140


          Gly Arg Pro Gln Pro Pro Ala Glu Glu Glu Leu Cys Ser Gly Lys Pro
          145                150                155                   160


          Phe Asp Ala Phe Thr Asp Leu Lys Asn Gly Ser Leu Phe Ala Phe Arg
                          165                170                   175


          Gly Gln Tyr Cys Tyr Glu Leu Asp Glu Lys Ala Val Arg Pro Gly Tyr
                      180                185                   190


          Pro Lys Leu Ile Arg Asp Val Trp Gly Ile Glu Gly Pro Ile Asp Ala
                  195                200                   205


          Ala Phe Thr Arg Ile Asn Cys Gln Gly Lys Thr Tyr Leu Phe Lys Gly
              210                215                220


          Ser Gln Tyr Trp Arg Phe Glu Asp Gly Val Leu Asp Pro Asp Tyr Pro
          225                230                235                   240


          Arg Asn Ile Ser Asp Gly Phe Asp Gly Ile Pro Asp Asn Val Asp Ala
                          245                250                   255


          Ala Leu Ala Leu Pro Ala His Ser Tyr Ser Gly Arg Glu Arg Val Tyr
                  260                265                270


          Phe Phe Lys Gly Lys Gln Tyr Trp Glu Tyr Gln Phe Gln His Gln Pro
                  275                280                285


          Ser Gln Glu Glu Cys Glu Gly Ser Ser Leu Ser Ala Val Phe Glu His
              290                295                300


          Phe Ala Met Met Gln Arg Asp Ser Trp Glu Asp Ile Phe Glu Leu Leu
          305                310                315                   320


          Phe Trp Gly Arg Thr Ser Ala Gly Thr Arg Gln Pro Gln Phe Ile Ser
                          325                330                   335


          Arg Asp Trp His Gly Val Pro Gly Gln Val Asp Ala Ala Met Ala Gly
                      340                345                   350


          Arg Ile Tyr Ile Ser Gly Met Ala Pro Arg Pro Ser Leu Ala Lys Lys
                  355                360                365


          Gln Arg Phe Arg His Arg Asn Arg Lys Gly Tyr Arg Ser Gln Arg Gly
              370                375                380
```

196

```
His Ser Arg Gly Arg Asn Gln Asn Ser Arg Arg Pro Ser Arg Ala Thr
385             390             395             400


Trp Leu Ser Leu Phe Ser Ser Glu Glu Ser Asn Leu Gly Ala Asn Asn
            405             410             415


Tyr Asp Asp Tyr Arg Met Asp Trp Leu Val Pro Ala Thr Cys Glu Pro
            420             425             430


Ile Gln Ser Val Phe Phe Phe Ser Gly Asp Lys Tyr Tyr Arg Val Asn
            435             440             445


Leu Arg Thr Arg Arg Val Asp Thr Val Asp Pro Pro Tyr Pro Arg Ser
    450             455             460


Ile Ala Gln Tyr Trp Leu Gly Cys Pro Ala Pro Gly His Leu
465             470             475
```

```
<210> 10
<211> 661
<212> PRT
<213> Homo sapiens

<400> 10
Met Arg Leu Lys Asn Leu Thr Phe Ile Ile Ile Leu Ile Ile Ser Gly
1               5               10              15


Glu Leu Tyr Ala Glu Glu Lys Pro Cys Gly Phe Pro His Val Glu Asn
            20              25              30


Gly Arg Ile Ala Gln Tyr Tyr Tyr Thr Phe Lys Ser Phe Tyr Phe Pro
            35              40              45


Met Ser Ile Asp Lys Lys Leu Ser Phe Phe Cys Leu Ala Gly Tyr Thr
    50              55              60


Thr Glu Ser Gly Arg Gln Glu Glu Gln Thr Thr Cys Thr Thr Glu Gly
65              70              75              80


Trp Ser Pro Glu Pro Arg Cys Phe Lys Lys Cys Thr Lys Pro Asp Leu
            85              90              95


Ser Asn Gly Tyr Ile Ser Asp Val Lys Leu Leu Tyr Lys Ile Gln Glu
            100             105             110


Asn Met Arg Tyr Gly Cys Ala Ser Gly Tyr Lys Thr Thr Gly Gly Lys
    115             120             125


Asp Glu Glu Val Val Gln Cys Leu Ser Asp Gly Trp Ser Ser Gln Pro
```

197

                    130                        135                        140

Thr Cys Arg Lys Glu His Glu Thr Cys Leu Ala Pro Glu Leu Tyr Asn
145                 150                 155                 160

Gly Asn Tyr Ser Thr Thr Gln Lys Thr Phe Lys Val Lys Asp Lys Val
                165                 170                 175

Gln Tyr Glu Cys Ala Thr Gly Tyr Tyr Thr Ala Gly Gly Lys Lys Thr
                180                 185                 190

Glu Glu Val Glu Cys Leu Thr Tyr Gly Trp Ser Leu Thr Pro Lys Cys
        195                 200                 205

Thr Lys Leu Lys Cys Ser Ser Leu Arg Leu Ile Glu Asn Gly Tyr Phe
    210                 215                 220

His Pro Val Lys Gln Thr Tyr Glu Glu Gly Asp Val Val Gln Phe Phe
225                 230                 235                 240

Cys His Glu Asn Tyr Tyr Leu Ser Gly Ser Asp Leu Ile Gln Cys Tyr
                245                 250                 255

Asn Phe Gly Trp Tyr Pro Glu Ser Pro Val Cys Glu Gly Arg Arg Asn
                260                 265                 270

Arg Cys Pro Pro Pro Pro Leu Pro Ile Asn Ser Lys Ile Gln Thr His
        275                 280                 285

Ser Thr Thr Tyr Arg His Gly Glu Ile Val His Ile Glu Cys Glu Leu
    290                 295                 300

Asn Phe Glu Ile His Gly Ser Ala Glu Ile Arg Cys Glu Asp Gly Lys
305                 310                 315                 320

Trp Thr Glu Pro Pro Lys Cys Ile Glu Gly Gln Glu Lys Val Ala Cys
                325                 330                 335

Glu Glu Pro Pro Phe Ile Glu Asn Gly Ala Ala Asn Leu His Ser Lys
                340                 345                 350

Ile Tyr Tyr Asn Gly Asp Lys Val Thr Tyr Ala Cys Lys Ser Gly Tyr
    355                 360                 365

Leu Leu His Gly Ser Asn Glu Ile Thr Cys Asn Arg Gly Lys Trp Thr
    370                 375                 380

Leu Pro Pro Glu Cys Val Glu Asn Asn Glu Asn Cys Lys His Pro Pro
385             390             395             400

Val Val Met Asn Gly Ala Val Ala Asp Gly Ile Leu Ala Ser Tyr Ala
            405             410             415

Thr Gly Ser Ser Val Glu Tyr Arg Cys Asn Glu Tyr Tyr Leu Leu Arg
            420             425             430

Gly Ser Lys Ile Ser Arg Cys Glu Gln Gly Lys Trp Ser Ser Pro Pro
            435             440             445

Val Cys Leu Glu Pro Cys Thr Val Asn Val Asp Tyr Met Asn Arg Asn
            450             455             460

Asn Ile Glu Met Lys Trp Lys Tyr Glu Gly Lys Val Leu His Gly Asp
465             470             475             480

Leu Ile Asp Phe Val Cys Lys Gln Gly Tyr Asp Leu Ser Pro Leu Thr
            485             490             495

Pro Leu Ser Glu Leu Ser Val Gln Cys Asn Arg Gly Glu Val Lys Tyr
            500             505             510

Pro Leu Cys Thr Arg Lys Glu Ser Lys Gly Met Cys Thr Ser Pro Pro
            515             520             525

Leu Ile Lys His Gly Val Ile Ile Ser Ser Thr Val Asp Thr Tyr Glu
            530             535             540

Asn Gly Ser Ser Val Glu Tyr Arg Cys Phe Asp His His Phe Leu Glu
545             550             555             560

Gly Ser Arg Glu Ala Tyr Cys Leu Asp Gly Met Trp Thr Thr Pro Pro
            565             570             575

Leu Cys Leu Glu Pro Cys Thr Leu Ser Phe Thr Glu Met Glu Lys Asn
            580             585             590

Asn Leu Leu Leu Lys Trp Asp Phe Asp Asn Arg Pro His Ile Leu His
            595             600             605

Gly Glu Tyr Ile Glu Phe Ile Cys Arg Gly Asp Thr Tyr Pro Ala Glu
            610             615             620

Leu Tyr Ile Thr Gly Ser Ile Leu Arg Met Gln Cys Asp Arg Gly Gln
625             630             635             640

199

```
Leu Lys Tyr Pro Arg Cys Ile Pro Arg Gln Ser Thr Leu Ser Tyr Gln
            645             650                 655


Glu Pro Leu Arg Thr
            660



<210> 11
<211> 163
<212> PRT
<213> Homo sapiens

<400> 11
Met Thr Val Lys Thr Leu His Gly Pro Ala Met Val Lys Tyr Leu Leu
1               5                   10                  15


Leu Ser Ile Leu Gly Leu Ala Phe Leu Ser Glu Ala Ala Ala Arg Lys
            20                  25                  30


Ile Pro Lys Val Gly His Thr Phe Phe Gln Lys Pro Glu Ser Cys Pro
            35                  40                  45


Pro Val Pro Gly Gly Ser Met Lys Leu Asp Ile Gly Ile Ile Asn Glu
            50                  55                  60


Asn Gln Arg Val Ser Met Ser Arg Asn Ile Glu Ser Arg Ser Thr Ser
65                  70                  75                  80


Pro Trp Asn Tyr Thr Val Thr Trp Asp Pro Asn Arg Tyr Pro Ser Glu
                85                  90                  95


Val Val Gln Ala Gln Cys Arg Asn Leu Gly Cys Ile Asn Ala Gln Gly
            100                 105                 110


Lys Glu Asp Ile Ser Met Asn Ser Val Pro Ile Gln Gln Glu Thr Leu
            115                 120                 125


Val Val Arg Arg Lys His Gln Gly Cys Ser Val Ser Phe Gln Leu Glu
            130                 135                 140


Lys Val Leu Val Thr Val Gly Cys Thr Cys Val Thr Pro Val Ile His
145                 150                 155                 160


His Val Gln



<210> 12
<211> 540
<212> PRT
<213> Homo sapiens
```

<400> 12

```
Met Gly Thr Thr Ala Arg Ala Ala Leu Val Leu Thr Tyr Leu Ala Val
1               5                   10                  15

Ala Ser Ala Ala Ser Glu Gly Gly Phe Thr Ala Thr Gly Gln Arg Gln
            20                  25                  30

Leu Arg Pro Glu His Phe Gln Glu Val Gly Tyr Ala Ala Pro Pro Ser
        35                  40                  45

Pro Pro Leu Ser Arg Ser Leu Pro Met Asp His Pro Asp Ser Ser Gln
    50                  55                  60

His Gly Pro Pro Phe Glu Gly Gln Ser Gln Val Gln Pro Pro Pro Ser
65                  70                  75                  80

Gln Glu Ala Thr Pro Leu Gln Gln Glu Lys Leu Leu Pro Ala Gln Leu
                85                  90                  95

Pro Ala Glu Lys Glu Val Gly Pro Pro Leu Pro Gln Glu Ala Val Pro
            100                 105                 110

Leu Gln Lys Glu Leu Pro Ser Leu Gln His Pro Asn Glu Gln Lys Glu
        115                 120                 125

Gly Thr Pro Ala Pro Phe Gly Asp Gln Ser His Pro Glu Pro Glu Ser
    130                 135                 140

Trp Asn Ala Ala Gln His Cys Gln Gln Asp Arg Ser Gln Gly Gly Trp
145                 150                 155                 160

Gly His Arg Leu Asp Gly Phe Pro Pro Gly Arg Pro Ser Pro Asp Asn
            165                 170                 175

Leu Asn Gln Ile Cys Leu Pro Asn Arg Gln His Val Val Tyr Gly Pro
        180                 185                 190

Trp Asn Leu Pro Gln Ser Ser Tyr Ser His Leu Thr Arg Gln Gly Glu
        195                 200                 205

Thr Leu Asn Phe Leu Glu Ile Gly Tyr Ser Arg Cys Cys His Cys Arg
    210                 215                 220

Ser His Thr Asn Arg Leu Glu Cys Ala Lys Leu Val Trp Glu Glu Ala
225                 230                 235                 240

Met Ser Arg Phe Cys Glu Ala Glu Phe Ser Val Lys Thr Arg Pro His
```

```
                    245                     250                         255


      Trp Cys Cys Thr Arg Gln Gly Glu Ala Arg Phe Ser Cys Phe Gln Glu
              260             265             270


      Glu Ala Pro Gln Pro His Tyr Gln Leu Arg Ala Cys Pro Ser His Gln
              275             280             285


      Pro Asp Ile Ser Ser Gly Leu Glu Leu Pro Phe Pro Pro Gly Val Pro
              290             295             300


      Thr Leu Asp Asn Ile Lys Asn Ile Cys His Leu Arg Arg Phe Arg Ser
      305             310             315             320


      Val Pro Arg Asn Leu Pro Ala Thr Asp Pro Leu Gln Arg Glu Leu Leu
                  325             330             335


      Ala Leu Ile Gln Leu Glu Arg Glu Phe Gln Arg Cys Cys Arg Gln Gly
                  340             345             350


      Asn Asn His Thr Cys Thr Trp Lys Ala Trp Glu Asp Thr Leu Asp Lys
                  355             360             365


      Tyr Cys Asp Arg Glu Tyr Ala Val Lys Thr His His His Leu Cys Cys
              370             375             380


      Arg His Pro Pro Ser Pro Thr Arg Asp Glu Cys Phe Ala Arg Arg Ala
      385             390             395             400


      Pro Tyr Pro Asn Tyr Asp Arg Asp Ile Leu Thr Ile Asp Ile Gly Arg
                  405             410             415


      Val Thr Pro Asn Leu Met Gly His Leu Cys Gly Asn Gln Arg Val Leu
                  420             425             430


      Thr Lys His Lys His Ile Pro Gly Leu Ile His Asn Met Thr Ala Arg
                  435             440             445


      Cys Cys Asp Leu Pro Phe Pro Glu Gln Ala Cys Cys Ala Glu Glu Glu
              450             455             460


      Lys Leu Thr Phe Ile Asn Asp Leu Cys Gly Pro Arg Arg Asn Ile Trp
      465             470             475             480


      Arg Asp Pro Ala Leu Cys Cys Tyr Leu Ser Pro Gly Asp Glu Gln Val
                  485             490             495
```

```
Asn Cys Phe Asn Ile Asn Tyr Leu Arg Asn Val Ala Leu Val Ser Gly
            500                 505             510


Asp Thr Glu Asn Ala Lys Gly Gln Gly Glu Gln Gly Ser Thr Gly Gly
            515                 520             525


Thr Asn Ile Ser Ser Thr Ser Glu Pro Lys Glu Glu
            530                 535             540


<210> 13
<211> 10
<212> PRT
<213> Homo sapiens

<400> 13
Met Ala Leu Pro Leu Arg Asp Arg Val Lys
1               5               10


<210> 14
<211> 5
<212> PRT
<213> Homo sapiens

<400> 14
Val Arg Leu Gly Ser
1               5


<210> 15
<211> 28
<212> PRT
<213> Homo sapiens

<400> 15
Gly Lys Glu Gly Arg Gly Pro Arg Pro His Ser Gln Pro Trp Leu Gly
1               5               10                  15


Glu Arg Val Gly Cys Ser His Ile Pro Pro Ser Ile
            20                  25


<210> 16
<211> 308
<212> PRT
<213> Homo sapiens

<400> 16
Met Pro Gly Gln Glu Leu Arg Thr Val Asn Gly Ser Gln Met Leu Leu
1               5               10                  15


Val Leu Leu Val Leu Ser Trp Leu Pro His Gly Gly Ala Leu Ser Leu
            20                  25                  30


Ala Glu Ala Ser Arg Ala Ser Phe Pro Gly Pro Ser Glu Leu His Ser
            35                  40                  45
```

```
Glu Asp Ser Arg Phe Arg Glu Leu Arg Lys Arg Tyr Glu Asp Leu Leu
    50              55              60

Thr Arg Leu Arg Ala Asn Gln Ser Trp Glu Asp Ser Asn Thr Asp Leu
65              70              75              80

Val Pro Ala Pro Ala Val Arg Ile Leu Thr Pro Glu Val Arg Leu Gly
            85              90              95

Ser Gly Gly His Leu His Leu Arg Ile Ser Arg Ala Ala Leu Pro Glu
            100             105             110

Gly Leu Pro Glu Ala Ser Arg Leu His Arg Ala Leu Phe Arg Leu Ser
        115             120             125

Pro Thr Ala Ser Arg Ser Trp Asp Val Thr Arg Pro Leu Arg Arg Gln
    130             135             140

Leu Ser Leu Ala Arg Pro Gln Ala Pro Ala Leu His Leu Arg Leu Ser
145             150             155             160

Pro Pro Pro Ser Gln Ser Asp Gln Leu Leu Ala Glu Ser Ser Ser Ala
            165             170             175

Arg Pro Gln Leu Glu Leu His Leu Arg Pro Gln Ala Ala Arg Gly Arg
            180             185             190

Arg Arg Ala Arg Ala Arg Asn Gly Asp His Cys Pro Leu Gly Pro Gly
        195             200             205

Arg Cys Cys Arg Leu His Thr Val Arg Ala Ser Leu Glu Asp Leu Gly
    210             215             220

Trp Ala Asp Trp Val Leu Ser Pro Arg Glu Val Gln Val Thr Met Cys
225             230             235             240

Ile Gly Ala Cys Pro Ser Gln Phe Arg Ala Ala Asn Met His Ala Gln
            245             250             255

Ile Lys Thr Ser Leu His Arg Leu Lys Pro Asp Thr Val Pro Ala Pro
            260             265             270

Cys Cys Val Pro Ala Ser Tyr Asn Pro Met Val Leu Ile Gln Lys Thr
    275             280             285

Asp Thr Gly Val Ser Leu Gln Thr Tyr Asp Asp Leu Leu Ala Lys Asp
    290             295             300
```

```
Cys His Cys Ile
305
```